# DEMANDE DE BREVET EUROPEEN

(11) **EP 3 578 662 A1**
(43) Date de publication de la demande: **11.12.2019**
(21) Numéro de dépôt: 19176373.9
(22) Date de dépôt: 24.05.2019
(51) Int. Cl.: C12N 15/90, C12N 15/74

(54) **OUTIL GENETIQUE OPTIMISÉ POUR MODIFIER LES BACTERIES DU GENRE CLOSTRIDIUM**

(30) Priorité: 04.06.2018 FR 1854835
(71) Demandeur: IFP Energies nouvelles, 92500 Rueil-Malmaison (FR)
(72) Inventeur: LOPES FERREIRA, Nicolas, 92500 RUEIL MALMAISON (FR); WASELS, François, 92500 RUEIL MALMAISON (FR); CHARTIER, Gwladys, 92500 RUEIL MALMAISON (FR)
(74) Mandataire: Cabinet Becker et Associés

(57) **Abrégé**

La présente invention concerne un outil génétique comprenant au moins deux acides nucléiques distincts optimisé pour faciliter la transformation et la modification par recombinaison homologue d'une bactérie du genre *Clostridium,* typiquement d'une bactérie solvantogène.

## Description

La présente invention concerne un outil génétique comprenant au moins deux acides nucléiques distincts optimisé pour faciliter la transformation et la modification par recombinaison homologue d'une bactérie du genre *Clostridium,* typiquement d'une bactérie solvantogène du genre *Clostridium.*

### ARRIERE-PLAN TECHNOLOGIQUE

Les bactéries appartenant au genre *Clostridium* sont des bacilles anaérobies strictes à Gram positif, capables de former des endospores et appartenant au phylum des Firmicutes. Ce genre contient de nombreuses espèces étudiées en raison de leur caractère pathogène ou de leur intérêt industriel et médical. Ainsi, *Clostridium tetani, Clostridium botulinum, Clostridium perfringens* et *Clostridium difficile* sont les agents respectivement responsables du tétanos, du botulisme, de gangrènes gazeuses et de colites pseudomembraneuses. *Clostridium novyi* et *Clostridium sporogenes* ont été utilisées dans des études ayant pour but la mise au point de thérapies anticancéreuses. Parallèlement, d'autres espèces telles que *Clostridium acetobutylicum*, *Clostridium butyricum* et *Clostridium beijerinckii,* non pathogènes pour l'homme, sont utilisées en fermentation.

Les espèces de *Clostridium* dites d'intérêt industriel sont capables de produire des composés d'intérêt tels que des acides et solvants à partir d'une large variété de sucres et substrats allant du glucose à la cellulose. La croissance des bactéries *Clostridium* productrices de solvants (« bactéries solvantogènes ») est dite biphasique. Des acides (acétiques et butyriques) sont produits lors de la phase exponentielle de croissance. Puis, lorsque la croissance cellulaire cesse et que les bactéries entrent en phase stationnaire, elles produisent des solvants.

La majorité des souches solvantogènes de *Clostridium* produit de l'Acétone, du Butanol et de l'Éthanol comme produits finaux. Ces souches sont désignées « souches ABE ». C'est par exemple le cas des souches DSM 792 (également désignée ATCC 824 ou encore LMG 5710) de *C. acetobutylicum* et NCIMB 8052 de *C. beijerinckii.* D'autres souches sont également capables de réduire tout ou partie de l'Acétone en Isopropanol, et sont désignées « souches IBE ». C'est le cas par exemple de la souche *C. beijerinckii* DSM 6423 (également désignée NRRL B-593, LMG 7814, LMG 7815) qui possède dans son génome un gène *adh* codant une alcool-déshydrogénase primaire/secondaire qui permet la réduction de l'acétone en isopropanol.

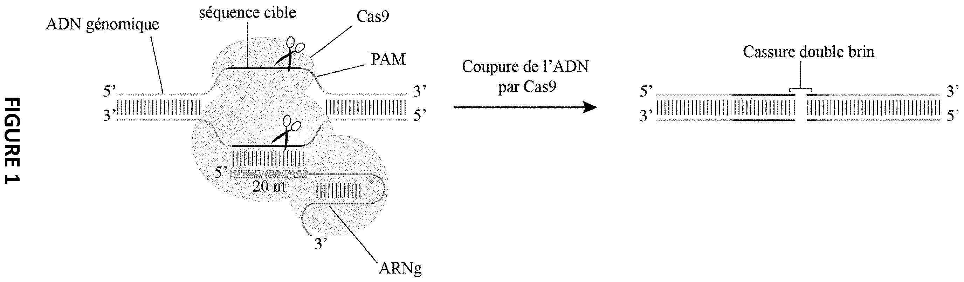

Bien qu'utilisées en industrie depuis plus d'un siècle, les connaissances sur les bactéries appartenant au genre *Clostridium* ont longtemps été limitées par les difficultés rencontrées pour les modifier génétiquement. Différents outils génétiques ont été conçus au cours des dernières années pour optimiser les souches de ce genre, la dernière génération étant basée sur l'utilisation de la technologie CRISPR (Clustered Regularly Interspaced Short Palindromic Repeats)/Cas (CRISPR-associated protein). Cette méthode est basée sur l'emploi d'une enzyme appelée nucléase (typiquement une nucléase de type Cas telle que la protéine Cas9 de *Streptococcus pyogenes*), qui va, guidée par une molécule d'ARN, réaliser une coupure double brin au sein d'une molécule d'ADN (séquence cible d'intérêt). La séquence de l'ARN guide (ARNg) déterminera le site de coupure de la nucléase, lui conférant ainsi une très forte spécificité (Figure 1).

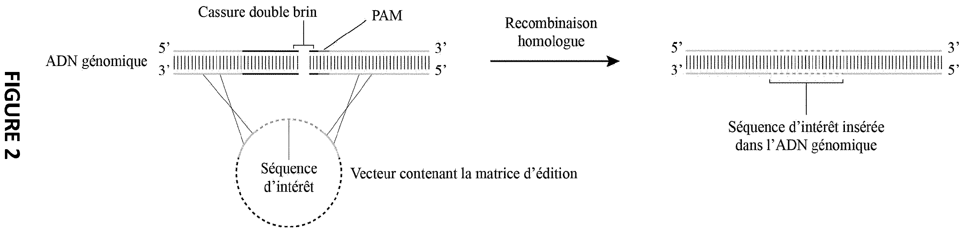

Une coupure double brin au sein d'une molécule d'ADN indispensable étant létale pour un organisme, la survie de ce dernier dépendra de sa capacité à la réparer (cf. par exemple Cui & Bikard, 2016). Chez les bactéries du genre *Clostridium,* la réparation d'une coupure double brin dépend d'un mécanisme de recombinaison homologue nécessitant une copie intacte de la séquence clivée. En fournissant à la bactérie un fragment d'ADN permettant d'effectuer cette réparation tout en modifiant la séquence originelle, il est possible de forcer le micro-organisme à intégrer les changements désirés au sein de son génome. La modification réalisée ne doit plus permettre le ciblage de l'ADN génomique par le complexe ribonucléoprotéique Cas9-ARNg, via la modification de la séquence cible ou du site PAM (Figure 2).

Différentes approches ont été décrites pour tenter de rendre fonctionnel cet outil génétique au sein de bactéries du genre *Clostridium.* Ces microorganismes sont en effet connus pour être difficiles à modifier génétiquement en raison de leurs faibles fréquences de transformation et de recombinaison homologue. Quelques approches sont basées sur l'emploi de Cas9, exprimée de manière constitutive chez *C. beijerinckii* et *C. ljungdahlii* (Wang *et al*, 2015 ; Huang *et al*, 2016) ou sous contrôle d'un promoteur inductible chez *C. beijerinckii, C. saccharoperbutylacetonicum* et *C. authoethanogenum* (Wang *et al,* 2016 ; Nagaraju *et al,* 2016; Wang *et al*, 2017). D'autres auteurs ont décrit l'utilisation d'une version modifiée de la nucléase, Cas9n, qui réalise des coupures simple brin, au lieu de coupures double brin, au sein du génome (Xu *et al*, 2015 ; Li *et al*, 2016). Ce choix est dû aux observations selon lesquelles la toxicité de Cas9 est trop importante pour son utilisation chez des bactéries du genre *Clostridium* dans les conditions expérimentales testées. Tous les outils décrits précédemment reposent sur l'utilisation d'un plasmide unique. Enfin, il est également possible d'utiliser des systèmes CRISPR/Cas endogènes lorsqu'ils ont été identifiés au sein du génome du micro-organisme, comme par exemple chez *C. pasteurianum* (Pyne *et al*, 2016).

A moins qu'ils n'utilisent (comme dans le dernier cas décrit ci-dessus) la machinerie endogène de la souche à modifier, les outils basés sur la technologie CRISPR présentent l'inconvénient majeur de limiter significativement la taille de l'acide nucléique d'intérêt (et donc le nombre de séquences codantes ou gènes) susceptible d'être inséré dans le génome bactérien (1,8 kb environ au mieux d'après Xu *et al.,* 2015).

Les inventeurs ont récemment mis au point et décrit un outil génétique plus performant de modification de bactéries, adapté aux bactéries du genre *Clostridium,* basé sur l'utilisation de deux acides nucléiques distincts, typiquement de deux plasmides (WO2017064439, Wasels et al., 2017 et Figure 3) qui résout notamment ce problème. Dans un mode de réalisation particulier, le premier acide nucléique de cet outil permet l'expression de *cas9* et un deuxième acide nucléique, spécifique de la modification à effectuer, contient une ou plusieurs cassettes d'expression d'ARNg ainsi qu'une matrice de réparation permettant le remplacement d'une portion de l'ADN bactérien ciblée par Cas9 par une séquence d'intérêt. La toxicité du système est limitée en plaçant *cas9* et/ou la (les) cassette(s) d'expression d'ARNg sous contrôle de promoteurs inductibles.

Les inventeurs sont également très récemment parvenus à modifier génétiquement des bactéries comprenant à l'état sauvage un gène conférant à la bactérie la résistance à un ou plusieurs antibiotiques afin de les rendre sensibles au(x)dit(s) antibiotique(s), ce qui a permis de faciliter l'utilisation de leur outil génétique basé sur l'utilisation d'au moins deux acides nucléiques. Ils sont ainsi parvenus à modifier génétiquement la souche *C. beijerinckii* DSM 6423 naturellement productrice d'isopropanol. Ils sont en particulier parvenus à éliminer de cette souche un plasmide naturel non essentiel pour la souche, identifié dans la présente description en tant que « pNF2 » (cf. FR18/73492). Les inventeurs ont aussi découvert et révèlent ici que l'élimination de ce plasmide pNF2 permet, dans le contexte de la présente invention, d'obtenir une bactérie *C. beijerinckii* DSM 6423 pour laquelle l'efficacité d'introduction de matériel génétique (i.e. de transformation) est augmentée d'un facteur compris entre environ 10¹ et 5 x 10³.

Les inventeurs décrivent dans le présent texte un outil génétique amélioré de modification des bactéries du genre *Clostridium* qui permet d'augmenter très significativement l'efficacité de transformation desdites bactéries et donc l'obtention, en nombre et quantité utile (en particulier dans un contexte de sélection de souches robustes pour une production à l'échelle industrielle), de bactéries mutantes (génétiquement modifiées) d'intérêt obtenues. Comme expliqué ci-dessous, les inventeurs ont notamment réussi à améliorer l'outil génétique selon l'invention, en utilisant une partie du plasmide pNF2 afin de concevoir des acides nucléiques particuliers porteurs d'une séquence permettant de modifier le matériel génétique d'une bactérie et/ou d'exprimer au sein d'une bactérie une séquence d'ADN absente du matériel génétique présent au sein de la version sauvage de ladite bactérie. Ces acides nucléiques et nouveaux outils améliorent de manière spectaculaire l'efficacité de transformation des bactéries, en particulier l'efficacité de transformation de bactéries préalablement débarrassées du ou des plasmides naturels qu'elles contiennent à l'état sauvage.

L'invention facilite ainsi de manière très avantageuse l'efficacité de transformation et l'exploitation de ces bactéries, en particulier à l'échelle industrielle.

### RESUME DE L'INVENTION

Les inventeurs décrivent, dans le contexte de la présente invention et pour la première fois, un outil génétique permettant la transformation optimisée, et la modification génétique par recombinaison homologue, d'une bactérie du genre *Clostridium* et/ou l'expression au sein de ladite bactérie d'une séquence d'ADN partiellement ou totalement absente du matériel génétique de ladite bactérie à l'état sauvage. Cet outil se caractérise typiquement i) en ce qu'il comprend :
- un « premier » acide nucléique codant au moins une endonucléase d'ADN, par exemple l'enzyme Cas9, dans lequel la séquence codant endonucléase d'ADN est placée sous le contrôle d'un promoteur, et
- au moins un « deuxième » acide nucléique contenant une matrice de réparation permettant, par un mécanisme de recombinaison homologue, le remplacement d'une portion de l'ADN bactérien ciblée par l'endonucléase par une séquence d'intérêt,
en ce que ii) au moins l'un desdits acides nucléiques code en outre un ou plusieurs ARN guides (ARNg) ou en ce que l'outil génétique comprend en outre un ou plusieurs ARN guides, chaque ARN guide comprenant une structure ARN de fixation à l'endonucléase d'ADN et une séquence complémentaire de la portion ciblée de l'ADN bactérien, et iii) en ce que au moins l'un desdits acides nucléiques comprend en outre une séquence codant une protéine anti-CRISPR placée sous le contrôle d'un promoteur inductible, ou en ce que l'outil génétique comprend en outre un « troisième » acide nucléique codant une protéine anti-CRISPR placée sous le contrôle d'un promoteur inductible.

Dans cet outil amélioré au moins un acide nucléique comprend une séquence codant une protéine anti-CRISPR (« acr »), placée sous le contrôle d'un promoteur inductible. Cette protéine anti-CRISPR permet de réprimer l'activité du complexe endonucléase d'ADN/ARN guide. L'expression de la protéine est régulée pour permettre son expression uniquement pendant l'étape de transformation de la bactérie.

Par rapport aux outils de l'art antérieur, cet outil présente l'avantage de faciliter considérablement la transformation des bactéries du genre *Clostridium* et donc l'obtention, en nombre et quantité utile, dans un contexte de production à l'échelle industrielle, de bactéries génétiquement modifiées d'intérêt.

Les inventeurs décrivent aussi, dans le contexte de la présente invention et pour la première fois, un acide nucléique (également identifié dans le présent texte en tant que acide nucléique « OPT ») facilitant la transformation des bactéries (en améliorant le maintien au sein desdites bactéries de l'ensemble du matériel génétique introduit). L'acide nucléique OPT comprend i) tout ou partie de la séquence SEQ ID NO : 126 et ii) une séquence permettant la modification du matériel génétique d'une bactérie et/ou l'expression au sein de ladite bactérie d'une séquence d'ADN partiellement ou totalement absente du matériel génétique présent au sein de la version sauvage de ladite bactérie. La séquence SEQ ID NO : 126 est également identifiée dans le présent texte en tant qu'acide nucléique « OREP ».

Les inventeurs sont parvenus à améliorer les fréquences de transformation d'un acide nucléique au sein de la bactérie *C. beijerinckii* DSM 6423 notamment en supprimant la séquence OREP au sein de ladite bactérie et en utilisant avantageusement tout ou partie de cette séquence OREP pour construire des acides nucléiques et/ou outils génétiques permettant la modification du matériel génétique d'une bactérie et/ou l'expression au sein de ladite bactérie d'une séquence d'ADN partiellement ou totalement absente du matériel génétique présent au sein de la version sauvage de ladite bactérie.

La séquence OREP comprend une séquence nucléotidique (SEQ ID NO : 127) codant pour une protéine impliquée dans la réplication d'un acide nucléique OPT d'intérêt. Cette protéine impliquée dans la réplication est également identifiée dans le présent texte en tant que protéine « REP » (SEQ ID NO : 128 - « MNNNNTESEELKEQSQLLLDKCTKKKKKNPKFSSYIEPLVSKKLSERIKECGDFLQMLSDLN LENSKLHRASFCGNRFCPMCSWRIACKDSLEISILMEHLRKEESKEFIFLTLTTPNVKGADLDN SIKAYNKAFKKLMERKEVKSIVKGYIRKLEVTYNLDKSSKSYNTYHPHFHVVLAVNRSYFKK QNLYINHHRWLSLWQESTGDYSITQVDVRKAKINDYKEVYELAKYSAKDSDYLINREVFTVF YKSLKGKQVLVFSGLFKDAHKMYKNGELDLYKKLDTIEYAYMVSYNWLKKKYDTSNIRELT EEEKQKFNKNLIEDVDIE »). La protéine REP possède un domaine conservé chez les firmicutes, nommé « COG 5655 » (Plasmid rolling circle replication initiator protein REP), de séquence SEQ ID NO : 129.

Est ainsi en particulier décrit dans le contexte de la présente invention un outil génétique comprenant au moins :
- un « premier » acide nucléique codant au moins une endonucléase d'ADN, dans lequel la séquence codant l'endonucléase d'ADN est placée sous le contrôle d'un promoteur, et
- un « autre » acide nucléique comprenant, ou consistant en, une séquence « d'acide nucléique OREP », i.e. comprenant, ou consistant en, i) tout ou partie de la séquence SEQ ID NO : 126 et ii) une séquence permettant la modification du matériel génétique d'une bactérie et/ou l'expression au sein de ladite bactérie d'une séquence d'ADN partiellement ou totalement absente du matériel génétique présent au sein de la version sauvage de ladite bactérie.

Dans un mode de réalisation particulier le « deuxième acide nucléique contenant une matrice de réparation » tel que décrit ci-dessus comprend cet « autre acide nucléique ».

Un procédé pour transformer, et typiquement pour modifier génétiquement par exemple par recombinaison homologue, une bactérie du genre *Clostridium,* typiquement une bactérie solvantogène du genre *Clostridium,* est également décrit, de même que la/les bactérie(s) obtenue (transformée(s) et typiquement modifiées génétiquement) à l'aide d'un tel procédé. Ce procédé comprend les étapes suivantes :
a) d'introduction dans la bactérie d'un outil génétique selon l'invention en présence d'un agent inducteur de l'expression de la protéine anti-CRISPR, et
b) de culture de la bactérie transformée obtenue à l'issue de l'étape a) sur un milieu ne contenant pas l'agent inducteur de l'expression de la protéine anti-CRISPR et permettant typiquement l'expression du complexe ribonucléoprotéique endonucléase d'ADN/ARNg.

Un exemple d'un tel procédé comprend avantageusement une étape de transformation de la bactérie par introduction dans ladite bactérie de tout ou partie d'un outil génétique tel que décrit dans le présent texte, en particulier d'un acide nucléique (« acide nucléique OPT ») comprenant, ou consistant en, i) tout ou partie de la séquence SEQ ID NO : 126 et ii) une séquence permettant la modification du matériel génétique d'une bactérie et/ou l'expression au sein de ladite bactérie d'une séquence d'ADN partiellement ou totalement absente du matériel génétique présent au sein de la version sauvage de ladite bactérie.

Les inventeurs décrivent également un kit pour transformer, et de préférence modifier génétiquement, une bactérie du genre *Clostridium,* ou pour produire au moins un solvant, par exemple un mélange de solvants, à l'aide d'une bactérie du genre *Clostridium.* Ce kit comprend un acide nucléique tel que décrit dans le présent texte ou les éléments de l'outil génétique tels que décrits dans le présent texte, et au moins un inducteur adapté au promoteur inductible de l'expression de la protéine anti-CRISPR sélectionné utilisé au sein de l'outil. Dans un mode de réalisation particulier, le kit comprend tout ou partie des éléments d'un outil génétique tel que décrit dans le présent texte.

Est également décrite l'utilisation d'un acide nucléique ou d'un outil génétique, divulgués pour la première fois dans le présent texte, pour transformer et éventuellement modifier génétiquement une bactérie du genre *Clostridium,* par exemple une bactérie du genre *Clostridium* possédant à l'état sauvage à la fois un chromosome bactérien et au moins une molécule d'ADN distincte de l'ADN chromosomique (typiquement un plasmide naturel).

Est aussi décrite l'utilisation d'un acide nucléique ou d'un outil génétique, du procédé pour transformer et de préférence modifier génétiquement, typiquement par recombinaison homologue, une bactérie du genre *Clostridium,* de la bactérie obtenue par ledit procédé et/ou d'un kit, divulgués pour la première fois dans le présent texte, pour permettre la production, de préférence à l'échelle industrielle, d'un solvant ou d'un mélange de solvants, de préférence d'acétone, de butanol, d'éthanol, d'isopropanol ou d'un mélange de ceux-ci, typiquement d'un mélange isopropanol/butanol, butanol/éthanol ou isopropanol/éthanol.

### DESCRIPTION DETAILLEE DE L'INVENTION

Bien qu'utilisées en industrie depuis plus d'un siècle, les connaissances sur les bactéries solvantogènes, en particulier appartenant au genre *Clostridium,* sont limitées par les difficultés rencontrées pour les modifier génétiquement. Par exemple, les bactéries du genre *Clostridium* naturellement productrice d'isopropanol, typiquement possédant dans leur génome un gène *adh* codant une alcool-déshydrogénase primaire/secondaire qui permet la réduction de l'acétone en isopropanol, se distinguent à la fois génétiquement et fonctionnellement des bactéries capables à l'état naturel d'une fermentation ABE.

L'outil génétique décrit dans le présent texte présente l'avantage de faciliter considérablement la transformation d'une bactérie du genre *Clostridium* par une séquence d'intérêt dans le but d'améliorer ses propriétés.

Cet outil se caractérise typiquement i) en ce qu'il comprend :
- un « premier » acide nucléique codant au moins une endonucléase d'ADN, par exemple l'enzyme Cas9, dans lequel la séquence codant l'endonucléase d'ADN est placée sous le contrôle d'un promoteur, et
- au moins un « deuxième » acide nucléique contenant une matrice de réparation permettant, par un mécanisme de recombinaison homologue, le remplacement d'une portion de l'ADN bactérien ciblée par l'endonucléase par une séquence d'intérêt,
en ce que ii) au moins l'un desdits acides nucléiques code en outre un ou plusieurs ARN guides (ARNg) ou en ce que l'outil génétique comprend en outre un ou plusieurs ARN guides, chaque ARN guide comprenant une structure ARN de fixation à l'endonucléase d'ADN et une séquence complémentaire de la portion ciblée de l'ADN bactérien, et
iii) en ce que au moins l'un desdits acides nucléiques comprend en outre une séquence codant une protéine anti-CRISPR placée sous le contrôle d'un promoteur inductible, ou en ce que l'outil génétique comprend en outre un « troisième » acide nucléique codant une protéine anti-CRISPR placée sous le contrôle d'un promoteur inductible.

Cet outil permet notamment l'insertion de grands fragments de séquences d'acide nucléique.

L'outil décrit par les inventeurs est utilisable pour transformer et/ou modifier génétiquement une bactérie d'intérêt, typiquement une bactérie telle que décrite dans le présent texte appartenant au genre *Clostridium,* de préférence une bactérie du genre *Clostridium* naturellement capable (i.e. capable à l'état sauvage) de produire de l'isopropanol, en particulier naturellement capable d'effectuer une fermentation IBE, de préférence une bactérie résistante naturellement à un ou plusieurs antibiotiques, telle qu'une bactérie C. *beijerinckii.* Une bactérie préférée possède à l'état sauvage à la fois un chromosome bactérien et au moins une molécule d'ADN distincte de l'ADN chromosomique.

Par « bactérie du genre *Clostridium* », on entend en particulier les espèces de *Clostridium* dites d'intérêt industriel, typiquement les bactéries solvantogènes ou acétogènes du genre *Clostridium.* L'expression « bactérie du genre *Clostridium* » englobe les bactéries sauvages ainsi que les souches dérivées de celles-ci modifiées génétiquement dans le but d'améliorer leur performance (par exemple surexprimant les gènes *ctfA*, *ctfB* et *adc*) sans avoir été exposées au Système CRISPR.

Par « espèce de *Clostridium* d'intérêt industriel » on entend les espèces capables de produire, par fermentation, des solvants et des acides tels que l'acide butyrique ou l'acide acétique, à partir de sucres ou d'oses, typiquement à partir de sucres comprenant 5 atomes de carbone tels que le xylose, l'arabinose ou le fructose, de sucres comprenant 6 atomes de carbones tels que le glucose ou le mannose, de polyosides tels que la cellulose ou les hémicelluloses et/ou de toute autre source de carbone assimilable et utilisable par les bactéries du genre *Clostridium* (CO, CO₂, et méthanol par exemple). Des exemples de bactéries solvantogènes d'intérêt sont les bactéries du genre *Clostridium* productrices d'acétone, de butanol, d'éthanol et/ou d'isopropanol, telles que les souches identifiées dans la littérature en tant que « souche ABE » [souches réalisant des fermentations permettant la production d'acétone, de butanol et d'éthanol] et « souche IBE » [souches réalisant des fermentations permettant la production d'isopropanol (par réduction de l'acétone), de butanol et d'éthanol]. Des bactéries solvantogènes du genre *Clostridium* peuvent être sélectionnées parmi *C. acetobutylicum*, *C. cellulolyticum, C. phytofermentans, C. beijerinckii, C. saccharobutylicum, C. saccharoperbutylacetonicum, C. sporogenes, C. butyricum, C. aurantibutyricum et C. tyrobutyricum,* de manière préférée parmi *C. acetobutylicum*, *C. beijerinckii, C. butyricum, C. tyrobutyricum* et *C. cellulolyticum*, et de manière encore plus préférée parmi *C. acetobutylicum* et *C. beijerinckii.*

Une bactérie capable de produire de l'isopropanol à l'état sauvage, en particulier capable d'effectuer une fermentation IBE à l'état sauvage, peut par exemple être une bactérie sélectionnée parmi une bactérie *C. beijerinckii,* une bactérie *C. diolis,* une bactérie *C. puniceum,* une bactérie *C. butyricum,* une bactérie *C. saccharoperbutylacetonicum*, une bactérie *C. botulinum*, une bactérie *C. drakei,* une bactérie *C. scatologenes*, une bactérie *C. perfringens,* et une bactérie *C. tunisiense,* de préférence une bactérie sélectionnée parmi une bactérie *C. beijerinckii,* une bactérie *C. diolis,* une bactérie *C. puniceum* et une bactérie *C. saccharoperbutylacetonicum.* Une bactérie naturellement capable de produire de l'isopropanol, en particulier capable d'effectuer une fermentation IBE à l'état sauvage, particulièrement préférée est une bactérie *C. beijerinckii.*

Les bactéries acétogènes d'intérêt sont des bactéries productrices d'acides et/ou de solvants à partir de CO₂ et H₂. Des bactéries acétogènes du genre *Clostridium* peuvent être sélectionnées par exemple parmi *C. aceticum, C. thermoaceticum*, *C. ljungdahlii*, *C. autoethanogenum*, *C. difficile, C. scatologenes* et *C. carboxydivorans.*

Dans un mode de réalisation particulier, la bactérie du genre *Clostridium* concernée est une « souche ABE », de préférence la souche DSM 792 (également désignée souche ATCC 824 ou encore LMG 5710) de C. *acetobutylicum*, ou la souche NCIMB 8052 de *C. beijerinckii.*

Dans un autre mode de réalisation particulier, la bactérie du genre *Clostridium* concernée est une « souche IBE », de préférence un sous-clade de C. *beijerinckii* sélectionné parmi DSM 6423, LMG 7814, LMG 7815, NRRL B-593, NCCB 27006, ou une bactérie *C. aurantibutyricum* DSZM 793 (Georges *et al.,* 1983), et un sous-clade d'une telle bactérie *C. beijerinckii* ou *C. aurantibutyricum* présentant au moins 90%, 95%, 96%, 97%, 98% ou 99% d'identité avec la souche DSM 6423. Une bactérie *C. beijerinckii,* ou un sous-clade de bactérie *C. beijerinckii,* particulièrement préféré(e) est dépourvu(e) du plasmide pNF2.

Les génomes respectifs des sous-clades LMG 7814, LMG 7815, NRRL B-593 et NCCB 27006 d'une part, et DSZM 793 d'autre part, présentent des pourcentages d'identité de séquence d'au moins 97% avec le génome du sous-clade DSM 6423.

Les inventeurs ont réalisé des tests fermentaires confirmant que les bactéries *C. beijerinckii* de sous-clade DSM 6423, LMG 7815 et NCCB 27006 sont capables de produire de l'isopropanol à l'état sauvage (cf. tableau 1).

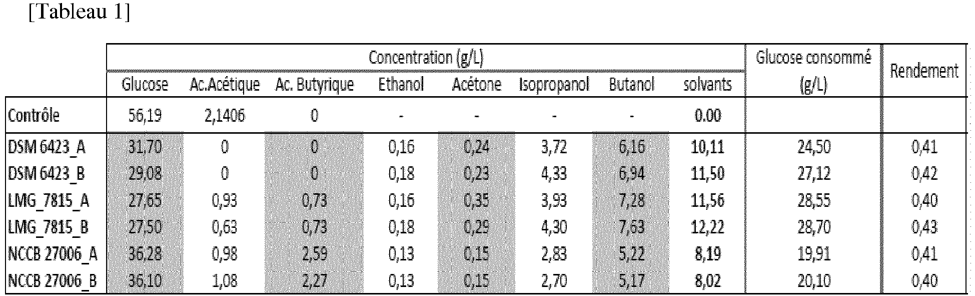

Bilan des essais de fermentation de glucose à l'aide des souches naturellement productrices d'isopropanol C. *beijerinckii* DSM 6423, LMG 7815 et NCCB 27006.Dans un mode de réalisation particulièrement préféré de l'invention, la bactérie C. *beijerinckii* est la bactérie de sous-clade DSM 6423.

Dans encore un autre mode de réalisation préféré de l'invention, la bactérie *C. beijerinckii* est une souche *C. beijerinckii* IFP963 Δ*catB* ΔpNF2 (enregistrée le 20 février 2019 sous le numéro de dépôt LMG P-31277 auprès de la collection BCCM-LMG, et également identifiée dans le présent texte en tant que *C. beijerinckii* DSM 6423 Δ*catB* ΔpNF2).

Le système CRISPR/endonucléase d'ADN contient deux éléments essentiels distincts, i.e. i) une endonucléase, dans le cas présent la nucléase associée au système CRISPR (Cas ou « CRISPR associated protein »), typiquement Cas9, et ii) un ARN guide. L'ARN guide se présente sous la forme d'un ARN chimérique qui consiste en la combinaison d'un ARN bactérien CRISPR (ARNcr) et d'un ARNtracr (trans-activating ARN CRISPR) (Jinek et al., Science 2012). L'ARNg combine la spécificité de ciblage de l'ARNcr correspondant aux "séquences espaçantes" qui servent de guides aux protéines Cas, et les propriétés conformationnelles de l'ARNtracr en un transcrit unique. Lorsque l'ARNg et la protéine Cas sont exprimés simultanément dans la cellule, la séquence génomique cible peut être modifiée de manière permanente grâce à une matrice de réparation fournie.

Au cours d'expérimentations récentes, les inventeurs sont avantageusement parvenus à transformer et à modifier génétiquement, une bactérie du genre *Clostridium* naturellement productrice d'isopropanol, la bactérie *C. beijerinckii* DSM 6423, ainsi que la souche de référence *C. acetobutylicum* DSM 792. Une partie des travaux décrits dans la partie expérimentale ont été réalisés au sein d'une souche capable de fermentation IBE, i.e. la souche *C. beijerinckii* DSM 6423 dont le génome et une analyse transcriptomique ont été décrits récemment par les inventeurs (Máté de Gerando *et al*., 2018).

Lors de l'assemblage du génome de cette souche, les inventeurs ont en particulier découvert, en plus du chromosome, la présence d'éléments génétiques mobiles (numéro d'accession PRJEB11626 - https://www.ebi.ac.uk/ena/data/view/PRJEB11626) : deux plasmides naturels (pNF1 et pNF2) et un bactériophage linéaire (Φ6423).

La souche *C. beijerinckii* DSM 6423 est naturellement sensible à l'érythromycine mais résistante au thiamphénicol. La demande de brevet n° FR18/73492 décrit une souche particulière, la souche *C. beijerinckii* DSM 6423 Δ*catB* (également identifiée dans le présent texte en tant que *C. beijerinckii* IFP962 Δ*catB*), rendue sensible au thiamphénicol. Dans un mode de réalisation particulier de l'invention, les inventeurs sont parvenus à supprimer de la souche *C. beijerinckii* DSM 6423 son plasmide naturel pNF2 et ont obtenu une souche *C. beijerinckii* DSM6423 Δ*catB* ΔpNF2. Cette souche a été enregistrée le 20 février 2019 sous le numéro de dépôt LMG P-31277 auprès de la collection BCCM-LMG. La description concerne également toute bactérie dérivée, clone, mutant ou version génétiquement modifiée de celle-ci. Elle concerne aussi plus généralement toute bactérie possédant à l'état sauvage à la fois un chromosome bactérien et au moins une molécule d'ADN distincte de l'ADN chromosomique (identifiée dans le présent texte en tant que « ADN (bactérien) non chromosomique » ou « plasmide (bactérien) naturel »), modifiée génétiquement à l'aide d'un acide nucléique et/ou outil génétique décrit(s) dans le présent texte de manière à ne plus comprendre au moins l'une de ses molécules d'ADN non chromosomique, typiquement plusieurs de ses molécules d'ADN non chromosomique (par exemple deux, trois ou quatre molécules d'ADN non chromosomique), de préférence l'ensemble de ses molécules d'ADN non chromosomique.

Les inventeurs ont observé que l'éviction du plasmide naturel pNF2 présente un avantage significatif pour l'introduction et le maintien d'éléments génétiques additionnels, naturels ou synthétiques (par exemple de cassette(s) d'expression ou de vecteur(s) plasmidique(s) d'expression). La souche DSM 6423 Δ*catB* ΔpNF2 peut ainsi être transformée avec une efficacité 10 à 5 x 10³ fois supérieure à son homologue sauvage ou à la souche DSM 6423 Δ*catB.*

Les inventeurs décrivent ainsi, dans la présente demande, une bactérie du genre *Clostridium* naturellement capable (i.e. capable à l'état sauvage) de produire de l'isopropanol, en particulier naturellement capable d'effectuer une fermentation IBE, qui a été modifiée génétiquement et a, du fait de cette modification génétique, en particulier perdu au moins un plasmide naturel (i.e. un plasmide naturellement présent au sein de la version sauvage de ladite bactérie), de préférence l'ensemble de ses plasmides naturels, ainsi que les outils, en particulier les outils génétiques, ayant permis son obtention. Ces outils présentent l'avantage de faciliter considérablement la transformation et la modification génétique des bactéries. Les expériences réalisées par les inventeurs ont démontré l'utilisation possible des outils et plus généralement de la technologie décrite dans le présent texte pour modifier génétiquement une bactérie du genre *Clostridium,* notamment des bactéries du genre *Clostridium* capables, à l'état sauvage, de produire de l'isopropanol, en particulier d'effectuer une fermentation IBE, en particulier celles porteuses d'un gène codant une enzyme responsable de la résistance à un antibiotique, en particulier un gène codant une amphénicol-O-acetyltransférase, par exemple une chloramphénicol-O-acetyltransférase ou une thiamphénicol-O-acetyltransférase.

Dans un mode de réalisation particulier, les inventeurs sont ainsi parvenus à rendre sensible à un antibiotique de la classe des amphénicols, une bactérie naturellement porteuse (porteuse à l'état sauvage) d'un gène codant une enzyme responsable de la résistance à ces antibiotiques.

D'autres bactéries préférées contiennent, à l'état sauvage, à la fois un chromosome bactérien et au moins une molécule d'ADN distincte de l'ADN chromosomique.

Des bactéries également préférées contiennent, à l'état sauvage, à la fois un chromosome bactérien et au moins une molécule d'ADN distincte de l'ADN chromosomique, ainsi qu'un gène conférant une résistance à un antibiotique. Dans un mode de réalisation particulier, ce gène code une amphénicol-O-acetyltransférase, par exemple une chloramphénicol-O-acetyltransférase ou une thiamphénicol-O-acetyltransférase.

Une bactérie particulière destinée à être transformée, et de préférence modifiée génétiquement, est de préférence une bactérie qui a été exposée à une première étape de transformation et à une première étape de modification génétique à l'aide d'un acide nucléique ou outil génétique selon l'invention ayant permis de supprimer au moins une molécule d'ADN extrachromosomique (typiquement au moins un plasmide) naturellement présente au sein de ladite bactérie à l'état sauvage.

Un objet décrit par les inventeurs concerne un acide nucléique (identifié dans le présent texte en tant que acide nucléique « OPT »), avantageusement utilisable pour faciliter la transformation des bactéries en améliorant le maintien au sein desdites bactéries de l'ensemble du matériel génétique introduit. Cet acide nucléique OPT comprend i) tout ou partie de la séquence SEQ ID NO : 126 (séquence « OREP ») ou d'un variant fonctionnel de celle-ci et ii) une séquence (également identifiée dans le présent texte en tant que « séquence d'intérêt ») permettant la modification du matériel génétique d'une bactérie et/ou l'expression au sein de ladite bactérie d'une séquence d'ADN partiellement ou totalement absente du matériel génétique présent au sein de la version sauvage de ladite bactérie.

La séquence OREP (SEQ ID NO : 126) comprend une séquence nucléotidique de séquence SEQ ID NO : 127. La séquence SEQ ID NO : 127 comprend de préférence une séquence codant pour une protéine impliquée dans la réplication de l'acide nucléique OPT. Une protéine considérée comme impliquée dans la réplication est également identifiée dans le présent texte en tant que protéine « REP » (SEQ ID NO : 128). La protéine REP possède un domaine conservé chez les Firmicutes, nommé « COG 5655 », de séquence SEQ ID NO : 129.

Dans un mode de réalisation particulier, l'acide nucléique OPT comprend une partie de la séquence OREP (SEQ ID NO : 126), typiquement un ou plusieurs fragments de la séquence OREP, de préférence au moins la séquence codant la protéine REP (SEQ ID NO : 128) ou un variant ou fragment fonctionnel de celle-ci (i.e. le fragment impliqué dans la réplication), typiquement la séquence SEQ ID NO : 127 ou un variant ou fragment de celle-ci codant le fragment impliqué, au sein de la protéine REP, dans la réplication d'un acide nucléique OPT. Le fragment fonctionnel de la séquence OREP codant le fragment, présent au sein de la protéine REP, impliqué dans la réplication d'un acide nucléique OPT, comprend le domaine de séquence SEQ ID NO : 129. Des exemples de tels fragments d'acide nucléique codant un fragment fonctionnel de la protéine REP, et variants de ceux-ci, sont susceptibles d'être facilement préparés par l'homme du métier. Un exemple typique de variant présente une homologie de séquence avec la séquence SEQ ID NO : 127 comprise entre 70% et 100%, de préférence entre 85 et 99%, de manière encore plus préférée entre 95 et 99%, par exemple de 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100%.

Dans un mode de réalisation préféré, le fragment ou variant fonctionnel de la séquence OREP code pour une protéine impliquée dans la réplication de l'acide nucléique OPT.

Dans un mode de réalisation préféré de l'invention, le fragment ou variant fonctionnel de la séquence OREP comprend, en plus de la séquence codant une protéine (par exemple la protéine REP) impliquée dans la réplication de l'acide nucléique OPT (par exemple une construction génétique de type plasmide) ou d'un variant ou fragment fonctionnel de celle-ci, un site de 1 à 150 bases, de préférence de 1 à 15 bases, par exemple une séquence riches en bases A et T (Rajewska *et al*.)*,* de préférence un site présent au sein du plasmide pNF2 de séquence SEQ ID NO : 118, permettant la fixation d'une protéine permettant la réplication de l'acide nucléique OPT.

La séquence d'intérêt permettant la modification du matériel génétique de la bactérie est typiquement une matrice de modification permettant, par exemple par un mécanisme de recombinaison homologue, le remplacement d'une portion du matériel génétique de la bactérie par une séquence d'intérêt. La séquence d'intérêt permettant la modification du matériel génétique de la bactérie peut aussi être une séquence reconnaissant (liant au moins en partie), et de préférence ciblant, i.e. reconnaissant et permettant la coupure, dans le génome d'une bactérie d'intérêt, d'au moins un brin i) d'une séquence cible, ii) d'une séquence contrôlant la transcription d'une séquence cible, ou iii) d'une séquence flanquant une séquence cible.

La séquence d'intérêt permettant l'expression au sein de ladite bactérie d'une séquence d'ADN partiellement ou totalement absente du matériel génétique présent au sein de la version sauvage de ladite bactérie permet typiquement à la bactérie d'exprimer une ou plusieurs protéines qu'elle est incapable d'exprimer, ou d'exprimer en quantité suffisante, à l'état sauvage.

Selon un aspect particulier, « l'acide nucléique OPT » comprend en outre iii) une séquence codant une endonucléase d'ADN, par exemple Cas9, et/ou iv) un ou plusieurs ARN guides (ARNg), chaque ARNg comprenant une structure ARN de fixation à l'endonucléase d'ADN et une séquence complémentaire de la portion ciblée du matériel génétique de la bactérie.

Selon un autre aspect particulier, « l'acide nucléique OPT » ne présente pas de méthylation au niveau des motifs reconnus par les méthyltransférases de type Dam et Dcm.

De manière préférée, « l'acide nucléique OPT » est sélectionné parmi une cassette d'expression et un vecteur, et est de préférence un plasmide, par exemple un plasmide ayant une séquence sélectionnée parmi SEQ ID NO : 119, SEQ ID NO : 123, SEQ ID NO : 124 et SEQ ID NO : 125.

Est ainsi en particulier décrit un outil génétique comprenant au moins :
- un premier acide nucléique codant au moins une endonucléase d'ADN, dans lequel la séquence codant l'endonucléase d'ADN est placée sous le contrôle d'un promoteur, et
- un autre acide nucléique (ou un « énième acide nucléique ») comprenant, ou consistant en, une séquence d'acide nucléique « OPT », i.e. une séquence comprenant i) tout ou partie de la séquence SEQ ID NO : 126 (« OREP ») et ii) une séquence permettant la modification du matériel génétique d'une bactérie et/ou l'expression au sein de ladite bactérie d'une séquence d'ADN partiellement ou totalement absente du matériel génétique présent au sein de la version sauvage de ladite bactérie, au moins l'un desdits acides nucléiques de cet outil génétique particulier comprenant de préférence en outre une séquence codant une protéine anti-CRISPR placée sous le contrôle d'un promoteur inductible, ou ledit outil génétique particulier comprenant de préférence en outre un troisième acide nucléique codant une protéine anti-CRISPR placée sous le contrôle d'un promoteur inductible.

Dans un mode de réalisation particulier le « deuxième » ou « énième acide nucléique contenant une matrice de réparation » tel que décrit ci-dessus comprend, ou consiste en, cet « autre acide nucléique ». Dans un autre mode de réalisation particulier le « premier acide nucléique » code en outre un ou plusieurs ARN guides (ARNg).

Par « acide nucléique », on entend au sens de l'invention, toute molécule d'ADN ou d'ARN naturelle, synthétique, semi-synthétique ou recombinante, éventuellement modifiée chimiquement (i.e. comprenant des bases non naturelles, des nucléotides modifiés comprenant par exemple une liaison modifiée, des bases modifiées et/ou des sucres modifiés), ou optimisée de manière à ce que les codons des transcrits synthétisés à partir des séquences codantes soient les codons les plus fréquemment trouvés chez une bactérie du genre *Clostridium* en vue de son utilisation chez celle-ci. Dans le cas du genre *Clostridium,* les codons optimisés sont typiquement des codons riches en bases adénines (« A ») et thymines (« T »).

L'outil génétique selon l'invention comprend un premier acide nucléique codant au moins une endonucléase, typiquement une nucléase de type Cas, par exemple Cas9 ou MAD7. Par « Cas9 » on entend une protéine Cas9 (aussi appelée CRISPR-associated protein 9, Csn1 ou Csx12) ou un fragment protéique, peptidique ou polypeptidique fonctionnel de celle-ci, i.e. capable d'interagir avec le/les ARN guides et d'exercer l'activité enzymatique (nucléasique) qui lui permet de réaliser la coupure double brin de l'ADN du génome cible. « Cas9 » peut ainsi désigner une protéine modifiée, par exemple tronquée afin de supprimer les domaines de la protéine non essentiels aux fonctions prédéfinies de la protéine, en particulier les domaines non nécessaires à l'interaction avec le/les ARNg.

La nucléase MAD7 (dont la séquence d'acides aminés correspond à la séquence SEQ ID NO : 72), également identifiée en tant que « Cas12 » ou « Cpf1 », peut autrement être avantageusement utilisée dans le cadre de la présente invention en la combinant avec un ou des ARNg connu(s) de l'homme de métier capable(s) de se lier à une telle nucléase (cf. Garcia-Doval *et al.,* 2017 et Stella S. *et al.,* 2017). Selon un aspect particulier, la séquence codant la nucléase MAD7 est une séquence optimisée pour être facilement exprimée dans des souches de *Clostridium,* de préférence la séquence SEQ ID NO : 71.

La séquence codant Cas9 (la protéine entière ou un fragment de celle-ci) telle qu'utilisée dans le cadre de l'invention peut être obtenue à partir de toute protéine Cas9 connue (Makarova *et al.,* 2011). Des exemples de protéines Cas9 utilisables dans la présente invention incluent, sans y être limités, les protéines Cas9 de *S. pyogenes, Streptococcus thermophilus, Streptococcus mutans, Campylobacter jejuni*, *Pasteurella multocida*, *Francisella novicida, Neisseria meningitidis, Neisseria lactamica* et *Legionella pneumophila* (cf. Fonfara *et al*, 2013 ; Makarova *et al*, 2015).

Dans un mode de réalisation particulier, la protéine Cas9, ou un fragment protéique, peptidique ou polypeptidique fonctionnel de celle-ci, codée par l'un des acides nucléiques de l'outil génétique selon l'invention comprend, ou consiste en, la séquence d'acides aminés SEQ ID NO : 75, ou toute autre séquence d'acides aminés ayant au moins 50%, de préférence au moins 60%, d'identité avec celle-ci, et contenant au minimum les deux acides aspartiques (« D ») occupant les positions 10 (« D10 ») et 840 (« D840 ») de la séquence d'acides aminés SEQ ID NO : 75.

Dans un mode de réalisation préféré, Cas9 comprend, ou consiste en, la protéine Cas9 (Numéro d'entrée NCBI : WP_010922251.1, SEQ ID NO : 75), codée par le gène *cas9* de la souche de *S. pyogenes* M1 GAS (Numéro d'entrée NCBI : NC_002737.2 SPy_1046, SEQ ID NO : 76) ou une version de celui-ci ayant subi une optimisation (« version optimisée ») à l'origine d'un transcrit contenant les codons utilisés préférentiellement par les bactéries du genre *Clostridium,* typiquement les codons riches en bases adénines (« A ») et thymines (« T »), permettant une expression facilitée de la protéine Cas9 au sein de ce genre bactérien. Ces codons optimisés respectent le biais d'usage de codons, bien connu de l'homme de l'art, spécifique à chaque souche bactérienne.

Dans les séquences peptidiques décrites dans ce document, les acides aminés sont représentés par leur code à une lettre selon la nomenclature suivante : C : cystéine; D : acide aspartique; E : acide glutamique; F : phénylalanine; G : glycine; H : histidine; I : isoleucine; K : lysine; L : leucine ; M : méthionine ; N : asparagine ; P : proline ; Q : glutamine ; R : arginine ; S : sérine ; T : thréonine ; V : valine ; W : tryptophane et Y : tyrosine.

Selon un mode de réalisation particulier, le domaine Cas9 est constitué d'une protéine Cas9 entière, de préférence la protéine Cas9 de *S. pyogenes* ou d'une version optimisée de celle-ci.

La séquence codant l'endonucléase d'ADN, par exemple Cas9, présente au sein de l'un des acides nucléiques de l'outil génétique selon l'invention est placée sous le contrôle d'un promoteur. Ce promoteur peut être un promoteur constitutif ou un promoteur inductible. Dans un mode de réalisation préféré, le promoteur contrôlant l'expression de Cas9 est un promoteur inductible.

Des exemples de promoteurs constitutifs utilisables dans le cadre de la présente invention peuvent être sélectionnés parmi le promoteur du gène *thl,* du gène *ptb,* du gène *adc,* de l'opéron BCS, ou un dérivé de ceux-ci, de préférence un dérivé fonctionnel mais plus court (tronqué) tel que le dérivé « miniPthl » du promoteur du gène *thl* de *C. acetobutylicum* (Dong *et al.,* 2012), ou tout autre promoteur, bien connu de l'homme de métier, permettant l'expression d'une protéine au sein d'une bactérie du genre *Clostridium.*

Des exemples de promoteurs inductibles utilisables dans le cadre de la présente invention peuvent être sélectionnés par exemple parmi un promoteur dont l'expression est contrôlée par le répresseur transcriptionnel TetR, par exemple le promoteur du gène *tetA* (gène de résistance à la tétracycline originellement présent sur le transposon Tn10 *d'E. coli)* ; un promoteur dont l'expression est contrôlée par la L-arabinose, par exemple le promoteur du gène *ptk* (Zhang *et al.,* 2015), de préférence en combinaison avec la cassette *araR* de régulation de l'expression de *C. acetobutylicum* de façon à construire un système ARAi (Zhang *et al.,* 2015) ; un promoteur dont l'expression est contrôlée par la laminaribiose (dimère de glucose β-1,3), par exemple le promoteur du gène *celC,* de préférence immédiatement suivi du gène répresseur glyR3 et du gène d'intérêt (Mearls *et al.* 2015) ou le promoteur du gène *celC* (Newcomb *et al.,* 2011) ; un promoteur dont l'expression est contrôlée par le lactose, par exemple le promoteur du gène *bgaL* (Banerjee *et al.,* 2014); un promoteur dont l'expression est contrôlée par le xylose, par exemple le promoteur du gène *xylB* (Nariya *et al.,* 2011) ; et un promoteur dont l'expression est contrôlée par l'exposition aux UV, par exemple le promoteur du gène *bcn* (Dupuy *et al.,* 2005).

Un promoteur dérivé de l'un des promoteurs décrits ci-dessus, de préférence un dérivé fonctionnel plus court (tronqué) peut également être utilisé dans le contexte de l'invention.

D'autres promoteurs inductibles utilisables dans le contexte de la présente invention sont également décrits par exemple dans les articles de Ransom *et al.* (2015), Currie *et al.* (2013) et Hartman *et al.* (2011).

Un promoteur inductible préféré est un promoteur dérivé de *tetA,* inductible à l'anhydrotétracycline (aTc ; moins toxique que la tétracycline et capable de lever l'inhibition du répresseur transcriptionnel TetR à plus faible concentration), choisi parmi Pcm-2tetO1 et Pcm-2tetO2/1 (Dong *et al*., 2012).

Un autre promoteur inductible préféré est un promoteur inductible par le xylose, par exemple le promoteur *xylB* de *Clostridium difficile* 630 (Nariya *et al.,* 2011).

Encore un autre promoteur inductible préféré est un promoteur inductible par le lactose, par exemple le promoteur du gène *bgaL* (Banerjee *et al.,* 2014).

Un acide nucléique d'intérêt particulier, typiquement une cassette ou un vecteur d'expression, comprend une ou plusieurs cassettes d'expression, chaque cassette codant un ARNg (ARN guide).

Le terme « ARN guide » ou « ARNg » désigne au sens de l'invention une molécule d'ARN capable d'interagir avec une endonucléase d'ADN telle que Cas9 afin de la guider vers une région cible du chromosome bactérien. La spécificité de coupure est déterminée par l'ARNg. Comme expliqué précédemment, chaque ARNg comprend deux régions :
- une première région (communément appelée région « SDS »), à l'extrémité 5' de l'ARNg, qui est complémentaire de la région chromosomique cible et qui imite l'ARNcr du système CRISPR endogène, et
- une seconde région (communément appelé région « handle »), à l'extrémité 3' de l'ARNg, qui mime les interactions d'appariement de bases entre l'ARNtracr (« trans-activating crRNA ») et l'ARNcr du système CRISPR endogène et présente une structure double brin en tige et boucle s'achevant en 3' par une séquence essentiellement simple brin. Cette seconde région est essentielle à la fixation de l'ARNg à l'endonucléase d'ADN.

La première région de l'ARNg (région « SDS ») varie selon la séquence chromosomique ciblée.

La région « SDS » de l'ARNg qui est complémentaire de la région chromosomique cible, comprend au moins 1 nucléotide, de préférence au moins 1, 2, 3, 4, 5, 10, 15, 20, 25, 30, 35 ou 40 nucléotides, typiquement entre 1 et 40 nucléotides. De préférence, cette région a une longueur de 20, 21, 22, 23, 24, 25, 26, 27, 28, 29 ou 30 nucléotides.

La seconde région de l'ARNg (région « handle ») a une structure en tige et boucle (ou structure en épingle à cheveux). Les régions « handle » des différents ARNg ne dépendent pas de la cible chromosomique choisie.

Selon un mode de réalisation particulier, la région « handle » comprend, ou consiste en, une séquence d'au moins 1 nucléotide, de préférence au moins 1, 50, 100, 200, 500 et 1000 nucléotides, typiquement entre 1 et 1000 nucléotides. De préférence, cette région a une longueur de 40 à 120 nucléotides.

La longueur totale d'un ARNg est en général de 50 à 1000 nucléotides, de préférence de 80 à 200 nucléotides, et de manière plus particulièrement préférée de 90 à 120 nucléotides. Selon un mode de réalisation particulier, un ARNg tel qu'utilisé dans la présente invention a une longueur comprise entre 95 et 110 nucléotides, par exemple une longueur d'environ 100 ou d'environ 110 nucléotides.

L'homme du métier peut aisément définir la séquence et la structure des ARNg selon la région chromosomique à cibler en utilisant des techniques bien connues (voir par exemple l'article de DiCarlo *et al.,* 2013).

La région/portion/séquence d'ADN ciblée au sein du chromosome bactérien peut correspondre à une portion d'ADN non codante ou à une portion d'ADN codante.

Dans un mode de réalisation particulier consistant à modifier une séquence donnée, la portion ciblée de l'ADN bactérien est essentielle à la survie bactérienne. Elle correspond par exemple à n'importe quelle région du chromosome bactérien ou à n'importe quelle région située sur de l'ADN non chromosomique, par exemple sur un élément génétique mobile, indispensable à la survie du micro-organisme dans des conditions de croissance particulières, par exemple un plasmide contenant un marqueur de résistance à un antibiotique lorsque les conditions de croissance prévues imposent de cultiver la bactérie en présence dudit antibiotique.

Dans un autre mode de réalisation particulier visant à supprimer un élément génétique non indispensable dans les conditions de croissance particulières associées à la culture du micro-organisme, la portion ciblée de l'ADN bactérien peut correspondre à n'importe quelle région dudit ADN bactérien non chromosomique, par exemple dudit élément génétique mobile.

Des exemples particuliers de portion d'ADN ciblée au sein d'une bactérie du genre *Clostridium* sont les séquences utilisées dans l'exemple 1 de la partie expérimentale. Il s'agit par exemple des séquences codant les gènes *bdhA* (SEQ ID NO : 77) et *bdhB* (SEQ ID NO : 78). La région/portion/séquence d'ADN ciblée est suivie d'une séquence « PAM » (« protospacer adjacent motif ») qui intervient dans la liaison à Cas9.

La région « SDS » d'un ARNg donné est identique (à 100%) ou identique à 80% au moins, de préférence à 85%, 90%, 95%, 96%, 97%, 98% ou 99% au moins à la région/portion/séquence d'ADN ciblée au sein du chromosome bactérien et est capable de s'hybrider à tout ou partie de la séquence complémentaire de ladite région/portion/séquence, typiquement à une séquence comprenant au moins 1 nucléotide, de préférence au moins 1, 2, 3, 4, 5, 10, 15, 20, 25, 30, 35 ou 40 nucléotides, typiquement entre 1 et 40 nucléotides, de préférence à une séquence comprenant 14, 15, 16, 17, 18, 19, 20, 21, 22, 23, 24, 25, 26, 27, 28, 29 ou 30 nucléotides.

Dans le procédé selon l'invention, un ou plusieurs ARNg ciblant une séquence ((« séquence cible », « séquence ciblée » ou « séquence reconnue ») peuvent être utilisés simultanément. Ces différents ARNg peuvent cibler des régions chromosomiques, ou des régions appartenant à l'ADN bactérien non chromosomique (par exemple aux éléments génétiques mobiles) éventuellement présents au sein du microorganisme, identiques ou différentes.

Les ARNg peuvent être introduits dans la cellule bactérienne sous la forme de molécules d'ARNg (matures ou précurseurs), sous la forme de précurseurs ou sous la forme d'un ou plusieurs acides nucléiques codant lesdits ARNg. Les ARNg sont de préférence introduits dans la cellule bactérienne sous la forme d'un ou plusieurs acides nucléiques codant lesdits ARNg.

Lorsque le ou les ARNg sont introduits dans la cellule directement sous la forme de molécules d'ARN, ces ARNg (matures ou précurseurs) peuvent contenir des nucléotides modifiés ou des modifications chimiques leur permettant, par exemple, d'augmenter leur résistance aux nucléases et d'augmenter ainsi leur durée de vie dans la cellule. Ils peuvent notamment comprendre au moins un nucléotide modifié ou non naturel tel que, par exemple, un nucléotide comportant une base modifiée, telle que l'inosine, la méthyl-5-désoxycytidine, la diméthylarnino-5-désoxyuridine, la désoxyuridine, la diamino-2,6-purine, la bromo-5-désoxyuridine ou toute autre base modifiée permettant l'hybridation. Les ARNg utilisés selon l'invention peuvent également être modifiés au niveau de la liaison internucléotidique comme par exemple les phosphorothioates, les H-phosphonates ou les alkyl-phosphonates, ou au niveau du squelette comme par exemple les alpha-oligonucléotides, les 2'-O-alkyl riboses ou les PNA (Peptide Nucleic Acids) (Egholm *et al.,* 1992).

Les ARNg peuvent être des ARN naturels, synthétiques ou produits par des techniques de recombinaison. Ces ARNg peuvent être préparés par toutes méthodes connues de l'homme du métier telles que, par exemple, la synthèse chimique, la transcription *in vivo* ou des techniques d'amplification.

Lorsque les ARNg sont introduits dans la cellule bactérienne sous la forme d'un ou plusieurs acides nucléiques, la ou les séquences codant le ou les ARNg sont placées sous le contrôle d'un promoteur d'expression. Ce promoteur peut être constitutif ou inductible.

Lorsque plusieurs ARNg sont utilisés, l'expression de chaque ARNg peut être contrôlée par un promoteur différent. De préférence, le promoteur utilisé est le même pour tous les ARNg. Un même promoteur peut dans un mode de réalisation particulier être utilisé pour permettre l'expression de plusieurs, par exemple de quelques-uns seulement, ou en d'autres termes de tout ou partie, des ARNg destinés à être exprimés.

Dans un mode de réalisation préféré, le/les promoteurs contrôlant l'expression du/des ARNg est/sont des promoteurs inductibles.

Des exemples de promoteurs constitutifs utilisables dans le cadre de la présente invention peuvent être sélectionnés parmi le promoteur du gène *thl,* du gène *ptb* ou de l'opéron BCS, ou un dérivé de ceux-ci, de préférence miniPthl, ou tout autre promoteur, bien connu de l'homme de métier, permettant la synthèse d'un ARN (codant ou non codant) au sein de *Clostridium.*

Des exemples de promoteurs inductibles utilisables dans le cadre de la présente invention peuvent être sélectionnés parmi le promoteur du gène *tetA,* du gène *xylA*, du gène *lacI,* ou du gène *bgaL,* ou un dérivé de ceux-ci, de préférence 2tetO1 ou tetO2/1. Un promoteur inductible préféré est 2tetO1.

Les promoteurs contrôlant l'expression de l'endonucléase d'ADN, par exemple Cas9, et du/des ARNg peuvent être identiques ou différents et constitutifs ou inductibles. Dans un mode de réalisation particulier et préféré de l'invention, les promoteurs contrôlant respectivement l'expression de l'endonucléase d'ADN ou du/des ARNg sont des promoteurs différents mais inductibles par le même agent inducteur.

Les promoteurs inductibles tels que décrits ci-dessus permettent de maîtriser avantageusement l'action du complexe ribonucléoprotéique endonucléase d'ADN/ARNg, par exemple Cas9/ARNg, et de faciliter la sélection de transformants ayant subi les modifications génétiques souhaitées.

L'outil génétique selon l'invention comprend avantageusement une séquence codant au moins une protéine anti-CRISPR (également identifiée dans le présent texte en tant que « protéine anti-CRISPR/endonucléase d'ADN » ou « protéine anti-CRISPR/Cas9 »), i.e. une protéine capable d'inhiber ou d'empêcher/de neutraliser l'action de Cas, et/ou une protéine capable d'inhiber ou d'empêcher/de neutraliser l'action d'un système CRISPR/Cas, par exemple d'un système CRISPR/Cas de type II lorsque la nucléase est une nucléase de type Cas9. Cette séquence est typiquement placée sous le contrôle d'un promoteur inductible différent des promoteurs contrôlant l'expression de l'endonucléase d'ADN et/ou du ou des ARNg, et est inductible par un autre agent inducteur. Dans un mode de réalisation préféré, la séquence codant la protéine anti-CRISPR est par ailleurs typiquement localisée sur l'un des au moins deux acides nucléiques présents au sein de l'outil génétique. Dans un mode de réalisation particulier, la séquence codant la protéine anti-CRISPR est localisée sur un acide nucléique distinct des deux premiers (typiquement un « troisième acide nucléique »). Dans encore un autre mode de réalisation particulier, à la fois la séquence codant la protéine anti-CRISPR et la séquence codant le répresseur transcriptionnel de ladite protéine anti-CRISPR sont intégrées dans le chromosome bactérien.

Dans un mode de réalisation préféré, la séquence codant une protéine anti-CRISPR est placée, au sein de l'outil génétique, sur l'acide nucléique codant l'endonucléase d'ADN (également identifié dans le présent texte en tant que « premier acide nucléique »). Dans un autre mode de réalisation, la séquence codant une protéine anti-CRISPR est placée, au sein de l'outil génétique, sur un acide nucléique différent de celui codant l'endonucléase d'ADN, par exemple sur l'acide nucléique identifié dans le présent texte en tant que « deuxième acide nucléique » ou encore sur un « énième » (typiquement un « troisième ») acide nucléique éventuellement compris dans l'outil génétique.

La protéine anti-CRISPR est typiquement une protéine « anti-Cas9 », i.e. une protéine capable d'inhiber ou d'empêcher/de neutraliser l'action de Cas9, et/ou une protéine capable d'inhiber ou d'empêcher/de neutraliser l'action d'un système CRISPR/Cas9 de type II.

La protéine anti-CRISPR est avantageusement une protéine « anti-Cas9 », ou une protéine « anti-MAD7 », i.e. une protéine capable d'inhiber ou d'empêcher/de neutraliser l'action de Cas9 ou de CAS7.

La protéine anti-CRISPR est avantageusement une protéine « anti-Cas9 », par exemple sélectionnée parmi AcrIIA1, AcrIIA2, AcrIIA3, AcrIIA4, AcrIIA5, AcrIIC1, AcrIIC2 et AcrIIC3 (Pawluk *et al*, 2018). De manière préférée la protéine « anti-Cas9 » est AcrIIA2 ou AcrIIA4. De manière encore plus préférée protéine « anti-Cas9 » est AcrIIA4. Une telle protéine est typiquement capable de limiter très significativement, idéalement d'empêcher, l'action de Cas9, par exemple en se fixant sur l'enzyme Cas9 (Dong *et al*, 2017 ; Rauch *et al*, 2017).

Une autre protéine anti-CRISPR avantageusement utilisable est une protéine « anti-MAD7 », par exemple la protéine AcrVAl (Marino *et al*, 2018).

Dans un mode de réalisation préféré, la protéine anti-CRISPR est capable d'inhiber, de préférence neutraliser, l'action de l'endonucléase d'ADN, de préférence durant la phase d'introduction des séquences d'acide nucléique de l'outil génétique dans la souche bactérienne d'intérêt.

Le promoteur contrôlant l'expression de la séquence codant la protéine anti-CRISPR est de préférence un promoteur inductible. Le promoteur inductible est associé à un gène exprimé de manière constitutive, typiquement responsable de l'expression d'une protéine permettant la répression transcriptionnelle à partir dudit promoteur inductible. Ce promoteur peut être par exemple sélectionné parmi le promoteur du gène *tetA,* du gène *xylA,* du gène *lacI,* ou du gène *bgaL,* ou un dérivé de ceux-ci.

Un exemple de promoteur inductible utilisable dans le contexte de l'invention est le promoteur Pbgal (inductible au lactose) présent, au sein de l'outil génétique et sur le même acide nucléique, au côté du gène *bgaR* exprimé de manière constitutive et dont le produit d'expression permet la répression transcriptionnelle à partir de Pbgal. En présence de l'agent inducteur, le lactose, la répression transcriptionnelle du promoteur Pbgal est levée, permettant la transcription du gène placé en aval de celui-ci. De manière préférée, le gène placé en aval correspond, dans le cadre de la présente invention, au gène codant la protéine anti-CRISPR, par exemple *acrIIA4.*

Le promoteur contrôlant l'expression de la protéine anti-CRISPR permet de maîtriser avantageusement l'action de l'endonucléase d'ADN, par exemple de l'enzyme Cas9 et d'ainsi faciliter la transformation des bactéries du genre *Clostridium* et l'obtention de transformants ayant subi les modifications génétiques souhaitées.

Par « acide nucléique », on entend au sens de l'invention, toute molécule d'ADN ou d'ARN naturelle, synthétique, semi-synthétique ou recombinante, éventuellement modifiée chimiquement (i.e. comprenant des bases non naturelles, des nucléotides modifiés comprenant par exemple une liaison modifiée, des bases modifiées et/ou des sucres modifiés), ou optimisée de manière à ce que les codons des transcrits synthétisés à partir des séquences codantes soient les codons les plus fréquemment trouvés chez une bactérie du genre *Clostridium* en vue de son utilisation chez celle-ci. Comme expliqué précédemment, dans le cas du genre *Clostridium,* les codons optimisés sont typiquement des codons riches en bases adénines (« A ») et thymines (« T »).

Chacun des acides nucléiques présents au sein de l'outil génétique selon l'invention, typiquement le « premier » acide nucléique et le « deuxième » ou « énième » acide nucléique, consiste en une entité distincte et correspond par exemple i) à une cassette d'expression (ou « construction ») telle qu'un acide nucléique comprenant au moins un promoteur transcriptionnel lié de manière opérationnelle (au sens où l'entend l'homme du métier) à une ou plusieurs séquences (codantes) d'intérêt, typiquement à un opéron comprenant plusieurs séquence codantes d'intérêt dont les produits d'expression concourent à la réalisation d'une fonction d'intérêt au sein de la bactérie, ou tel qu'un acide nucléique comprenant en outre une séquence activatrice et/ou un terminateur de transcription ; ou ii) à un vecteur, circulaire ou linéaire, simple ou double brin, par exemple un plasmide, un phage, un cosmide, un chromosome artificiel ou synthétique, comprenant une ou plusieurs cassettes d'expression telles que définies ci-dessus. De manière préférée, le vecteur est un plasmide.

Les acides nucléiques d'intérêt, typiquement les cassettes et vecteurs d'expression, peuvent être construits par des techniques classiques bien connues de l'homme du métier et peuvent comporter un(e) ou plusieurs promoteurs, origines de réplication bactérienne (séquences ORI), séquences de terminaison, gènes de sélection, par exemple gènes de résistance à un antibiotique, et séquences (« régions flanquées ») permettant l'insertion ciblée de la cassette ou du vecteur. Par ailleurs, ces cassettes et vecteurs d'expression peuvent être intégrés au sein du génome par des techniques bien connues de l'homme du métier.

Des séquences ORI d'intérêt peuvent être choisies parmi pIP404, pAMβ1, repH (origine de réplication chez *C. acetobutylicum*), ColEl ou rep (origine de réplication chez *E. coli*), ou toute autre origine de réplication permettant le maintien du vecteur, typiquement du plasmide, au sein d'une cellule de *Clostridium.*

Dans le contexte de la présente invention, une séquence ORI préférée est celle présente au sein de la séquence OREP (SEQ ID NO : 126) du plasmide pNF2 (SEQ ID NO : 118).

Des séquences de terminaison d'intérêt peuvent être choisies parmi celles des gènes *adc*, *thl,* de l'opéron *bcs,* ou de tout autre terminateur, bien connu de l'homme de l'art, permettant l'arrêt de la transcription au sein de *Clostridium.*

Des gènes de sélection (gènes de résistance) d'intérêt peuvent être choisis parmi *ermB*, *catP*, *bla, tetA, tetM*, et/ou tout autre gène de résistance à l'ampicilline, à l'érythromycine, au chloramphenicol, au thiamphénicol, à la spectinomycine, à la tétracycline ou à tout autre antibiotique pouvant être utilisé pour sélectionner des bactéries du genre *Clostridium* bien connu de l'homme de l'art.

Un vecteur particulier comprend une ou plusieurs cassettes d'expression, chaque cassette codant un ARNg.

Dans un mode de réalisation particulier, l'invention concerne un outil génétique comprenant en tant que « premier » acide nucléique tel qu'identifié dans les revendications un vecteur plasmidique dont la séquence est celle de SEQ ID NO : 23.

Dans un mode de réalisation particulier, l'invention concerne un outil génétique comprenant en tant que « deuxième » ou « énième » acide nucléique un vecteur plasmidique dont la séquence est sélectionnée parmi l'une des séquences SEQ ID NO : 79, SEQ ID NO : 80, SEQ ID NO : 119, SEQ ID NO : 123, SEQ ID NO : 124 et SEQ ID NO : 125.

Dans encore un autre mode de réalisation particulier, l'invention concerne un outil génétique comprenant un vecteur plasmidique dont la séquence est sélectionnée parmi l'une des séquences SEQ ID NO : 119, SEQ ID NO : 123, SEQ ID NO : 124 et SEQ ID NO : 125 en tant que « acide nucléique OPT ». Dans un autre mode de réalisation particulier, l'outil génétique comprend plusieurs (par exemple au moins deux ou trois) séquences parmi SEQ ID NO : 23, 79, 80, 119, 123, 124 et 125, lesdites séquences étant différentes les unes des autres.

La séquence d'intérêt est introduite dans le génome bactérien via un mécanisme de recombinaison homologue guidé par une matrice de réparation sélectionnée (selon la technologie CRISPR). La séquence d'intérêt vient remplacer la portion ciblée au sein du génome bactérien. Le processus de recombinaison permet ainsi la modification ou la délétion total(le) ou partiel(le) de la portion ciblée au sein du génome de la bactérie ou permet l'insertion de fragments (dans un mode de réalisation particulier d'insertion de grands fragments) d'acide nucléique dans le génome de la bactérie. La matrice de réparation sélectionnée peut en effet comprendre tout ou partie de la séquence ciblée du génome bactérien ou une version plus ou moins modifiée de celle-ci selon la nature de la transformation souhaitée. Tout comme la portion d'ADN ciblée, la matrice peut ainsi elle-même comprendre une ou plusieurs séquences d'acide nucléique ou portions de séquence d'acide nucléique correspondant à des séquences naturelles et/ou synthétiques, codantes et/ou non codantes. La matrice peut également comprendre une ou plusieurs séquences « étrangères », i.e. naturellement absentes du génome des bactéries appartenant au genre *Clostridium* ou du génome d'espèces particulières dudit genre. La matrice peut aussi comprendre une combinaison de séquences telles que décrites ci-dessus. L'outil génétique selon l'invention permet à la matrice de réparation de guider l'incorporation au sein du génome bactérien des bactéries du genre *Clostridium* d'un acide nucléique d'intérêt, typiquement d'une séquence ou portion de séquence d'ADN comprenant au moins 1 paire de base (pb), de préférence au moins 1, 2, 3, 4, 5, 10, 15, 20, 50, 100, 1 000, 10 000, 100 000 ou 1 000 000 pb, typiquement entre 1 pb et 20 kb ou entre 1 pb et 10 kb, de préférence entre 10 pb et 10 kb ou entre 1 kb et 10 kb, par exemple entre 1pb et 5 kb, entre 2 kb et 5 kb, ou encore entre 2,5 ou 3 kb et 5 kb.

Les inventeurs décrivent des exemples d'acide nucléique d'intérêt, typiquement de séquences d'ADN d'intérêt, permettant l'expression au sein d'une bactérie d'une séquence d'ADN partiellement ou totalement absente du matériel génétique présent au sein de la version sauvage de ladite bactérie.

Dans un mode de réalisation particulier, l'expression de la séquence d'ADN d'intérêt permet à la bactérie du genre *Clostridium* de fermenter (typiquement simultanément) plusieurs sucres différents, par exemple au moins deux sucres différents, typiquement au moins deux sucres différents parmi les sucres comprenant 5 atomes de carbone (tels que le glucose ou le mannose) et/ou parmi les sucres comprenant 6 atomes de carbone (tels que le xylose, l'arabinose ou le fructose), de préférence au moins trois sucres différents, sélectionnés par exemple parmi glucose, xylose et mannose ; glucose, arabinose et mannose ; et glucose xylose et arabinose.

Dans un autre mode de réalisation particulier, la séquence d'ADN d'intérêt code au moins un produit d'intérêt, de préférence un produit favorisant la production de solvant par la bactérie du genre *Clostridium,* typiquement au moins une protéine d'intérêt, par exemple une enzyme ; une protéine membranaire tel qu'un transporteur ; une protéine de maturation d'autres protéines (protéine chaperonne) ; un facteur de transcription ; ou une combinaison de ceux-ci.

Dans un mode de réalisation préféré, la séquence d'ADN d'intérêt favorise la production de solvant et est typiquement sélectionnée parmi une séquence codant i) une enzyme, par exemple une enzyme impliquée dans la conversion des aldéhydes en alcool, par exemple sélectionnée parmi une séquence codant une alcool déshydrogénase (par exemple une séquence sélectionnée parmi *adh, adhE, adhE1*, *adhE2, bdhA, bdhB* et *bdhC*), une séquence codant une transférase (par exemple une séquence sélectionnée parmi *ctfA*, *ctfB*, *atoA* et *atoB*), une séquence codant une décarboxylase (par exemple *adc*), une séquence codant une hydrogénase (par exemple une séquence sélectionnée parmi *etfA*, *etfB* et *hydA*), et une combinaison de celles-ci, ii) une protéine membranaire, par exemple une séquence codant une phosphotransférase (par exemple une séquence sélectionnée parmi *glcG*, *bglC*, *cbe4532, cbe4533, cbe4982, cbe4983, cbe0751*), iii) un facteur de transcription (par exemple une séquence sélectionnée parmi *sigE, sigF, sigG, sigH, sigK*) et iv) une combinaison de ceux-ci.

Les inventeurs décrivent par ailleurs des exemples d'acide nucléique d'intérêt reconnaissant (liant au moins en partie), et de préférence ciblant, i.e. reconnaissant et permettant la coupure, dans le génome d'une bactérie d'intérêt, d'au moins un brin i) d'une séquence cible, ii) d'une séquence contrôlant la transcription d'une séquence cible, ou iii) d'une séquence flanquant une séquence cible.

La séquence reconnue est également identifiée dans le présent texte en tant que « séquence cible » ou « séquence ciblée ».

Un outil génétique comprenant, ou consistant en, un tel acide nucléique d'intérêt est également décrit. Dans ce cas, l'acide nucléique d'intérêt est typiquement présent au sein du « deuxième » ou « énième » acide nucléique d'un outil génétique tel que décrit dans le présent texte.

L'acide nucléique d'intérêt est utilisé typiquement dans le contexte de la présente description pour supprimer la séquence reconnue du génome de la bactérie ou pour modifier son expression, par exemple pour moduler/réguler son expression, en particulier l'inhiber, de préférence pour la modifier de manière à rendre ladite bactérie incapable d'exprimer une protéine, en particulier une protéine fonctionnelle, à partir de ladite séquence.

Lorsque la séquence cible est une séquence codant une enzyme permettant à la bactérie d'intérêt de croître dans un milieu de culture contenant un antibiotique vis-à-vis duquel elle lui confère une résistance, une séquence contrôlant la transcription d'une telle séquence ou une séquence flanquant une telle séquence, l'antibiotique est typiquement un antibiotique appartenant à la classe des amphénicols. Des exemples d'amphénicols d'intérêt dans le contexte de la présente description sont le chloramphénicol, le thiamphénicol, l'azidamfénicol et le florfénicol (Schwarz S. *et al.,* 2004), en particulier le chloramphénicol et le thiamphénicol.

Dans un mode de réalisation particulier, l'acide nucléique d'intérêt comprend au moins une région complémentaire de la séquence cible identique à 100% ou identique à 80% au moins, de préférence à 85%, 90%, 95%, 96%, 97%, 98% ou 99% au moins à la région/portion/séquence d'ADN ciblée au sein du génome bactérien et est capable de s'hybrider à tout ou partie de la séquence complémentaire de ladite région/portion/séquence, typiquement à une séquence comprenant au moins 1 nucléotide, de préférence au moins 1, 2, 3, 4, 5, 10, 14, 15, 20, 25, 30, 35 ou 40 nucléotides, typiquement entre 1, 10 ou 20 et 1000 nucléotides, par exemple entre 1, 10 ou 20 et 900, 800, 700, 600, 500, 400, 300 ou 200 nucléotides, entre 1, 10 ou 20 et 100 nucléotides, entre 1, 10 ou 20 et 50 nucléotides, ou entre 1, 10 ou 20 et 40 nucléotides, par exemple entre 10 et 40 nucléotides, entre 10 et 30 nucléotides, entre 10 et 20 nucléotides, entre 20 et 30 nucléotides, entre 15 et 40 nucléotides, entre 15 et 30 nucléotides ou entre 15 et 20 nucléotides, de préférence à une séquence comprenant 14, 15, 16, 17, 18, 19, 20, 21, 22, 23, 24, 25, 26, 27, 28, 29 ou 30 nucléotides. La région complémentaire de la séquence cible présente au sein de l'acide nucléique d'intérêt peut correspondre à la région « SDS » d'un ARN guide (ARNg) utilisé dans un outil CRISPR tel que décrit dans le présent texte.

Dans un autre mode de réalisation particulier décrit, l'acide nucléique d'intérêt comprend au moins deux régions complémentaires chacune d'une séquence cible, identiques à 100% ou identiques à 80% au moins, de préférence à 85%, 90%, 95%, 96%, 97%, 98% ou 99% au moins à ladite région/portion/séquence d'ADN ciblée au sein du génome bactérien. Ces régions sont capables de s'hybrider à tout ou partie de la séquence complémentaire de ladite région/portion/séquence, typiquement à une séquence telle que décrite ci-dessus comprenant au moins 1 nucléotide, de préférence au moins 100 nucléotides, typiquement entre 100 et 1000 nucléotides. Les régions complémentaires de la séquence cible présentes au sein de l'acide nucléique d'intérêt peuvent reconnaître, de préférence cibler, les régions flanquantes en 5' et en 3' de la séquence ciblée dans un outil de modification génétique tel que décrit dans le présent texte.

Selon un aspect particulier, la séquence cible est une séquence codant une amphénicol-O-acetyltransférase, par exemple une chloramphénicol-O-acetyltransférase ou une thiamphénicol-O-acetyltransférase, contrôlant la transcription d'une telle séquence ou flanquant une telle séquence, au sein du génome d'une bactérie d'intérêt, par exemple du genre *Clostridium,* capable de croître dans un milieu de culture contenant un ou plusieurs antibiotiques appartenant à la classe des amphénicols, par exemple du chloramphénicol et/ou du thiamphénicol.

La séquence reconnue est par exemple la séquence SEQ ID NO : 18 correspondant au gène *catB* (CIBE_3859) codant une chloramphénicol-O-acetyltransférase de C. *beijerinckii* DSM 6423 ou une séquence d'acides aminés identique à au moins 70%, 75%, 80%, 85%, 90% ou 95% à ladite chloramphénicol-O-acetyltransférase, ou une séquence comprenant tout ou au moins 70%, 75%, 80%, 85%, 90%, 95%, 96%, 97%, 98% ou 99% de la séquence SEQ ID NO : 18. Autrement formulé, la séquence reconnue peut être une séquence comprenant au moins 1 nucléotide, de préférence au moins 1, 2, 3, 4, 5, 10, 15, 20, 25, 30, 35 ou 40 nucléotides, typiquement entre 1 et 40 nucléotides, de préférence une séquence comprenant 14, 15, 16, 17, 18, 19, 20, 21, 22, 23, 24, 25, 26, 27, 28, 29 ou 30 nucléotides de la séquence SEQ ID NO : 18.

Des exemples de séquences d'acides aminés identiques à au moins 70% à la chloramphénicol-O-acetyltransférase codée par la séquence SEQ ID NO : 18 correspondent aux séquences identifiées dans la base de données NCBI sous les références suivantes : WP_077843937.1, SEQ ID NO : 44 (WP_063843219.1), SEQ ID NO : 45 (WP_078116092.1), SEQ ID NO : 46 (WP_077840383.1), SEQ ID NO : 47 (WP_077307770.1), SEQ ID NO : 48 (WP_103699368.1), SEQ ID NO : 49 (WP_087701812.1), SEQ ID NO : 50 (WP_017210112.1), SEQ ID NO : 51 (WP_077831818.1), SEQ ID NO : 52 (WP_012059398.1), SEQ ID NO : 53 (WP_077363893.1), SEQ ID NO : 54 (WP_015393553.1), SEQ ID NO : 55 (WP_023973814.1), SEQ ID NO : 56 (WP_026887895.1), SEQ ID NO 57 (AWK51568.1), SEQ ID NO : 58 (WP_003359882.1), SEQ ID NO : 59 (WP_091687918.1), SEQ ID NO : 60 (WP_055668544.1), SEQ ID NO : 61 (KGK90159.1), SEQ ID NO : 62 (WP_032079033.1), SEQ ID NO : 63 (WP_029163167.1), SEQ ID NO : 64 (WP_017414356.1), SEQ ID NO : 65 (WP_073285202.1), SEQ ID NO : 66 (WP_063843220.1), et SEQ ID NO : 67 (WP_021281995.1).

Des exemples de séquences d'acides aminés identiques à au moins 75% à la chloramphénicol-O-acetyltransférase codée par la séquence SEQ ID NO : 18 correspondent aux séquences WP_077843937.1, WP_063843219.1, WP_078116092.1, WP_077840383.1, WP_077307770.1, WP_103699368.1, WP_087701812.1, WP_017210112.1, WP_077831818.1, WP_012059398.1, WP_077363893.1, WP_015393553.1, WP_023973814.1, WP_026887895.1 AWK51568.1, WP_003359882.1, WP_091687918.1, WP_055668544.1 et KGK90159.1.

Des exemples de séquences d'acides aminés identiques à au moins 90% à la chloramphénicol-O-acetyltransférase codée par la séquence SEQ ID NO : 18, sont les séquences WP_077843937.1, WP_063843219.1, WP_078116092.1, WP_077840383.1, WP_077307770.1, WP_103699368.1, WP_087701812.1, WP_017210112.1, WP_077831818.1, WP_012059398.1, WP_077363893.1, WP_015393553.1, WP_023973814.1, WP_026887895.1 et AWK51568.1.

Des exemples de séquences d'acides aminés identiques à au moins 95% à la chloramphénicol-O-acetyltransférase codée par la séquence SEQ ID NO : 18 correspondent aux séquences WP_077843937.1, WP_063843219.1, WP 078116092.1, WP_077840383.1, WP_077307770.1, WP_103699368.1, WP_087701812.1, WP_017210112.1, WP_077831818.1, WP 012059398.1, WP_077363893.1, WP_015393553.1, WP_023973814.1, et WP_026887895.1.

Des séquences d'acides aminés préférées, identiques à au moins 99% à la chloramphénicol-O-acetyltransférase codée par la séquence SEQ ID NO : 18, sont les séquences WP_077843937.1, SEQ ID NO : 44 (WP_063843219.1) et SEQ ID NO : 45 (WP_078116092.1).

Une séquence particulière identique à la séquence SEQ ID NO : 18 est la séquence identifiée dans la base de données NCBI sous la référence WP_077843937.1.

Selon un exemple particulier, la séquence cible est la séquence SEQ ID NO : 68 correspondant au gène *catQ* codant une chloramphénicol-O-acetyltransférase de C. *perfringens* dont la séquence d'acides aminés correspond à SEQ ID NO : 66 (WP_063843220.1), ou une séquence identique à au moins 70%, 75%, 80%, 85%, 90% ou 95% à ladite chloramphénicol-O-acetyltransférase, ou une séquence comprenant tout ou au moins 70%, 75%, 80%, 85%, 90%, 95%, 96%, 97%, 98% ou 99% de la séquence SEQ ID NO : 68.

Autrement formulé, la séquence reconnue peut être une séquence comprenant au moins 1 nucléotide, de préférence au moins 1, 2, 3, 4, 5, 10, 15, 20, 25, 30, 35 ou 40 nucléotides, typiquement entre 1 et 40 nucléotides, de préférence une séquence comprenant 14, 15, 16, 17, 18 , 19, 20, 21, 22, 23, 24, 25, 26, 27, 28, 29 ou 30 nucléotides de la séquence SEQ ID NO : 68.

Dans encore un autre exemple particulier, la séquence reconnue est sélectionnée parmi une séquence d'acide nucléique *catB* (SEQ ID NO : 18), *catQ* (SEQ ID NO 68), *catD* (SEQ ID NO : 69, Schwarz S. *et al.,* 2004) ou *catP* (SEQ ID NO : 70, Schwarz S. *et al.,* 2004) connue de l'homme du métier, présente naturellement au sein d'une bactérie ou introduite artificiellement dans une telle bactérie.

Comme indiqué précédemment, selon un autre exemple particulier, la séquence cible peut aussi être une séquence contrôlant la transcription d'une séquence codante telle que décrite précédemment (codant une enzyme permettant à la bactérie d'intérêt de croître dans un milieu de culture contenant un antibiotique vis-à-vis duquel elle lui confère une résistance), typiquement une séquence promotrice, par exemple la séquence promotrice (SEQ ID NO : 73) du gène *catB* ou celle (SEQ ID NO : 74) du géne *catQ.*

L'acide nucléique d'intérêt reconnaît alors, et est donc typiquement capable de se lier à une séquence contrôlant la transcription d'une séquence codante telle que décrite précédemment.

Selon un autre exemple particulier, la séquence cible peut être une séquence flanquant une séquence codante telle que décrite précédemment, par exemple une séquence flanquant le gène *catB* de séquence SEQ ID NO : 18 ou une séquence identique à au moins 70% à celle-ci. Une telle séquence flanquante comprend typiquement 1, 10 ou 20 et 1000 nucléotides, par exemple entre 1, 10 ou 20 et 900, 800, 700, 600, 500, 400, 300 ou 200 nucléotides, entre 1, 10 ou 20 et 100 nucléotides, entre 1, 10 ou 20 et 50 nucléotides, ou entre 1, 10 ou 20 et 40 nucléotides, par exemple entre 10 et 40 nucléotides, entre 10 et 30 nucléotides, entre 10 et 20 nucléotides, entre 20 et 30 nucléotides, entre 15 et 40 nucléotides, entre 15 et 30 nucléotides ou entre 15 et 20 nucléotides.

Selon un aspect particulier, la séquence cible correspond à la paire de séquences flanquant une telle séquence codante, chaque séquence flanquante comprenant typiquement au moins 20 nucléotides, typiquement entre 100 et 1000 nucléotides, de préférence entre 200 et 800 nucléotides.

Dans le contexte de la présente description, un exemple particulier d'acide nucléique d'intérêt, utilisé pour transformer et/ou modifier génétiquement une bactérie d'intérêt, est un fragment d'ADN i) reconnaissant une séquence codant, ii) contrôlant la transcription d'une séquence codant, ou iii) flanquant une séquence codant, une enzyme d'intérêt, de préférence une amphénicol-O-acetyltransférase, par exemple une chloramphénicol-O-acetyltransférase ou une thiamphénicol-O-acetyltransférase, au sein du génome d'une bactérie, par exemple d'une bactérie du genre *Clostridium* telle que décrite précédemment

Un exemple d'acide nucléique d'intérêt selon l'invention est capable de supprimer la séquence (« séquence cible ») reconnue du génome de la bactérie ou de modifier son expression, par exemple de la moduler, en particulier de l'inhiber, de préférence de la modifier de manière à rendre ladite bactérie incapable d'exprimer une protéine, par exemple une amphénicol-O-acetyltransférase, en particulier une protéine fonctionnelle, à partir de ladite séquence.

Dans un mode de réalisation particulier où la séquence reconnue codant une enzyme est une séquence conférant à la bactérie une résistance au chloramphénicol et/ou au thiamphénicol, le gène de sélection utilisé n'est pas un gène de résistance au chloramphénicol et/ou au thiamphénicol, et n'est de préférence aucun des gènes *catB, catQ, catD* ou *catP.*

Dans un mode de réalisation particulier, l'acide nucléique d'intérêt comprend un ou plusieurs ARN guides (ARNg) ciblant une séquence codant, contrôlant la transcription d'une séquence codant, ou flanquant une séquence codant, une enzyme d'intérêt, en particulier une amphénicol-O-acetyltransférase, et/ou une matrice de modification (également identifiée dans le présent texte en tant que « matrice d'édition »), par exemple une matrice permettant d'éliminer ou de modifier tout ou partie de la séquence cible, de préférence dans le but d'inhiber ou supprimer l'expression de la séquence cible, typiquement une matrice comprenant des séquences homologues (correspondant) aux séquences situées en amont et en aval de la séquence cible telles que décrites précédemment, typiquement des séquences (homologues auxdites séquences situées en amont et en aval de la séquence cible) comprenant chacune entre 10 ou 20 paires de base et 1000, 1500 ou 2000 paires de base, par exemple entre 100, 200, 300, 400 ou 500 paires de base et 1000, 1200, 1300, 1400 ou 1500 paires de base, de préférence entre 100 et 1500 ou entre 100 et 1000 paires de bases, et de manière encore plus préférée entre 500 et 1000 paires de base ou entre 200 et 800 paires de base.

Dans un mode de réalisation particulier, l'acide nucléique d'intérêt utilisé pour transformer et/ou modifier génétiquement une bactérie d'intérêt, est un acide nucléique ne présentant pas de méthylation aux niveaux des motifs reconnus par les méthyltransférases de type Dam et Dcm (préparé à partir d'une bactérie *Escherichia coli* présentant le génotype *dam- dcm-*).

Lorsque la bactérie d'intérêt à transformer et/ou modifier génétiquement est une bactérie *C. beijerinckii,* en particulier appartenant à l'un des sous-clades DSM 6423, LMG 7814, LMG 7815, NRRL B-593 et NCCB 27006, l'acide nucléique d'intérêt utilisé comme outil génétique, par exemple le plasmide, est un acide nucléique ne présentant pas de méthylation aux niveaux des motifs reconnus par les méthyltransférases de type Dam et Dcm, typiquement un acide nucléique dont l'adénosine (« A ») du motif GATC et/ou la deuxième cytosine « C » du motif CCWGG (W pouvant correspondre à une adénosine (« A ») ou à une thymine (« T »)) sont déméthylés.

Un acide nucléique ne présentant pas de méthylation aux niveaux des motifs reconnus par les méthyltransférases de type Dam et Dcm peut typiquement être préparé à partir d'une bactérie *Escherichia coli* présentant le génotype *dam⁻ dcm⁻* (par exemple *Escherichia coli* INV 110, Invitrogen). Ce même acide nucléique peut comporter d'autres méthylations réalisées par exemple par des méthyltransférases de type EcoKI, cette dernière visant les adénines (« A ») des motifs AAC(N6)GTGC et GCAC(N6)GTT (N pouvant correspondre à n'importe quel base).

Dans un mode de réalisation particulier, la séquence ciblée correspond à un gène codant une amphénicol-O-acetyltransférase par exemple une chloramphénicol-O-acetyltransférase tel que le gène *catB,* à une séquence contrôlant la transcription de ce gène, ou à une séquence flanquant ce gène.

Un acide nucléique d'intérêt particulier décrit par les inventeurs est par exemple un vecteur, de préférence un plasmide, par exemple le plasmide pCas9ind-Δc*atB* de séquence SEQ ID NO : 21 ou le plasmide pCas9ind-gRNA_*catB* de séquence SEQ ID NO : 38 décrit dans la partie expérimentale de la présente description (cf. exemple 2), en particulier une version de ladite séquence ne présentant pas de méthylation au niveau des motifs reconnus par les méthyltransférases de type Dam et Dcm.

La présente description concerne aussi l'utilisation d'un acide nucléique d'intérêt pour transformer et/ou modifier génétiquement une bactérie d'intérêt telle que décrite dans le présent texte.

La présente invention concerne par ailleurs un procédé pour transformer, et typiquement modifier génétiquement par recombinaison homologue, une bactérie du genre *Clostridium,* de préférence une bactérie solvantogène du genre *Clostridium.* Ce procédé comprend avantageusement une étape de transformation de la bactérie par introduction dans ladite bactérie d'un outil génétique selon l'invention tel que décrit dans la présente demande, en particulier d'un acide nucléique d'intérêt décrit dans le présent texte, de préférence d'un « acide nucléique OPT » comprenant, ou consistant en, i) tout ou partie de la séquence SEQ ID NO : 126 (OREP) et ii) une séquence permettant la modification du matériel génétique d'une bactérie et/ou l'expression au sein de ladite bactérie d'une séquence d'ADN partiellement ou totalement absente du matériel génétique présent au sein de la version sauvage de ladite bactérie. Le procédé peut comprendre en outre une étape d'obtention, de récupération, de sélection ou d'isolement de la bactérie transformée, i.e. de la bactérie présentant la ou les recombinaisons/modifications/optimisations recherchées.

La présente invention est typiquement avantageusement mise en oeuvre dès lors que l'outil de modification génétique sélectionné pour transformer, et de préférence modifier génétiquement, une bactérie du genre *Clostridium,* est destiné à être utilisé sur une bactérie, telle que C. *beijerinckii,* porteuse à l'état sauvage d'un gène codant une enzyme responsable de la résistance à un ou plusieurs antibiotiques et/ou porteuse à l'état sauvage d'au moins une séquence d'ADN extra-chromosomique, et que la mise en oeuvre dudit outil génétique comprend une étape de transformation de ladite bactérie à l'aide d'un acide nucléique permettant l'expression d'un marqueur de résistance à un antibiotique auquel cette bactérie est résistante à l'état sauvage et/ou une étape de sélection des bactéries transformées et/ou modifiées génétiquement à l'aide dudit antibiotique (auquel la bactérie est résistante à l'état sauvage), de préférence de sélection parmi lesdites bactéries des bactéries ayant perdu ladite séquence d'ADN extra-chromosomique.

Une modification avantageusement réalisable grâce à la présente invention, typiquement à l'aide d'un outil de modification génétique CRISPR, consiste à supprimer une séquence indésirable, par exemple une séquence codant une enzyme conférant à la bactérie la résistance à un ou plusieurs antibiotiques, ou à rendre cette séquence indésirable non fonctionnelle. Une autre modification avantageusement réalisable grâce à la présente invention consiste à modifier génétiquement une bactérie afin d'améliorer ses performances, par exemple ses performances dans la production d'un solvant ou d'un mélange de solvants d'intérêt, ladite bactérie ayant préalablement déjà été modifiée grâce à l'invention pour la rendre sensible à un antibiotique auquel elle était résistante à l'état sauvage, et/ou pour la débarrasser d'une séquence d'ADN extra-chromosomique présente au sein de la forme sauvage de ladite bactérie.

Le procédé selon l'invention est basé sur l'utilisation de (met en oeuvre) la technologie / l'outil génétique CRISPR (Clustered Regularly Interspaced Short Palindromic Repeats), en particulier l'outil génétique CRISPR/Cas (CRISPR-associated protein).

La présente invention peut être mise en oeuvre à l'aide d'un outil génétique CRISPR/Cas classique utilisant un unique plasmide comprenant une nucléase, un ARNg et une matrice de réparation tel que décrit par Wang *et al.* (2015).

L'homme du métier peut aisément définir la séquence et la structure des ARNg selon la région chromosomique ou l'élément génétique mobile à cibler en utilisant des techniques bien connues (voir par exemple l'article de DiCarlo *et al.,* 2013).

Les inventeurs ont mis au point et décrit un outil génétique de modification de bactéries, adapté aux bactéries du genre *Clostridium,* également utilisable dans le contexte de la présente invention, basé sur l'utilisation de deux plasmides (cf. WO2017/064439, Wasels et al., 2017, et Figure 15 associée à la présente description).

Dans un mode de réalisation particulier, le « premier » plasmide de cet outil permet l'expression de la nucléase *Cas* et un « deuxième » plasmide, spécifique de la modification à effectuer, contient une ou plusieurs cassettes d'expression d'ARNg (ciblant typiquement des régions différentes de l'ADN bactérien) ainsi qu'une matrice de réparation permettant, par un mécanisme de recombinaison homologue, le remplacement d'une portion de l'ADN bactérien ciblée par Cas par une séquence d'intérêt. Le gène *cas* et/ou la (les) cassette(s) d'expression d'ARNg sont placés sous le contrôle de promoteurs d'expression constitutifs ou inductibles, de préférence inductibles, connus de l'homme du métier (par exemple décrits dans la demande WO2017/064439 et incorporés par référence à la présente description), et de préférence différents mais inductibles par le même agent inducteur.

Les ARNg susceptibles d'être utilisés correspondent aux ARNg tels que décrits précédemment dans le présent texte.

Un procédé particulier selon l'invention permettant de transformer, et typiquement modifier génétiquement par recombinaison homologue, une bactérie solvantogène du genre *Clostridium,* comprend, dans l'ordre, les étapes suivantes :
a) l'introduction dans la bactérie d'un acide nucléique ou d'un outil génétique tels que décrits dans la présente demande en présence d'un agent inducteur de l'expression de la protéine anti-CRISPR, et
b) la culture de la bactérie transformée obtenue à l'issue de l'étape a) sur un milieu ne contenant pas (ou dans des conditions n'impliquant pas) l'agent inducteur de l'expression de la protéine anti-CRISPR, permettant typiquement l'expression du complexe ribonucléoprotéique endonucléase d'ADN/ARNg, typiquement Cas/ARNg (afin de stopper la production de ladite protéine anti-CRISPR et de permettre l'action de l'endonucléase).

L'introduction dans la bactérie des éléments (acides nucléiques ou ARNg) de l'outil génétique selon l'invention est réalisée par toute méthode, directe ou indirecte, connue de l'homme du métier, par exemple par transformation, conjugaison, microinjection, transfection, électroporation, etc., de préférence par électroporation (Mermelstein *et al*, 1993).

L'agent inducteur de l'expression de la protéine anti-CRISPR est présent en quantité suffisante pour induire ladite expression. Dans le cas du promoteur Pbgal, l'agent inducteur, le lactose, permet de lever l'inhibition d'expression (répression transcriptionnelle) de la protéine anti-CRISPR liée à l'expression de la protéine BgaR.

L'agent inducteur de l'expression de la protéine anti-CRISPR est de préférence utilisé à une concentration comprise entre environ 1 mM et environ 1M, de préférence entre environ 10 mM et environ 100 mM, par exemple environ 40 mM.

Dans un mode de réalisation préféré, la protéine anti-CRISPR est capable d'inhiber, de préférence neutraliser, l'action de la nucléase, de préférence durant la phase d'introduction des séquences d'acide nucléique de l'outil génétique dans la souche bactérienne d'intérêt.

Comme démontré dans la partie expérimentale, l'invention permet avantageusement la transformation des bactéries du genre *Clostridium* contenant une cassette d'expression de Cas9 et une cassette d'expression d'une protéine anti-CRISPR telle que AcrIIA4 avec n'importe quel acide nucléique contenant une cassette d'expression d'un ARNg.

La bactérie transformée obtenue à l'issue de l'étape a) du procédé décrit ci-dessus est ensuite cultivée sur un milieu ne contenant pas l'agent inducteur de l'expression de la protéine anti-CRISPR (afin de stopper la production de ladite protéine anti-CRISPR et de permettre l'action de la nucléase).

Dans un mode de réalisation particulier, le procédé comprend en outre, pendant ou après l'étape b), une étape d'induction de l'expression du ou des promoteurs inductibles contrôlant l'expression de la nucléase et/ou du ou des ARN guides lorsqu'un tel ou de tels promoteurs sont présents au sein de l'outil génétique, afin de permettre la modification génétique d'intérêt de la bactérie une fois ledit outil génétique introduit dans ladite bactérie. L'induction est typiquement réalisée à l'aide d'une substance permettant de lever l'inhibition d'expression liée au promoteur inductible sélectionné.

L'étape d'induction, lorsqu'elle est présente, peut être mise en oeuvre par toute méthode de culture sur un milieu permettant l'expression du complexe ribonucléoprotéique endonucléase d'ADN/ARNg connue de l'homme du métier après introduction dans la bactérie cible de l'outil génétique selon l'invention. Elle est par exemple réalisée par mise en contact de la bactérie avec une substance adéquate, présente en quantité suffisante ou par exposition à la lumière UV. Cette substance permet de lever l'inhibition d'expression liée au promoteur inductible sélectionné. Lorsque le promoteur sélectionné est un promoteur inductible à l'anhydrotétracycline (aTc), choisi parmi Pcm-2tetO1 et Pcm-tetO2/1, l'aTc est de préférence utilisée à une concentration comprise entre environ 1 ng/ml et environ 5000 ng/ml, de préférence entre environ 10 ng/ml et 1000 ng/ml, 10 ng/ml et 800 ng/ml, 10 ng/ml et 500 ng/ml, 100 ng/ml ou 200 ng/ml et environ 800 ng/ml ou 1000 ng/ml, ou entre environ 100 ng/ml ou 200 ng/ml et environ 500 ng/ml, 600 ng/ml ou 700 ng/ml, par exemple environ 50 ng/ml, 100 ng/ml, 150 ng/ml, 200 ng/ml, 250 ng/ml, 300 ng/ml, 350 ng/ml, 400 ng/ml, 450 ng/ml, 500 ng/ml, 550 ng/ml, 600 ng/ml, 650 ng/ml, 700 ng/ml, 750 ng/ml ou 800 ng/ml. Dans un mode de réalisation particulier, l'aTc est de préférence utilisée à une concentration comprise entre environ 200 ng/ml et environ 1000 ng/ml ou entre environ 200 ng/ml et environ 800 ng/ml, par exemple environ 500 ng/ml.

Dans un mode de réalisation particulier, le procédé comprend une étape c) additionnelle d'élimination de l'acide nucléique contenant la matrice de réparation (la cellule bactérienne étant alors considérée comme « curée » dudit acide nucléique) et/ou d'élimination du/des ARNs guides ou séquences codant le/les ARNs guides introduits avec l'outil génétique lors de l'étape a).

Dans un autre mode de réalisation particulier, le procédé comprend une ou plusieurs étapes additionnelles, postérieure(s) à l'étape b) ou à l'étape c), d'introduction d'un énième, par exemple troisième, quatrième, cinquième, etc., acide nucléique contenant une matrice de réparation distincte de celle(s) déjà introduite(s) et d'une ou plusieurs cassettes d'expression d'ARN guides permettant l'intégration de la séquence d'intérêt contenue dans ladite matrice de réparation distincte dans une zone ciblée du génome de la bactérie, en présence d'un agent inducteur de l'expression de la protéine anti-CRISPR, chaque étape additionnelle étant suivie d'une étape de culture de la bactérie ainsi transformée sur un milieu ne contenant pas l'agent inducteur de l'expression de la protéine anti-CRISPR, permettant typiquement l'expression du complexe ribonucléoprotéique Cas/ARNg, par exemple Cas9/ARNg.

Dans un mode de réalisation particulier du procédé selon l'invention, la bactérie est transformée à l'aide d'un acide nucléique ou d'un outil génétique tels ceux décrits plus haut, utilisant (par exemple codant) une enzyme responsable de la coupure d'au moins un brin de la séquence cible d'intérêt, dans lequel l'enzyme est dans un mode particulier une nucléase, de préférence une nucléase de type Cas, préférentiellement sélectionnée parmi une enzyme Cas9 et une enzyme MAD7. Dans un exemple de mode de réalisation, la séquence cible d'intérêt est une séquence, par exemple le gène *catB*, codant une enzyme conférant à la bactérie la résistance à un ou plusieurs antibiotiques, de préférence à un ou plusieurs antibiotiques appartenant à la classe des amphénicols, typiquement une amphénicol-O-acetyltransférase telle qu'une chloramphénicol-O-acetyltransférase, une séquence contrôlant la transcription de la séquence codante ou une séquence flanquant ladite séquence codante.

Lorsqu'elle est utilisée la protéine anti-CRISPR est typiquement une protéine « anti-Cas » telle que décrite précédemment. La protéine anti-CRISPR est avantageusement une protéine « anti-Cas9 » ou une protéine « anti-MAD7 ».

Tout comme la portion d'ADN ciblée (« séquence reconnue »), la matrice d'édition/de réparation peut elle-même comprendre une ou plusieurs séquences d'acide nucléique ou portions de séquence d'acide nucléique correspondant à des séquences naturelles et/ou synthétiques, codantes et/ou non codantes. La matrice peut également comprendre une ou plusieurs séquences « étrangères », i.e. naturellement absentes du génome des bactéries appartenant au genre *Clostridium,* ou du génome d'espèces particulières dudit genre. La matrice peut aussi comprendre une combinaison de séquences.

L'outil génétique utilisé dans le cadre de la présente invention permet à la matrice de réparation de guider l'incorporation au sein du génome bactérien d'un acide nucléique d'intérêt, typiquement d'une séquence ou portion de séquence d'ADN comprenant au moins 1 paire de base (pb), de préférence au moins 1, 2, 3, 4, 5, 10, 15, 20, 50, 100, 1 000, 10 000, 100 000 ou 1 000 000 pb, typiquement entre 1 pb et 20 kb, par exemple 2, 3, 4, 5, 6, 7, 8, 9, 10, 11, 12 ou 13 kb, ou entre 1 pb et 10 kb, de préférence entre 10 pb et 10 kb ou entre 1 kb et 10 kb, par exemple entre 1pb et 5 kb, entre 2 kb et 5 kb, ou encore entre 2,5 ou 3 kb et 5 kb.

Dans un mode de réalisation particulier, l'expression de la séquence d'ADN d'intérêt permet à la bactérie appartenant au phylum des Firmicutes, en particulier du genre *Clostridium,* du genre *Bacillus* ou du genre *Lactobacillus*, de fermenter (typiquement simultanément) plusieurs sucres différents, par exemple au moins deux sucres différents, typiquement au moins deux sucres différents parmi les sucres comprenant 5 atomes de carbone (tels que le glucose ou le mannose) et/ou parmi les sucres comprenant 6 atomes de carbone (tels que le xylose, l'arabinose ou le fructose), de préférence au moins trois sucres différents, sélectionnés par exemple parmi glucose, xylose et mannose ; glucose, arabinose et mannose ; et glucose xylose et arabinose.

Dans un autre mode de réalisation particulier, la séquence d'ADN d'intérêt code au moins un produit d'intérêt, de préférence un produit favorisant la production de solvant par la bactérie modifiée, typiquement au moins une protéine d'intérêt, par exemple une enzyme ; une protéine membranaire tel qu'un transporteur ; une protéine de maturation d'autres protéines (protéine chaperonne) ; un facteur de transcription ; ou une combinaison de ceux-ci.

De manière particulièrement avantageuse, l'outil génétique selon l'invention permet l'introduction de séquences d'intérêt de petites tailles aussi bien que de grandes tailles, en une étape, i.e. à l'aide d'un seul acide nucléique (typiquement « l'acide nucléique OPT », le « deuxième » ou le « énième » acide nucléique tel que décrit dans le présent texte) ou en plusieurs étapes, i.e. à l'aide de plusieurs acides nucléiques (typiquement le « deuxième » et le ou les « énième » acides nucléiques tels que décrits dans le présent texte), de préférence en une étape.

Dans mode de réalisation particulier de l'invention, les acides nucléiques tels que ce « énième » acide nucléique, et outils génétiques décrits dans le présent texte, permettent de supprimer la portion ciblée de l'ADN bactérien ou de la remplacer par une séquence plus courte (par exemple par une séquence délétée d'au moins une paire de bases) et/ou non fonctionnelle. Dans un mode de réalisation particulier préféré de l'invention, le « deuxième » ou « énième » acide nucléique permet avantageusement d'introduire dans la bactérie, par exemple dans le génome bactérien, un acide nucléique d'intérêt comprenant au moins une paire de base, et jusqu'à 2, 3, 4, 5, 6, 7, 8, 9, 10, 11, 12, 13, 14 ou 15 kb.

Les acides nucléiques d'intérêt peuvent être insérés dans le chromosome bactérien au niveau de régions identiques ou différentes selon les ARNg utilisés.

Grâce à l'invention, typiquement grâce à l'outil génétique et au procédé selon l'invention, il est à présent possible de transformer systématiquement, et de modifier efficacement (fréquence de recombinaison homologue performante), substantiellement (incorporation possible au sein du génome de la bactérie d'un acide nucléique d'intérêt de grande taille) et de manière stable (le maintien des bactéries transformées en contact avec des antibiotiques n'est pas nécessaire), les bactéries du genre *Clostridium* de manière à obtenir des bactéries transformées d'intérêt, par exemple des mutants améliorés présentant une différence génotypique ou phénotypique par rapport à la bactérie dont elle est issue, typiquement des bactéries exploitables sur le plan industriel, par exemple des bactéries utilisables dans la production de solvants ou biocarburants.

Un autre objet de l'invention concerne une bactérie du genre *Clostridium,* typiquement une bactérie solvantogène du genre *Clostridium,* obtenue à l'aide du procédé et/ou transformée et idéalement modifiée génétiquement à l'aide de l'outil génétique selon l'invention, ainsi que toute bactérie dérivée, clone, mutant ou version génétiquement modifiée de celle-ci, et leurs utilisations. Une telle bactérie exprime le ou les acides nucléiques d'intérêt introduits dans son génome par recombinaison homologue à l'aide de la matrice de réparation. Une telle bactérie peut comprendre tout ou partie de l'outil génétique selon l'invention, typiquement une nucléase telle que Cas9 ou un acide nucléique codant une nucléase telle que Cas9.

Un exemple de bactérie ainsi transformée et/ou génétiquement modifiée grâce à l'invention est une bactérie n'exprimant plus une enzyme lui conférant la résistance à un ou plusieurs antibiotiques, en particulier une bactérie n'exprimant plus une amphénicol-O-acetyltransférase, par exemple une bactérie exprimant à l'état sauvage le gène *catB*, et dépourvue dudit gène *catB* ou incapable d'exprimer ledit gène *catB* une fois transformée et/ou génétiquement modifiée grâce à l'invention. La bactérie ainsi transformée et/ou génétiquement modifiée grâce à l'invention est rendue sensible à un amphénicol, par exemple à un amphénicol tel que décrit dans le présent texte, en particulier au chloramphénicol ou au thiamphénicol.

Un exemple particulier de bactérie génétiquement modifiée préférée selon l'invention est la bactérie identifiée dans la présente description en tant que C. *beijerinckii* IFP962 Δ*catB* (également identifiée dans le présent texte en tant que C. *beijerinckii* DSM 6423 Δ*catB*) telle qu'enregistrée sous le numéro de dépôt LMG P-31151 auprès de la Belgian Co-ordinated Collections of Micro-organisms (« BCCM », K.L. Ledeganckstraat 35, B-9000 Gent - Belgique) le 6 décembre 2018.

Un autre exemple particulier de bactérie génétiquement modifiée préférée selon l'invention est la bactérie identifiée dans la présente description en tant que C. *beijerinckii* est une souche C. *beijerinckii* IFP963 Δ*catB* ΔpNF2 telle qu'enregistrée sous le numéro de dépôt LMG P-31277 auprès de la collection BCCM-LMG le 20 février 2019.

La description concerne également toute bactérie dérivée, clone, mutant ou version génétiquement modifiée de l'une desdites bactéries, par exemple toute bactérie dérivée, clone, mutant ou version génétiquement modifiée restant sensible à un amphénicol tel que le thiamphénicol et/ou le chloramphénicol.

Selon un mode de réalisation particulier, la bactérie transformée et/ou génétiquement modifiée selon l'invention, par exemple la bactérie *C. beijerinckii* DSM 6423 Δ*catB* ou la bactérie *C. beijerinckii* DSM6423 Δ*catB* ΔpNF2, est encore susceptible d'être transformée, et de préférence modifiée génétiquement. Elle peut l'être à l'aide d'un acide nucléique, par exemple d'un plasmide tel que décrit dans la présente description, par exemple dans la partie expérimentale. Un exemple d'acide nucléique susceptible d'être avantageusement utilisé est le plasmide pCas9_{acr} de séquence SEQ ID NO : 23 (décrit dans la partie expérimentale de la présente description) ou encore un plasmide sélectionné parmi pCas9_{ind} (SEQ ID NO : 22), pCas9_{cond} (SEQ ID NO : 133) et pMAD7 (SEQ ID NO : 134).

Un aspect particulier de l'invention concerne l'utilisation d'une bactérie modifiée génétiquement décrite dans le présent texte, de préférence la bactérie C. *beijerinckii* IFP962 Δ*catB* déposée sous le numéro LMG P-31151, de manière encore plus préférée la bactérie C. *beijerinckii* IFP963 Δ*catB* ΔpNF2 déposée sous le numéro LMG P-31277, ou d'une version génétiquement modifiée de l'une de celles-ci, par exemple à l'aide de l'un des acides nucléiques, outils génétiques ou procédés décrits dans le présent texte, pour produire, grâce à l'expression du ou des acides nucléiques d'intérêt introduits volontairement dans son génome, un ou plusieurs solvants, de préférence au moins l'isopropanol, de préférence à l'échelle industrielle.

Dans un mode de réalisation particulier, la bactérie du genre *Clostridium* selon l'invention, obtenue à l'aide du procédé et de l'outil génétique selon l'invention, n'est en mesure de produire un ou plusieurs solvants que grâce à l'expression du ou des acides nucléiques d'intérêt introduits volontairement dans son génome.

L'invention concerne aussi un kit (trousse) pour transformer, et typiquement modifier génétiquement, une bactérie du genre *Clostridium* comprenant tout ou partie des éléments de l'outil génétique tels que décrits dans le présent texte, typiquement i) un premier acide nucléique codant une endonucléase d'ADN telle que Cas9, dans lequel la séquence codant l'endonucléase est placée sous le contrôle d'un promoteur, et ii) au moins un deuxième acide nucléique codant une matrice de réparation permettant, par un mécanisme de recombinaison homologue, le remplacement d'une portion de l'ADN bactérien ciblée par l'endonucléase par une séquence d'intérêt, et au moins un inducteur adapté au promoteur inductible de l'expression de la protéine anti-CRISPR sélectionnée utilisé au sein de l'outil. Le kit peut en outre comprendre un ou plusieurs inducteurs adaptés au(x) promoteur(s) inductible(s) sélectionné(s) éventuellement utilisé(s) au sein de l'outil pour contrôler l'expression de l'endonucléase et/ou d'un ou de plusieurs ARN guides.

Est également décrit un kit comprenant (i) un acide nucléique tel que décrit dans le présent texte, par exemple « un acide nucléique OPT » ou un fragment d'ADN reconnaissant une séquence cible dans une bactérie telle que décrite dans le présent texte, et (ii) au moins un outil, de préférence plusieurs outils, sélectionné(s) parmi les éléments d'un outil de modification génétique tel que décrit dans le présent texte permettant de transformer, et typiquement modifier génétiquement une telle bactérie, en vue de produire un variant amélioré de ladite bactérie; un acide nucléique en tant qu'ARNg ; un acide nucléique en tant que matrice de réparation ; un « acide nucléique OPT » ; au moins une paire d'amorces, par exemple une paire d'amorces telle que décrite dans le contexte de la présente invention ; et un inducteur permettant l'expression d'une protéine codée par ledit outil, par exemple d'une nucléase de type Cas9 ou MAD7.

L'outil de modification génétique pour transformer, et typiquement modifier génétiquement une bactérie telle que décrite dans le présent texte, peut-être par exemple sélectionné parmi un « acide nucléique OPT », un outil CRISPR, un outil basé sur l'utilisation d'introns de type II et un outil d'échange allélique, comme expliqué plus haut.

Dans un mode de réalisation particulier, le kit comprend tout ou partie des éléments d'un outil génétique tel que décrit dans le présent texte.

Un kit particulier pour transformer, et de préférence modifier génétiquement, une bactérie appartenant au phylum des Firmicutes telle que décrite dans le présent texte, ou pour produire au moins un solvant, par exemple un mélange de solvants, à l'aide d'une telle bactérie, comprend un acide nucléique comprenant, ou consistant en, i) tout ou partie de la séquence SEQ ID NO : 126 et ii) une séquence permettant la modification du matériel génétique d'une bactérie et/ou l'expression au sein de ladite bactérie d'une séquence d'ADN partiellement ou totalement absente du matériel génétique présent au sein de la version sauvage de ladite bactérie ; ainsi que au moins un inducteur adapté au promoteur inductible de l'expression de la protéine anti-CRISPR sélectionné utilisé au sein d'un outil génétique décrit dans le présent texte.

Le kit peut en outre comprendre un ou plusieurs inducteurs adaptés au(x) promoteur(s) inductible(s) sélectionné(s) éventuellement utilisé(s) au sein de l'outil génétique pour contrôler l'expression de la nucléase utilisée et/ou d'un ou de plusieurs ARN guides.

Un kit particulier selon l'invention permet l'expression d'une endonucléase, par exemple d'une protéine Cas9 ou MAD7 comprenant une étiquette (ou « tag »).Les kits selon l'invention peuvent en outre comprendre un ou plusieurs consommables tels qu'un milieu de culture, au moins une bactérie compétente du genre *Clostridium* (i.e. conditionnée en vue de la transformation), au moins un ARNg, une nucléase, par exemple une protéine Cas9 ou MAD7, une ou plusieurs molécules de sélection, ou encore une notice explicative.

L'invention concerne typiquement un kit pour la mise en oeuvre du procédé de transformation et idéalement de modification génétique décrit dans le présent texte, et/ou pour la production de solvant(s) (au moins un solvant) à l'aide d'une bactérie du genre *Clostridium.*

L'invention concerne aussi les utilisations possibles des acides nucléiques, de l'outil génétique, du procédé, ou du kit selon l'invention pour transformer, et typiquement modifier génétiquement, une bactérie du genre *Clostridium,* typiquement une bactérie solvantogène du genre *Clostridium,* par exemple pour générer des variants améliorés d'une bactérie du genre *Clostridium.*

La description concerne en particulier l'utilisation d'un kit selon l'invention, ou de l'un ou de plusieurs des éléments de ce kit, pour la mise en oeuvre d'un procédé décrit dans le présent texte de transformation, et idéalement de modification génétique, d'une bactérie telle que décrite dans le présent texte, typiquement une bactérie du genre *Clostridium* (par exemple la bactérie *C. beijerinckii* DSM 6423 Δ*catB* déposée sous le numéro LMG P-31151), de préférence une bactérie possédant à l'état sauvage à la fois un chromosome bactérien et au moins une molécule d'ADN distincte de l'ADN chromosomique (typiquement un plasmide naturel), de manière préférée entre toute de la bactérie *C. beijerinckii* DSM 6423 Δ*catB* ΔpNF2 déposée sous le numéro LMG P-31277.

L'invention concerne enfin les utilisations possibles des acides nucléiques, de l'outil génétique, du procédé, du kit ou d'une bactérie du genre *Clostridium* transformée selon l'invention, en particulier pour permettre la production de solvant(s) ou biocarburant(s), ou de mélanges de ceux-ci, typiquement à l'échelle industrielle. Des solvants susceptibles d'être produits sont typiquement l'acétone, le butanol, l'éthanol, l'isopropanol ou un mélange de ceux-ci, typiquement un mélange éthanol/isopropanol, butanol/isopropanol, ou éthanol/butanol, de préférence un mélange isopropanol/butanol.

Dans un mode de réalisation particulier, le ratio du mélange éthanol/isopropanol est au moins égal à 1/4. Ce ratio est de préférence compris entre 1/3 et 1, et est de manière encore plus préférée égal à 1. Dans un mode de réalisation particulier, le ratio du mélange éthanol/butanol est au moins égal à 1/4. Ce ratio est de préférence compris entre 1/3 et 1, et est de manière encore plus préférée égal à 1.

Dans un mode de réalisation particulier, le ratio du mélange isopropanol/butanol est au moins égal à 1/4. Ce ratio est de préférence compris entre 1/3 et 1, et est de manière encore plus préférée égal à 1. L'utilisation de bactéries transformées selon l'invention permet typiquement la production par an à l'échelle industrielle d'au moins 100 tonnes d'acétone, d'au moins 100 tonnes d'éthanol, d'au moins 1000 tonnes d'isopropanol, d'au moins 1800 tonnes de butanol, ou d'au moins 40 000 tonnes d'un mélange de ceux-ci.

Les exemples et figures ci-après ont pour but d'illustrer plus pleinement l'invention sans pour autant en limiter la portée.

### FIGURES

**Figure 1** : Système CRISPR/Cas9 utilisé pour l'édition du génome en tant qu'outil génétique permettant de créer, à l'aide de la nucléase Cas9, une ou des coupures double brin dans l'ADN génomique dirigée(s) par l'ARNg.
   ARNg, ARN guide ; PAM, Protospacer Adjacent Motif. Figure modifiée depuis Jinek *et al*, 2012.
**Figure 2** : Réparation par recombinaison homologue d'une coupure double brin induite par Cas9. PAM, Protospacer Adjacent Motif.

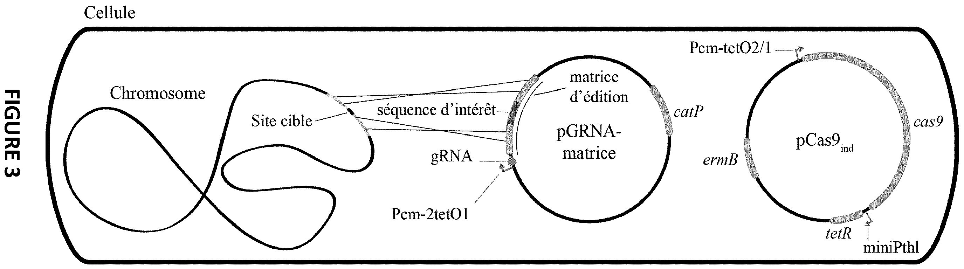

**Figure 3** **:** Utilisation de CRISPR/Cas9 chez *Clostridium.*
   *ermB*, gène de résistance à l'érythromycine ; *catP* (SEQ ID NO : 70), gène de résistance au thiamphénicol/chloramphenicol ; *tetR*, gène dont le produit d'expression réprime la transcription à partir de Pcm-tetO2/1; Pcm-2tetO1 et Pcm-tetO2/1, promoteurs inductibles à l'anhydrotétracycycline, « aTc » (Dong *et al*., 2012) ; miniPthl, promoteur constitutif (Dong *et al*., 2012).

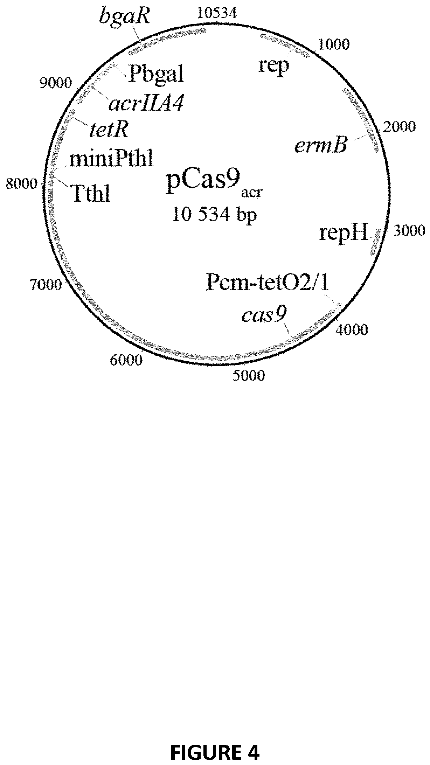

**Figure 4** : Carte du plasmide pCas9_{acr} (SEQ ID NO : 23).
   *ermB*, gène de résistance à l'érythromycine ; rep, origine de réplication chez *E. coli* ; repH, origine de réplication chez *C. acetobutylicum* ; Tthl, terminateur thiolase ; miniPthl, promoteur constitutif (Dong *et al.,* 2012) ; Pcm-tetO2/1, promoteur réprimé par le produit de *tetR* et inductible par l' anhydrotétracycline, « aTc » (Dong *et al*., 2012) ; Pbgal, promoteur réprimé par le produit de *lacR* et inductible par le lactose (Hartman *et al.,* 2011) ; *acrIIA4,* gène codant la protéine anti-CRISPR AcrII14 ; *bgaR,* gène dont le produit d'expression réprime la transcription à partir de Pbgal.

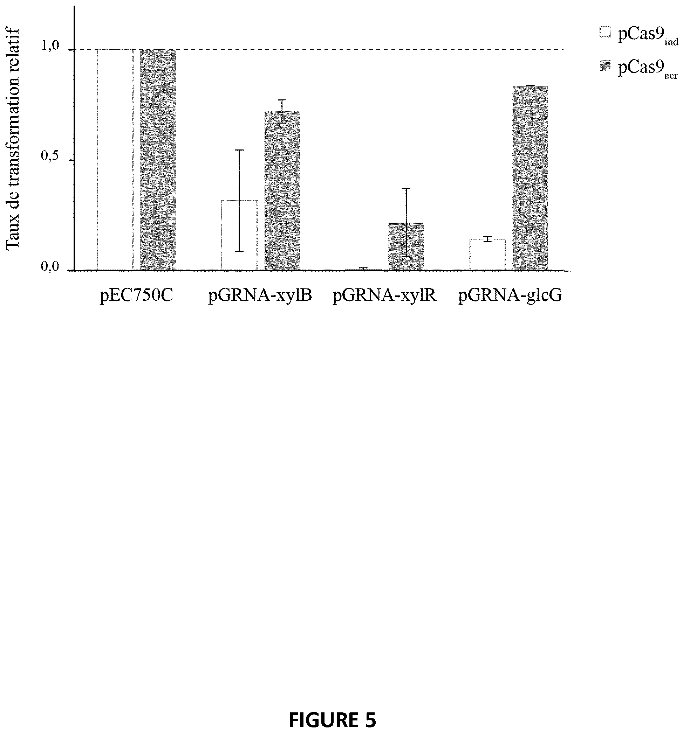

**Figure 5** ***:*** Taux de transformation relatifs de *C. acetobutylicum* DSM 792 contenant pCas9_{ind} (SEQ ID NO : 22) ou pCas9_{acr} (SEQ ID N : 23). Les fréquences sont exprimées en nombre de transformants obtenus par µg d'ADN utilisé lors de la transformation, rapportées aux fréquences de transformation de pEC750C (SEQ ID NO : 106), et représentent les moyennes d'au moins deux expériences indépendantes.

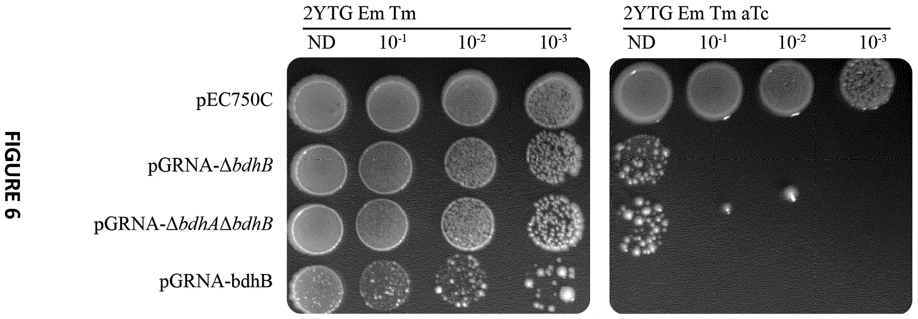

**Figure 6** : Induction du système CRISPR/Cas9 chez des transformants de la souche DSM 792 contenant pCas9_{acr} et un plasmide d'expression de l'ARNg ciblant *bdhB*, avec (SEQ ID NO : 79 et SEQ ID NO : 80) ou sans (SEQ ID NO : 105) matrice de réparation. Em, érythromycine ; Tm, thiamphénicol ; aTc, anhydrotétracycline ; ND, non dilué.

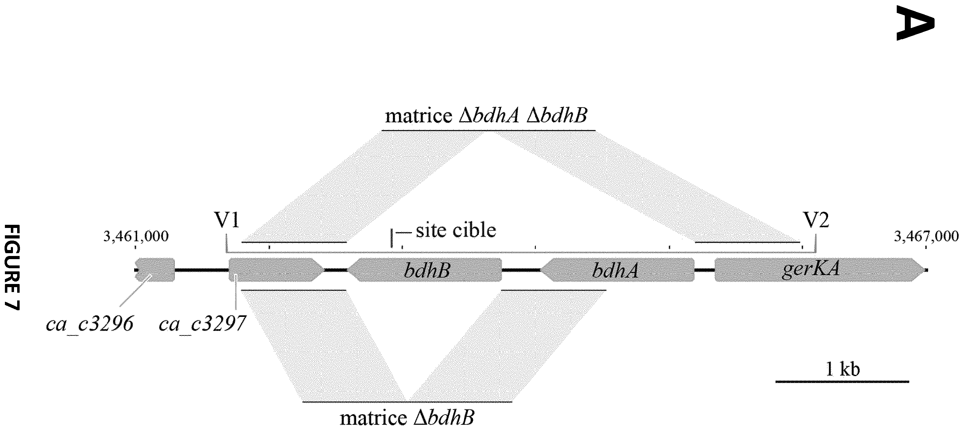

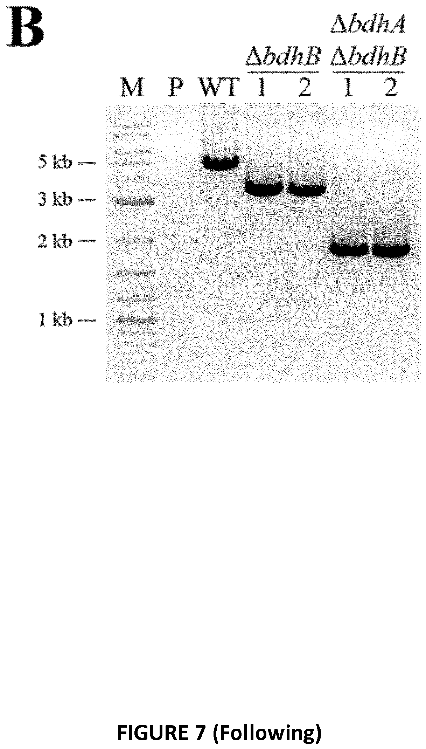

**Figure 7** : Modification du locus *bdh* de C. *acetobutylicum* DSM792 via le système CRISPR/Cas9.
   A, organisation génétique du locus *bdh.* Les homologies entre matrice de réparation et ADN génomique sont mises en évidence à l'aide de parallélogrammes gris clair. Les sites d'hybridation des amorces V1 et V2 sont également représentés.
   B, amplification du locus *bdh* à l'aide des amorces V1 et V2. M, marqueur de taille 2-log (NEB) ; P, plasmide pGRNA*-*Δ*bdhA*Δ*bdhB* ; WT, souche sauvage.

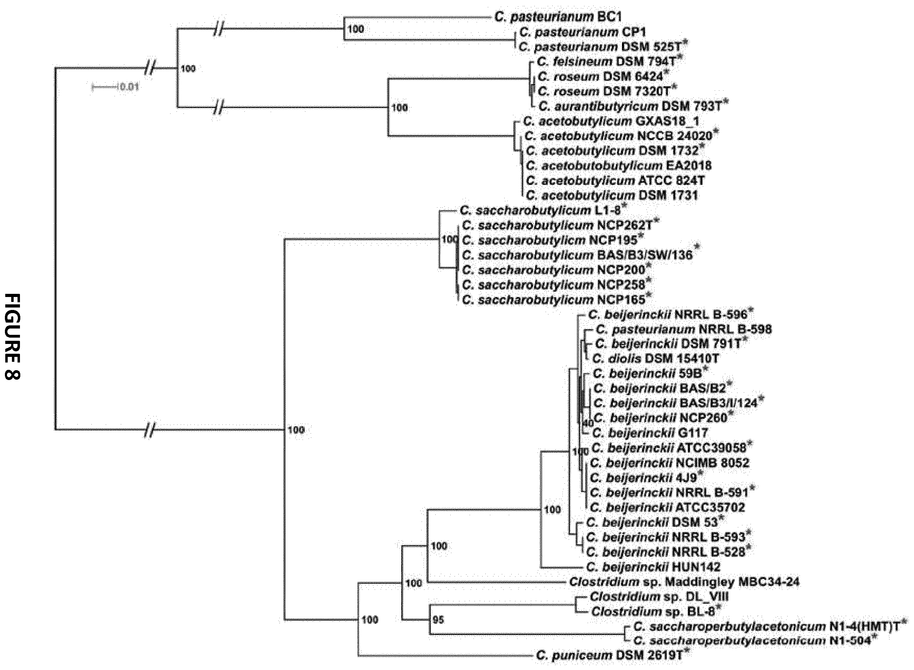

**Figure 8** : Classement de 30 souches de *Clostridium* solvantogènes, d'après Poehlein *et al.,* 2017. A noter que le sous-clade C. *beijerinckii* NRRL B-593 est également identifié dans la littérature en tant que C. *beijerinckii* DSM 6423.

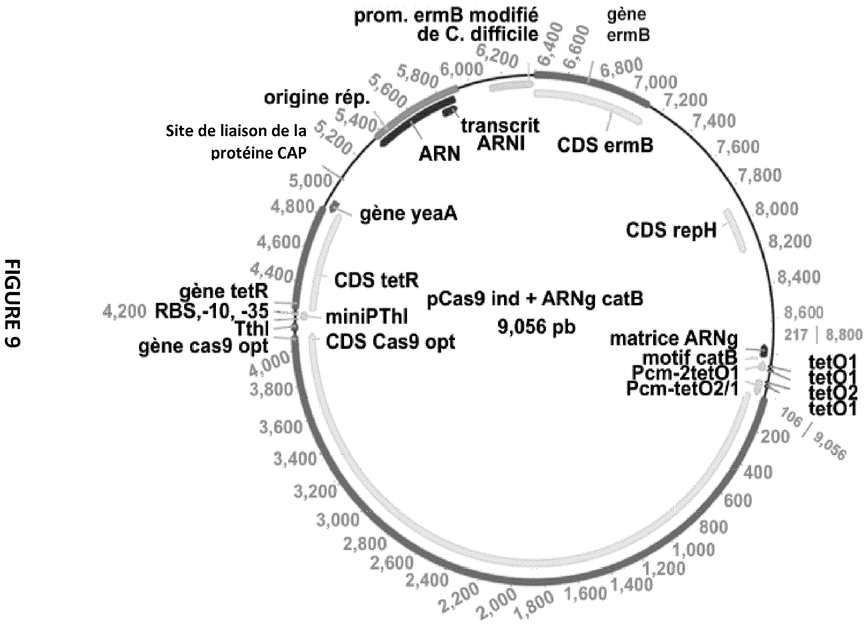

**Figure 9** **:** Carte du plasmide pCas9ind-Δc*atB*

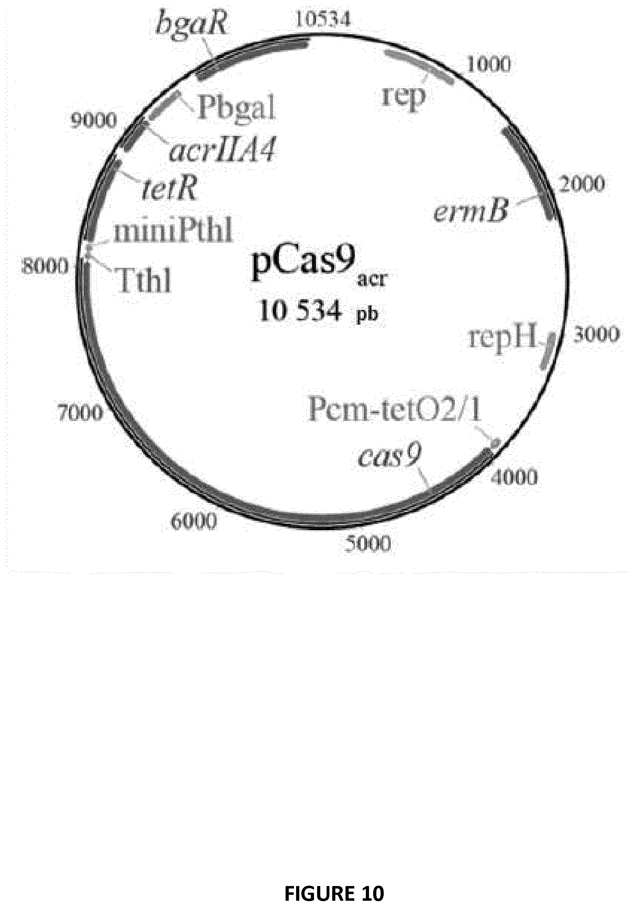

**Figure 10** : Carte du plasmide pCas9acr

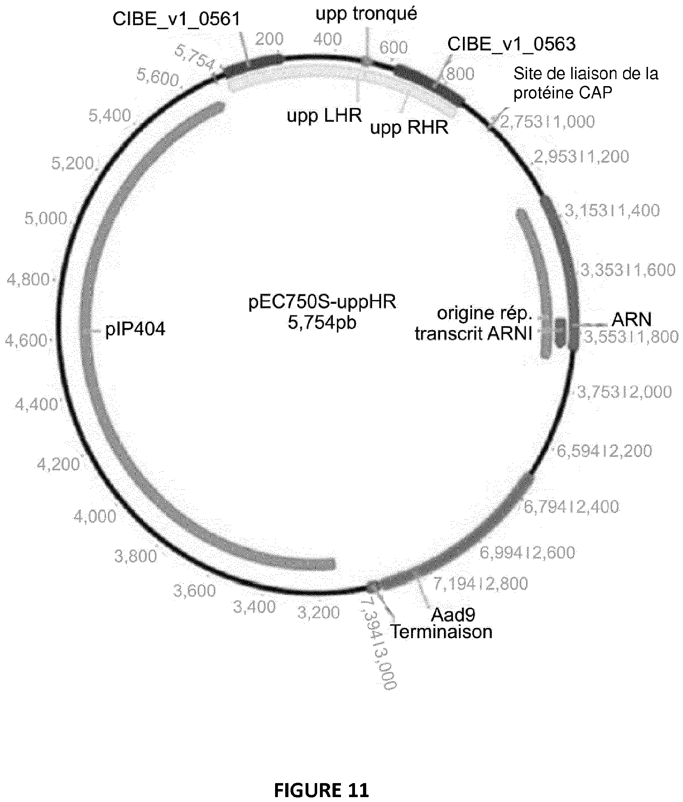

**Figure 11** : Carte du plasmide pEC750S-*upp*HR

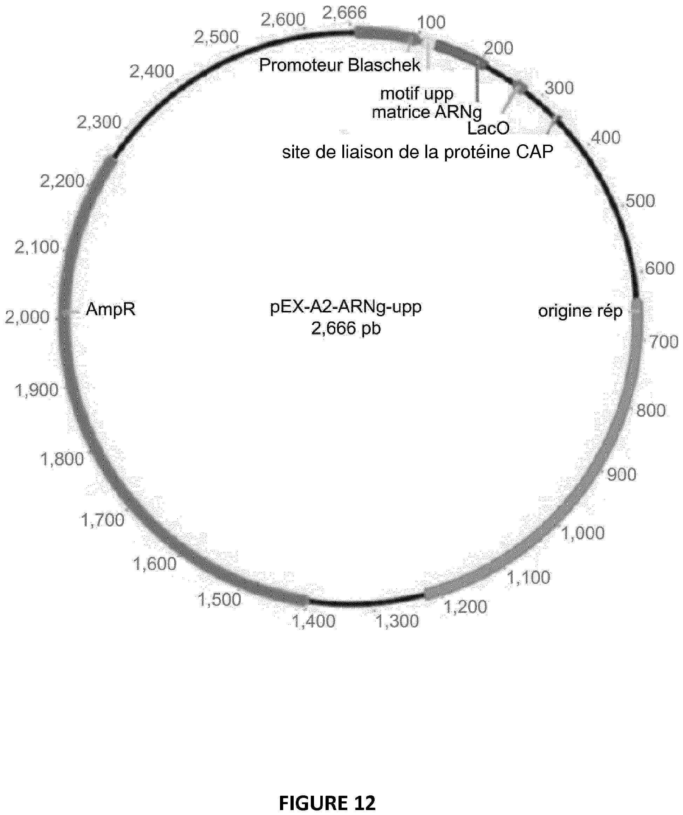

**Figure 12** **:** Carte du plasmide pEX-A2-gRNA-*upp*.

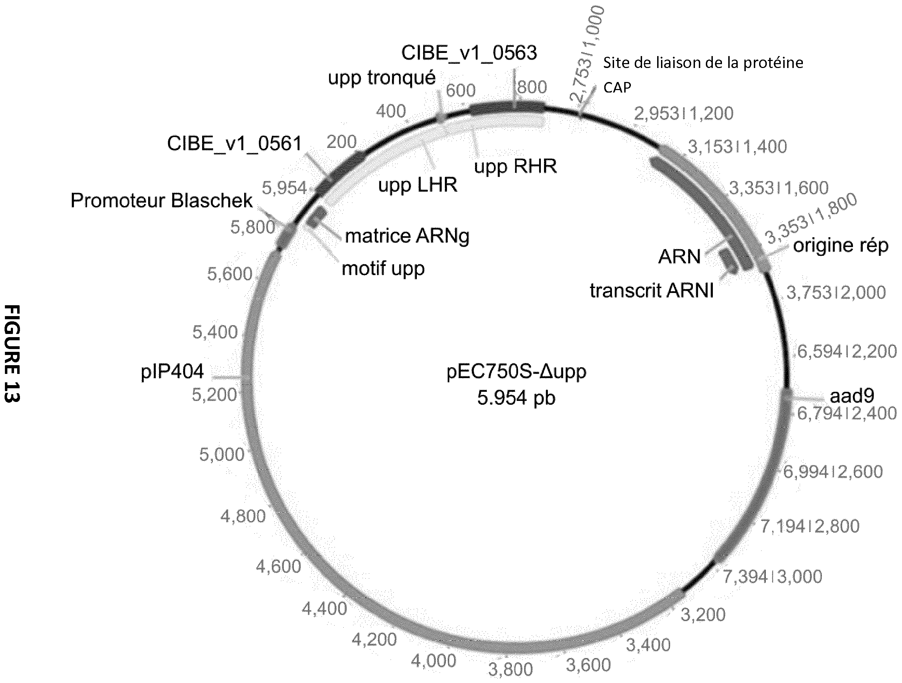

**Figure 13** **:** Carte du plasmide pEC750S-Δ*upp*.

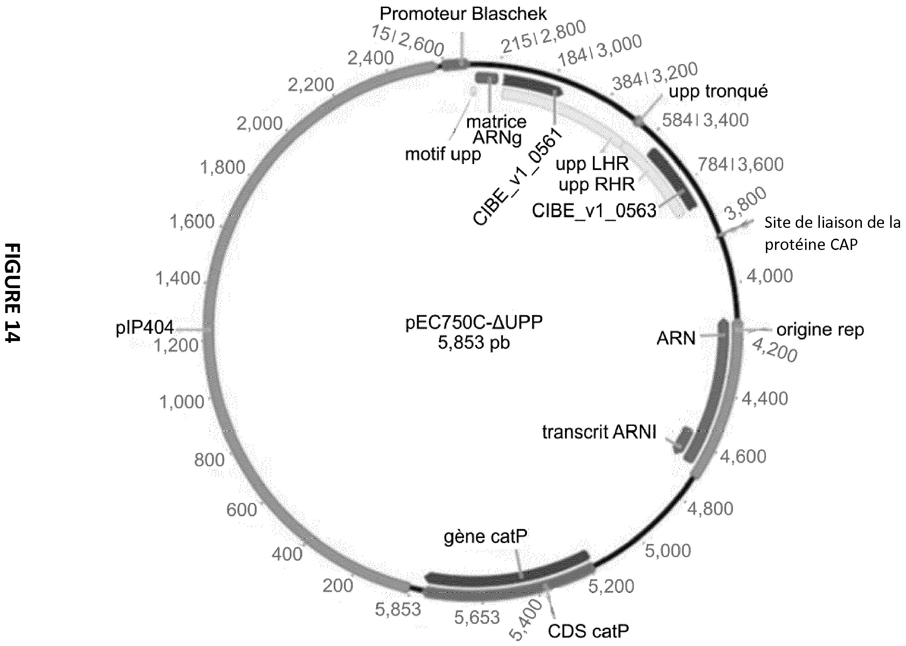

**Figure 14** **:** Carte du plasmide pEC750C-Δ*upp*

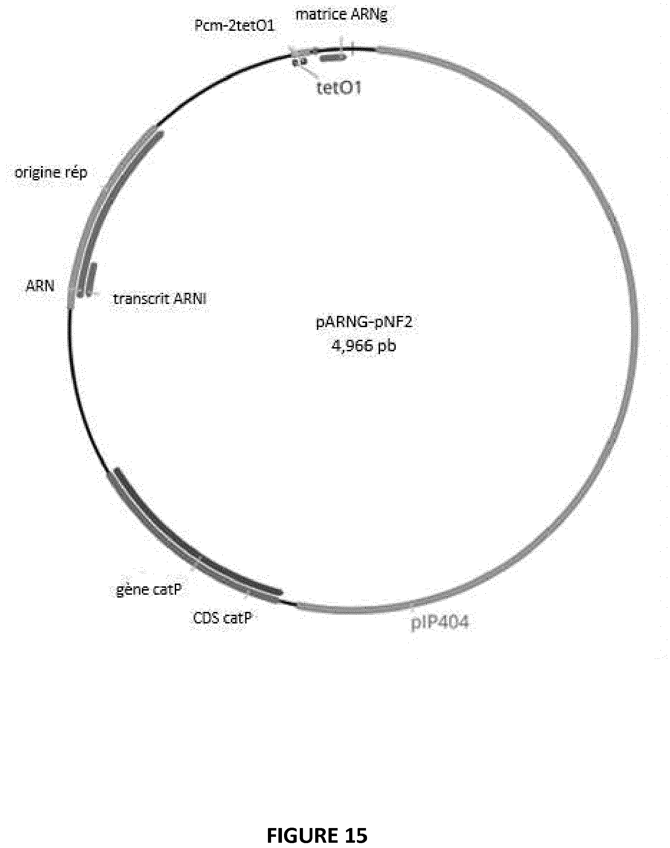

**Figure 15** **:** Carte du pGRNA-pNF2

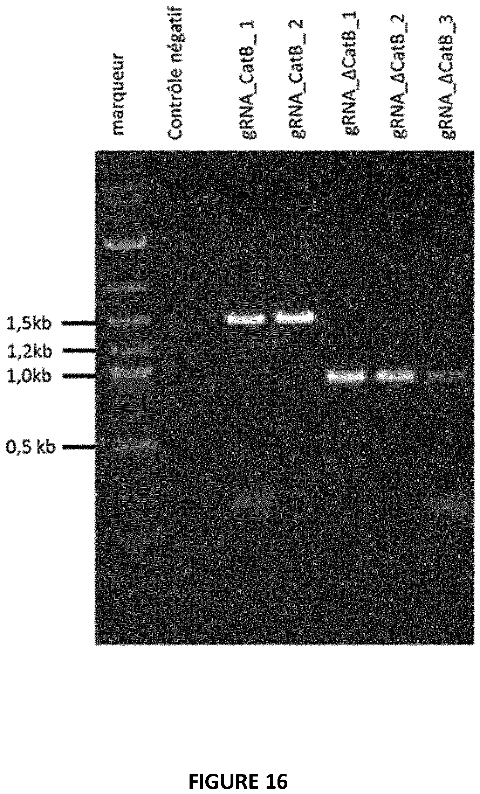

**Figure 16** : Amplification PCR du gène catB dans les clones issus de la transformation bactérienne de la souche *C. beijerinckii* DSM 6423.
   Amplification d'environ 1,5 kb si la souche possède encore le gène *catB*, ou d'environ 900 pb si ce gène est délété.

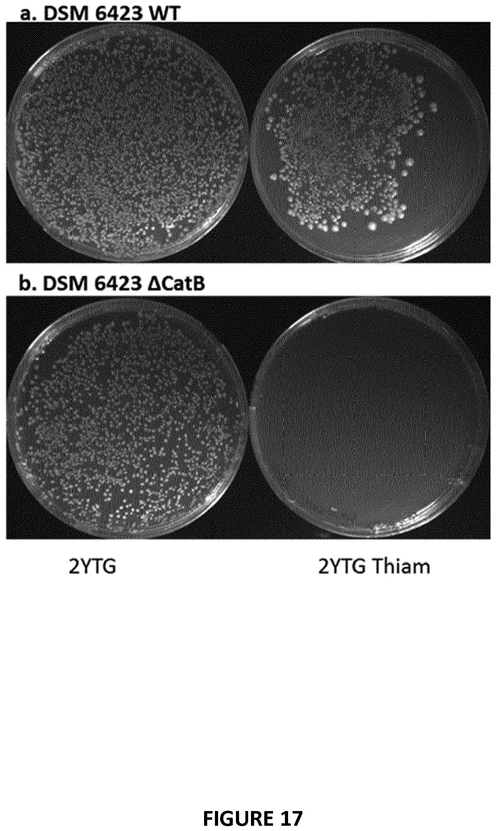

**Figure 17** **:** Croissance des souches *C. beijerinckii* DSM 6423 WT *et* Δ*catB* sur milieu 2YTG et milieu sélectif 2YTG thiamphénicol.

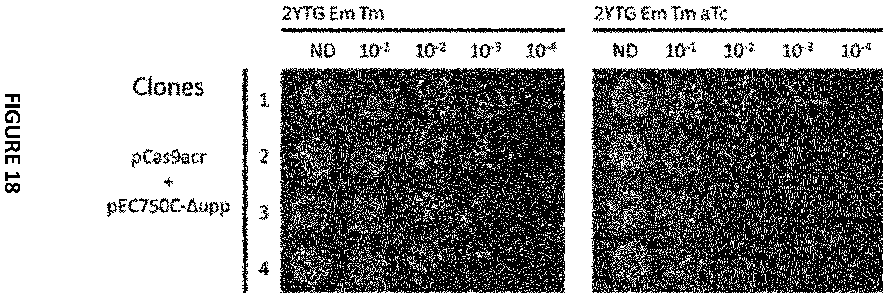

**Figure 18** **:** Induction du système CRISPR/Cas9acr chez des transformants de la souche *C. beijerinckii* DSM 6423 contenant pCas9_{acr} et un plasmide d'expression de l'ARNg ciblant *upp,* avec ou sans matrice de réparation. Légende : Em, érythromycine ; Tm, thiamphénicol ; aTc, anhydrotétracycline ; ND, non dilué.

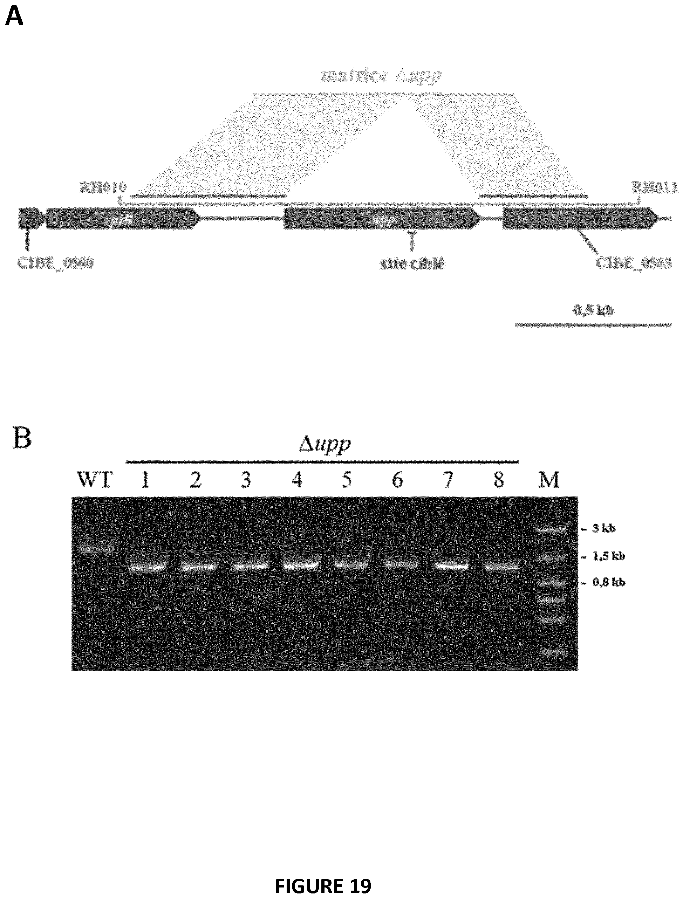

**Figure 19** **:** La Figure 19A représente la Modification du locus *upp* de *C. beijerinckii* DSM 6423 via le système CRISPR/Cas9. La Figure 19A représente l'organisation génétique du locus *upp* : gènes, site cible de l'ARNg et matrices de réparation, associées aux régions d'homologies correspondantes sur l'ADN génomique. Les sites d'hybridation des amorces pour la vérification par PCR (RH010 et RH011) sont également indiqués.
   La Figure 19B représente la Modification du locus *upp* de *C. beijerinckii* DSM 6423 via le système CRISPR/Cas9. La Figure 19B représente l'amplification du locus upp à l'aide des amorces RH010 et RH011. Une amplification de 1680 pb est attendue dans le cas d'un gène sauvage, contre 1090 pb pour un gène upp modifié. M, marqueur de taille 100 bp - 3 kb (Lonza) ; WT, souche sauvage.

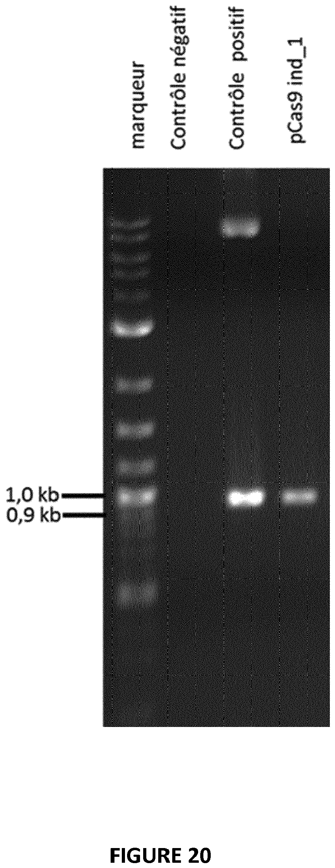

**Figure 20** : Amplification PCR vérifiant la présence du plasmide pCas9_{ind}. dans la souche *C. beijerinckii* 6423 Δ*catB.*

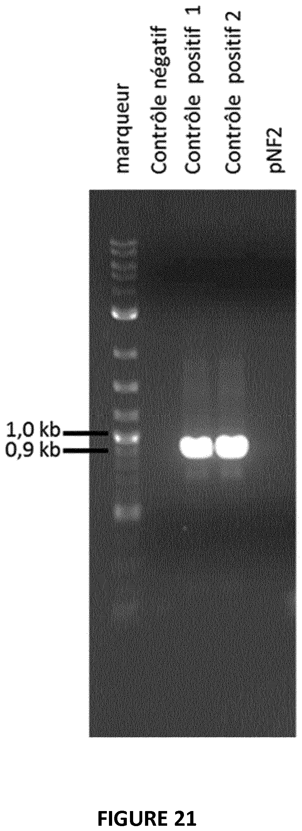

**Figure 21** **:** Amplification PCR (≈900 pb) vérifiant la présence ou non du plasmide naturel pNF2 avant induction (contrôle positif 1 et 2) puis après induction sur milieu contenant de l'aTc du système CRISPR-Cas9.

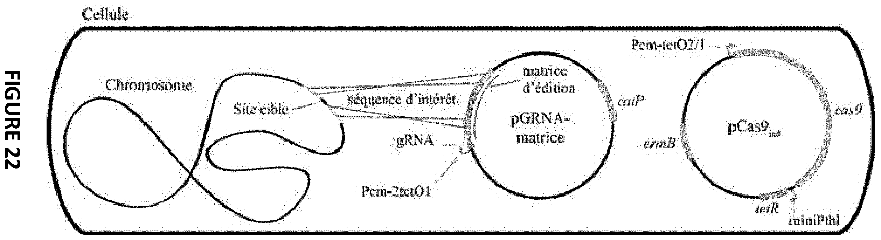

**Figure 22** **:** Outil génétique de modification de bactéries, adapté aux bactéries du genre *Clostridium,* basé sur l'utilisation de deux plasmides (cf. WO2017/064439, Wasels et al., 2017).

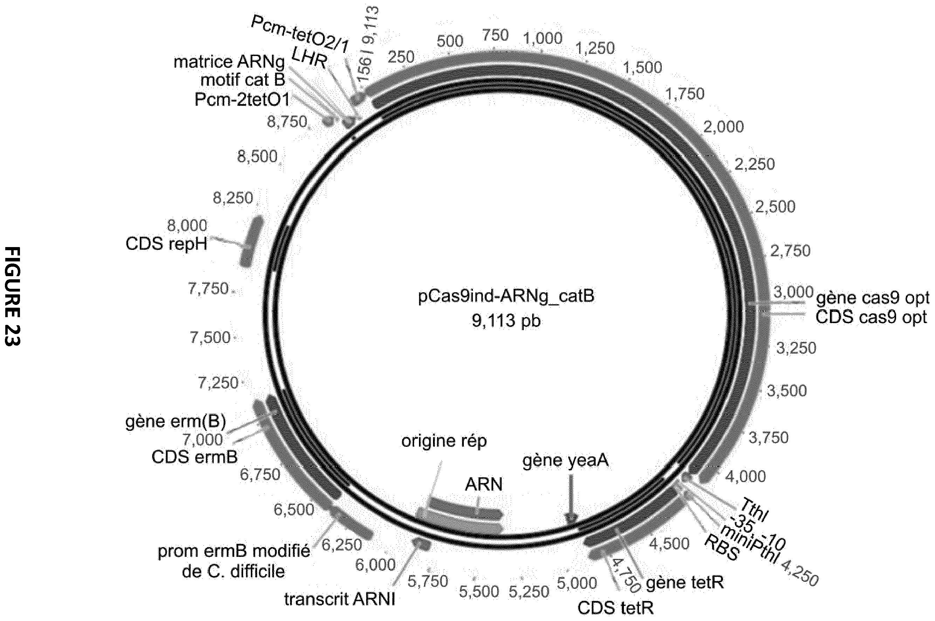

**Figure 23** : Carte du plasmide pCas9ind-gRNA_*catB*.

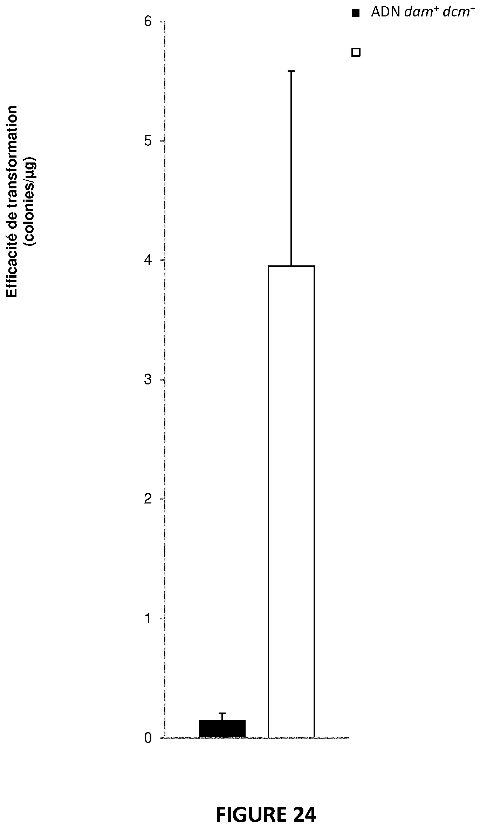

**Figure 24** **:** Efficacité de transformation (en colonies observées par µg d'ADN transformé) pour 20 µg de plasmide pCas9_{ind} dans la souche de *C. beijerinckii* DSM6423. Les barres d'erreur représentent l'erreur standard de la moyenne pour un triplicat biologique.

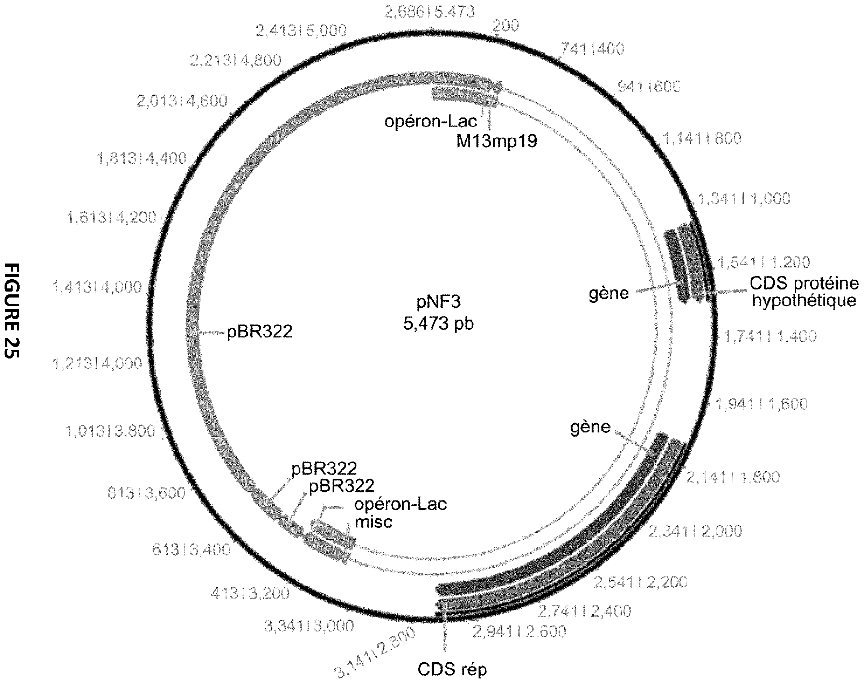

**Figure 25** : Carte du plasmide pNF3.

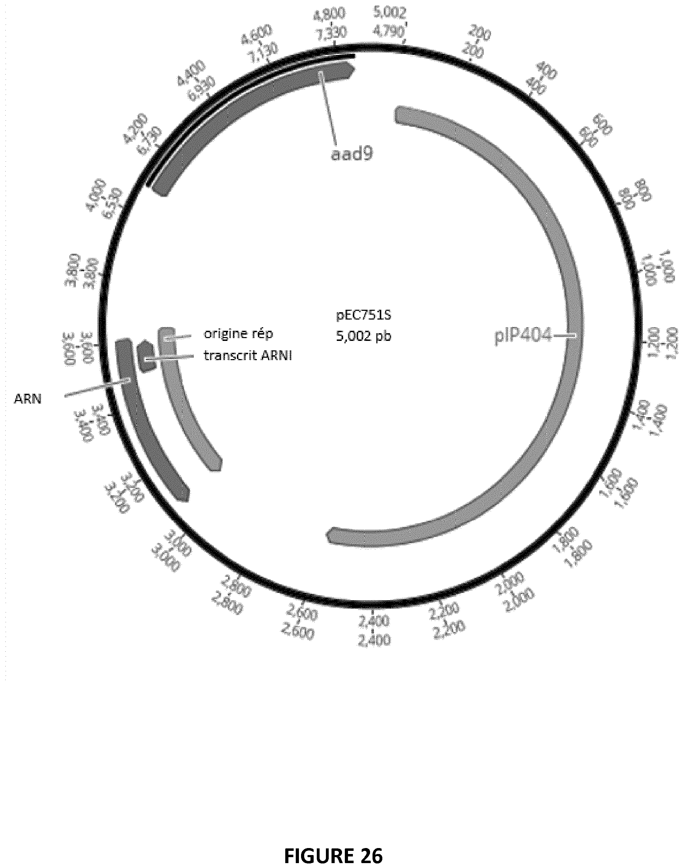

**Figure 26** : Carte du plasmide pEC751S.

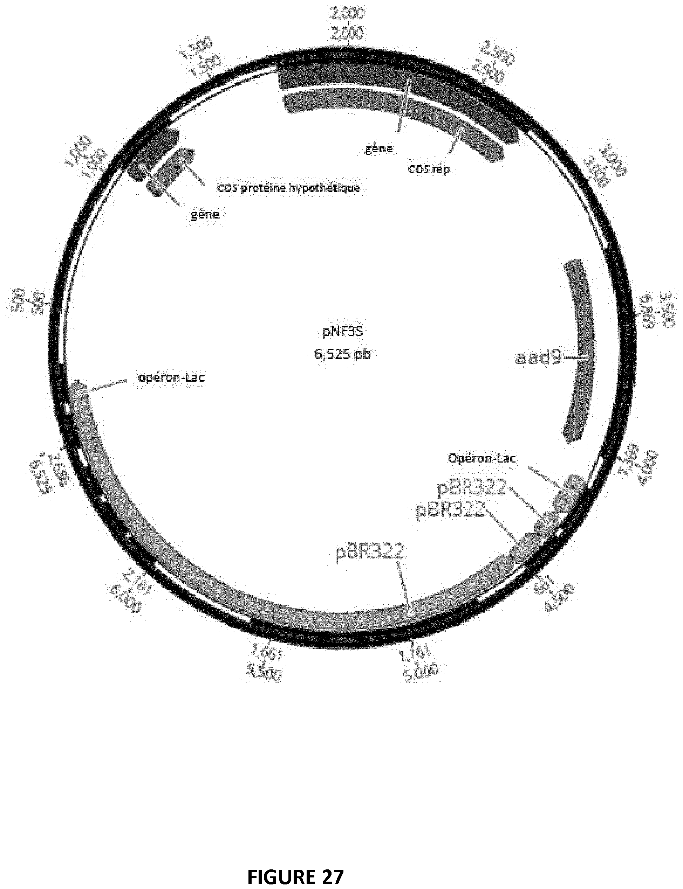

**Figure 27** : Carte du plasmide pNF3S.

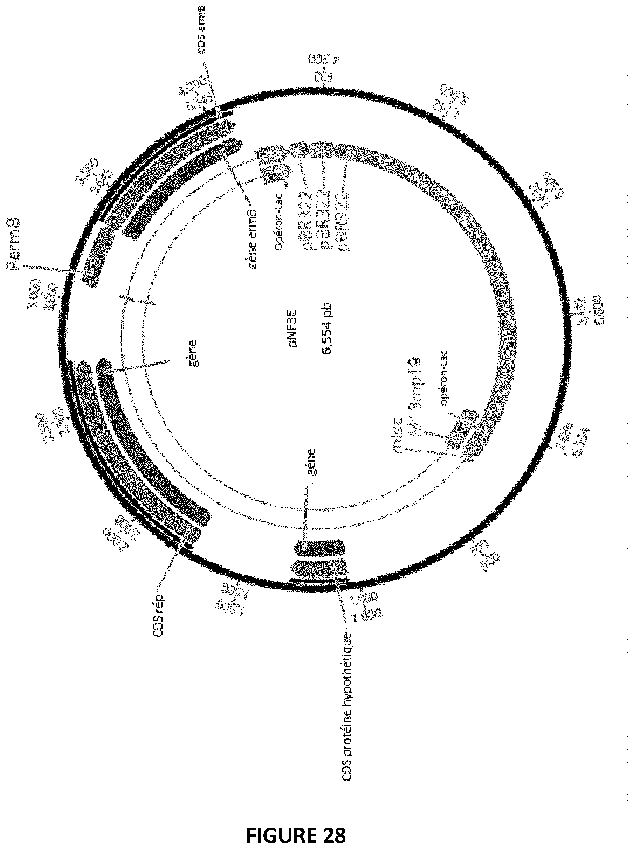

**Figure 28** : Carte du plasmide pNF3E.

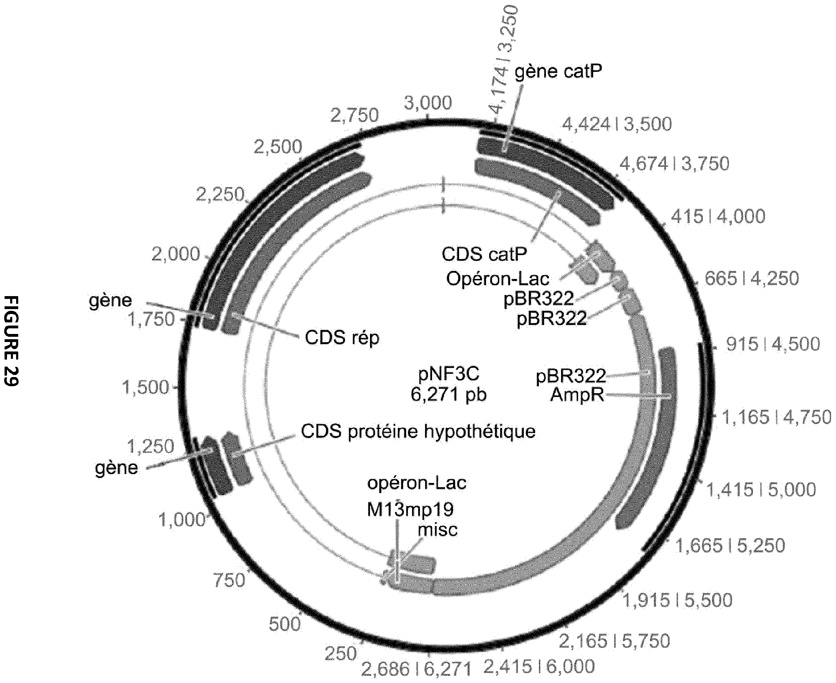

**Figure 29** : Carte du plasmide pNF3C.

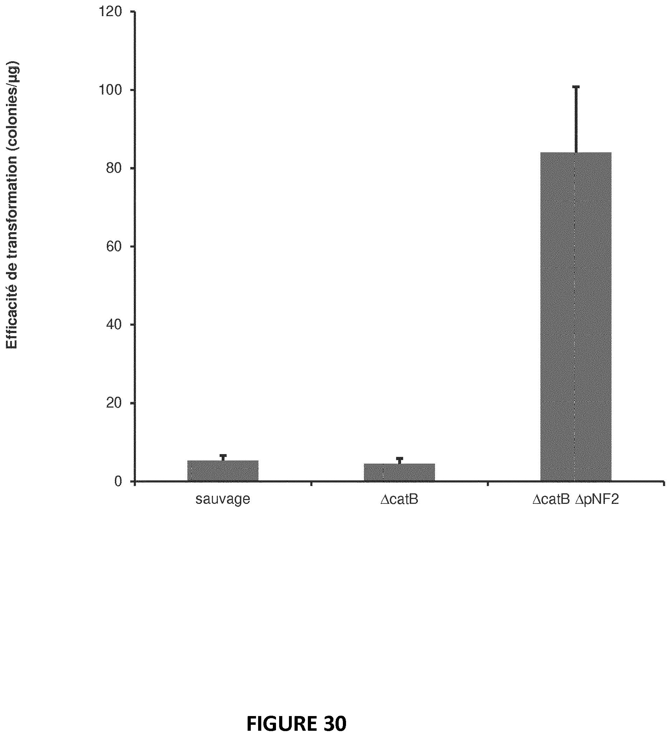

**Figure 30** : Efficacité de transformation (en colonies observées par µg d'ADN transformé) du plasmide pCas9_{ind} dans trois souches de *C. beijerinckii* DSM 6423. Les barres d'erreur correspondent à l'écart standard de la moyenne pour un duplicat biologique.

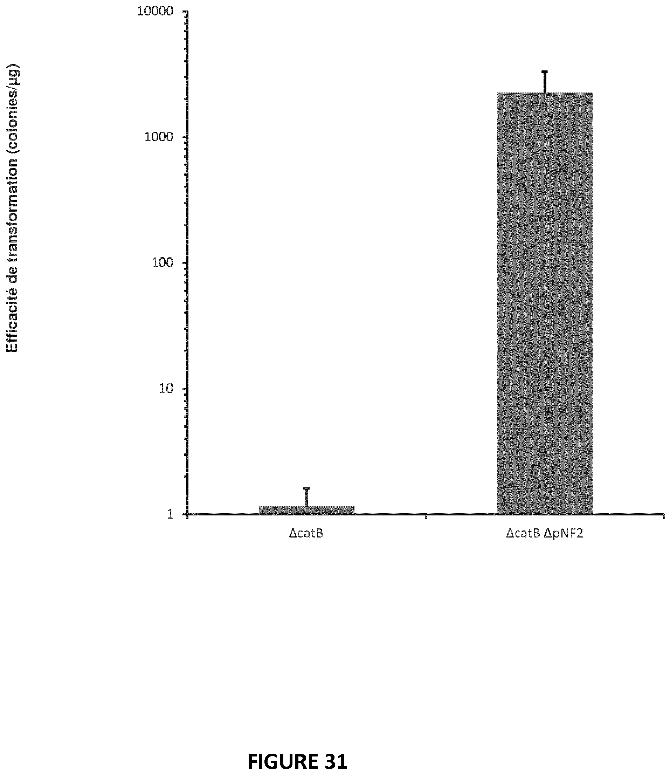

**Figure 31** : Efficacité de transformation (en colonies observées par µg d'ADN transformé) du plasmide pEC750C dans deux souches dérivées de *C. beijerinckii* DSM 6423. Les barres d'erreur correspondent à l'écart standard de la moyenne pour un duplicat biologique.

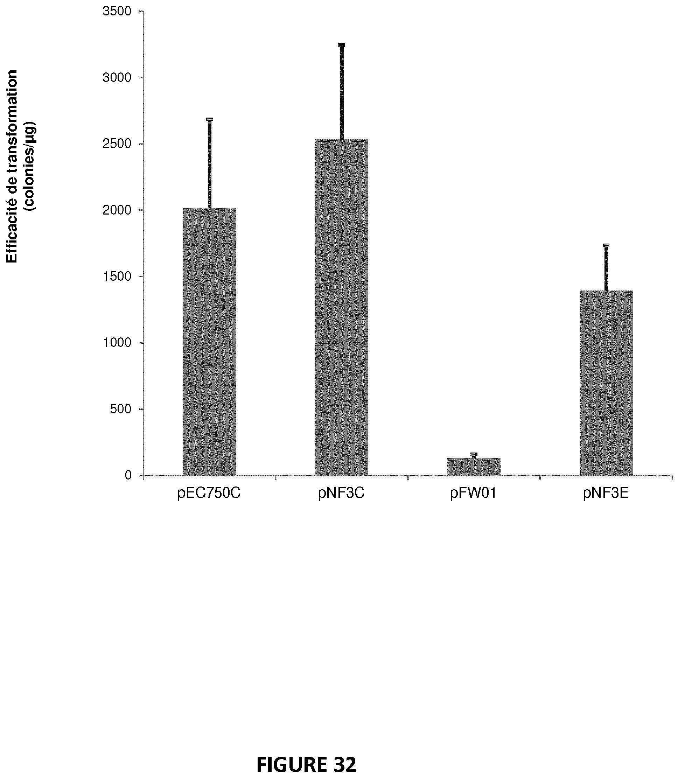

**Figure 32** **:** Efficacité de transformation (en colonies observées par µg d'ADN transformé) des plasmides pEC750C, pNF3C, pFW01 et pNF3E dans la souche *C. beijerinckii* DSM 6423Δ*catB* ΔpNF2. Les barres d'erreur correspondent à l'écart standard de la moyenne pour un triplicat biologique.

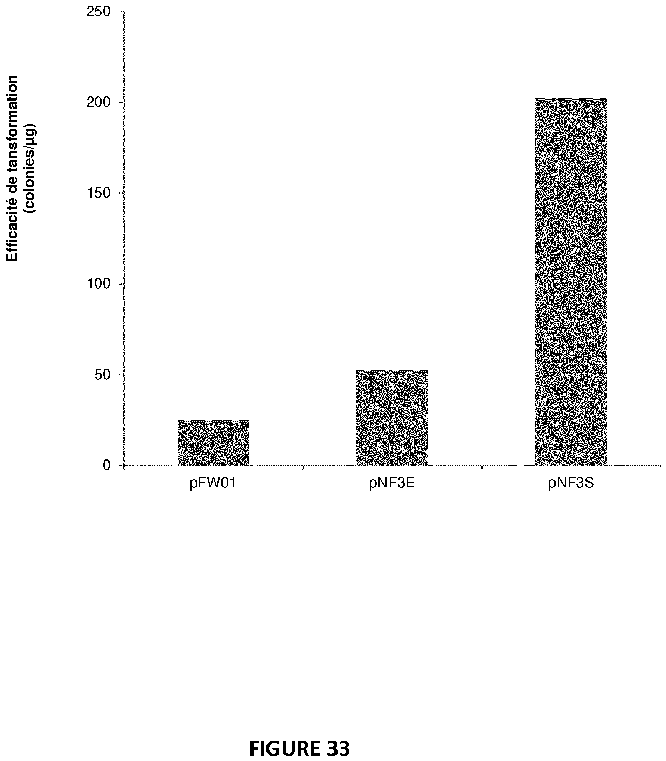

**Figure 33** **:** Efficacité de transformation (en colonies observées par µg d'ADN transformé) des plasmides pFW01, pNF3E et pNF3S dans la souche *C. beijerinckii* NCIMB 8052.

### EXEMPLES

### EXEMPLE n°1

### Matériels et méthodes

### Conditions de culture

*C. acetobutylicum* DSM 792 a été cultivée en milieu 2YTG (Tryptone 16 g.l⁻¹, extrait de levure 10 g.l⁻¹, glucose 5 g.l⁻¹, NaCl 4 g.l⁻¹). *E. coli* NEB10B a été cultivée en milieu LB (Tryptone 10 g.l⁻¹, extrait de levure 5 g.l⁻¹, NaCl 5 g.l⁻¹). Les milieux solides ont été réalisés en ajoutant 15 g.l⁻¹ d'agarose aux milieux liquides. De l'érythromycine (à des concentrations de 40 ou 500 mg.l⁻¹ respectivement en milieu 2YTG ou LB), du chloramphénicol (25 ou 12,5 mg.l⁻¹ respectivement en LB solide ou liquide) et du thiamphénicol (15 mg.l⁻¹ en milieu 2YTG) ont été utilisés lorsque nécessaire.

### Manipulation des acides nucléiques

Toutes les enzymes et kits utilisés l'ont été en suivant les recommandations des fournisseurs.

### Construction des plasmides

Le plasmide pCas9_{acr} (SEQ ID NO : 23), présenté en Figure 4, a été construit en clonant le fragment (SEQ ID NO : 1) contenant *bgaR* et *acrIIA4* sous le contrôle du promoteur Pbgal synthétisé par Eurofins Genomics au niveau du site SacI du vecteur pCas9_{ind} (Wasels *et al*, 2017).

Le plasmide pGRNA_{ind} (SEQ ID NO : 82) a été construit en clonant une cassette d'expression (SEQ ID NO : 83) d'un ARNg sous contrôle du promoteur Pcm-2tetO1 (Dong *et al*, 2012) synthétisée par Eurofins Genomics au niveau du site SacI du vecteur pEC750C (SEQ ID NO : 106) (Wasels *et al*, 2017).

Les plasmides pGRNA-xylB (SEQ ID NO : 102), pGRNA-xylR (SEQ ID NO : 103), pGRNA-glcG (SEQ ID NO : 104) et pGRNA-bdhB (SEQ ID NO : 105) ont été construits en clonant les couples d'amorces respectifs 5'-TCATGATTTCTCCATATTAGCTAG-3' et 5'-AAACCTAGCTAATATGGAGAAATC-3', 5'-TCATGTTACACTTGGAACAGGCGT-3' et 5'-AAACACGCCTGTTCCAAGTGTAAC-3', 5'-TCATTTCCGGCAGTAGGATCCCCA-3' et 5'-AAACTGGGGATCCTACTGCCGGAA-3', 5'-TCATGCTTATTACGACATAACACA-3' et 5'-AAACTGTGTTATGTCGTAATAAGC-3' au sein du plasmide pGRNA_{ind} (SEQ ID NO : 82) digéré par BsaI.

Le plasmide pGRNA-Δ*bdhB* (SEQ ID NO : 79) a été construit en clonant le fragment d'ADN obtenu par assemblage par PCR chevauchante des produits PCR obtenus avec les amorces 5'-ATGCATGGATCCAAACGAACCCAAAAAGAAAGTTTC-3' et 5'-GGTTGATTTCAAATCTGTGTAAACCTACCG-3' d'une part, 5'-ACACAGATTTGAAATCAACCACTTTAACCC-3' et 5'-ATGCATGTCGACTCTTAAGAACATGTATAAAGTATGG-3' d'autre part, dans le vecteur pGRNA-bdhB digéré par BamHI et SacI.

Le plasmide pGRNA-Δ*bdhA*Δ*bdhB* (SEQ ID NO : 80) a été construit en clonant le fragment d'ADN obtenu par assemblage par PCR chevauchante des produits PCR obtenus avec les amorces 5'-ATGCATGGATCCAAACGAACCCAAAAAGAAAGTTTC-3' et 5'-GCTAAGTTTTAAATCTGTGTAAACCTACCG-3' d'une part, 5'-ACACAGATTTAAAACTTAGCATACTTCTTACC-3' et 5'-ATGCATGTCGACCTTCTAATCTCCTCTACTATTTTAG -3' d'autre part, dans le vecteur pGRNA-bdhB digéré par BamHI et SacI.

### Transformation

*C. acetobutylicum* DSM 792 a été transformée selon le protocole décrit par Mermelstein *et al*, 1993. La sélection de transformants de *C. acetobutylicum* DSM 792 contenant déjà un plasmide d'expression de Cas9 (pCas9_{ind} ou pCas9_{acr}) transformés avec un plasmide contenant une cassette d'expression d'un ARNg a été réalisée sur milieu 2YTG solide contenant de l'érythromycine (40 mg.l⁻¹), du thiamphénicol (15 mg.l⁻¹) et du lactose (40 nM).

### Induction de l'expression de cas9

L'induction de l'expression de *cas9* a été réalisée via la croissance des transformants obtenus sur un milieu 2YTG solide contenant de l'érythromycine (40 mg.l⁻¹), du thiamphénicol (15 mg.l⁻¹) et l'agent inducteur de l'expression de *cas9* et de l'ARNg, l'aTc (1 mg.l⁻¹).

### Amplification du locus bdh

Le contrôle de l'édition du génome de *C. acetobutylicum* DSM 792 au niveau du locus des gènes *bdhA* et *bdhB* a été réalisé par PCR à l'aide de l'enzyme Q5® High-Fidelity DNA Polymerase (NEB) à l'aide des amorces V1 (5'-ACACATTGAAGGGAGCTTTT-3') et V2 (5'-GGCAACAACATCAGGCCTTT-3').

### Résultats

### Efficacité de transformation

Afin d'évaluer l'impact de l'insertion du gène *acrIIA4* sur la fréquence de transformation du plasmide d'expression de *cas9,* différents plasmides d'expression d'ARNg ont été transformés dans la souche DSM 792 contenant pCas9_{ind} (SEQ ID NO : 22) ou pCas9_{acr} (SEQ ID NO : 23), et les transformants ont été sélectionnés sur un milieu supplémenté en lactose. Les fréquences de transformations obtenues sont présentées en Figure 5.

### Génération de mutants ΔbdhB et ΔbdhAΔbdhB

Le plasmide de ciblage contenant la cassette d'expression de l'ARNg ciblant *bdhB* (pGRNA-bdhB - SEQ ID NO : 105) ainsi que deux plasmides dérivés contenant des matrices de réparation permettant la délétion du gène *bdhB* seul (pGRNA-Δ*bdhB* - SEQ ID NO : 79) ou des gènes *bdhA* et *bdhB* (pGRNA-Δ*bdhA*Δ*bdhB* - SEQ ID NO : 80) ont été transformés dans la souche DSM 792 contenant pCas9_{ind} (SEQ ID NO : 22) ou pCas9_{acr} (SEQ ID NO : 23). Les fréquences de transformation obtenues sont présentées dans le Tableau 2 :

**Tableau 2 : Fréquences de transformation de la souche DSM 792 contenant pCas9_{ind} ou pCas9_{acr} avec des plasmides ciblant bdhB. Les fréquences sont exprimées en nombre de transformants obtenus par µg d'ADN utilisé lors de la transformation, et représentent les moyennes d'au moins deux expériences indépendantes.**

| | DSM 792 | |
|---|---|---|
| | pCas9_{ind} | pCas9_{acr} |
| pEC750C | 32,6 ± 27,1 ufc.µg⁻¹ | 24,9 ± 27,8 ufc.µg⁻¹ |
| pGRNA-bdhB | 0 ufc.µg⁻¹ | 17,0 ± 10,7 ufc.µg⁻¹ |
| pGRNA-Δ*bdhB* | 0 ufc.µg⁻¹ | 13,3 ± 4,8 ufc.µg⁻¹ |
| pGRNA-Δ*bdhA*Δ*bdhB* | 0 ufc.µg⁻¹ | 33,1 ± 13,4 ufc.µg⁻¹ |

Les transformants obtenus ont subi une phase d'induction de l'expression du système CRISPR/Cas9 via un passage sur milieu supplémenté en anhydrotétracycline, aTc (Figure 6).

Les modifications désirées ont été confirmées par PCR sur l'ADN génomique de deux colonies résistantes à l'aTc (Figure 7).

### Conclusions

L'outil génétique basé sur CRISPR/Cas9 décrit dans Wasels *et al.* (2017) utilise deux plasmides :
- le premier plasmide, pCas9_{ind}, contient *cas9* sous contrôle d'un promoteur inductible à l'aTc, et
- le deuxième plasmide, dérivé de pEC750C, contient la cassette d'expression d'un ARNg (placé sous le contrôle d'un second promoteur inductible à l'aTc) ainsi qu'une matrice d'édition permettant de réparer la cassure double brin induite par le système.

Cependant, les inventeurs ont observé que certains ARNg semblaient encore être trop toxiques, malgré le contrôle de leur expression ainsi que de celle de Cas9 à l'aide de promoteurs inductibles à l'aTc, limitant en conséquence l'efficacité de transformation des bactéries par l'outil génétique et donc la modification du chromosome.

Afin d'améliorer cet outil génétique, le plasmide d'expression de *cas9* a été modifié, via l'insertion d'un gène anti-CRISPR, *acrIIA4*, sous contrôle d'un promoteur inductible au lactose. Les efficacités de transformation de différents plasmides d'expression d'ARNg ont ainsi pu être améliorées de manière très significative, permettant l'obtention de transformants pour tous les plasmides testés.

Il a également été possible de réaliser l'édition du locus *bdhB* au sein du génome de *C. acetobutylicum* DSM 792, à l'aide de plasmides qui n'avaient pas pu être introduits dans la souche DSM 792 contenant pCas9_{ind}. Les fréquences de modification observées sont les mêmes que celles observées précédemment (Wasels *et al*, 2017), avec 100% des colonies testées modifiées.

En conclusion, la modification du plasmide d'expression de *cas9* permet un meilleur contrôle du complexe ribonucléoprotéique Cas9-ARNg, facilitant avantageusement l'obtention de transformants dans lesquels l'action de Cas9 peut être déclenchée afin d'obtenir des mutants d'intérêt.

### EXEMPLE n°2

### Matériels et méthodes

### Conditions de culture

*C. beijerinckii* DSM 6423 a été cultivée en milieu 2YTG (Tryptone 16 g L⁻¹, extrait de levure 10 g L⁻¹, glucose 5 g L⁻¹, NaCl 4 g L⁻¹). *E. coli* NEB 10-beta et INV110 ont été cultivées en milieu LB (Tryptone 10 g L⁻¹, extrait de levure 5 g L⁻¹, NaCl 5 g L⁻¹). Les milieux solides ont été réalisés en ajoutant 15 g L⁻¹ d'agarose aux milieux liquides. De l'érythromycine (à des concentrations de 20 ou 500 mg L⁻¹ respectivement en milieu 2YTG ou LB), du chloramphénicol (25 ou 12,5 mg L⁻¹ respectivement en LB solide ou liquide), du thiamphénicol (15 mg L⁻¹ en milieu 2YTG) ou de la spectinomycine (à des concentrations de 100 ou 650 mg L⁻¹ respectivement en milieu LB ou 2YTG) ont été utilisés si nécessaire.

### Acides nucléiques et vecteurs plasmidiques

Toutes les enzymes et kits utilisés l'ont été en suivant les recommandations des fournisseurs.

Les tests de PCR sur colonie ont respecté le protocole suivant :
Une colonie isolée de *C. beijerinckii* DSM 6423 est resuspendue dans 100 µL de Tris 10 mM pH 7,5 EDTA 5 mM. Cette solution est chauffée à 98 °C pendant 10 min sans agitation. 0,5 µL de ce lysat bactérien peuvent alors être utilisés comme matrice de PCR dans des réactions de 10 µL avec la Phire (Thermo Scientific), la Phusion (Thermo Scientific), la Q5 (NEB) ou la KAPA2G Robust (Sigma-Aldrich) polymerase.

La liste des amorces ayant servies à l'ensemble des constructions (nom/séquence d'ADN) est détaillée ci-dessous :

| | |
|---|---|
| ΔcatB_fwd : | TGTTATGGATTATAAGCGGCTCGAGGACGTCAAACCATGTTAATCATTGC |
| ΔcatB_rev : | AATCTATCACTGATAGGGACTCGAGCAATTTCACCAAAGAATTCGCTAGC |
| ΔcatB_gRNA_rev | AATCTATCACTGATAGGGACTCGAGGGGCAAAAGTGTAAAGACAAGCTTC |
| RH076 : | CATATAATAAAAGGAAACCTCTTGATCG |
| RH077 : | ATTGCCAGCCTAACACTTGG |
| RH001 : | ATCTCCATGGACGCGTGACGTCGACATAAGGTACCAGGAATTAGAGCAGC |
| RH002 : | TCTATCTCCAGCTCTAGACCATTATTATTCCTCCAAGTTTGCT |
| RH003 : | ATAATGGTCTAGAGCTGGAGATAGATTATTTGGTACTAAG |
| RH004 : | TATGACCATGATTACGAATTCGAGCTCGAAGCGCTTATTATTGCATTAGC |
| pEX-fwd : | CAGATTGTACTGAGAGTGCACC |
| pEX-rev : | GTGAGCGGATAACAATTTCACAC |
| pEC750C-fwd : | CAATATTCCACAATATTATATTATAAGCTAGC |
| M13-rev : | CAGGAAACAGCTATGAC |
| RH010 : | CGGATATTGCATTACCAGTAGC |
| RH011 : | TTATCAATCTCTTACACATGGAGC |
| RH025 : | TAGTATGCCGCCATTATTACGACA |
| RH134 : | GTCGACGTGGAATTGTGAGC |
| pNF2_fwd : | GGGCGCACTTATACACCACC |
| pNF2_rev : | TGCTACGCACCCCCTAAAGG |
| RH021 | ACTTGGGTCGACCACGATAAAACAAGGTTTTAAGG |
| RH022 | TACCAGGGATCCGTATTAATGTAACTATGATATCAATTCTTG |
| aad9-fwd2 | ATGCATGGTCCCAATGAATAGGTTTACACTTACTTTAGTTTTATGG |
| aad9-rev | ATGCGAGTTAACAACTTCTAAAATCTGATTACCAATTAG |
| RH031 | ATGCATGGATCCCAATGAATAGGTTTACACTTACTTTAGTTTTATGG |
| RH032 | ATGCGAGAGCTCAACTTCTAAAATCTGATTACCAATTAG |
| RH138 | ATGCATGGATCCGTCTGACAGTTACCAGGTCC |
| RH139 | ATGCGAGAGCTCCAATTGTTCAAAAAAATAATGGCGGAG |
| RH140 | ATGCATGGATCCCGGCAGTTTTTCTTTTTCGG |
| RH141 | ATGCGAGAGCTCGGTTAAATACTAGTTTTTAGTTACAGAC |

Les vecteurs plasmidiques suivants ont été préparés :
- Plasmide N°1 : pEX-A258-Δ*catB* (SEQ ID NO : 17)
   Il contient le fragment d'ADN synthétisé Δ*catB* cloné dans le plasmide pEX-A258. Ce fragment Δ*catB* comprend i) une cassette d'expression d'un ARN guide ciblant le gène *catB* (gène de résistance au chloramphénicol codant une chloramphénicol-O-acetyltransférase - SEQ ID NO : 18) de C. *beijerinckii* DSM6423 sous le contrôle d'un promoteur inductible à l'anhydrotetracycline (cassette d'expression : SEQ ID NO : 19), et ii) une matrice d'édition (SEQ ID NO : 20) comprenant 400 pb homologues situées en amont et en aval du gène *catB.*
- Plasmide N°2 : pCas9ind-Δc*atB* (cf. Figure 9 et SEQ ID NO : 21)
   Il contient le fragment Δ*catB* amplifié par PCR (amorces Δ*catB_*fwd et Δ*catB_*rev) et cloné dans le pCas9ind (décrit dans la demande de brevet WO2017/064439 - SEQ ID NO : 22) après digestion des différents ADN par l'enzyme de restriction XhoI.
- Plasmide N°3 : pCas9acr (cf. Figure 10 et SEQ ID NO : 23)
- Plasmide N°4 : pEC750S-*upp*HR (cf. Figure 11 et SEQ ID NO : 24)
   Il contient une matrice de réparation (SEQ ID NO : 25) utilisée pour la délétion du gène *upp* et constituée par deux fragments d'ADN homologues en amont et en aval du gène *upp* (tailles respectives : 500 (SEQ ID NO : 26) et 377 (SEQ ID NO : 27) paires de bases). L'assemblage a été obtenu à l'aide du système de clonage Gibson (New England Biolabs, Gibson assembly Master Mix 2X). Pour ce faire, les parties amont et aval ont été amplifiée par PCR à partir de l'ADN génomique de la souche DSM 6423 (cf. Maté de Gerando *et al*., 2018 et numéro d'accession PRJEB11626 (https://www.ebi.ac.uk/ena/data/view/PRJEB11626)) à l'aide des amorces respectives RH001 / RH002 et RH003 / RH004. Ces deux fragments ont ensuite été assemblés dans le pEC750S linéarisé au préalable par restriction enzymatique (enzymes de restriction SalI et SacI).
- Plasmide N°5 : pEX-A2-gRNA-*upp* (cf. Figure 12 et SEQ ID NO : 28)
   Ce plasmide comprend le fragment d'ADN gRNA-upp correspondant à une cassette d'expression (SEQ ID NO : 29) d'un ARN guide ciblant le gène *upp* (protospacer ciblant upp (SEQ ID NO :31)) sous le contrôle d'un promoteur constitutif (ARN non codant de séquence SEQ ID NO : 30), inséré dans un plasmide de réplication nommé pEX-A2.
- Plasmide N°6 : pEC750S-Δ*upp* (cf. Figure 13 et SEQ ID NO : 32)
   Il possède comme base le plasmide pEC750S-*upp*HR (SEQ ID NO : 24) et contient en plus le fragment d'ADN comportant une cassette d'expression d'un ARN guide ciblant le gène *upp* sous le contrôle d'un promoteur constitutif.
   Ce fragment a été inséré dans un pEX-A2, appelé pEX-A2-gRNA-upp. L'insert a ensuite été amplifié par PCR avec les amorces pEX-fwd et pEX-rev, puis digéré avec les enzymes de restriction XhoI et NcoI. Enfin, ce fragment a été cloné par ligation dans le pEC750S-*upp*HR préalablement digéré par les mêmes enzymes de restriction pour obtenir le pEC750S-Δ*upp*.
- Plasmide N°7 : pEC750C-Δupp (cf. Figure 14 et SEQ ID NO : 33)
   La cassette comportant l'ARN guide ainsi que la matrice de réparation ont ensuite été amplifiés avec les amorces pEC750C-fwd et M13-rev. L'amplicon a été digéré par restriction enzymatique avec les enzymes XhoI et SacI, puis cloné par ligation enzymatique dans le pEC750C pour obtenir le pEC750C-Δ*upp*.
- Plasmide N°8 : pGRNA-pNF2 (cf. Figure 15 et SEQ ID NO : 34)
   Ce plasmide a comme base pEC750C et contient une cassette d'expression d'un ARN guide ciblant le plasmide pNF2 (SEQ ID NO : 118).
- Plasmide N°9 : pCas9ind-gRNA_*catB* (cf. Figure 23 et SEQ ID NO : 38).
   Il contient la séquence codant pour l'ARN guide ciblant le locus *catB* amplifiée par PCR (amorces ΔcatB_fwd et ΔcatB_gRNA_rev) et clonée dans le pCas9ind (décrit dans la demande de brevet WO2017/064439) après digestion des différents ADN par l'enzyme de restriction XhoI et ligation.
- Plasmide N°10 : pNF3 (cf. Figure 25 et SEQ ID NO : 119)
   Il contient une partie du pNF2, comprenant notamment l'origine de réplication et un gène codant pour une protéine de réplication de plasmide (CIBE_p20001), amplifiée avec les amorces RH021 et RH022. Ce produit de PCR a ensuite été cloné au niveau des sites de restriction SalI et BamHI dans le plasmide pUC19 (SEQ ID NO : 117).
- Plasmide N°11 : pEC751S (cf. Figure 26 et SEQ ID NO : 121)
   Il contient tous les éléments du pEC750C (SEQ ID NO : 106), sauf le gène de résistance au chloramphénicol *catP* (SEQ ID NO : 70). Ce dernier a été remplacé par le gène *aad9* d'*Enterococcus faecalis* (SEQ ID NO : 130), qui confère une résistance à la spectinomycine. Cet élément a été amplifié avec les amorces aad9-fwd2 et aad9-rev à partir du plasmide pMTL007S-E1 (SEQ ID NO : 120) et cloné dans les sites AvaII et HpaI du pEC750C, à la place du gène *catP* (SEQ ID NO : 70).
- Plasmide N°12 : pNF3S (cf. Figure 27 et SEQ ID NO : 123)
   Il contient tous les éléments du pNF3, avec une insertion du gène *aad9* (amplifié avec les amorces RH031 et RH032 à partir du pEC751S) entre les sites BamHI et SacI.
- Plasmide N°13 : pNF3E (cf. Figure 28 et SEQ ID NO : 124)
   Il contient tous les éléments du pNF3, avec une insertion du gène *ermB* de *Clostridium difficile* (SEQ ID NO : 131) sous le contrôle du promoteur miniPthl. Cet élément a été amplifié à partir du pFW01 avec les amorces RH138 et RH139 et cloné entre les sites BamHI et SacI du pNF3E.
- Plasmide N°14 : pNF3C (cf. Figure 29 et SEQ ID NO : 125)
   Il contient tous les éléments du pNF3, avec une insertion du gène *catP* de *Clostridium perfringens* (SEQ ID NO : 70). Cet élément a été amplifié à partir du pEC750C avec les amorces RH140 et RH141 et cloné entre les sites BamHI et SacI du pNF3E.

### Résultats n°1

### Transformation de la souche C. beijerinckii DSM 6423

Les plasmides ont été introduits et répliqués dans une souche de *E. coli dam⁻ dcm⁻* (INV110, Invitrogen). Ceci permet d'éliminer les méthylations de type Dam et Dcm sur le plasmide pCas9ind-Δ*catB* avant de l'introduire par transformation dans la souche DSM 6423 selon le protocole décrit par Mermelstein *et al.* (1993), avec les modifications suivantes : la souche est transformée avec une quantité plus importante de plasmide (20 µg), à une DO₆₀₀ de 0,8, et à l'aide des paramètres d'électroporation suivants : 100 Ω, 25 µF, 1400 V. L'étalement sur boite de Pétri contenant de l'érythromycine (20 µg/mL) a ainsi permis d'obtenir des transformants de *C. beijerinckii* DSM 6423 contenant le plasmide pCas9ind-Δ*catB*.

### Induction de l'expression de cas9 et obtention de la souche C. beijerinckii DSM 6423 ΔcatB

Plusieurs colonies résistantes à l'érythromycine ont ensuite été reprises dans 100 µL de milieu de culture (2YTG) puis diluées en série jusqu'à un facteur de dilution de 10⁴ dans du milieu de culture. Pour chaque colonie, huit µL de chaque dilution ont été déposés sur une boîte de Pétri contenant de l'érythromycine et de l'anhydrotétracycline (200 ng/mL) permettant d'induire l'expression du gène codant la nucléase Cas9.

Après extraction d'ADN génomique, la délétion du gène *catB* au sein des clones ayant poussés sur cette boite a été vérifiée par PCR, à l'aide des amorces RH076 et RH077 (cf. Figure 16).

### Vérification de la sensibilité de la souche C. beijerinckii DSM 6423 ΔcatB au thiamphénicol

Pour s'assurer que la délétion du gène *catB* confère bien une sensibilité nouvelle au thiamphénicol, des analyses comparatives sur milieu gélosé ont été réalisées. Des précultures de *C. beijerinckii* DSM 6423 et *C. beijerinckii* DSM 6423 Δ*catB* ont été réalisées sur milieu 2YTG puis 100 µL de ces précultures ont été étalés sur des milieux gélosés 2YTG supplémentée ou non en thiamphénicol à une concentration de 15 mg/L. La figure 17 permet d'observer que seule la souche initiale *C. beijerinckii* DSM 6423 est capable de pousser sur un milieu supplémenté en thiamphénicol.

### Délétion du gène upp par l'outil CRISPR-Cas9 dans la souche C. beijerinckii DSM 6423 ΔcatB

Un clone de la souche *C. beijerinckii* DSM 6423 Δ*catB* a été au préalable transformé avec le vecteur pCas9_{acr} ne présentant pas de méthylation aux niveaux des motifs reconnus par les méthyltransférases de type dam et dcm (préparé à partir d'une bactérie *Escherichia coli* présentant le génotype *dam⁻ dcm⁻* ). La vérification de la présence du plasmide pCas9_{acr} maintenu dans la souche *C. beijerinckii* DSM 6423 a été vérifiée par PCR sur colonie avec les amorces RH025 et RH134.

Un clone résistant à l'érythromycine a ensuite été transformé avec pEC750C-Δ*upp* préalablement déméthylé. Les colonies ainsi obtenues ont été sélectionnées sur du milieu contenant de l'érythromycine (20 µg/mL), du thiamphénicol (15 µg/mL) et du lactose (40 mM).

Plusieurs de ces clones ont ensuite été resuspendus dans 100 µL de milieu de culture (2YTG) puis dilués en série dans du milieu de culture (jusqu'à un facteur de dilution de 10⁴). Cinq µL de chaque dilution ont été déposés sur une boîte de Pétri contenant de l'érythromycine, du thiamphénicol et de l'anhydrotétracycline (200 ng/mL) (cf. Figure 18).

Pour chaque clone, deux colonies résistantes à l'aTc ont été testées par colonie PCR avec des amorces destinées à amplifier le locus *upp* (cf. Figure 19).

### Délétion du plasmide naturel pNF2 par l'outil CRISPR-Cas9 dans la souche C. beijerinckii DSM 6423 ΔcatB

Un clone de la souche *C. beijerinckii* DSM 6423 Δ*catB* a été au préalable transformé avec le vecteur pCas9_{ind} ne présentant pas de méthylation aux niveaux des motifs reconnus par les méthyltransférases de type Dam et Dcm (préparé à partir d'une bactérie *Escherichia coli* présentant le génotype *dam⁻ dcm*). La présence du plasmide pCas9_{ind} au sein de la souche *C. beijerinckii* DSM6423 a été vérifiée par PCR avec les amorces pCas9_{ind}_fwd (SEQ ID NO : 42) et pCas9_{ind}_rev (SEQ ID NO : 43) (cf. Figure 20).

Un clone résistant à l'érythromycine a ensuite été utilisé pour transformer le pGRNA-pNF2, préparé à partir d'une bactérie *Escherichia coli* présentant le génotype *dam⁻ dcm⁻.*

Plusieurs colonies obtenues sur du milieu contenant de l'érythromycine (20 µg/mL) et du thiamphénicol (15 µg/mL) ont été resuspendues dans du milieu de culture et diluées en série jusqu'à un facteur de dilution de 10⁴. Huit µL de chaque dilution ont été déposés sur une boîte de Pétri contenant de l'érythromycine, du thiamphénicol et de l'anhydrotétracycline (200 ng/mL) afin d'induire l'expression du système CRISPR/Cas9.

L'absence du plasmide naturel pNF2 a été vérifiée par PCR avec les amorces pNF2_fwd (SEQ ID NO: 39) et pNF2_rev (SEQ ID NO : 40) (cf. Figure 21).

### Conclusions

Au cours de ce travail, les inventeurs sont parvenus à introduire et maintenir différents plasmides au sein de la souche *Clostridium beijerinckii* DSM 6423. Ils sont parvenus à supprimer le gène *catB* à l'aide d'un outil de type CRISPR-Cas9 basé sur l'utilisation d'un seul plasmide. La sensibilité au thiamphénicol des souches recombinantes obtenues a été confirmée par des tests en milieu gélosé. Cette délétion leur a permis d'utiliser plus efficacement l'outil CRISPR-Cas9 nécessitant deux plasmides décrit dans la demande de brevet FR1854835. Deux exemples permettant de démontrer l'intérêt de cette application ont été réalisés : la délétion du gène *upp* et l'élimination d'un plasmide naturel non essentiel pour la souche *Clostridium beijerinckii* DSM 6423.

### Résultats n°2

### Transformation des souches de C. beijerinckii

Les plasmides préparés dans la souche d'*E. coli* NEB 10-beta sont également utilisés pour transformer la souche *C. beijerinckii* NCIMB 8052. En revanche, pour *C. beijerinckii* DSM 6423, les plasmides sont préalablement introduits et répliqués dans une souche de *E. coli dam⁻ dcm⁻* (INV110, Invitrogen). Ceci permet d'éliminer les méthylations de type Dam et Dcm sur les plasmides d'intérêt avant de les introduire par transformation dans la souche DSM 6423.

La transformation est autrement réalisée similairement pour chaque souche, c'est-à-dire selon le protocole décrit par Mermelstein *et al.* 1992, avec les modifications suivantes : la souche est transformée avec une quantité plus importante de plasmide (5-20 µg), à une DO₆₀₀ de 0,6-0,8, et les paramètres d'électroporation sont 100 Ω, 25 µF, 1400 V. Après 3h de régénération dans du 2YTG, les bactéries sont étalées sur boîte de Pétri (2YTG agar) contenant l'antibiotique souhaité (erythromycine : 20-40 µg/mL ; thiamphénicol : 15 µg/mL ; spectinomycine : 650 µg/mL).

### Comparaison des efficacités de transformation des souches de C. beijerinckii DSM 6423

Des transformations ont été réalisées en duplicat biologique dans les souches de *C. beijerinckii* suivantes : DSM 6423 sauvage, DSM 6423 Δ*catB* et DSM 6423 Δ*catB* ΔpNF2 (Figure 30). Pour cela, le vecteur pCas9_{ind}, notablement difficile à utiliser pour modifier une bactérie car ne permettant pas de bonnes efficacités de transformation, a été utilisé. Il comporte de plus un gène conférant à la souche une résistance à l'érythromycine, antibiotique pour lequel les trois souches sont sensibles.

Les résultats indiquent une augmentation de l'efficacité de transformation d'un facteur d'environ 15-20 imputable à la perte du plasmide naturel pNF2.

L'efficacité de transformation a également été testée pour le plasmide pEC750C, qui confère une résistance au thiamphénicol, uniquement dans les souches DSM 6423 Δ*catB* et DSM 6423 Δ*catB* ΔpNF2, puisque la souche sauvage est résistante à cet antibiotique (Figure 31). Pour ce plasmide, le gain en efficacité de transformation est encore plus flagrant (amélioration d'un facteur d'environ 2000).

### Comparaison des efficacités de transformation des plasmides pNF3 avec d'autres plasmides

Afin de déterminer l'efficacité de transformation de plasmides contenant l'origine de réplication du plasmide naturel pNF2, les plasmides pNF3E et pNF3C ont été introduits dans la souche *C. beijerinckii* DSM 6423 Δ*catB* ΔpNF2. L'utilisation de vecteurs contenant des gènes de résistance à l'érythromycine ou au chloamphénicol permet de comparer l'efficacité de transformation du vecteur en fonction de la nature du gène de résistance. Les plasmides pFW01 et pEC750C ont également été transformés. Ces deux plasmides contiennent des gènes de résistance à des antibiotiques différents (respectivement l'érythromycine et le thiamphénicol) et sont couramment utilisés pour transformer *C. beijerinckii* et *C. acetobutylicum.*

Comme montré dans la Figure 32, les vecteurs basés sur le pNF3 présentent une excellente efficacité de transformation, et sont notamment utilisables chez *C. beijerinckii* DSM 6423 Δ*catB* ΔpNF2. En particulier, le pNF3E (qui contient un gène de résistance à l'érythromycine) montre une efficacité de transformation nettement supérieure à celle de pFW01, qui comprend le même gène de résistance. Ce même plasmide n'a pas pu être introduit dans la souche *C. beijerinckii* DSM 6423 sauvage (0 colonies obtenues avec 5 µg de plasmides transformés en duplicat biologique), ce qui démontre l'impact de la présence du plasmide naturel pNF2.

### Vérification de la transformabilité des plasmides pNF3 dans d'autres souches/espèces

Pour illustrer la possibilité d'utiliser ce nouveau plasmide dans d'autres souches solvantogènes de *Clostridium,* les inventeurs ont réalisé une analyse comparative des efficacités de transformation des plasmides pFW01, pNF3E et pNF3S dans la souche ABE *C. beijerinckii* NCIMB 8052 (Figure 33). La souche NCIMB 8052 étant naturellement résistante au thiamphénicol, le pNF3S, conférant une résistance à la spectinomycine, a été utilisé à la place du pNF3C.

Les résultats démontrent que la souche NCIMB 8052 est transformable avec les plasmides basés sur le pNF3, ce qui prouve que ces vecteurs sont applicables à l'espèce *C. beijerinckii* au sens large.

L'applicabilité de la suite de vecteurs de synthèse basés sur le pNF3 a également été testée dans la souche référence DSM 792 de *C. acetobutylicum.* Un essai de transformation a ainsi montré la possibilité de transformer cette souche par le plasmide pNF3C (Efficacité de transformation de 3 colonies observées par µg d'ADN transformé contre 120 colonies/µg pour le plasmide pEC750C).

### Vérification de la compatibilité des plasmides pNF3 avec l'outil génétique décrit dans la demande FR18/73492

La demande de brevet FR18/73492 décrit la souche Δ*catB* ainsi que l'utilisation d'un système CRISPR/Cas9 à deux plasmides nécessitant l'utilisation d'un gène de résistance à l'érythromycine et d'un gène de résistance au thiamphénicol. Pour démontrer l'intérêt de la nouvelle suite de plasmides pNF3, le vecteur pNF3C a été transformé dans la souche Δ*catB* contenant déjà le plasmide pCas9_{acr}. La transformation, réalisée en duplicat, a montré une efficacité de transformation de 0,625 ± 0,125 colonies/µg d'ADN (moyenne ± erreur standard), ce qui prouve qu'un vecteur basé sur le pNF3C peut être utilisé en combinaison avec le pCas9_{acr} dans la souche Δ*catB*.

Parallèlement à ces résultats, une partie du plasmide pNF2 comprenant son origine de réplication (SEQ ID NO : 118) a pu être réutilisée avec succès pour créer une nouvelle suite de vecteurs navettes (SEQ ID NO : 119, 123, 124 et 125), modifiables à façon, permettant notamment leur réplication dans une souche *d'E. coli* ainsi que leur réintroduction chez *C. beijerinckii* DSM 6423. Ces nouveaux vecteurs présentent des efficacités de transformation avantageuses pour réaliser de l'édition génétique par exemple chez *C. beijerinckii* DSM 6423 et ses dérivées, en particulier à l'aide de l'outil CRISPR/Cas9 comprenant deux acides nucléiques différents.

Ces nouveaux vecteurs ont également pu être testés avec succès dans une autre souche de *C. beijerinckii* (NCIMB 8052), et d'espèce de *Clostridium* (en particulier *C. acetobutylicum*), démontrant leur applicabilité dans d'autres organismes du phylum des Firmicutes. Un test est également réalisé sur *Bacillus.*

### Conclusions

Ces résultats démontrent que la suppression du plasmide naturel pNF2 augmente significativement les fréquences de transformation de la bactérie qui le contenait (d'un facteur d'environ 15 pour le pFW01 et d'un facteur d'environ 2000 pour le pEC750C). Ce résultat est particulièrement intéressant dans le cas des bactéries du genre *Clostridium,* connues pour être difficiles à transformer, et en particulier pour la souche *C. beijerinckii* DSM 6423 qui souffre naturellement d'une efficacité de transformation faible (inférieure à 5 colonies/µg de plasmide).

### REFERENCES

- Banerjee, A., Leang, C., Ueki, T., Nevin, K. P., & Lovley, D. R. (2014). Lactose-inducible system for metabolic engineering of Clostridium ljungdahlii. Applied and environmental microbiology, 80(8), 2410-2416.
- Chen J.-S., Hiu S.F. (1986) Acetone-butanol-isopropanol production by Clostridium beijerinckii (synonym,Clostridium butylicum). Biotechnol. Lett. 8:371-376.
- Cui, L., & Bikard, D. (2016). Consequences of Cas9 cleavage in the chromosome of Escherichia coli. Nucleic acids research, 44(9), 4243-4251.
- Currie, D. H., Herring, C. D., Guss, A. M., Olson, D. G., Hogsett, D. A., & Lynd, L. R. (2013). Functional heterologous expression of an engineered full length CipA from Clostridium thermocellum in Thermoanaerobacterium saccharolyticum. Biotechnology for biofuels, 6(1), 32.
- DiCarlo, J. E., Norville, J. E., Mali, P., Rios, X., Aach, J., & Church, G. M. (2013). Genome engineering in Saccharomyces cerevisiae using CRISPR-Cas systems. Nucleic acids research, 41(7), 4336-4343.
- Dong, H., Tao, W., Zhang, Y., & Li, Y. (2012). Development of an anhydrotetracycline-inducible gene expression system for solvent-producing Clostridium acetobutylicum: A useful tool for strain engineering. Metabolic engineering, 14(1), 59-67.
- Dong, D., Guo, M., Wang, S., Zhu, Y., Wang, S., Xiong, Z., ... & Huang, Z. (2017). Structural basis of CRISPR-SpyCas9 inhibition by an anti-CRISPR protein. Nature, 546(7658), 436.
- Dupuy, B., Mani, N., Katayama, S., & Sonenshein, A. L. (2005). Transcription activation of a UV-inducible Clostridium perfringens bacteriocin gene by a novel σ factor. Molecular microbiology, 55(4), 1196-1206.
- Egholm, M., Buchardt, O., Nielsen, P. E., & Berg, R. H. (1992). Peptide nucleic acids (PNA). Oligonucleotide analogs with an achiral peptide backbone. Journal of the American Chemical Society, 114(5), 1895-1897.
- Fonfara, I., Le Rhun, A., Chylinski, K., Makarova, K. S., Lecrivain, A. L., Bzdrenga, J., ... & Charpentier, E. (2013). Phylogeny of Cas9 determines functional exchangeability of dual-RNA and Cas9 among orthologous type II CRISPR-Cas systems. Nucleic acids research, 42(4), 2577-2590.
- Garcia-Doval C, Jinek M. Molecular architectures and mechanisms of Class 2 CRISPR-associated nucleases. Curr Opin Struct Biol. 2017 Dec;47:157-166. doi: 10.1016/j.sbi.2017.10.015 Ajouter au projet Citavi par DOI. Epub 2017 Nov 3. Review.
- George H.A., Johnson J.L., Moore W. E. C., Holdeman, L. V., Chen J. S. (1983) Acetone, Isopropanol, and Butanol Production by Clostridium beijerinckii (syn. Clostridium butylicum) and Clostridium aurantibutyricum. Appl. Env. Microbiol. 45:1160-1163.
- Gonzales y Tucker RD, Frazee B. View from the front lines: an emergency medicine perspective on clostridial infections in injection drug users. Anaerobe. 2014 Dec; 30:108-15.
- Hartman, A. H., Liu, H., & Melville, S. B. (2011). Construction and characterization of a lactose-inducible promoter system for controlled gene expression in Clostridium perfringens. Applied and environmental microbiology, 77(2), 471-478.
- Heap, J. T., Ehsaan, M., Cooksley, C. M., Ng, Y. K., Cartman, S. T., Winzer, K., & Minton, N. P. (2012). Integration of DNA into bacterial chromosomes from plasmids without a counter-selection marker. Nucleic acids research, 40(8), e59-e59.
- Heap, J. T., Kuehne, S. A., Ehsaan, M., Cartman, S. T., Cooksley, C. M., Scott, J. C., & Minton, N. P. (2010). The ClosTron: mutagenesis in Clostridium refined and streamlined. Journal of microbiological methods, 80(1), 49-55.
- Heap, J. T., Pennington, O. J., Cartman, S. T., Carter, G. P., & Minton, N. P. (2007). The ClosTron: a universal gene knock-out system for the genus Clostridium. Journal of microbiological methods, 70(3), 452-464.
- Heap, J. T., Pennington, O. J., Cartman, S. T., & Minton, N. P. (2009). A modular system for Clostridium shuttle plasmids. Journal of microbiological methods, 78(1), 79-85.
- Hidalgo-Cantabrana, C., O'Flaherty, S., & Barrangou, R. (2017). CRISPR-based engineering of next-generation lactic acid bacteria. Current opinion in microbiology, 37, 79-87.
- Hiu S.F., Zhu C.-X., Yan R.-T., Chen J.-S. (1987) Butanol-ethanol dehydrogenase and butanol-ethanol-isopropanol dehydrogenase: different alcohol dehydrogenases in two strains of Clostridium beijerinckii (Clostridium butylicum). Appl. Env. Microbiol. 53:697-703.
- Huang, H., Chai, C., Li, N., Rowe, P., Minton, N. P., Yang, S., & Gu, Y. (2016). CRISPR/Cas9-based efficient genome editing in Clostridium ljungdahlii, an autotrophic gas-fermenting bacterium. ACS synthetic biology, 5(12), 1355-1361.
- Huggins, A.S., Bannam, T.L. and Rood, J.I. (1992) Comparative sequence analysis of the catB gene from Clostridium butyricum. Antimicrob. Agents Chemother. 36, 2548-2551.
- Ismaiel A.A., Zhu C.X., Colby G.D., Chen, J. S. (1993). Purification and characterization of a primary-secondary alcohol dehydrogenase from two strains of Clostridium beijerinckii. J. Bacteriol. 175:5097-5105.
- Jinek, M., Chylinski, K., Fonfara, I., Hauer, M., Doudna, J. A., & Charpentier, E. (2012). A programmable dual-RNA-guided DNA endonuclease in adaptive bacterial immunity. Science, 337(6096), 816-821.
- Jones D.T., Woods D.R. (1986) Acetone-butanol fermentation revisited. Microbiological Reviews 50:484-524.
- Kolek J., Sedlar K., Provaznik I., Patakova P. (2016). Dam and Dcm methylations prevent gene transfer into Clostridium pasteurianum NRRL B-598: development of methods for electrotransformation, conjugation, and sonoporation. Biotechnol Biofuels. 9:14.
- Li, Q., Chen, J., Minton, N. P., Zhang, Y., Wen, Z., Liu, J., ... & Gu, Y. (2016). CRISPR-based genome editing and expression control systems in Clostridium acetobutylicum and Clostridium beijerinckii. Biotechnology journal, 11(7), 961-972.
- Makarova, K. S., Haft, D. H., Barrangou, R., Brouns, S. J., Charpentier, E., Horvath, P., ... & Van Der Oost, J. (2011). Evolution and classification of the CRISPR-Cas systems. Nature Reviews Microbiology, 9(6), 467.
- Makarova, K. S., Wolf, Y. I., Alkhnbashi, O. S., Costa, F., Shah, S. A., Saunders, S. J., ... & Horvath, P. (2015). An updated evolutionary classification of CRISPR-Cas systems. Nature Reviews Microbiology, 13(11), 722.
- Marino, N. D., Zhang, J. Y., Borges, A. L., Sousa, A. A., Leon, L. M., Rauch, B. J., ... & Bondy-Denomy, J. (2018). Discovery of widespread type I and type V CRISPR-Cas inhibitors. Science, 362(6411), 240-242.
- Máté de Gérando, H., Wasels, F., Bisson, A., Clément, B., Bidard, F., Jourdier E., Lopez-Contreras A., Lopes Ferreira N. (2018). Genome and transcriptome of the natural isopropanol producer Clostridium beijerinckii DSM 6423. BMC genomics. 19:242.
- Mearls, E. B., Oison, D. G., Herring, C. D., & Lynd, L. R. (2015). Development of a regulatable plasmid-based gene expression system for Clostridium thermocellum. Applied microbiology and biotechnology, 99(18), 7589-7599.
- Mermelstein, L. D., & Papoutsakis, E. T. (1993). In vivo methylation in Escherichia coli by the Bacillus subtilis phage phi 3T I methyltransferase to protect plasmids from restriction upon transformation of Clostridium acetobutylicum ATCC 824. Applied and environmental microbiology, 59(4), 1077-1081.
- Mermelstein L.D., Welker N.E., Bennett G.N., Papoutsakis E.T. (1992). Expression of cloned homologous fermentative genes in Clostridium acetobutylicum ATCC 824 10:190-195.
- Mermelstein L.D., Welker N.E., Bennett G.N., Papoutsakis E.T. (1993). Expression of cloned homologous fermentative genes in Clostridium acetobutylicum ATCC 824 10:190-195.
- Moon HG, Jang YS, Cho C, Lee J, Binkley R, Lee SY. One hundred years of clostridial butanol fermentation. FEMS Microbiol Lett. 2016 Feb;363(3).
- Nagaraju, S., Davies, N. K., Walker, D. J. F., Köpke, M., & Simpson, S. D. (2016). Genome editing of Clostridium autoethanogenum using CRISPR/Cas9. Biotechnology for biofuels, 9(1), 219.
- Nariya, H., Miyata, S., Kuwahara, T., & Okabe, A. (2011). Development and characterization of a xylose-inducible gene expression system for Clostridium perfringens. Applied and environmental microbiology, 77(23), 8439-8441.
- Newcomb, M., Millen, J., Chen, C. Y., & Wu, J. D. (2011). Co-transcription of the celC gene cluster in Clostridium thermocellum. Applied microbiology and biotechnology, 90(2), 625-634.
- Pawluk, A., Davidson, A. R., & Maxwell, K. L. (2018). Anti-CRISPR: Discovery, mechanism and function. Nature Reviews Microbiology, 16(1), 12.
- Poehlein A., Solano J.D.M., Flitsch S.K., Krabben P., Winzer K., Reid S.J., Jones D.T., Green E., Minton N.P., Daniel R., Dürre P. (2017). Microbial solvent formation revisited by comparative genome analysis. Biotechnol Biofuels. 10:58.
- Pyne, M. E., Bruder, M. R., Moo-Young, M., Chung, D. A., & Chou, C. P. (2016). Harnessing heterologous and endogenous CRISPR-Cas machineries for efficient markerless genome editing in Clostridium. Scientific reports, 6*.*
- Rauch, B. J., Silvis, M. R., Hultquist, J. F., Waters, C. S., McGregor, M. J., Krogan, N. J., & Bondy-Denomy, J. (2017). Inhibition of CRISPR-Cas9 with bacteriophage proteins. Cell, 168(1-2), 150-158.
- Rajewska M., Wegrzyn K, Konieczny I., FEMS Microbiol Rev. 2012 Mar; 36(2). AT-rich region and repeated sequences - the essential elements of replication origins of bacterial replicons :408-34.
- Ransom, E. M., Ellermeier, C. D., & Weiss, D. S. (2015). Use of mCherry red fluorescent protein for studies of protein localization and gene expression in Clostridium difficile. Applied and environmental microbiology, 81(5), 1652-1660.
- Rogers P., Chen J.-S., Zidwick M. (2006) in The prokaryotes. 3rd edition, Vol. 1, edited by Dworkin M (Springer, New York, USA,2006). 3rd edition, Vol. 1, pp. 672-755.
- Schwarz S, Kehrenberg C, Doublet B, Cloeckaert A. Molecular basis of bacterial resistance to chloramphénicol and florfenicol. FEMS Microbiol Rev. 2004 Nov;28(5):519-42.
- Stella S, Alcón P, Montoya G. Class 2 CRISPR-Cas RNA-guided endonucleases: Swiss Army knives of genome editing. Nat Struct Mol Biol. 2017 Nov;24(11):882-892. doi: 10.1038/nsmb.3486.
- Wang, S., Dong, S., Wang, P., Tao, Y., & Wang, Y. (2017). Genome Editing in Clostridium saccharoperbutylacetonicum N1-4 with the CRISPR-Cas9 System. Applied and Environmental Microbiology, 83(10), e00233-17.
- Wang Y, Li X, Milne CB, et al. Development of a gene knockout system using mobile group II introns (Targetron) and genetic disruption of acid production pathways in Clostridium beijerinckii. Appl Environ Microbiol. 2013; 79(19): 5853-63.
- Wang, Y., Zhang, Z. T., Seo, S. O., Choi, K., Lu, T., Jin, Y. S., & Blaschek, H. P. (2015). Markerless chromosomal gene deletion in Clostridium beijerinckii using CRISPR/Cas9 system. Journal of biotechnology, 200, 1-5.
- Wang, Y., Zhang, Z. T., Seo, S. O., Lynn, P., Lu, T., Jin, Y. S., & Blaschek, H. P. (2016). Bacterial genome editing with CRISPR-Cas9: deletion, Integration, single nucleotide modification, and desirable "clean" mutant selection in Clostridium beijerinckii as an example. ACS synthetic biology, 5(7), 721-732.
- Wasels, F., Jean-Marie, J., Collas, F., López-Contreras, A. M., & Ferreira, N. L. (2017 Sept). A two-plasmid inducible CRISPR/Cas9 genome editing tool for Clostridium acetobutylicum. Journal of microbiological methods, 140, 5-11.
- Xu, T., Li, Y., Shi, Z., Hemme, C. L., Li, Y., Zhu, Y., ... & Zhou, J. (2015). Efficient genome editing in Clostridium cellulolyticum via CRISPR-Cas9 nickase. Applied and environmental microbiology, 81(13), 4423-4431.
- Yadav, R., Kumar, V., Baweja, M., & Shukla, P. (2018). Gene editing and genetic engineering approaches for advanced probiotics: A Review. Critical reviews in food science and nutrition, 58(10), 1735-1746.
- Yue Chen, Bruce A. McClane, Derek J. Fisher, Julian I. Rood, Phalguni Gupta; Construction of an Alpha Toxin Gene Knockout Mutant of Clostridium perfringens Type A by Use of a Mobile Group II Intron; Appl. Environ. Microbiol. Nov 2005, 71 (11) 7542-7547; DOI: 10.1128/AEM.71.11.7542-7547.2005.
- Zhang, J., Liu, Y. J., Cui, G. Z., & Cui, Q. (2015). A novel arabinose-inducible genetic operation system developed for Clostridium cellulolyticum. Biotechnology for biofuels, 8(1), 36.
- Zhang C., Tinggang L. Jianzhong H. (2018) Characterization and genome analysis of a butanol-isopropanol-producing Clostridium beijerinckii strain BGS1. Biotechnol Biofuels (2018) 11:280.
- Zhong, J., Karberg, M., & Lambowitz, A. M. (2003). Targeted and random bacterial gene disruption using a group II intron (targetron) vector containing a retrotransposition-activated selectable marker. Nucleic acids research, 31(6), 1656-1664.

## Revendications

1. Outil génétique permettant la transformation, et la modification génétique par recombinaison homologue, d'une bactérie du genre *Clostridium* caractérisé i) en ce qu'il comprend :
- un premier acide nucléique codant au moins Cas9, dans lequel la séquence codant Cas9 est placée sous le contrôle d'un promoteur, et
- au moins un deuxième acide nucléique contenant une matrice de réparation permettant, par un mécanisme de recombinaison homologue, le remplacement d'une portion de l'ADN bactérien ciblée par Cas9 par une séquence d'intérêt,
en ce que ii) au moins l'un desdits acides nucléiques code en outre un ou plusieurs ARN guides (ARNg) ou en ce que l'outil génétique comprend en outre un ou plusieurs ARN guides, chaque ARN guide comprenant une structure ARN de fixation à l'enzyme Cas9 et une séquence complémentaire de la portion ciblée de l'ADN bactérien, et
iii) en ce que au moins l'un desdits acides nucléiques comprend en outre une séquence codant une protéine anti-CRISPR placée sous le contrôle d'un promoteur inductible, ou en ce que l'outil génétique comprend en outre un troisième acide nucléique codant une protéine anti-CRISPR placée sous le contrôle d'un promoteur inductible.

2. Outil selon la revendication 1, **caractérisé en ce que** la bactérie du genre *Clostridium* est une bactérie solvantogène sélectionnée parmi *C. acetobutylicum, C. cellulolyticum*, *C. phytofermentans, C. beijerinckii, C. saccharobutylicum*, *C. saccharoperbutylacetonicum, C. sporogenes, C. butyricum, C. aurantibutyricum, C. tyrobutyricum.*

3. Outil selon la revendication 2, **caractérisé en ce que** lorsque la bactérie est *C*. *acetobutylicum* ladite bactérie *C*. *acetobutylicum* est la souche DSM 792 (également désignée ATCC 824 ou LMG 5710), et lorsque la bactérie solvantogène est *C*. *beijerinckii* ladite bactérie *C*. *beijerinckii* est la souche NCIMB 8052 ou la souche DSM 6423 (également désignée NRRL B-593, LMG 7814 ou LMG 7815).

4. Outil selon l'une des revendications 1 à 3, **caractérisé en ce que** la séquence codant une protéine anti-CRISPR est portée par le premier acide nucléique.

5. Outil selon l'une des revendications 1 à 4, **caractérisé en ce que** la protéine anti-CRISPR est la protéine AcrIIA2 ou la protéine AcrIIA4.

6. Outil selon l'une des revendications 1 à 5, **caractérisé en ce que** l'expression de la séquence d'ADN d'intérêt permet à la bactérie du genre *Clostridium* de fermenter au moins deux sucres différents parmi les sucres comprenant 6 atomes de carbone et/ou parmi les sucres comprenant 5 atomes de carbone.

7. Outil selon l'une des revendications 1 à 6, **caractérisé en ce que** la séquence d'intérêt code au moins un produit favorisant la production de solvant par la bactérie du genre *Clostridium,* par exemple au moins une enzyme impliquée dans la conversion des aldéhydes en alcool, une protéine membranaire, un facteur de transcription, ou une combinaison de ceux-ci.

8. Outil selon l'une des revendications 1 à 7, **caractérisé en ce que** chacun des acides nucléiques présents au sein de l'outil appartient à une cassette d'expression distincte ou à un vecteur, par exemple un plasmide, distinct.

9. Procédé pour transformer, et modifier génétiquement par recombinaison homologue, une bactérie du genre *Clostridium,* **caractérisé en ce qu'**il comprend une étape de transformation de la bactérie par introduction dans ladite bactérie d'un outil génétique selon l'une des revendications 1 à 8.

10. Procédé selon la revendication 9, **caractérisé en ce qu'**il comprend les étapes suivantes :
a) l'introduction dans la bactérie d'un outil génétique selon l'une des revendications 1 à 8 en présence d'un agent inducteur de l'expression de la protéine anti-CRISPR, et
b) la culture de la bactérie transformée obtenue à l'issue de l'étape a) sur un milieu ne contenant pas l'agent inducteur de l'expression de la protéine anti-CRISPR.

11. Procédé selon la revendication 9 ou 10, **caractérisé en ce qu'**il comprend une étape c) additionnelle d'élimination de l'acide nucléique contenant la matrice de réparation et/ou du/des ARNs guides ou séquences codant le/les ARNs guides introduits avec l'outil génétique lors de l'étape a).

12. Procédé selon l'une des revendications 9 à 11, **caractérisé en ce qu'**il comprend une ou plusieurs étapes additionnelles, postérieure(s) à l'étape b) ou à l'étape c), d'introduction d'un énième acide nucléique contenant une matrice de réparation distincte de celle(s) déjà introduite(s) et d'une ou plusieurs cassettes d'expression d'ARN guides permettant l'intégration de la séquence d'intérêt contenue dans ladite matrice de réparation distincte dans une zone ciblée du génome de la bactérie, en présence d'un agent inducteur de l'expression de la protéine anti-CRISPR, chaque étape additionnelle étant suivie d'une étape de culture de la bactérie ainsi transformée sur un milieu ne contenant pas l'agent inducteur de l'expression de la protéine anti-CRISPR et permettant l'expression du complexe ribonucléoprotéique Cas9/ARNg.

13. Kit pour transformer et de préférence modifier génétiquement une bactérie du genre *Clostridium* ou pour produire au moins un solvant à l'aide d'une bactérie du genre *Clostridium* comprenant les éléments de l'outil génétique tels que décrits dans l'une des revendications 1 à 8 et au moins un inducteur adapté au promoteur inductible de l'expression de la protéine anti-CRISPR sélectionné utilisé au sein de l'outil.

14. Utilisation de l'outil génétique selon l'une des revendications 1 à 8, du procédé selon l'une des revendications 9 à 12 ou d'un kit selon la revendication 13, pour permettre la production d'un solvant ou d'un mélange de solvants à l'échelle industrielle, de préférence d'acétone, de butanol, d'éthanol, d'isopropanol ou d'un mélange de ceux-ci, typiquement d'un mélange isopropanol/butanol.


SEQUENCE LISTING

<110>  IFP Energies nouvelles

<120>  Outil génétique optimisé pour modifier les bactéries du genre
       Clostridium

<130>  B2767

<160>  134

<170>  PatentIn version 3.5

<210>  1
<211>  50
<212>  DNA
<213>  Artificial Sequence

<220>
<223>  Amorce deltacatB-fwd

<400>  1
tgttatggat tataagcggc tcgaggacgt caaaccatgt taatcattgc                50


<210>  2
<211>  50
<212>  DNA
<213>  Artificial Sequence

<220>
<223>  Amorce deltacatB-rev

<400>  2
aatctatcac tgatagggac tcgagcaatt tcaccaaaga attcgctagc                50


<210>  3
<211>  28
<212>  DNA
<213>  Artificial Sequence

<220>
<223>  Amorce RH076

<400>  3
catataataa aaggaaacct cttgatcg                                        28


<210>  4
<211>  20
<212>  DNA
<213>  Artificial Sequence

<220>
<223>  Amorce RH077

<400>  4
attgccagcc taacacttgg                                                 20


<210>  5
<211>  50

<212>  DNA
<213>  Artificial Sequence

<220>
<223>  Amorce RH001

<400>  5
atctccatgg acgcgtgacg tcgacataag gtaccaggaa ttagagcagc                50


<210>  6
<211>  43
<212>  DNA
<213>  Artificial Sequence

<220>
<223>  Amorce RH002

<400>  6
tctatctcca gctctagacc attattattc ctccaagttt gct                       43


<210>  7
<211>  40
<212>  DNA
<213>  Artificial Sequence

<220>
<223>  Amorce RH003

<400>  7
ataatggtct agagctggag atagattatt tggtactaag                           40


<210>  8
<211>  50
<212>  DNA
<213>  Artificial Sequence

<220>
<223>  Amorce RH004

<400>  8
tatgaccatg attacgaatt cgagctcgaa gcgcttatta ttgcattagc                50


<210>  9
<211>  22
<212>  DNA
<213>  Artificial Sequence

<220>
<223>  Amorce pEX-fwd

<400>  9
cagattgtac tgagagtgca cc                                              22


<210>  10
<211>  23
<212>  DNA
<213>  Artificial Sequence

<220>
<223>  Amorce pEX-rev

<400>  10
gtgagcggat aacaatttca cac                                              23


<210>  11
<211>  32
<212>  DNA
<213>  Artificial Sequence

<220>
<223>  Amorce pEC750C-fwd

<400>  11
caatattcca caatattata ttataagcta gc                                    32


<210>  12
<211>  17
<212>  DNA
<213>  Artificial Sequence

<220>
<223>  Amorce M13-rev

<400>  12
caggaaacag ctatgac                                                     17


<210>  13
<211>  22
<212>  DNA
<213>  Artificial Sequence

<220>
<223>  Amorce RH010

<400>  13
cggatattgc attaccagta gc                                               22


<210>  14
<211>  24
<212>  DNA
<213>  Artificial Sequence

<220>
<223>  Amorce RH011

<400>  14
ttatcaatct cttacacatg gagc                                             24


<210>  15
<211>  24
<212>  DNA
<213>  Artificial Sequence

<220>
<223>  Amorce RH025

<400>  15
tagtatgccg ccattattac gaca                                              24


<210>  16
<211>  20
<212>  DNA
<213>  Artificial Sequence

<220>
<223>  Amorce RH134

<400>  16
gtcgacgtgg aattgtgagc                                                   20


<210>  17
<211>  3658
<212>  DNA
<213>  Artificial Sequence

<220>
<223>  pEX-A258-deltacatB

<400>  17
ctcgagctgc agcaaaaaaa gcaccgactc ggtgccactt tttcaagttg ataacggact      60

agccttattt taacttgcta tttctagctc taaaactgtg gtctctcttt tcgttgatgg     120

tggaatgata agggtttgca ccttaatttc tcctattgag aaaatcgtct cttctcagac     180

gtcaaaccat gttaatcatt gcttttatca aaaataggat ccactctatc attgatagag     240

tttgaaactc tatcattgat agagtataat atctttgttc atgtacatca tgctatctgt     300

gagttttaga gctagaaata gcaagttaaa ataaggctag tccgttatca acttgaaaaa     360

gtggcaccga gtcggtgctt tttttgaagc ttgtctttac acttttgccc attaattttt     420

gagttcctta tttttaggga gcttttatta tttttatcat gaaaatttca taaaatactc     480

ataaactaag gatgtcttca taatcagatt agtactccat tttcaatcca tttaatctgg     540

gaatatgata ttttaattac gtattattta agatatatta acgtgtaata taataccccg     600

caaatattaa ttatcacata catatccccc ctttattggg gcattttttg tacccattat     660

tttagtattg tgcagtactt aaataaaaaa atgccgcaaa ttcattttta ttgaataatg     720

cggtatttct tctattcttt atttttatta ctctataaat aatgtaatca agacatgact     780

atctaaatat atgatatctt aattcataat tcgggcctcc taaaaatttt cgtaattcta     840

ttttagaagg cttttttccg tgacctagcc atttcaatct cctttttaca atgatattta     900

cgctttagtt tattatagca cattctgtaa taccgaacta ttcaattttc agagaccatt     960

ttttattgat tcataactta agaatactac gaattactct aatattttac tttttcttat    1020

ctcttgttat tttaacatcg gaattactac taatattaat ttttattttt ccatccgcat    1080

ttgctccaac atttttttaa ctatactttc cttttgttaa taaattatgt tattgttgaa    1140

caatataaga aaagtgcgta acatttttta ttaaaaataa ttaggtattt ctatctgtgg 1200

ggtaccctcg aggtggcagc tctagagcta gcgaattctt tggtgaaatt gttatccgct 1260

cacaattcca cacaacatac gagccggaag cataaagtgt aaagcctggg gtgcctaatg 1320

agtgagctaa ctcacattaa ttgcgttgcg ctcactgccc gctttccagt cgggaaacct 1380

gtcgtgccag ctgcattaat gaatcggcca acgcgcgggg agaggcggtt tgcgtattgg 1440

gcgctcttcc gcttcctcgc tcactgactc gctgcgctcg gtcgttcggc tgcggcgagc 1500

ggtatcagct cactcaaagg cggtaatacg gttatccaca gaatcagggg ataacgcagg 1560

aaagaacatg tgagcaaaag gccagcaaaa ggccaggaac cgtaaaaagg ccgcgttgct 1620

ggcgtttttc cataggctcc gcccccctga cgagcatcac aaaaatcgac gctcaagtca 1680

gaggtggcga aacccgacag gactataaag ataccaggcg tttccccctg gaagctccct 1740

cgtgcgctct cctgttccga ccctgccgct taccggatac ctgtccgcct ttctcccttc 1800

gggaagcgtg gcgctttctc atagctcacg ctgtaggtat ctcagttcgg tgtaggtcgt 1860

tcgctccaag ctgggctgtg tgcacgaacc ccccgttcag cccgaccgct gcgccttatc 1920

cggtaactat cgtcttgagt ccaacccggt aagacacgac ttatcgccac tggcagcagc 1980

cactggtaac aggattagca gagcgaggta tgtaggcggt gctacagagt tcttgaagtg 2040

gtggcctaac tacggctaca ctagaagaac agtatttggt atctgcgctc tgctgaagcc 2100

agttaccttc ggaaaaagag ttggtagctc ttgatccggc aaacaaacca ccgctggtag 2160

cggtggtttt tttgtttgca agcagcagat tacgcgcaga aaaaaaggat ctcaagaaga 2220

tcctttgatc ttttctacgg ggtctgacgc tcagtggaac gaaaactcac gttaagggat 2280

tttggtcatg agattatcaa aaaggatctt cacctagatc cttttaaatt aaaaatgaag 2340

ttttaaatca atctaaagta tatatgagta aacttggtct gacagttacc aatgcttaat 2400

cagtgaggca cctatctcag cgatctgtct atttcgttca tccatagttg cctgactccc 2460

cgtcgtgtag ataactacga tacgggaggg cttaccatct ggccccagtg ctgcaatgat 2520

accgcgcgaa ccacgctcac cggctccaga tttatcagca ataaaccagc cagccggaag 2580

ggccgagcgc agaagtggtc ctgcaacttt atccgcctcc atccagtcta ttaattgttg 2640

ccgggaagct agagtaagta gttcgccagt taatagtttg cgcaacgttg ttgccattgc 2700

tacaggcatc gtggtgtcac gctcgtcgtt tggtatggct tcattcagct ccggttccca 2760

acgatcaagg cgagttacat gatcccccat gttgtgcaaa aaagcggtta gctccttcgg 2820

tcctccgatc gttgtcagaa gtaagttggc cgcagtgtta tcactcatgg ttatggcagc 2880

actgcataat tctcttactg tcatgccatc cgtaagatgc ttttctgtga ctggtgagta 2940

ctcaaccaag tcattctgag aatagtgtat gcggcgaccg agttgctctt gcccggcgtc 3000

aatacgggat aataccgcgc cacatagcag aactttaaaa gtgctcatca ttggaaaacg 3060

ttcttcgggg cgaaaactct caaggatctt accgctgttg agatccagtt cgatgtaacc 3120

cactcgtgca cccaactgat cttcagcatc ttttactttc accagcgttt ctgggtgagc 3180

aaaaacagga aggcaaaatg ccgcaaaaaa gggaataagg gcgacacgga aatgttgaat 3240

actcatactc ttcctttttc aatattattg aagcatttat cagggttatt gtctcatgag 3300

cggatacata tttgaatgta tttagaaaaa taaacaaata ggggttccgc gcacatttcc 3360

ccgaaaagtg ccacctgacg tctaagaaac cattattatc atgacattaa cctataaaaa 3420

taggcgtatc acgaggccct ttcgtctcgc gcgtttcggt gatgacggtg aaaacctctg 3480

acacatgcag ctcccggaga cggtcacagc ttgtctgtaa gcggatgccg ggagcagaca 3540

agcccgtcag ggcgcgtcag cgggtgttgg cgggtgtcgg ggctggctta actatgcggc 3600

atcagagcag attgtactga gagtttggca attggtcgac ctcgagggcg cgcccgta 3658

<210> 18
<211> 660
<212> DNA
<213> Clostridium beijerinckii

<400> 18
atgaatttta atttgataga tattaatcat tggagtagaa agccatactt tgaacattat 60

ttaaacaatg tgaaatgtac ttatagtatg actgccaata tagaaataac tgatttattg 120

tatgaaatta aacttaaaaa tattaaattt tatcctaccc ttatttatat gattgcaact 180

gtggttaata agcataaaga attccgtatt tgttttgatc atgaaggtag tttaggatat 240

tgggatagca tgaatccaag ctatactatt tttcataaag aaaacgaaac attttcaagt 300

atttggacgg aatataacaa aagtttttta cgtttttata gtgattatct tgacgatata 360

aaaaactatg gaaatatcat gaagtttact ccgaaatcaa atgaacctga caatacattt 420

tctgtatcaa gcattccttg ggtgagtttt acaggattta acttgaatgt gtataatgaa 480

ggaacatatt taattcctat ttttactgca ggaaagtatt tcaaacaaga aaataaaata 540

tttattccta tatcaataca agtacatcat gctatctgtg acggttatca tgctagtaga 600

tttattaatg aaatgcaaga attagcattt agttttcaag aatggttaga aaataaataa 660

<210> 19
<211> 160
<212> DNA
<213> Artificial Sequence

<220>
<223> Cassette d'expression ARNg

<400> 19
actctatcat tgatagagtt tgaaactcta tcattgatag agtataatat ctttgttcat 60

gtacatcatg ctatctgtga gttttagagc tagaaatagc aagttaaaat aaggctagtc 120

cgttatcaac ttgaaaaagt ggcaccgagt cggtgctttt                    160

<210>  20
<211>  808
<212>  DNA
<213>  Artificial Sequence

<220>
<223>  Matrice d'édition

<400>  20
gtctttacac ttttgcccat taatttttga gttccttatt tttagggagc ttttattatt      60

tttatcatga aaatttcata aaatactcat aaactaagga tgtcttcata atcagattag     120

tactccattt tcaatccatt taatctggga atatgatatt ttaattacgt attatttaag     180

atatattaac gtgtaatata ataccccgca aatattaatt atcacataca tatcccccct      240

ttattggggc attttttgta cccattattt tagtattgtg cagtacttaa ataaaaaaat      300

gccgcaaatt catttttatt gaataatgcg gtatttcttc tattctttat ttttattact     360

ctataaataa tgtaatcaag acatgactat ctaaatatat gatatcttaa ttcataattc     420

gggcctccta aaaattttcg taattctatt ttagaaggct tttttccgtg acctagccat      480

ttcaatctcc tttttacaat gatatttacg ctttagttta ttatagcaca ttctgtaata     540

ccgaactatt caattttcag agaccatttt ttattgattc ataacttaag aatactacga     600

attactctaa tattttactt tttcttatct cttgttattt taacatcgga attactacta     660

atattaattt ttatttttcc atccgcattt gctccaacat ttttttaact atactttcct     720

tttgttaata aattatgtta ttgttgaaca atataagaaa agtgcgtaac attttttatt    780

aaaaataatt aggtatttct atctgtgg                                        808

<210>  21
<211>  9954
<212>  DNA
<213>  Artificial Sequence

<220>
<223>  pCas9ind-deltacatB

<400>  21
catggataaa aagtacagta ttggtctaga cataggaact aactctgttg ggtgggctgt      60

tataacagat gaatataaag ttccatcaaa aaaatttaaa gtattaggaa acactgatag     120

acattcaata aaaaaaaact tgataggtgc tttattattc gattcaggag agactgctga     180

agctacacgt ttaaaaagaa cagctagacg tagatataca agaagaaaaa ataggatatg     240

ttatcttcaa gaaattttta gtaatgaaat ggcaaaagtt gatgattcat tctttcacag     300

actagaagaa agtttcttag ttgaagaaga taagaagcat gaaagacacc ctatttttgg     360

taatatcgta gatgaagtag catatcatga gaagtatcca actatctatc atttaagaaa 420

gaaattagtt gattctacag ataaagctga tctgagatta atatatttag ctttagctca 480

tatgattaaa tttagaggac attttttaat agaaggtgat ttaaacccag acaacagcga 540

tgtagataaa ttatttatcc aattagttca aacttataat caattattcg aagagaatcc 600

aattaatgca agtggtgtag acgctaaggc tatattatca gctagattat caaaatctag 660

aagattagaa aatctaatag ctcaacttcc tggagaaaag aaaaatggac tttttgggaa 720

cctaatagct ctctcactcg gactaacacc aaattttaaa agcaattttg atcttgctga 780

agacgcaaag ttacaactat caaaggatac atacgatgat gatttagata atttgttagc 840

tcaaataggt gatcaatatg ctgatttgtt tcttgcagca aaaaacttaa gtgatgcaat 900

tttactatca gatatactta gagtaaatac agaaataaca aaggctcctt tatcagcaag 960

tatgattaaa cgatatgatg agcatcatca agatttaaca ttattaaagg cacttgtaag 1020

acaacaatta ccagaaaaat ataaagaaat tttctttgat caatctaaaa atggatatgc 1080

tggatatata gacggtggag caagtcaaga agagttttat aaatttataa agcctatttt 1140

agaaaaaatg gatggaactg aagaattact tgttaaactt aacagagaag atttacttag 1200

aaaacaaaga acttttgata atggttcaat tcctcaccaa attcatttag gagaattaca 1260

tgctatacta agaagacaag aagattttta tccatttctt aaagataata gagaaaaaat 1320

tgaaaaaatt ttaactttta gaataccata ttatgtagga ccacttgcaa ggggaaattc 1380

aagatttgca tggatgacta gaaaatcaga agaaactata accccgtgga attttgaaga 1440

agtagtagat aaaggagcta gtgctcaatc atttatagaa agaatgacaa attttgataa 1500

gaatcttcct aacgaaaagg ttttgccaaa gcatagcctt ctttatgagt attttacagt 1560

ttataatgag cttactaaag taaaatacgt tacagaagga atgagaaaac cagcattttt 1620

gtctggtgaa caaaagaaag caatagtaga cctattattt aaaacaaata ggaaggttac 1680

cgtaaagcaa cttaaagaag attacttcaa aaaaattgaa tgctttgata gtgttgaaat 1740

atcaggagtt gaagatagat ttaatgcttc acttggtaca tatcacgatc tcttaaaaat 1800

tataaaagat aaggattttt tagataatga agaaaatgaa gatattcttg aagatatagt 1860

attaacattg acactttttg aagatagaga aatgatagaa gaaagattaa aaacatatgc 1920

acatcttttt gatgataagg ttatgaagca acttaaaaga agaagatata caggttgggg 1980

acgtttgtca agaaagctaa ttaatggtat tagagataaa caatcaggaa agactattct 2040

cgattttctt aaatcagatg gatttgctaa tagaaacttt atgcaattaa ttcatgatga 2100

ttctcttact ttcaaagagg atattcaaaa ggctcaagtt tctggacaag gcgatagctt 2160

acacgaacac attgctaacc ttgcagggag ccccgctatc aaaaaaggaa ttttacaaac 2220

agttaaagtt gtagatgaac ttgttaaagt tatgggaaga cacaaacctg agaatatagt 2280

tatagaaatg gccagagaaa atcaaacaac acaaaaagga caaaaaaatt ctagagagag 2340

aatgaagaga attgaagaag gaataaaaga gctaggatca caaatattaa aagaacatcc 2400

agttgaaaat actcaattgc aaaatgaaaa gttatatttg tattacttac aaaatggaag 2460

agatatgtat gttgatcaag aactcgatat taatagatta agtgactatg atgttgatca 2520

tattgttcct caatcatttt taaaagatga ttcaatcgat aacaaagtat taactagatc 2580

agataaaaat agaggaaagt cagataatgt accatctgaa gaagttgtta aaaaaatgaa 2640

gaactattgg agacaacttt taaatgcaaa gctaattaca caaagaaaat ttgacaattt 2700

aacaaaagca gaaagaggag gattaagcga attagacaaa gctggattta taaaaagaca 2760

acttgttgag acaagacaaa taactaagca tgttgctcaa atacttgatt caagaatgaa 2820

tacaaaatat gatgaaaatg ataaattaat cagagaagta aaagtaataa cattaaagtc 2880

aaaattagta tcagatttca gaaaggattt tcaattttac aaagttcgtg aaataaataa 2940

ctatcatcat gctcatgatg catacttaaa tgctgttgta ggaactgctc ttattaagaa 3000

atatcctaaa ctagaaagcg aatttgttta tggagattat aaagtttatg atgtgcgcaa 3060

aatgatcgcg aaatccgaac aagaaatcgg taaggctaca gcaaaatatt tcttttatag 3120

taatataatg aattttttta agacagaaat aactttggct aatggtgaaa tcagaaaaag 3180

accacttatc gaaacaaatg gagagacagg agaaatagta tgggataaag gaagagattt 3240

tgctactgtt agaaaagtac taagtatgcc acaagtaaat atcgtaaaga aaactgaagt 3300

tcaaactgga ggtttctcta aggaatcaat tttacctaag agaaattcag ataagttaat 3360

tgcaaggaaa aaagattggg acccaaaaaa atacggtggt tttgatagtc caacagttgc 3420

ctatagtgtt cttgtagtag cgaaagttga gaaaggtaag tcaaaaaagt tgaaaagcgt 3480

aaaagaactt cttggtatca caattatgga aagatcttca tttgaaaaaa atccaattga 3540

ctttttagaa gctaagggtt ataaagaagt taaaaaggat ttaatcataa aactaccaaa 3600

gtatagtcta tttgaactcg aaaacggaag aaaacgaatg ctcgctagcg caggagaact 3660

tcaaaaagga aatgaacttg cgctgccatc aaagtatgta aatttcttat atttagcttc 3720

tcattatgag aaattaaaag gatcaccaga ggataatgaa caaaagcaac tatttgtaga 3780

acaacacaaa cattatttag atgaaataat agaacaaata tctgaatttt ctaaaagagt 3840

tatacttgcc gacgcaaatc tagataaggt gctttcagcg tataataaac acagagataa 3900

accaataaga gaacaagcag aaaacattat ccatcttttt acattaacta atcttggtgc 3960

accagctgca tttaagtact ttgatacaac aatagataga aaaagataca catctactaa 4020

agaagtatta gacgcaactt taatacatca atctattaca gggctttatg aaacaagaat 4080

tgatttaagt caactaggcg gagattaagt cgacaaagta ttgttaaaaa taactctgta 4140

gaattataaa ttagttctac agagttattt tttgacccgg gtatattgat aaaaataata 4200

atagtgggta taattaagtt gttaggaggt tagttagaat gatgtcaaga ttagataaaa 4260

gtaaagtgat taacagcgca ttagagctgc ttaatgaggt cggaatcgaa ggtttaacaa 4320

cccgtaaact cgcccagaag ctaggtgtag agcagcctac attgtattgg catgtaaaaa 4380

ataagcgggc tttgctcgac gccttagcca ttgagatgtt agataggcac catactcact 4440

tttgcccttt agaaggggaa agctggcaag attttttacg taataacgct aaaagtttta 4500

gatgtgcttt actaagtcat cgcgatggag caaaagtaca tttaggtaca cggcctacag 4560

aaaaacagta tgaaactctc gaaaatcaat tagccttttt atgccaacaa ggtttttcac 4620

tagagaatgc attatatgca ctcagcgctg tggggcattt tactttaggt tgcgtattgg 4680

aagatcaaga gcatcaagtc gctaaagaag aaagggaaac acctactact gatagtatgc 4740

cgccattatt acgacaagct atcgaattat ttgatcacca aggtgcagag ccagccttct 4800

tattcggcct tgaattgatc atatgcggat tagaaaaaca acttaaatgt gaaagtgggt 4860

cttaaaagca gcataacctt tttccgtgat ggtaacttca cggtaaccaa gatgtcgagt 4920

tgagctcgaa ttcgtaatca tggtcatagc tgtttcctgt gtgaaattgt tatccgctca 4980

caattccaca caacatacga gccggaagca taaagtgtaa agcctggggt gcctaatgag 5040

tgagctaact cacattaatt gcgttgcgct cactgcccgc tttccagtcg ggaaacctgt 5100

cgtgccagct gcattaatga atcggccaac gcgcggggag aggcggtttg cgtattgggc 5160

gctcttccgc ttcctcgctc actgactcgc tgcgctcggt cgttcggctg cggcgagcgg 5220

tatcagctca ctcaaaggcg gtaatacggt tatccacaga atcaggggat aacgcaggaa 5280

agaacatgtg agcaaaaggc cagcaaaagg ccaggaaccg taaaaaggcc gcgttgctgg 5340

cgtttttcca taggctccgc ccccctgacg agcatcacaa aaatcgacgc tcaagtcaga 5400

ggtggcgaaa cccgacagga ctataaagat accaggcgtt tccccctgga agctccctcg 5460

tgcgctctcc tgttccgacc ctgccgctta ccggatacct gtccgccttt ctcccttcgg 5520

gaagcgtggc gctttctcat agctcacgct gtaggtatct cagttcggtg taggtcgttc 5580

gctccaagct gggctgtgtg cacgaacccc ccgttcagcc cgaccgctgc gccttatccg 5640

gtaactatcg tcttgagtcc aacccggtaa gacacgactt atcgccactg gcagcagcca 5700

ctggtaacag gattagcaga gcgaggtatg taggcggtgc tacagagttc ttgaagtggt 5760

ggcctaacta cggctacact agaaggacag tatttggtat ctgcgctctg ctgaagccag 5820

ttaccttcgg aaaaagagtt ggtagctctt gatccggcaa acaaaccacc gctggtagcg 5880

gtggtttttt tgtttgcaag cagcagatta cgcgcagaaa aaaaggatct caagaagatc 5940

ctttgatctt ttctacgggg tctgacgctc agtggaacga aaactcacgt taagggattt 6000

tggtcatgag attatcaaaa aggatcttca cctagatcct tttaaattaa aaatgaagtt 6060

ttaaatcaat ctaaagtata tatgagtaaa cttggtctga cagttaccag gtccactgcc 6120

gggcctcttg cgggatcaaa agaaaaacga aatgatacac caatcagtgc aaaaaaagat 6180

ataatgggag ataagacggt tcgtgttcgt gctgacttgc accatatcat aaaaatcgaa 6240

acagcaaaga atggcggaaa cgtaaaagaa gttatggaaa taagacttag aagcaaactt 6300

aagagtgtgt tgatagtgca gtatcttaaa attttgtata ataggaattg aagttaaatt 6360

agatgctaaa aatttgtaat taagaaggag tgattacatg aacaaaaata taaaatattc 6420

tcaaaacttt ttaacgagtg aaaaagtact caaccaaata ataaaacaat tgaatttaaa 6480

agaaaccgat accgtttacg aaattggaac aggtaaaggg catttaacga cgaaactggc 6540

taaaataagt aaacaggtaa cgtctattga attagacagt catctattca acttatcgtc 6600

agaaaaatta aaactgaata ctcgtgtcac tttaattcac caagatattc tacagtttca 6660

attccctaac aaacagaggt ataaaattgt tgggagtatt ccttaccatt taagcacaca 6720

aattattaaa aaagtggttt ttgaaagcca tgcgtctgac atctatctga ttgttgaaga 6780

aggattctac aagcgtacct tggatattca ccgaacacta gggttgctct tgcacactca 6840

agtctcgatt cagcaattgc ttaagctgcc agcggaatgc tttcatccta aaccaaaagt 6900

aaacagtgtc ttaataaaac ttacccgcca taccacagat gttccagata aatattggaa 6960

gctatatacg tactttgttt caaaatgggt caatcgagaa tatcgtcaac tgtttactaa 7020

aaatcagttt catcaagcaa tgaaacacgc caaagtaaac aatttaagta ccgttactta 7080

tgagcaagta ttgtctattt ttaatagtta tctattattt aacgggagga aataattcta 7140

tgagtcccta ggcaggcctc cgccattatt tttttgaaca attgacaatt catttcttat 7200

tttttattaa gtgatagtca aaaggcataa cagtgctgaa tagaaagaaa tttacagaaa 7260

agaaaattat agaatttagt atgattaatt atactcattt atgaatgttt aattgaatac 7320

aaaaaaaaat acttgttatg tattcaatta cgggttaaaa tatagacaag ttgaaaaatt 7380

taataaaaaa ataagtcctc agctcttata tattaagcta ccaacttagt atataagcca 7440

aaacttaaat gtgctaccaa cacatcaagc cgttagagaa ctctatctat agcaatattt 7500

caaatgtacc gacatacaag agaaacatta actatatata ttcaatttat gagattatct 7560

taacagatat aaatgtaaat tgcaataagt aagatttaga agtttatagc ctttgtgtat 7620

tggaagcagt acgcaaaggc ttttttattt gataaaaatt agaagtatat ttattttttc 7680

ataattaatt tatgaaaatg aaagggggtg agcaaagtga cagaggaaag cagtatctta 7740

tcaaataaca aggtattagc aatatcatta ttgactttag cagtaaacat tatgactttt 7800

atagtgcttg tagctaagta gtacgaaagg gggagcttta aaaagctcct tggaatacat 7860

agaattcata aattaattta tgaaaagaag ggcgtatatg aaaacttgta aaaattgcaa 7920

agagtttatt aaagatactg aaatatgcaa aatacattcg ttgatgattc atgataaaac 7980

agtagcaacc tattgcagta aatacaatga gtcaagatgt ttacataaag ggaaagtcca 8040

atgtattaat tgttcaaaga tgaaccgata tggatggtgt gccataaaaa tgagatgttt 8100

tacagaggaa gaacagaaaa aagaacgtac atgcattaaa tattatgcaa ggagctttaa 8160

aaaagctcat gtaaagaaga gtaaaaagaa aaaataattt atttattaat ttaatattga 8220

gagtgccgac acagtatgca ctaaaaaata tatctgtggt gtagtgagcc gatacaaaag 8280

gatagtcact cgcattttca taatacatct tatgttatga ttatgtgtcg gtgggacttc 8340

acgacgaaaa cccacaataa aaaaagagtt cggggtaggg ttaagcatag ttgaggcaac 8400

taaacaatca agctaggata tgcagtagca gaccgtaagg tcgttgttta ggtgtgttgt 8460

aatacatacg ctattaagat gtaaaaatac ggataccaat gaagggaaaa gtataatttt 8520

tggatgtagt ttgtttgttc atctatgggc aaactacgtc caaagccgtt tccaaatctg 8580

ctaaaaagta tatcctttct aaaatcaaag tcaagtatga aatcataaat aaagtttaat 8640

tttgaagtta ttatgatatt atgtttttct attaaaataa attaagtata tagaatagtt 8700

taataatagt atatacttaa tgtgataagt gtctgacagt gtcacagaaa ggatgattgt 8760

tatggattat aagcggctcg aggacgtcaa accatgttaa tcattgcttt tatcaaaaat 8820

aggatccact ctatcattga tagagtttga aactctatca ttgatagagt ataatatctt 8880

tgttcatgta catcatgcta tctgtgagtt ttagagctag aaatagcaag ttaaaataag 8940

gctagtccgt tatcaacttg aaaaagtggc accgagtcgg tgcttttttt gaagcttgtc 9000

tttacacttt tgcccattaa tttttgagtt ccttattttt agggagcttt tattattttt 9060

atcatgaaaa tttcataaaa tactcataaa ctaaggatgt cttcataatc agattagtac 9120

tccattttca atccatttaa tctgggaata tgatatttta attacgtatt atttaagata 9180

tattaacgtg taatataata ccccgcaaat attaattatc acatacatat ccccccttta 9240

ttggggcatt ttttgtaccc attattttag tattgtgcag tacttaaata aaaaaatgcc 9300

gcaaattcat ttttattgaa taatgcggta tttcttctat tctttatttt tattactcta 9360

taaataatgt aatcaagaca tgactatcta aatatatgat atcttaattc ataattcggg 9420

cctcctaaaa attttcgtaa ttctatttta gaaggctttt ttccgtgacc tagccatttc 9480

aatctccttt ttacaatgat atttacgctt tagtttatta tagcacattc tgtaataccg 9540

aactattcaa ttttcagaga ccatttttta ttgattcata acttaagaat actacgaatt 9600

actctaatat tttacttttt cttatctctt gttattttaa catcggaatt actactaata 9660

ttaattttta tttttccatc cgcatttgct ccaacatttt tttaactata ctttcctttt 9720

gttaataaat tatgttattg ttgaacaata taagaaaagt gcgtaacatt ttttattaaa 9780

aataattagg tatttctatc tgtggggtac cctcgaggtg gcagctctag agctagcgaa 9840

ttctttggtg aaattgctcg agtccctatc agtgatagat tgaaactcta tcattgatag 9900

agtataatat ctttgttcat tagagcgata aacttgaatt tgagagggaa cttc 9954

<210> 22
<211> 8874
<212> DNA
<213> Artificial Sequence

<220>
<223> pCas9ind

<400> 22
catggataaa aagtacagta ttggtctaga cataggaact aactctgttg ggtgggctgt 60

tataacagat gaatataaag ttccatcaaa aaaatttaaa gtattaggaa acactgatag 120

acattcaata aaaaaaaact tgataggtgc tttattattc gattcaggag agactgctga 180

agctacacgt ttaaaaagaa cagctagacg tagatataca agaagaaaaa ataggatatg 240

ttatcttcaa gaaattttta gtaatgaaat ggcaaaagtt gatgattcat tctttcacag 300

actagaagaa agtttcttag ttgaagaaga taagaagcat gaaagacacc ctatttttgg 360

taatatcgta gatgaagtag catatcatga gaagtatcca actatctatc atttaagaaa 420

gaaattagtt gattctacag ataaagctga tctgagatta atatatttag ctttagctca 480

tatgattaaa tttagaggac attttttaat agaaggtgat ttaaacccag acaacagcga 540

tgtagataaa ttatttatcc aattagttca aacttataat caattattcg aagagaatcc 600

aattaatgca agtggtgtag acgctaaggc tatattatca gctagattat caaaatctag 660

aagattagaa aatctaatag ctcaacttcc tggagaaaag aaaaatggac tttttgggaa 720

cctaatagct ctctcactcg gactaacacc aaattttaaa agcaattttg atcttgctga 780

agacgcaaag ttacaactat caaaggatac atacgatgat gatttagata atttgttagc 840

tcaaataggt gatcaatatg ctgatttgtt tcttgcagca aaaaacttaa gtgatgcaat 900

tttactatca gatatactta gagtaaatac agaaataaca aaggctcctt tatcagcaag 960

tatgattaaa cgatatgatg agcatcatca agatttaaca ttattaaagg cacttgtaag 1020

acaacaatta ccagaaaaat ataaagaaat tttctttgat caatctaaaa atggatatgc 1080

tggatatata gacggtggag caagtcaaga agagttttat aaatttataa agcctatttt 1140

agaaaaaatg gatggaactg aagaattact tgttaaactt aacagagaag atttacttag 1200

aaaacaaaga acttttgata atggttcaat tcctcaccaa attcatttag gagaattaca 1260

tgctatacta agaagacaag aagattttta tccatttctt aaagataata gagaaaaaat 1320

tgaaaaaatt ttaactttta gaataccata ttatgtagga ccacttgcaa ggggaaattc 1380

aagatttgca tggatgacta gaaaatcaga agaaactata accccgtgga attttgaaga 1440

agtagtagat aaaggagcta gtgctcaatc atttatagaa agaatgacaa attttgataa 1500
gaatcttcct aacgaaaagg ttttgccaaa gcatagcctt ctttatgagt attttacagt 1560
ttataatgag cttactaaag taaaatacgt tacagaagga atgagaaaac cagcattttt 1620
gtctggtgaa caaaagaaag caatagtaga cctattattt aaaacaaata ggaaggttac 1680
cgtaaagcaa cttaaagaag attacttcaa aaaaattgaa tgctttgata gtgttgaaat 1740
atcaggagtt gaagatagat ttaatgcttc acttggtaca tatcacgatc tcttaaaaat 1800
tataaaagat aaggattttt tagataatga agaaaatgaa gatattcttg aagatatagt 1860
attaacattg acactttttg aagatagaga aatgatagaa gaaagattaa aaacatatgc 1920
acatcttttt gatgataagg ttatgaagca acttaaaaga agaagatata caggttgggg 1980
acgtttgtca agaaagctaa ttaatggtat tagagataaa caatcaggaa agactattct 2040
cgattttctt aaatcagatg gatttgctaa tagaaacttt atgcaattaa ttcatgatga 2100
ttctcttact ttcaaagagg atattcaaaa ggctcaagtt tctggacaag gcgatagctt 2160
acacgaacac attgctaacc ttgcagggag ccccgctatc aaaaaaggaa ttttacaaac 2220
agttaaagtt gtagatgaac ttgttaaagt tatgggaaga cacaaacctg agaatatagt 2280
tatagaaatg gccagagaaa atcaaacaac acaaaaagga caaaaaaatt ctagagagag 2340
aatgaagaga attgaagaag gaataaaaga gctaggatca caaatattaa aagaacatcc 2400
agttgaaaat actcaattgc aaaatgaaaa gttatatttg tattacttac aaaatggaag 2460
agatatgtat gttgatcaag aactcgatat taatagatta agtgactatg atgttgatca 2520
tattgttcct caatcatttt taaaagatga ttcaatcgat aacaaagtat taactagatc 2580
agataaaaat agaggaaagt cagataatgt accatctgaa gaagttgtta aaaaaatgaa 2640
gaactattgg agacaacttt taaatgcaaa gctaattaca caaagaaaat ttgacaattt 2700
aacaaaagca gaaagaggag gattaagcga attagacaaa gctggattta taaaaagaca 2760
acttgttgag acaagacaaa taactaagca tgttgctcaa atacttgatt caagaatgaa 2820
tacaaaatat gatgaaaatg ataaattaat cagagaagta aaagtaataa cattaaagtc 2880
aaaattagta tcagatttca gaaaggattt tcaattttac aaagttcgtg aaataaataa 2940
ctatcatcat gctcatgatg catacttaaa tgctgttgta ggaactgctc ttattaagaa 3000
atatcctaaa ctagaaagcg aatttgttta tggagattat aaagtttatg atgtgcgcaa 3060
aatgatcgcg aaatccgaac aagaaatcgg taaggctaca gcaaaatatt tcttttatag 3120
taatataatg aattttttta agacagaaat aactttggct aatggtgaaa tcagaaaaag 3180
accacttatc gaaacaaatg gagagacagg agaaatagta tgggataaag gaagagattt 3240
tgctactgtt agaaaagtac taagtatgcc acaagtaaat atcgtaaaga aaactgaagt 3300
tcaaactgga ggtttctcta aggaatcaat tttacctaag agaaattcag ataagttaat 3360

tgcaaggaaa aaagattggg acccaaaaaa atacggtggt tttgatagtc caacagttgc 3420

ctatagtgtt cttgtagtag cgaaagttga gaaaggtaag tcaaaaaagt tgaaaagcgt 3480

aaaagaactt cttggtatca caattatgga aagatcttca tttgaaaaaa atccaattga 3540

ctttttagaa gctaagggtt ataaagaagt taaaaaggat ttaatcataa aactaccaaa 3600

gtatagtcta tttgaactcg aaaacggaag aaaacgaatg ctcgctagcg caggagaact 3660

tcaaaaagga aatgaacttg cgctgccatc aaagtatgta aatttcttat atttagcttc 3720

tcattatgag aaattaaaag gatcaccaga ggataatgaa caaaagcaac tatttgtaga 3780

acaacacaaa cattatttag atgaaataat agaacaaata tctgaatttt ctaaaagagt 3840

tatacttgcc gacgcaaatc tagataaggt gctttcagcg tataataaac acagagataa 3900

accaataaga gaacaagcag aaaacattat ccatcttttt acattaacta atcttggtgc 3960

accagctgca tttaagtact ttgatacaac aatagataga aaaagataca catctactaa 4020

agaagtatta gacgcaactt taatacatca atctattaca gggctttatg aaacaagaat 4080

tgatttaagt caactaggcg gagattaagt cgacaaagta ttgttaaaaa taactctgta 4140

gaattataaa ttagttctac agagttattt tttgacccgg gtatattgat aaaaataata 4200

atagtgggta taattaagtt gttaggaggt tagttagaat gatgtcaaga ttagataaaa 4260

gtaaagtgat taacagcgca ttagagctgc ttaatgaggt cggaatcgaa ggtttaacaa 4320

cccgtaaact cgcccagaag ctaggtgtag agcagcctac attgtattgg catgtaaaaa 4380

ataagcgggc tttgctcgac gccttagcca ttgagatgtt agataggcac catactcact 4440

tttgcccttt agaaggggaa agctggcaag attttttacg taataacgct aaaagtttta 4500

gatgtgcttt actaagtcat cgcgatggag caaaagtaca tttaggtaca cggcctacag 4560

aaaaacagta tgaaactctc gaaaatcaat tagccttttt atgccaacaa ggtttttcac 4620

tagagaatgc attatatgca ctcagcgctg tggggcattt tactttaggt tgcgtattgg 4680

aagatcaaga gcatcaagtc gctaaagaag aaagggaaac acctactact gatagtatgc 4740

cgccattatt acgacaagct atcgaattat ttgatcacca aggtgcagag ccagccttct 4800

tattcggcct tgaattgatc atatgcggat tagaaaaaca acttaaatgt gaaagtgggt 4860

cttaaaagca gcataacctt tttccgtgat ggtaacttca cggtaaccaa gatgtcgagt 4920

tgagctcgaa ttcgtaatca tggtcatagc tgtttcctgt gtgaaattgt tatccgctca 4980

caattccaca caacatacga gccggaagca taaagtgtaa agcctggggt gcctaatgag 5040

tgagctaact cacattaatt gcgttgcgct cactgcccgc tttccagtcg ggaaacctgt 5100

cgtgccagct gcattaatga atcggccaac gcgcggggag aggcggtttg cgtattgggc 5160

gctcttccgc ttcctcgctc actgactcgc tgcgctcggt cgttcggctg cggcgagcgg 5220

tatcagctca ctcaaaggcg gtaatacggt tatccacaga atcaggggat aacgcaggaa 5280

agaacatgtg agcaaaaggc cagcaaaagg ccaggaaccg taaaaaggcc gcgttgctgg 5340

cgtttttcca taggctccgc ccccctgacg agcatcacaa aaatcgacgc tcaagtcaga 5400

ggtggcgaaa cccgacagga ctataaagat accaggcgtt tccccctgga agctccctcg 5460

tgcgctctcc tgttccgacc ctgccgctta ccggatacct gtccgccttt ctcccttcgg 5520

gaagcgtggc gctttctcat agctcacgct gtaggtatct cagttcggtg taggtcgttc 5580

gctccaagct gggctgtgtg cacgaacccc ccgttcagcc cgaccgctgc gccttatccg 5640

gtaactatcg tcttgagtcc aacccggtaa gacacgactt atcgccactg gcagcagcca 5700

ctggtaacag gattagcaga gcgaggtatg taggcggtgc tacagagttc ttgaagtggt 5760

ggcctaacta cggctacact agaaggacag tatttggtat ctgcgctctg ctgaagccag 5820

ttaccttcgg aaaaagagtt ggtagctctt gatccggcaa acaaaccacc gctggtagcg 5880

gtggtttttt tgtttgcaag cagcagatta cgcgcagaaa aaaaggatct caagaagatc 5940

ctttgatctt ttctacgggg tctgacgctc agtggaacga aaactcacgt taagggattt 6000

tggtcatgag attatcaaaa aggatcttca cctagatcct tttaaattaa aaatgaagtt 6060

ttaaatcaat ctaaagtata tatgagtaaa cttggtctga cagttaccag gtccactgcc 6120

gggcctcttg cgggatcaaa agaaaaacga aatgatacac caatcagtgc aaaaaaagat 6180

ataatgggag ataagacggt tcgtgttcgt gctgacttgc accatatcat aaaaatcgaa 6240

acagcaaaga atggcggaaa cgtaaaagaa gttatggaaa taagacttag aagcaaactt 6300

aagagtgtgt tgatagtgca gtatcttaaa attttgtata ataggaattg aagttaaatt 6360

agatgctaaa aatttgtaat taagaaggag tgattacatg aacaaaaata taaaatattc 6420

tcaaaacttt ttaacgagtg aaaaagtact caaccaaata ataaaacaat tgaatttaaa 6480

agaaaccgat accgtttacg aaattggaac aggtaaaggg catttaacga cgaaactggc 6540

taaaataagt aaacaggtaa cgtctattga attagacagt catctattca acttatcgtc 6600

agaaaaatta aaactgaata ctcgtgtcac tttaattcac caagatattc tacagtttca 6660

attccctaac aaacagaggt ataaaattgt tgggagtatt ccttaccatt taagcacaca 6720

aattattaaa aaagtggttt ttgaaagcca tgcgtctgac atctatctga ttgttgaaga 6780

aggattctac aagcgtacct tggatattca ccgaacacta gggttgctct tgcacactca 6840

agtctcgatt cagcaattgc ttaagctgcc agcggaatgc tttcatccta aaccaaaagt 6900

aaacagtgtc ttaataaaac ttacccgcca taccacagat gttccagata aatattggaa 6960

gctatatacg tactttgttt caaaatgggt caatcgagaa tatcgtcaac tgtttactaa 7020

aaatcagttt catcaagcaa tgaaacacgc caaagtaaac aatttaagta ccgttactta 7080

tgagcaagta ttgtctattt ttaatagtta tctattattt aacgggagga aataattcta 7140

tgagtcccta ggcaggcctc cgccattatt tttttgaaca attgacaatt catttcttat 7200

tttttattaa gtgatagtca aaaggcataa cagtgctgaa tagaaagaaa tttacagaaa 7260

agaaaattat agaatttagt atgattaatt atactcattt atgaatgttt aattgaatac 7320

aaaaaaaaat acttgttatg tattcaatta cgggttaaaa tatagacaag ttgaaaaatt 7380

taataaaaaa ataagtcctc agctcttata tattaagcta ccaacttagt atataagcca 7440

aaacttaaat gtgctaccaa cacatcaagc cgttagagaa ctctatctat agcaatattt 7500

caaatgtacc gacatacaag agaaacatta actatatata ttcaatttat gagattatct 7560

taacagatat aaatgtaaat tgcaataagt aagatttaga agtttatagc ctttgtgtat 7620

tggaagcagt acgcaaaggc ttttttattt gataaaaatt agaagtatat ttattttttc 7680

ataattaatt tatgaaaatg aaagggggtg agcaaagtga cagaggaaag cagtatctta 7740

tcaaataaca aggtattagc aatatcatta ttgactttag cagtaaacat tatgactttt 7800

atagtgcttg tagctaagta gtacgaaagg gggagcttta aaaagctcct tggaatacat 7860

agaattcata aattaattta tgaaaagaag ggcgtatatg aaaacttgta aaaattgcaa 7920

agagtttatt aaagatactg aaatatgcaa aatacattcg ttgatgattc atgataaaac 7980

agtagcaacc tattgcagta aatacaatga gtcaagatgt ttacataaag ggaaagtcca 8040

atgtattaat tgttcaaaga tgaaccgata tggatggtgt gccataaaaa tgagatgttt 8100

tacagaggaa gaacagaaaa aagaacgtac atgcattaaa tattatgcaa ggagctttaa 8160

aaaagctcat gtaaagaaga gtaaaaagaa aaaataattt atttattaat ttaatattga 8220

gagtgccgac acagtatgca ctaaaaaata tatctgtggt gtagtgagcc gatacaaaag 8280

gatagtcact cgcattttca taatacatct tatgttatga ttatgtgtcg gtgggacttc 8340

acgacgaaaa cccacaataa aaaaagagtt cggggtaggg ttaagcatag ttgaggcaac 8400

taaacaatca agctaggata tgcagtagca gaccgtaagg tcgttgttta ggtgtgttgt 8460

aatacatacg ctattaagat gtaaaaatac ggataccaat gaagggaaaa gtataatttt 8520

tggatgtagt ttgtttgttc atctatgggc aaactacgtc caaagccgtt tccaaatctg 8580

ctaaaaagta tatcctttct aaaatcaaag tcaagtatga aatcataaat aaagtttaat 8640

tttgaagtta ttatgatatt atgtttttct attaaaataa attaagtata tagaatagtt 8700

taataatagt atatacttaa tgtgataagt gtctgacagt gtcacagaaa ggatgattgt 8760

tatggattat aagcggctcg agtccctatc agtgatagat tgaaactcta tcattgatag 8820

agtataatat ctttgttcat tagagcgata aacttgaatt tgagagggaa cttc 8874

<210> 23
<211> 10534
<212> DNA

<213>  Artificial Sequence

<220>
<223>  pCas9acr

<400>  23
cgaattcgta atcatggtca tagctgtttc ctgtgtgaaa ttgttatccg ctcacaattc      60

cacacaacat acgagccgga agcataaagt gtaaagcctg gggtgcctaa tgagtgagct     120

aactcacatt aattgcgttg cgctcactgc ccgctttcca gtcgggaaac ctgtcgtgcc     180

agctgcatta atgaatcggc caacgcgcgg ggagaggcgg tttgcgtatt gggcgctctt     240

ccgcttcctc gctcactgac tcgctgcgct cggtcgttcg gctgcggcga gcggtatcag     300

ctcactcaaa ggcggtaata cggttatcca cagaatcagg ggataacgca ggaaagaaca     360

tgtgagcaaa aggccagcaa aaggccagga accgtaaaaa ggccgcgttg ctggcgtttt     420

tccataggct ccgcccccct gacgagcatc acaaaaatcg acgctcaagt cagaggtggc     480

gaaacccgac aggactataa agataccagg cgtttccccc tggaagctcc ctcgtgcgct     540

ctcctgttcc gaccctgccg cttaccggat acctgtccgc ctttctccct tcgggaagcg     600

tggcgctttc tcatagctca cgctgtaggt atctcagttc ggtgtaggtc gttcgctcca     660

agctgggctg tgtgcacgaa ccccccgttc agcccgaccg ctgcgcctta tccggtaact     720

atcgtcttga gtccaacccg gtaagacacg acttatcgcc actggcagca gccactggta     780

acaggattag cagagcgagg tatgtaggcg gtgctacaga gttcttgaag tggtggccta     840

actacggcta cactagaagg acagtatttg gtatctgcgc tctgctgaag ccagttacct     900

tcggaaaaag agttggtagc tcttgatccg gcaaacaaac caccgctggt agcggtggtt     960

tttttgtttg caagcagcag attacgcgca gaaaaaaagg atctcaagaa gatcctttga    1020

tcttttctac ggggtctgac gctcagtgga acgaaaactc acgttaaggg attttggtca    1080

tgagattatc aaaaaggatc ttcacctaga tccttttaaa ttaaaaatga agttttaaat    1140

caatctaaag tatatatgag taaacttggt ctgacagtta ccaggtccac tgccgggcct    1200

cttgcgggat caaaagaaaa acgaaatgat acaccaatca gtgcaaaaaa agatataatg    1260

ggagataaga cggttcgtgt tcgtgctgac ttgcaccata tcataaaaat cgaaacagca    1320

aagaatggcg gaaacgtaaa agaagttatg gaaataagac ttagaagcaa acttaagagt    1380

gtgttgatag tgcagtatct taaaattttg tataatagga attgaagtta aattagatgc    1440

taaaaatttg taattaagaa ggagtgatta catgaacaaa aatataaaat attctcaaaa    1500

ctttttaacg agtgaaaaag tactcaacca aataataaaa caattgaatt taaaagaaac    1560

cgataccgtt tacgaaattg gaacaggtaa agggcattta acgacgaaac tggctaaaat    1620

aagtaaacag gtaacgtcta ttgaattaga cagtcatcta ttcaacttat cgtcagaaaa    1680

attaaaactg aatactcgtg tcactttaat tcaccaagat attctacagt ttcaattccc    1740

taacaaacag aggtataaaa ttgttgggag tattccttac catttaagca cacaaattat 1800

taaaaaagtg gtttttgaaa gccatgcgtc tgacatctat ctgattgttg aagaaggatt 1860

ctacaagcgt accttggata ttcaccgaac actagggttg ctcttgcaca ctcaagtctc 1920

gattcagcaa ttgcttaagc tgccagcgga atgctttcat cctaaaccaa aagtaaacag 1980

tgtcttaata aaacttaccc gccataccac agatgttcca gataaatatt ggaagctata 2040

tacgtacttt gtttcaaaat gggtcaatcg agaatatcgt caactgttta ctaaaaatca 2100

gtttcatcaa gcaatgaaac acgccaaagt aaacaattta agtaccgtta cttatgagca 2160

agtattgtct atttttaata gttatctatt atttaacggg aggaaataat tctatgagtc 2220

cctaggcagg cctccgccat tatttttttg aacaattgac aattcatttc ttatttttta 2280

ttaagtgata gtcaaaaggc ataacagtgc tgaatagaaa gaaatttaca gaaaagaaaa 2340

ttatagaatt tagtatgatt aattatactc atttatgaat gtttaattga atacaaaaaa 2400

aaatacttgt tatgtattca attacgggtt aaaatataga caagttgaaa aatttaataa 2460

aaaaataagt cctcagctct tatatattaa gctaccaact tagtatataa gccaaaactt 2520

aaatgtgcta ccaacacatc aagccgttag agaactctat ctatagcaat atttcaaatg 2580

taccgacata caagagaaac attaactata tatattcaat ttatgagatt atcttaacag 2640

atataaatgt aaattgcaat aagtaagatt tagaagttta tagcctttgt gtattggaag 2700

cagtacgcaa aggctttttt atttgataaa aattagaagt atatttattt tttcataatt 2760

aatttatgaa aatgaaaggg ggtgagcaaa gtgacagagg aaagcagtat cttatcaaat 2820

aacaaggtat tagcaatatc attattgact ttagcagtaa acattatgac ttttatagtg 2880

cttgtagcta agtagtacga aagggggagc tttaaaaagc tccttggaat acatagaatt 2940

cataaattaa tttatgaaaa gaagggcgta tatgaaaact tgtaaaaatt gcaaagagtt 3000

tattaaagat actgaaatat gcaaaataca ttcgttgatg attcatgata aaacagtagc 3060

aacctattgc agtaaataca atgagtcaag atgtttacat aaagggaaag tccaatgtat 3120

taattgttca aagatgaacc gatatggatg gtgtgccata aaaatgagat gttttacaga 3180

ggaagaacag aaaaaagaac gtacatgcat taaatattat gcaaggagct ttaaaaaagc 3240

tcatgtaaag aagagtaaaa agaaaaaata atttatttat taatttaata ttgagagtgc 3300

cgacacagta tgcactaaaa aatatatctg tggtgtagtg agccgataca aaaggatagt 3360

cactcgcatt ttcataatac atcttatgtt atgattatgt gtcggtggga cttcacgacg 3420

aaaacccaca ataaaaaaag agttcggggt agggttaagc atagttgagg caactaaaca 3480

atcaagctag gatatgcagt agcagaccgt aaggtcgttg tttaggtgtg ttgtaataca 3540

tacgctatta agatgtaaaa atacggatac caatgaaggg aaaagtataa tttttggatg 3600

tagtttgttt gttcatctat gggcaaacta cgtccaaagc cgtttccaaa tctgctaaaa 3660
agtatatcct ttctaaaatc aaagtcaagt atgaaatcat aaataaagtt taattttgaa 3720
gttattatga tattatgttt ttctattaaa ataaattaag tatatagaat agtttaataa 3780
tagtatatac ttaatgtgat aagtgtctga cagtgtcaca gaaaggatga ttgttatgga 3840
ttataagcgg ctcgagtccc tatcagtgat agattgaaac tctatcattg atagagtata 3900
atatctttgt tcattagagc gataaacttg aatttgagag ggaacttcca tggataaaaa 3960
gtacagtatt ggtctagaca taggaactaa ctctgttggg tgggctgtta taacagatga 4020
atataaagtt ccatcaaaaa aatttaaagt attaggaaac actgatagac attcaataaa 4080
aaaaaacttg ataggtgctt tattattcga ttcaggagag actgctgaag ctacacgttt 4140
aaaaagaaca gctagacgta gatatacaag aagaaaaaat aggatatgtt atcttcaaga 4200
aatttttagt aatgaaatgg caaaagttga tgattcattc tttcacagac tagaagaaag 4260
tttcttagtt gaagaagata agaagcatga aagacaccct atttttggta atatcgtaga 4320
tgaagtagca tatcatgaga agtatccaac tatctatcat ttaagaaaga aattagttga 4380
ttctacagat aaagctgatc tgagattaat atatttagct ttagctcata tgattaaatt 4440
tagaggacat tttttaatag aaggtgattt aaacccagac aacagcgatg tagataaatt 4500
atttatccaa ttagttcaaa cttataatca attattcgaa gagaatccaa ttaatgcaag 4560
tggtgtagac gctaaggcta tattatcagc tagattatca aaatctagaa gattagaaaa 4620
tctaatagct caacttcctg gagaaaagaa aaatggactt tttgggaacc taatagctct 4680
ctcactcgga ctaacaccaa attttaaaag caattttgat cttgctgaag acgcaaagtt 4740
acaactatca aaggatacat acgatgatga tttagataat ttgttagctc aaataggtga 4800
tcaatatgct gatttgtttc ttgcagcaaa aaacttaagt gatgcaattt tactatcaga 4860
tatacttaga gtaaatacag aaataacaaa ggctccttta tcagcaagta tgattaaacg 4920
atatgatgag catcatcaag atttaacatt attaaaggca cttgtaagac aacaattacc 4980
agaaaaatat aaagaaattt tctttgatca atctaaaaat ggatatgctg gatatataga 5040
cggtggagca agtcaagaag agttttataa atttataaag cctattttag aaaaaatgga 5100
tggaactgaa gaattacttg ttaaacttaa cagagaagat ttacttagaa aacaaagaac 5160
ttttgataat ggttcaattc ctcaccaaat tcatttagga gaattacatg ctatactaag 5220
aagacaagaa gatttttatc catttcttaa agataataga gaaaaaattg aaaaaatttt 5280
aacttttaga ataccatatt atgtaggacc acttgcaagg ggaaattcaa gatttgcatg 5340
gatgactaga aaatcagaag aaactataac cccgtggaat tttgaagaag tagtagataa 5400
aggagctagt gctcaatcat ttatagaaag aatgacaaat tttgataaga atcttcctaa 5460
cgaaaaggtt ttgccaaagc atagccttct ttatgagtat tttacagttt ataatgagct 5520

tactaaagta aaatacgtta cagaaggaat gagaaaacca gcatttttgt ctggtgaaca 5580

aaagaaagca atagtagacc tattatttaa aacaaatagg aaggttaccg taaagcaact 5640

taaagaagat tacttcaaaa aaattgaatg ctttgatagt gttgaaatat caggagttga 5700

agatagattt aatgcttcac ttggtacata tcacgatctc ttaaaaatta taaaagataa 5760

ggatttttta gataatgaag aaaatgaaga tattcttgaa gatatagtat taacattgac 5820

actttttgaa gatagagaaa tgatagaaga aagattaaaa acatatgcac atctttttga 5880

tgataaggtt atgaagcaac ttaaaagaag aagatataca ggttggggac gtttgtcaag 5940

aaagctaatt aatggtatta gagataaaca atcaggaaag actattctcg attttcttaa 6000

atcagatgga tttgctaata gaaactttat gcaattaatt catgatgatt ctcttacttt 6060

caaagaggat attcaaaagg ctcaagtttc tggacaaggc gatagcttac acgaacacat 6120

tgctaacctt gcagggagcc ccgctatcaa aaaaggaatt ttacaaacag ttaaagttgt 6180

agatgaactt gttaaagtta tgggaagaca caaacctgag aatatagtta tagaaatggc 6240

cagagaaaat caaacaacac aaaaaggaca aaaaaattct agagagagaa tgaagagaat 6300

tgaagaagga ataaaagagc taggatcaca aatattaaaa gaacatccag ttgaaaatac 6360

tcaattgcaa aatgaaaagt tatatttgta ttacttacaa aatggaagag atatgtatgt 6420

tgatcaagaa ctcgatatta atagattaag tgactatgat gttgatcata ttgttcctca 6480

atcattttta aaagatgatt caatcgataa caaagtatta actagatcag ataaaaatag 6540

aggaaagtca gataatgtac catctgaaga agttgttaaa aaaatgaaga actattggag 6600

acaactttta aatgcaaagc taattacaca aagaaaattt gacaatttaa caaaagcaga 6660

aagaggagga ttaagcgaat tagacaaagc tggatttata aaaagacaac ttgttgagac 6720

aagacaaata actaagcatg ttgctcaaat acttgattca agaatgaata caaaatatga 6780

tgaaaatgat aaattaatca gagaagtaaa agtaataaca ttaaagtcaa aattagtatc 6840

agatttcaga aaggattttc aattttacaa agttcgtgaa ataaataact atcatcatgc 6900

tcatgatgca tacttaaatg ctgttgtagg aactgctctt attaagaaat atcctaaact 6960

agaaagcgaa tttgtttatg gagattataa agtttatgat gtgcgcaaaa tgatcgcgaa 7020

atccgaacaa gaaatcggta aggctacagc aaaatatttc ttttatagta atataatgaa 7080

tttttttaag acagaaataa ctttggctaa tggtgaaatc agaaaaagac cacttatcga 7140

aacaaatgga gagacaggag aaatagtatg ggataaagga agagattttg ctactgttag 7200

aaaagtacta agtatgccac aagtaaatat cgtaaagaaa actgaagttc aaactggagg 7260

tttctctaag gaatcaattt tacctaagag aaattcagat aagttaattg caaggaaaaa 7320

agattgggac ccaaaaaaat acggtggttt tgatagtcca acagttgcct atagtgttct 7380

tgtagtagcg aaagttgaga aaggtaagtc aaaaaagttg aaaagcgtaa aagaacttct 7440

tggtatcaca attatggaaa gatcttcatt tgaaaaaaat ccaattgact ttttagaagc 7500

taagggttat aaagaagtta aaaaggattt aatcataaaa ctaccaaagt atagtctatt 7560

tgaactcgaa aacggaagaa aacgaatgct cgctagcgca ggagaacttc aaaaaggaaa 7620

tgaacttgcg ctgccatcaa agtatgtaaa tttcttatat ttagcttctc attatgagaa 7680

attaaaagga tcaccagagg ataatgaaca aaagcaacta tttgtagaac aacacaaaca 7740

ttatttagat gaaataatag aacaaatatc tgaattttct aaaagagtta tacttgccga 7800

cgcaaatcta gataaggtgc tttcagcgta taataaacac agagataaac caataagaga 7860

acaagcagaa aacattatcc atctttttac attaactaat cttggtgcac cagctgcatt 7920

taagtacttt gatacaacaa tagatagaaa aagatacaca tctactaaag aagtattaga 7980

cgcaacttta atacatcaat ctattacagg gctttatgaa acaagaattg atttaagtca 8040

actaggcgga gattaagtcg acaaagtatt gttaaaaata actctgtaga attataaatt 8100

agttctacag agttattttt tgacccgggt atattgataa aaataataat agtgggtata 8160

attaagttgt taggaggtta gttagaatga tgtcaagatt agataaaagt aaagtgatta 8220

acagcgcatt agagctgctt aatgaggtcg gaatcgaagg tttaacaacc cgtaaactcg 8280

cccagaagct aggtgtagag cagcctacat tgtattggca tgtaaaaaat aagcgggctt 8340

tgctcgacgc cttagccatt gagatgttag ataggcacca tactcacttt tgccctttag 8400

aaggggaaag ctggcaagat tttttacgta ataacgctaa aagttttaga tgtgctttac 8460

taagtcatcg cgatggagca aaagtacatt taggtacacg gcctacagaa aaacagtatg 8520

aaactctcga aaatcaatta gcctttttat gccaacaagg tttttcacta gagaatgcat 8580

tatatgcact cagcgctgtg gggcatttta ctttaggttg cgtattggaa gatcaagagc 8640

atcaagtcgc taaagaagaa agggaaacac ctactactga tagtatgccg ccattattac 8700

gacaagctat cgaattattt gatcaccaag gtgcagagcc agccttctta ttcggccttg 8760

aattgatcat atgcggatta gaaaaacaac ttaaatgtga aagtgggtct taaaagcagc 8820

ataacctttt tccgtgatgg taacttcacg gtaaccaaga tgtcgagttg agctcttagt 8880

tcaactcact ttttaaggtg attgtttgca tgtcattata aaattcttct tcatcctcgt 8940

attcttgatt ccaaccgttt ttaaatgcag atatgaattt ttcaactatt gattcatttt 9000

cactttcaga aattacatac tcgtttccat cattattaac tctaataatt agctgtgtta 9060

tactattgct atccgtacca ctcaatttca ctgtgtaatc tttgtttttt atttctctaa 9120

ttaagtcatt aatattcatt tcagccctcc tgtgaaattg ttatccgctc acaattccac 9180

gtcgactacc gcggattcta gattctgcag tatcttcatg gtattcattt tttaatatca 9240

ttttaccctc ccaatacatt taaaataatt atgtattcat gaaacatgat tgtatattta 9300

agaaacataa ttccatataa atcatttttc aaaatagttt ttacccataa ttaaatgtta 9360

atatgtaaat taatctttta gaatagttaa aaagttctaa aatatgttat aatgtttctt 9420

ataatcttat aaattttaat aactaatata taaagatatt tctttaaaat attcttatat 9480

ttagaagaat ttattttaaa ataaaaagct tttatgttga taaactgctt tgcaaagctc 9540

tcatgtaaat gtttaatata agactactat aaaattggct aattttatag gttaggaggt 9600

agaaatgcaa atattgtgga aaaagtatgt taaagaaaac tttgaaatga atgtagatga 9660

atgtggtata gaacaaggta taccaggatt aggatataac tatgaagtat tgaaaaatgc 9720

tgttattcat tacgtaacta agggatatgg aacttttaaa tttaatggta aggtatataa 9780

cttaaaacaa ggtgatattt ttatactact aaaaggtatg caagttgagt atgtggcttc 9840

tattgatgat ccttgggaat actactggat aggatttagt ggttcaaatg ctaatgagta 9900

tttaaataga acttctatta ctaactcctg tgttgctaat tgtgaagaaa actcaaaaat 9960

tccacagata atattaaata tgtgcgaaat atcaaaaact tataatcctt caagatctga 10020

tgacatacta ttactaaaag aactttactc attattgtac gcacttatag aagaattccc 10080

aaaacctttt gaatacaaag ataaggaatt acacacatat attcaagatg ctcttaattt 10140

cattaattct aattacatgc atagcataac tgttcaagaa attgctgatt atgtgaactt 10200

aagtagaagt tatttatata aaatgttcat aaaaaacctt ggaatttctc ctcaaagata 10260

tttaataaac cttagaatgt acaaagccac ccttttatta aaaagcacta aacttcctat 10320

aggagaagtc gcaagtagtg taggttatag tgactccctg ttattttcaa aaactttttc 10380

aaaacatttt tcaatgtctc cactaaatta cagaaataat caagtaaata aaccaagtat 10440

ataaatttaa aatacagctt taaaacaaaa aaatttcaaa aataaaaagt ataacagagg 10500

cgtaaattaa aacctctgtt atactttttg agct 10534

<210> 24
<211> 5754
<212> DNA
<213> Artificial Sequence

<220>
<223> pEC750S-uppHR

<400> 24
ataaggtacc aggaattaga gcagcgctat gttcagatac atttagtgct catgcaacaa 60

gagaacataa taatgctaat atattaacta tgggtcaaag ggttgttgga gcaggtcttg 120

ctttagatat agtaaaaaca tttatatcag ctaaatttga aggagatagg caccaaaaaa 180

gaatagataa gatttcagat attgaaaaaa agtatacaca ttagaaaaaa gcagctatgc 240

tgcaaataag atcaatttat attagaaaaa agcagctatg ctgcaaataa gatcaattta 300

tattagaaaa aagcagctat gctgcaaata agatcaattt atattagaaa aaagcagcta 360
tgctacaaat aagatcaatt tatattagaa aaaagtagct atgctgcaac aatattaatt 420
tatattacta gaaagctaaa tggggtatat aaatataaag ggctataaat actaaaagca 480
aacttggagg aataataatg gtctagagct ggagatagat tatttggtac taagtaatta 540
gtaatctatt agaattaaaa gctatctaca taagtttctg aatgacccaa gataatttta 600
ctggggggaa tatagaaaat ggagagacga gataagaaaa attattactt ggatattgct 660
gaaacagttt tagagagagg aacctgtcta aggagaaact atggttctat aattgttaaa 720
aatgatgaaa taatttctac tggatacaca ggagcaccta gaggtagaaa aaattgcatg 780
gatttgaata gttgcataag agaaaagttg aaagttccaa gaggtactca ttatgagttg 840
tgtaggagtg tacatagtga agctaatgca ataataagcg cttcgagctc gaattcgtaa 900
tcatggtcat agctgtttcc tgtgtgaaat tgttatccgc tcacaattcc acacaacata 960
cgagccggaa gcataaagtg taaagcctgg ggtgcctaat gagtgagcta actcacatta 1020
attgcgttgc gctcactgcc cgctttccag tcgggaaacc tgtcgtgcca gctgcattaa 1080
tgaatcggcc aacgcgcggg gagaggcggt ttgcgtattg ggcgctcttc cgcttcctcg 1140
ctcactgact cgctgcgctc ggtcgttcgg ctgcggcgag cggtatcagc tcactcaaag 1200
gcggtaatac ggttatccac agaatcaggg gataacgcag gaaagaacat gtgagcaaaa 1260
ggccagcaaa aggccaggaa ccgtaaaaag gccgcgttgc tggcgttttt ccataggctc 1320
cgcccccctg acgagcatca caaaaatcga cgctcaagtc agaggtggcg aaacccgaca 1380
ggactataaa gataccaggc gtttccccct ggaagctccc tcgtgcgctc tcctgttccg 1440
accctgccgc ttaccggata cctgtccgcc tttctccctt cgggaagcgt ggcgctttct 1500
catagctcac gctgtaggta tctcagttcg gtgtaggtcg ttcgctccaa gctgggctgt 1560
gtgcacgaac cccccgttca gcccgaccgc tgcgccttat ccggtaacta tcgtcttgag 1620
tccaacccgg taagacacga cttatcgcca ctggcagcag ccactggtaa caggattagc 1680
agagcgaggt atgtaggcgg tgctacagag ttcttgaagt ggtggcctaa ctacggctac 1740
actagaagaa cagtatttgg tatctgcgct ctgctgaagc cagttacctt cggaaaaaga 1800
gttggtagct cttgatccgg caaacaaacc accgctggta gcggtggttt ttttgtttgc 1860
aagcagcaga ttacgcgcag aaaaaaagga tctcaagaag atcctttgat cttttctacg 1920
gggtctgacg ctcagtggaa cgaaaactca cgttaaggga ttttggtcat gagattatca 1980
aaaaggatct tcacctagat ccttttaaat taaaaatgaa gttttaaatc aatctaaagt 2040
atatatgagt aaacttggtc tgacagttac caaagctagc ttaatactag tatatactta 2100
atgtgataag tgtctgacag ctgaccggtc taaagaggtc cgccaatgaa atctataaat 2160
aaactaaatt aagtttattt aattaacaac tatggatata aaataggtac taatcaaaat 2220

agtgaggagg atatatttga atacatacga acaaattaat aaagtgaaaa aaatacttcg 2280

gaaacattta aaaaataacc ttattggtac ttacatgttt ggatcaggag ttgagagtgg 2340

actaaaacca aatagtgatc ttgacttttt agtcgtcgta tctgaaccat tgacagatca 2400

aagtaaagaa atacttatac aaaaaattag acctatttca aagaaaatag gagataaaag 2460

caacttacga tatattgaat taacaattat tattcagcaa gaaatggtac cgtggaatca 2520

tcctcccaaa caagaattta tttatggaga atggttacaa gagctttatg aacaaggata 2580

cattcctcag aaggaattaa attcagattt aaccataatg ctttaccaag caaaacgaaa 2640

aaataaaaga atatacggaa attatgactt agaggaatta ctacctgata ttccattttc 2700

tgatgtgaga agagccatta tggattcgtc agaggaatta atagataatt atcaggatga 2760

tgaaaccaac tctatattaa ctttatgccg tatgatttta actatggaca cgggtaaaat 2820

cataccaaaa gatattgcgg gaaatgcagt ggctgaatct tctccattag aacataggga 2880

gagaattttg ttagcagttc gtagttatct tggagagaat attgaatgga ctaatgaaaa 2940

tgtaaattta actataaact atttaaataa cagattaaaa aaattataaa aaaattgaaa 3000

aaatggtgga aacacttttt tcaatttttt tgttttatta tttaatattt gggaaatatt 3060

cattctaatt ggtaatcaga ttttagaagt tgttaacttc aggtttgtct gtaactaaaa 3120

actagtattt aacctaggat caaaaaaatt tccaataatc ccactctaag ccacaaacac 3180

gccctataaa atcccgcttt aatcccactt tgagacacat gtaatattac tttacgccct 3240

agtatagtga taatttttta cattcaatgc cacgcaaaaa aataaagggg cactataata 3300

aaagttcctt cggaactaac taaagtaaaa aattatcttt acaacctccc caaaaaaaag 3360

aacaggtaca aagtacccta taatacaagc gtaaaaaaaa tgagggtaaa aataaaaaaa 3420

taaaaaaata aaaaaataaa aaaataaaaa aataaaaaaa taaaaaaata taaaaataaa 3480

aaaatataaa aataaaaaaa tataaaaata aaaaaataaa aaaatataaa aataaaaaaa 3540

taaaaaaata taaaaatatt ttttatttaa agtttgaaaa aaattttttt atattatata 3600

atctttgaag aaaagaatat aaaaaatgag cctttataaa agcccatttt ttttcatata 3660

cgtaatatga cgttctaatg tttttattgg tacttctaac attagagtaa tttctttatt 3720

tttaaagcct ttttctttaa gggcttttat tttttttctt aatacattta attcctcttt 3780

ttttgttgct tttcctttag cttttaattg ctcttgataa ttttttttac ctctaatatt 3840

ttctcttctc ttatattcct ttttagaaat tattattgtc atatattttt gttcttcttc 3900

tgtaatttct aataactcta taagagtttc attcttatac ttatattgct tatttttatc 3960

taaataacat ctttcagcac ttctagttgc tcttataact tctctttcac ttaaatgttg 4020

tctaaacata ctattaagtt ctaaaacatc atttaatgcc ttctcaatgt cttctgtaaa 4080

gctacaaaga taatatctat ataaaaataa tataagctct ctgtgtcctt ttaaatcata 4140

ttctcttagt tcacaaagtt ttattatgtc ttgtattctt ccataatata aacttctttc 4200

tctataaata taatttattt tgcttggtct accctttttc ctttcatatg gttttaattc 4260

aggtaaaaat ccattttgta tttctcttaa gtcataaata tattcgtact catctaatat 4320

attgactact gtttttgatt tagagtttat acttcctgga actcttaata ttctcgttgc 4380

atctaaggct tgtctatctg ctccaaagta ttttaattga ttatataaat attcttgaac 4440

cgctttccat aatggtaatg ctttactagg tactgcattt attatccata ttaaatacat 4500

tcctcttcca ctatctatta catagtttgg tataggaata ctttgattaa aataattctt 4560

ttctaagtcc attaatacct ggtctttagt tttgccagtt ttataataat ccaagtctat 4620

aaacagtgta tttaactctt ttatattttc taatcgccta cacggcttat aaaaggtatt 4680

tagagttata tagatatttt catcactcat atctaaatct tttaattcag cgtatttata 4740

gtgccattgg ctatatcctt ttttatctat aacgctcctg gttatccacc ctttacttct 4800

actatgaata ttatctatat agttcttttt attcagcttt aatgcgtttc tcacttattc 4860

acctcccctt ctgtaaaact aagaaaatta tatcatattt tcaataatta ttaactattc 4920

ttaaactctt aataaaaaat agagtaagtc cccaattgaa acttaatcta ttttttatgt 4980

tttaatttat tatttttatt aaaatatttt aaactaaatt aaatgattct ttttaatttt 5040

ttactatttc attccataat atattactat aattatttac aaataatatt tcttcatttg 5100

taatatttag atgatttact aattttagtt tttatatatt aaataattaa tgtataattt 5160

atataaaaaa tcaaaggagc ttataaatta tgattatttc caaagatact aaagatttaa 5220

tttttttcaa ttttaacaat actttttgta atattatgtt taaatttaat tgtatttttt 5280

tcatataata aagccgttga agtaaaccaa tccattttcc ttatgatgtt attattaaat 5340

ttaagtttta taataatatc tttattatat ttattgtttt taaaaaaact agtgaaattt 5400

ctagtgaaat ttccggcttt attaaactta tttttaggaa ttttattttc attttcatct 5460

ttacaggatt tgattatatc tttaaatatg ttttatcaaa tattatcttt ttctaaattt 5520

atatatattt ttattatatt tattattata tatattttat ttttaagttt ctttctaaca 5580

gctattaaaa agaaacttaa aaataaaaac acgtactcta aaccaataaa taaaactatt 5640

tttattattg ctgccttgat tggaatagtt tttagtaaaa ttaatttcaa tattccacaa 5700

tattatatta taagctagca ggcctcgaga tctccatgga cgcgtgacgt cgac 5754

<210> 25
<211> 884
<212> DNA
<213> Artificial Sequence

<220>

<223>  Matrice de réparation

<400>  25
ataaggtacc aggaattaga gcagcgctat gttcagatac atttagtgct catgcaacaa      60

gagaacataa taatgctaat atattaacta tgggtcaaag ggttgttgga gcaggtcttg     120

ctttagatat agtaaaaaca tttatatcag ctaaatttga aggagatagg caccaaaaaa     180

gaatagataa gatttcagat attgaaaaaa agtatacaca ttagaaaaaa gcagctatgc     240

tgcaaataag atcaatttat attagaaaaa agcagctatg ctgcaaataa gatcaattta     300

tattagaaaa aagcagctat gctgcaaata agatcaattt atattagaaa aaagcagcta     360

tgctacaaat aagatcaatt tatattagaa aaaagtagct atgctgcaac aatattaatt     420

tatattacta gaaagctaaa tggggtatat aaatataaag ggctataaat actaaaagca     480

aacttggagg aataataatg gtctagagct ggagatagat tatttggtac taagtaatta     540

gtaatctatt agaattaaaa gctatctaca taagtttctg aatgacccaa gataatttta     600

ctggggggaa tatagaaaat ggagagacga gataagaaaa attattactt ggatattgct     660

gaaacagttt tagagagagg aacctgtcta aggagaaact atggttctat aattgttaaa     720

aatgatgaaa taatttctac tggatacaca ggagcaccta gaggtagaaa aaattgcatg     780

gatttgaata gttgcataag agaaaagttg aaagttccaa gaggtactca ttatgagttg     840

tgtaggagtg tacatagtga agctaatgca ataataagcg cttc                      884

<210>  26
<211>  500
<212>  DNA
<213>  Artificial Sequence

<220>
<223>  Fragment amont gène upp

<400>  26
ataaggtacc aggaattaga gcagcgctat gttcagatac atttagtgct catgcaacaa      60

gagaacataa taatgctaat atattaacta tgggtcaaag ggttgttgga gcaggtcttg     120

ctttagatat agtaaaaaca tttatatcag ctaaatttga aggagatagg caccaaaaaa     180

gaatagataa gatttcagat attgaaaaaa agtatacaca ttagaaaaaa gcagctatgc     240

tgcaaataag atcaatttat attagaaaaa agcagctatg ctgcaaataa gatcaattta     300

tattagaaaa aagcagctat gctgcaaata agatcaattt atattagaaa aaagcagcta     360

tgctacaaat aagatcaatt tatattagaa aaaagtagct atgctgcaac aatattaatt     420

tatattacta gaaagctaaa tggggtatat aaatataaag ggctataaat actaaaagca     480

aacttggagg aataataatg                                                 500

<210>  27

<211>  377
<212>  DNA
<213>  Artificial Sequence

<220>
<223>  Fragment aval gène upp

<400>  27
gctggagata gattatttgg tactaagtaa ttagtaatct attagaatta aaagctatct      60

acataagttt ctgaatgacc caagataatt ttactggggg gaatatagaa aatggagaga     120

cgagataaga aaaattatta cttggatatt gctgaaacag ttttagagag aggaacctgt     180

ctaaggagaa actatggttc tataattgtt aaaaatgatg aaataatttc tactggatac     240

acaggagcac ctagaggtag aaaaaattgc atggatttga atagttgcat aagagaaaag     300

ttgaaagttc caagaggtac tcattatgag ttgtgtagga gtgtacatag tgaagctaat     360

gcaataataa gcgcttc                                                    377


<210>  28
<211>  2666
<212>  DNA
<213>  Artificial Sequence

<220>
<223>  pEX-A2-gRNA-upp

<400>  28
ctcgagtatt tttgataaaa gcaatgatta acatggtttg acgtctgaga agagacgatt      60

ttctcaatag gagaaattaa ggtgcaaacc cttatcattc caccatgatc cacctgtagc     120

aagcatgttt tagagctaga aatagcaagt taaaataagg ctagtccgtt atcaacttga     180

aaaagtggca ccgagtcggt gctttttttg ccatggacct gcttttgctc gcttggatcc     240

gaattcctgt gtgaaattgt tatccgctca caattccaca caacatacga gccggaagca     300

taaagtgtaa agcctggggt gcctaatgag tgagctaact cacattaatt gcgttgcgct     360

cactgcccgc tttccagtcg ggaaacctgt cgtgccagct gcattaatga atcggccaac     420

gcgcggggag aggcggtttg cgtattgggc gctcttccgc ttcctcgctc actgactcgc     480

tgcgctcggt cgttcggctg cggcgagcgg tatcagctca ctcaaaggcg gtaatacggt     540

tatccacaga atcaggggat aacgcaggaa agaacatgtg agcaaaaggc cagcaaaagg     600

ccaggaaccg taaaaaggcc gcgttgctgg cgtttttcca taggctccgc ccccctgacg     660

agcatcacaa aaatcgacgc tcaagtcaga ggtggcgaaa cccgacagga ctataaagat     720

accaggcgtt tccccctgga agctccctcg tgcgctctcc tgttccgacc ctgccgctta     780

ccggatacct gtccgccttt ctcccttcgg gaagcgtggc gctttctcat agctcacgct     840

gtaggtatct cagttcggtg taggtcgttc gctccaagct gggctgtgtg cacgaacccc     900

ccgttcagcc cgaccgctgc gccttatccg gtaactatcg tcttgagtcc aacccggtaa     960

gacacgactt atcgccactg gcagcagcca ctggtaacag gattagcaga gcgaggtatg 1020

taggcggtgc tacagagttc ttgaagtggt ggcctaacta cggctacact agaagaacag 1080

tatttggtat ctgcgctctg ctgaagccag ttaccttcgg aaaaagagtt ggtagctctt 1140

gatccggcaa acaaaccacc gctggtagcg gtggtttttt tgtttgcaag cagcagatta 1200

cgcgcagaaa aaaaggatct caagaagatc ctttgatctt ttctacgggg tctgacgctc 1260

agtggaacga aaactcacgt taagggattt tggtcatgag attatcaaaa aggatcttca 1320

cctagatcct tttaaattaa aaatgaagtt ttaaatcaat ctaaagtata tatgagtaaa 1380

cttggtctga cagttaccaa tgcttaatca gtgaggcacc tatctcagcg atctgtctat 1440

ttcgttcatc catagttgcc tgactccccg tcgtgtagat aactacgata cgggagggct 1500

taccatctgg ccccagtgct gcaatgatac cgcgagaccc acgctcaccg gctccagatt 1560

tatcagcaat aaaccagcca gccggaaggg ccgagcgcag aagtggtcct gcaactttat 1620

ccgcctccat ccagtctatt aattgttgcc gggaagctag agtaagtagt tcgccagtta 1680

atagtttgcg caacgttgtt gccattgcta caggcatcgt ggtgtcacgc tcgtcgtttg 1740

gtatggcttc attcagctcc ggttcccaac gatcaaggcg agttacatga tcccccatgt 1800

tgtgcaaaaa agcggttagc tccttcggtc ctccgatcgt tgtcagaagt aagttggccg 1860

cagtgttatc actcatggtt atggcagcac tgcataattc tcttactgtc atgccatccg 1920

taagatgctt ttctgtgact ggtgagtact caaccaagtc attctgagaa tagtgtatgc 1980

ggcgaccgag ttgctcttgc ccggcgtcaa tacgggataa taccgcgcca catagcagaa 2040

ctttaaaagt gctcatcatt ggaaaacgtt cttcggggcg aaaactctca aggatcttac 2100

cgctgttgag atccagttcg atgtaaccca ctcgtgcacc caactgatct tcagcatctt 2160

ttactttcac cagcgtttct gggtgagcaa aaacaggaag gcaaaatgcc gcaaaaaagg 2220

gaataagggc gacacggaaa tgttgaatac tcatactctt cctttttcaa tattattgaa 2280

gcatttatca gggttattgt ctcatgagcg gatacatatt tgaatgtatt tagaaaaata 2340

aacaaatagg ggttccgcgc acatttcccc gaaaagtgcc acctgacgtc taagaaacca 2400

ttattatcat gacattaacc tataaaaata ggcgtatcac gaggcccttt cgtctcgcgc 2460

gtttcggtga tgacggtgaa aacctctgac acatgcagct cccggagacg gtcacagctt 2520

gtctgtaagc ggatgccggg agcagacaag cccgtcaggg cgcgtcagcg ggtgttggcg 2580

ggtgtcgggg ctggcttaac tatgcggcat cagagcagat tgtactgaga gtgcaccaat 2640

tgggtaccga gctcgcggcc gcaagc 2666

<210> 29
<211> 203
<212> DNA

<213>  Artificial Sequence

<220>
<223>  Cassette d'expression d'un ARNg

<400>  29
tatttttgat aaaagcaatg attaacatgg tttgacgtct gagaagagac gattttctca      60

ataggagaaa ttaaggtgca aacccttatc attccaccat gatccacctg tagcaagcat     120

gttttagagc tagaaatagc aagttaaaat aaggctagtc cgttatcaac ttgaaaaagt     180

ggcaccgagt cggtgctttt ttt                                             203


<210>  30
<211>  100
<212>  DNA
<213>  Artificial Sequence

<220>
<223>  Promoteur constitutif

<400>  30
tatttttgat aaaagcaatg attaacatgg tttgacgtct gagaagagac gattttctca      60

ataggagaaa ttaaggtgca aacccttatc attccaccat                           100


<210>  31
<211>  20
<212>  DNA
<213>  Artificial Sequence

<220>
<223>  Protospacer ciblant upp

<400>  31
gatccacctg tagcaagcat                                                  20


<210>  32
<211>  5954
<212>  DNA
<213>  Artificial Sequence

<220>
<223>  pEC750S-deltaupp

<400>  32
ataaggtacc aggaattaga gcagcgctat gttcagatac atttagtgct catgcaacaa      60

gagaacataa taatgctaat atattaacta tgggtcaaag ggttgttgga gcaggtcttg     120

ctttagatat agtaaaaaca tttatatcag ctaaatttga aggagatagg caccaaaaaa     180

gaatagataa gatttcagat attgaaaaaa agtatacaca ttagaaaaaa gcagctatgc     240

tgcaaataag atcaatttat attagaaaaa agcagctatg ctgcaaataa gatcaattta     300

tattagaaaa aagcagctat gctgcaaata agatcaattt atattagaaa aaagcagcta     360

tgctacaaat aagatcaatt tatattagaa aaaagtagct atgctgcaac aatattaatt     420

tatattacta gaaagctaaa tggggtatat aaatataaag ggctataaat actaaaagca 480

aacttggagg aataataatg gtctagagct ggagatagat tatttggtac taagtaatta 540

gtaatctatt agaattaaaa gctatctaca taagtttctg aatgacccaa gataatttta 600

ctggggggaa tatagaaaat ggagagacga gataagaaaa attattactt ggatattgct 660

gaaacagttt tagagagagg aacctgtcta aggagaaact atggttctat aattgttaaa 720

aatgatgaaa taatttctac tggatacaca ggagcaccta gaggtagaaa aaattgcatg 780

gatttgaata gttgcataag agaaaagttg aaagttccaa gaggtactca ttatgagttg 840

tgtaggagtg tacatagtga agctaatgca ataataagcg cttcgagctc gaattcgtaa 900

tcatggtcat agctgtttcc tgtgtgaaat tgttatccgc tcacaattcc acacaacata 960

cgagccggaa gcataaagtg taaagcctgg ggtgcctaat gagtgagcta actcacatta 1020

attgcgttgc gctcactgcc cgctttccag tcgggaaacc tgtcgtgcca gctgcattaa 1080

tgaatcggcc aacgcgcggg gagaggcggt ttgcgtattg ggcgctcttc cgcttcctcg 1140

ctcactgact cgctgcgctc ggtcgttcgg ctgcggcgag cggtatcagc tcactcaaag 1200

gcggtaatac ggttatccac agaatcaggg gataacgcag gaaagaacat gtgagcaaaa 1260

ggccagcaaa aggccaggaa ccgtaaaaag gccgcgttgc tggcgttttt ccataggctc 1320

cgcccccctg acgagcatca caaaaatcga cgctcaagtc agaggtggcg aaacccgaca 1380

ggactataaa gataccaggc gtttccccct ggaagctccc tcgtgcgctc tcctgttccg 1440

accctgccgc ttaccggata cctgtccgcc tttctccctt cgggaagcgt ggcgctttct 1500

catagctcac gctgtaggta tctcagttcg gtgtaggtcg ttcgctccaa gctgggctgt 1560

gtgcacgaac cccccgttca gcccgaccgc tgcgccttat ccggtaacta tcgtcttgag 1620

tccaacccgg taagacacga cttatcgcca ctggcagcag ccactggtaa caggattagc 1680

agagcgaggt atgtaggcgg tgctacagag ttcttgaagt ggtggcctaa ctacggctac 1740

actagaagaa cagtatttgg tatctgcgct ctgctgaagc cagttacctt cggaaaaaga 1800

gttggtagct cttgatccgg caaacaaacc accgctggta gcggtggttt ttttgtttgc 1860

aagcagcaga ttacgcgcag aaaaaaagga tctcaagaag atcctttgat cttttctacg 1920

gggtctgacg ctcagtggaa cgaaaactca cgttaaggga ttttggtcat gagattatca 1980

aaaaggatct tcacctagat ccttttaaat taaaaatgaa gttttaaatc aatctaaagt 2040

atatatgagt aaacttggtc tgacagttac caaagctagc ttaatactag tatatactta 2100

atgtgataag tgtctgacag ctgaccggtc taaagaggtc cgccaatgaa atctataaat 2160

aaactaaatt aagtttattt aattaacaac tatggatata aaataggtac taatcaaaat 2220

agtgaggagg atatatttga atacatacga acaaattaat aaagtgaaaa aaatacttcg 2280

gaaacattta aaaaataacc ttattggtac ttacatgttt ggatcaggag ttgagagtgg 2340

actaaaacca aatagtgatc ttgacttttt agtcgtcgta tctgaaccat tgacagatca 2400

aagtaaagaa atacttatac aaaaaattag acctatttca aagaaaatag gagataaaag 2460

caacttacga tatattgaat taacaattat tattcagcaa gaaatggtac cgtggaatca 2520

tcctcccaaa caagaattta tttatggaga atggttacaa gagctttatg aacaaggata 2580

cattcctcag aaggaattaa attcagattt aaccataatg ctttaccaag caaaacgaaa 2640

aaataaaaga atatacggaa attatgactt agaggaatta ctacctgata ttccattttc 2700

tgatgtgaga agagccatta tggattcgtc agaggaatta atagataatt atcaggatga 2760

tgaaaccaac tctatattaa ctttatgccg tatgatttta actatggaca cgggtaaaat 2820

cataccaaaa gatattgcgg gaaatgcagt ggctgaatct tctccattag aacataggga 2880

gagaattttg ttagcagttc gtagttatct tggagagaat attgaatgga ctaatgaaaa 2940

tgtaaattta actataaact atttaaataa cagattaaaa aaattataaa aaaattgaaa 3000

aaatggtgga aacacttttt tcaatttttt tgttttatta tttaatattt gggaaatatt 3060

cattctaatt ggtaatcaga ttttagaagt tgttaacttc aggtttgtct gtaactaaaa 3120

actagtattt aacctaggat caaaaaaatt tccaataatc ccactctaag ccacaaacac 3180

gccctataaa atcccgcttt aatcccactt tgagacacat gtaatattac tttacgccct 3240

agtatagtga taatttttta cattcaatgc cacgcaaaaa aataaagggg cactataata 3300

aaagttcctt cggaactaac taaagtaaaa aattatcttt acaacctccc caaaaaaaag 3360

aacaggtaca aagtacccta taatacaagc gtaaaaaaaa tgagggtaaa aataaaaaaa 3420

taaaaaaata aaaaaataaa aaaataaaaa aataaaaaaa taaaaaaata taaaaataaa 3480

aaaatataaa aataaaaaaa tataaaaata aaaaaataaa aaaatataaa aataaaaaaa 3540

taaaaaaata taaaaatatt ttttatttaa agtttgaaaa aaattttttt atattatata 3600

atctttgaag aaaagaatat aaaaaatgag cctttataaa agcccatttt ttttcatata 3660

cgtaatatga cgttctaatg tttttattgg tacttctaac attagagtaa tttctttatt 3720

tttaaagcct ttttctttaa gggcttttat tttttttctt aatacattta attcctcttt 3780

ttttgttgct tttcctttag cttttaattg ctcttgataa ttttttttac ctctaatatt 3840

ttctcttctc ttatattcct ttttagaaat tattattgtc atatattttt gttcttcttc 3900

tgtaatttct aataactcta taagagtttc attcttatac ttatattgct tatttttatc 3960

taaataacat ctttcagcac ttctagttgc tcttataact tctctttcac ttaaatgttg 4020

tctaaacata ctattaagtt ctaaaacatc atttaatgcc ttctcaatgt cttctgtaaa 4080

gctacaaaga taatatctat ataaaaataa tataagctct ctgtgtcctt ttaaatcata 4140

ttctcttagt tcacaaagtt ttattatgtc ttgtattctt ccataatata aacttctttc 4200

tctataaata taatttattt tgcttggtct accctttttc ctttcatatg gttttaattc 4260

aggtaaaaat ccattttgta tttctcttaa gtcataaata tattcgtact catctaatat 4320

attgactact gtttttgatt tagagtttat acttcctgga actcttaata ttctcgttgc 4380

atctaaggct tgtctatctg ctccaaagta ttttaattga ttatataaat attcttgaac 4440

cgctttccat aatggtaatg ctttactagg tactgcattt attatccata ttaaatacat 4500

tcctcttcca ctatctatta catagtttgg tataggaata ctttgattaa aataattctt 4560

ttctaagtcc attaatacct ggtctttagt tttgccagtt ttataataat ccaagtctat 4620

aaacagtgta tttaactctt ttatattttc taatcgccta cacggcttat aaaaggtatt 4680

tagagttata tagatatttt catcactcat atctaaatct tttaattcag cgtatttata 4740

gtgccattgg ctatatcctt ttttatctat aacgctcctg gttatccacc ctttacttct 4800

actatgaata ttatctatat agttcttttt attcagcttt aatgcgtttc tcacttattc 4860

acctcccctt ctgtaaaact aagaaaatta tatcatattt tcaataatta ttaactattc 4920

ttaaactctt aataaaaaat agagtaagtc cccaattgaa acttaatcta ttttttatgt 4980

tttaatttat tatttttatt aaaatatttt aaactaaatt aaatgattct ttttaatttt 5040

ttactatttc attccataat atattactat aattatttac aaataatatt tcttcatttg 5100

taatatttag atgatttact aattttagtt tttatatatt aaataattaa tgtataattt 5160

atataaaaaa tcaaaggagc ttataaatta tgattatttc caaagatact aaagatttaa 5220

tttttttcaa ttttaacaat actttttgta atattatgtt taaatttaat tgtatttttt 5280

tcatataata aagccgttga agtaaaccaa tccattttcc ttatgatgtt attattaaat 5340

ttaagtttta taataatatc tttattatat ttattgtttt taaaaaaact agtgaaattt 5400

ctagtgaaat ttccggcttt attaaactta tttttaggaa ttttattttc attttcatct 5460

ttacaggatt tgattatatc tttaaatatg ttttatcaaa tattatcttt ttctaaattt 5520

atatatattt ttattatatt tattattata tatattttat ttttaagttt ctttctaaca 5580

gctattaaaa agaaacttaa aaataaaaac acgtactcta aaccaataaa taaaactatt 5640

tttattattg ctgccttgat tggaatagtt tttagtaaaa ttaatttcaa tattccacaa 5700

tattatatta taagctagca cgcctcgagt atttttgata aaagcaatga ttaacatggt 5760

ttgacgtctg agaagagacg attttctcaa taggagaaat taaggtgcaa acccttatca 5820

ttccaccatg atccacctgt agcaagcatg ttttagagct agaaatagca agttaaaata 5880

aggctagtcc gttatcaact tgaaaaagtg gcaccgagtc ggtgcttttt ttgccatgga 5940

cgcgtgacgt cgac 5954

<210> 33

<211>  5853
<212>  DNA
<213>  Artificial Sequence

<220>
<223>  pEC750C-deltaupp

<400>  33
atcaaaaaaa tttccaataa tcccactcta agccacaaac acgccctata aaatcccgct      60

ttaatcccac tttgagacac atgtaatatt actttacgcc ctagtatagt gataattttt     120

tacattcaat gccacgcaaa aaaataaagg ggcactataa taaaagttcc ttcggaacta     180

actaaagtaa aaaattatct ttacaacctc cccaaaaaaa agaacaggta caaagtaccc     240

tataatacaa gcgtaaaaaa aatgagggta aaaataaaaa aataaaaaaa taaaaaaata     300

aaaaaataaa aaaataaaaa aataaaaaaa tataaaaata aaaaaatata aaaataaaaa     360

aatataaaaa taaaaaaata aaaaaatata aaaataaaaa aataaaaaaa tataaaaata     420

ttttttattt aaagtttgaa aaaaattttt ttatattata taatctttga agaaaagaat     480

ataaaaaatg agcctttata aaagcccatt ttttttcata tacgtaatat gacgttctaa     540

tgtttttatt ggtacttcta acattagagt aatttcttta tttttaaagc ctttttcttt     600

aagggctttt attttttttc ttaatacatt taattcctct ttttttgttg cttttccttt     660

agcttttaat tgctcttgat aatttttttt acctctaata ttttctcttc tcttatattc     720

ctttttagaa attattattg tcatatattt ttgttcttct tctgtaattt ctaataactc     780

tataagagtt tcattcttat acttatattg cttattttta tctaaataac atctttcagc     840

acttctagtt gctcttataa cttctctttc acttaaatgt tgtctaaaca tactattaag     900

ttctaaaaca tcatttaatg ccttctcaat gtcttctgta aagctacaaa gataatatct     960

atataaaaat aatataagct ctctgtgtcc ttttaaatca tattctctta gttcacaaag    1020

ttttattatg tcttgtattc ttccataata taaacttctt tctctataaa tataatttat    1080

tttgcttggt ctaccctttt tcctttcata tggttttaat tcaggtaaaa atccattttg    1140

tatttctctt aagtcataaa tatattcgta ctcatctaat atattgacta ctgtttttga    1200

tttagagttt atacttcctg gaactcttaa tattctcgtt gcatctaagg cttgtctatc    1260

tgctccaaag tattttaatt gattatataa atattcttga accgctttcc ataatggtaa    1320

tgctttacta ggtactgcat ttattatcca tattaaatac attcctcttc cactatctat    1380

tacatagttt ggtataggaa tactttgatt aaaataattc ttttctaagt ccattaatac    1440

ctggtcttta gttttgccag ttttataata atccaagtct ataaacagtg tatttaactc    1500

ttttatattt tctaatcgcc tacacggctt ataaaaggta tttagagtta tatagatatt    1560

ttcatcactc atatctaaat cttttaattc agcgtattta tagtgccatt ggctatatcc    1620

ttttttatct ataacgctcc tggttatcca ccctttactt ctactatgaa tattatctat    1680

atagttcttt ttattcagct ttaatgcgtt tctcacttat tcacctcccc ttctgtaaaa 1740

ctaagaaaat tatatcatat tttcaataat tattaactat tcttaaactc ttaataaaaa 1800

atagagtaag tccccaattg aaacttaatc tattttttat gttttaattt attattttta 1860

ttaaaatatt ttaaactaaa ttaaatgatt ctttttaatt ttttactatt tcattccata 1920

atatattact ataattattt acaaataata tttcttcatt tgtaatattt agatgattta 1980

ctaattttag tttttatata ttaaataatt aatgtataat ttatataaaa aatcaaagga 2040

gcttataaat tatgattatt tccaaagata ctaaagattt aatttttttc aattttaaca 2100

atactttttg taatattatg tttaaattta attgtatttt tttcatataa taaagccgtt 2160

gaagtaaacc aatccatttt ccttatgatg ttattattaa atttaagttt tataataata 2220

tctttattat atttattgtt tttaaaaaaa ctagtgaaat ttctagtgaa atttccggct 2280

ttattaaact tatttttagg aattttattt tcattttcat ctttacagga tttgattata 2340

tctttaaata tgttttatca aatattatct ttttctaaat ttatatatat ttttattata 2400

tttattatta tatatatttt atttttaagt ttctttctaa cagctattaa aaagaaactt 2460

aaaaataaaa acacgtactc taaaccaata aataaaacta tttttattat tgctgccttg 2520

attggaatag tttttagtaa aattaatttc aatattccac aatattatat tataagctag 2580

cacgcctcga gtatttttga taaaagcaat gattaacatg gtttgacgtc tgagaagaga 2640

cgattttctc aataggagaa attaaggtgc aaacccttat cattccacca tgatccacct 2700

gtagcaagca tgttttagag ctagaaatag caagttaaaa taaggctagt ccgttatcaa 2760

cttgaaaaag tggcaccgag tcggtgcttt ttttgccatg gacgcgtgac gtcgacataa 2820

ggtaccagga attagagcag cgctatgttc agatacattt agtgctcatg caacaagaga 2880

acataataat gctaatatat taactatggg tcaaagggtt gttggagcag gtcttgcttt 2940

agatatagta aaaacattta tatcagctaa atttgaagga gataggcacc aaaaaagaat 3000

agataagatt tcagatattg aaaaaaagta tacacattag aaaaaagcag ctatgctgca 3060

aataagatca atttatatta gaaaaaagca gctatgctgc aaataagatc aatttatatt 3120

agaaaaaagc agctatgctg caaataagat caatttatat tagaaaaaag cagctatgct 3180

acaaataaga tcaatttata ttagaaaaaa gtagctatgc tgcaacaata ttaatttata 3240

ttactagaaa gctaaatggg gtatataaat ataaagggct ataaatacta aaagcaaact 3300

tggaggaata ataatggtct agagctggag atagattatt tggtactaag taattagtaa 3360

tctattagaa ttaaaagcta tctacataag tttctgaatg acccaagata attttactgg 3420

ggggaatata gaaaatggag agacgagata agaaaaatta ttacttggat attgctgaaa 3480

cagttttaga gagaggaacc tgtctaagga gaaactatgg ttctataatt gttaaaaatg 3540

atgaaataat ttctactgga tacacaggag cacctagagg tagaaaaaat tgcatggatt 3600

tgaatagttg cataagagaa aagttgaaag ttccaagagg tactcattat gagttgtgta 3660

ggagtgtaca tagtgaagct aatgcaataa taagcgcttc gagctcgaat tcgtaatcat 3720

ggtcatagct gtttcctgtg tgaaattgtt atccgctcac aattccacac aacatacgag 3780

ccggaagcat aaagtgtaaa gcctggggtg cctaatgagt gagctaactc acattaattg 3840

cgttgcgctc actgcccgct ttccagtcgg gaaacctgtc gtgccagctg cattaatgaa 3900

tcggccaacg cgcggggaga ggcggtttgc gtattgggcg ctcttccgct tcctcgctca 3960

ctgactcgct gcgctcggtc gttcggctgc ggcgagcggt atcagctcac tcaaaggcgg 4020

taatacggtt atccacagaa tcaggggata acgcaggaaa gaacatgtga gcaaaaggcc 4080

agcaaaaggc caggaaccgt aaaaaggccg cgttgctggc gtttttccat aggctccgcc 4140

cccctgacga gcatcacaaa aatcgacgct caagtcagag gtggcgaaac ccgacaggac 4200

tataaagata ccaggcgttt ccccctggaa gctccctcgt gcgctctcct gttccgaccc 4260

tgccgcttac cggatacctg tccgcctttc tcccttcggg aagcgtggcg ctttctcata 4320

gctcacgctg taggtatctc agttcggtgt aggtcgttcg ctccaagctg ggctgtgtgc 4380

acgaaccccc cgttcagccc gaccgctgcg ccttatccgg taactatcgt cttgagtcca 4440

acccggtaag acacgactta tcgccactgg cagcagccac tggtaacagg attagcagag 4500

cgaggtatgt aggcggtgct acagagttct tgaagtggtg gcctaactac ggctacacta 4560

gaagaacagt atttggtatc tgcgctctgc tgaagccagt taccttcgga aaaagagttg 4620

gtagctcttg atccggcaaa caaaccaccg ctggtagcgg tggttttttt gtttgcaagc 4680

agcagattac gcgcagaaaa aaaggatctc aagaagatcc tttgatcttt tctacggggt 4740

ctgacgctca gtggaacgaa aactcacgtt aagggatttt ggtcatgaga ttatcaaaaa 4800

ggatcttcac ctagatcctt ttaaattaaa aatgaagttt taaatcaatc taaagtatat 4860

atgagtaaac ttggtctgac agttaccaaa gctagcttaa tactagtata tacttaatgt 4920

gataagtgtc tgacagctga ccggtctaaa gaggtcccta gcgcctacgg ggaatttgta 4980

tcgataaggg gtacaaattc ccactaagcg ctcggccggg gatcgatccc cgggtacgta 5040

cccggcagtt tttctttttc ggcaagtgtt caagaagtta ttaagtcggg agtgcagtcg 5100

aagtgggcaa gttgaaaaat tcacaaaaat gtggtataat atctttgttc attagagcga 5160

taaacttgaa tttgagaggg aacttagatg gtatttgaaa aaattgataa aaatagttgg 5220

aacagaaaag agtattttga ccactacttt gcaagtgtac cttgtaccta cagcatgacc 5280

gttaaagtgg atatcacaca aataaaggaa aagggaatga aactatatcc tgcaatgctt 5340

tattatattg caatgattgt aaaccgccat tcagagttta ggacggcaat caatcaagat 5400

ggtgaattgg ggatatatga tgagatgata ccaagctata caatatttca caatgatact 5460

gaaacatttt ccagcctttg gactgagtgt aagtctgact ttaaatcatt tttagcagat 5520

tatgaaagtg atacgcaacg gtatggaaac aatcatagaa tggaaggaaa gccaaatgct 5580

ccggaaaaca tttttaatgt atctatgata ccgtggtcaa ccttcgatgg ctttaatctg 5640

aatttgcaga aaggatatga ttatttgatt cctattttta ctatggggaa atattataaa 5700

gaagataaca aaattatact tcctttggca attcaagttc atcacgcagt atgtgacgga 5760

tttcacattt gccgttttgt aaacgaattg caggaattga taaatagtta acttcaggtt 5820

tgtctgtaac taaaaactag tatttaacct agg 5853

<210> 34
<211> 4966
<212> DNA
<213> Artificial Sequence

<220>
<223> pGRNA-pNF2

<400> 34
agctcggtac ccggggatcc tctagagtcg acgtcacgcg tccatggaga tctcgaggcg 60

tgctagctta taatataata ttgtggaata ttgaaattaa ttttactaaa aactattcca 120

atcaaggcag caataataaa aatagtttta tttattggtt tagagtacgt gtttttattt 180

ttaagtttct ttttaatagc tgttagaaag aaacttaaaa ataaaatata tataataata 240

aatataataa aaatatatat aaatttagaa aaagataata tttgataaaa catatttaaa 300

gatataatca aatcctgtaa agatgaaaat gaaaataaaa ttcctaaaaa taagtttaat 360

aaagccggaa atttcactag aaatttcact agttttttta aaaacaataa atataataaa 420

gatattatta taaaacttaa atttaataat aacatcataa ggaaaatgga ttggtttact 480

tcaacggctt tattatatga aaaaaataca attaaattta aacataatat tacaaaaagt 540

attgttaaaa ttgaaaaaaa ttaaatcttt agtatctttg gaaataatca taatttataa 600

gctcctttga ttttttatat aaattataca ttaattattt aatatataaa aactaaaatt 660

agtaaatcat ctaaatatta caaatgaaga aatattattt gtaaataatt atagtaatat 720

attatggaat gaaatagtaa aaaattaaaa agaatcattt aatttagttt aaaatatttt 780

aataaaaata ataaattaaa acataaaaaa tagattaagt ttcaattggg gacttactct 840

attttttatt aagagtttaa gaatagttaa taattattga aaatatgata taattttctt 900

agttttacag aaggggaggt gaataagtga gaaacgcatt aaagctgaat aaaaagaact 960

atatagataa tattcatagt agaagtaaag ggtggataac caggagcgtt atagataaaa 1020

aaggatatag ccaatggcac tataaatacg ctgaattaaa agatttagat atgagtgatg 1080

aaaatatcta tataactcta aatacctttt ataagccgtg taggcgatta gaaaatataa 1140

aagagttaaa tacactgttt atagacttgg attattataa aactggcaaa actaaagacc 1200
aggtattaat ggacttagaa aagaattatt ttaatcaaag tattcctata ccaaactatg 1260
taatagatag tggaagagga atgtatttaa tatggataat aaatgcagta cctagtaaag 1320
cattaccatt atggaaagcg gttcaagaat atttatataa tcaattaaaa tactttggag 1380
cagatagaca agccttagat gcaacgagaa tattaagagt tccaggaagt ataaactcta 1440
aatcaaaaac agtagtcaat atattagatg agtacgaata tatttatgac ttaagagaaa 1500
tacaaaatgg atttttacct gaattaaaac catatgaaag gaaaaagggt agaccaagca 1560
aaataaatta tatttataga gaaagaagtt tatattatgg aagaatacaa gacataataa 1620
aactttgtga actaagagaa tatgatttaa aaggacacag agagcttata ttatttttat 1680
atagatatta tctttgtagc tttacagaag acattgagaa ggcattaaat gatgttttag 1740
aacttaatag tatgtttaga caacatttaa gtgaaagaga agttataaga gcaactagaa 1800
gtgctgaaag atgttattta gataaaaata agcaatataa gtataagaat gaaactctta 1860
tagagttatt agaaattaca gaagaagaac aaaaatatat gacaataata atttctaaaa 1920
aggaatataa gagaagagaa aatattagag gtaaaaaaaa ttatcaagag caattaaaag 1980
ctaaaggaaa agcaacaaaa aaagaggaat taaatgtatt aagaaaaaaa ataaaagccc 2040
ttaaagaaaa aggctttaaa aataaagaaa ttactctaat gttagaagta ccaataaaaa 2100
cattagaacg tcatattacg tatatgaaaa aaaatgggct tttataaagg ctcatttttt 2160
atattctttt cttcaaagat tatataatat aaaaaaattt ttttcaaact ttaaataaaa 2220
aatattttta tattttttta tttttttatt tttatatttt tttatttttt tatttttata 2280
tttttttatt tttatatttt tttattttta tattttttta tttttttatt tttttatttt 2340
tttatttttt tattttttta tttttttatt tttaccctca ttttttttac gcttgtatta 2400
tagggtactt tgtacctgtt cttttttttg gggaggttgt aaagataatt ttttacttta 2460
gttagttccg aaggaacttt tattatagtg cccctttatt tttttgcgtg gcattgaatg 2520
taaaaaatta tcactatact agggcgtaaa gtaatattac atgtgtctca aagtgggatt 2580
aaagcgggat tttatagggc gtgtttgtgg cttagagtgg gattattgga aatttttttg 2640
atcctaggtt aaatactagt ttttagttac agacaaacct gaagttaact atttatcaat 2700
tcctgcaatt cgtttacaaa acggcaaatg tgaaatccgt cacatactgc gtgatgaact 2760
tgaattgcca aaggaagtat aattttgtta tcttctttat aatatttccc catagtaaaa 2820
ataggaatca aataatcata tcctttctgc aaattcagat taaagccatc gaaggttgac 2880
cacggtatca tagatacatt aaaaatgttt tccggagcat ttggctttcc ttccattcta 2940
tgattgtttc cataccgttg cgtatcactt tcataatctg ctaaaaatga tttaaagtca 3000
gacttacact cagtccaaag gctggaaaat gtttcagtat cattgtgaaa tattgtatag 3060

cttggtatca tctcatcata tatccccaat tcaccatctt gattgattgc cgtcctaaac 3120

tctgaatggc ggtttacaat cattgcaata taataaagca ttgcaggata tagtttcatt 3180

cccttttcct ttatttgtgt gatatccact ttaacggtca tgctgtaggt acaaggtaca 3240

cttgcaaagt agtggtcaaa atactctttt ctgttccaac tatttttatc aattttttca 3300

aataccatct aagttccctc tcaaattcaa gtttatcgct ctaatgaaca aagatattat 3360

accacatttt tgtgaatttt tcaacttgcc cacttcgact gcactcccga cttaataact 3420

tcttgaacac ttgccgaaaa agaaaaactg ccgggtacgt acccggggat cgatccccgg 3480

ccgagcgctt agtgggaatt tgtacccctt atcgatacaa attccccgta ggcgctaggg 3540

acctctttag accggtcagc tgtcagacac ttatcacatt aagtatatac tagtattaag 3600

ctagctttgg taactgtcag accaagttta ctcatatata ctttagattg atttaaaact 3660

tcatttttaa tttaaaagga tctaggtgaa gatccttttt gataatctca tgaccaaaat 3720

cccttaacgt gagttttcgt tccactgagc gtcagacccc gtagaaaaga tcaaaggatc 3780

ttcttgagat cctttttttc tgcgcgtaat ctgctgcttg caaacaaaaa aaccaccgct 3840

accagcggtg gtttgtttgc cggatcaaga gctaccaact ctttttccga aggtaactgg 3900

cttcagcaga gcgcagatac caaatactgt tcttctagtg tagccgtagt taggccacca 3960

cttcaagaac tctgtagcac cgcctacata cctcgctctg ctaatcctgt taccagtggc 4020

tgctgccagt ggcgataagt cgtgtcttac cgggttggac tcaagacgat agttaccgga 4080

taaggcgcag cggtcgggct gaacgggggg ttcgtgcaca cagcccagct tggagcgaac 4140

gacctacacc gaactgagat acctacagcg tgagctatga gaaagcgcca cgcttcccga 4200

agggagaaag gcggacaggt atccggtaag cggcagggtc ggaacaggag agcgcacgag 4260

ggagcttcca gggggaaacg cctggtatct ttatagtcct gtcgggtttc gccacctctg 4320

acttgagcgt cgatttttgt gatgctcgtc aggggggcgg agcctatgga aaaacgccag 4380

caacgcggcc tttttacggt tcctggcctt ttgctggcct tttgctcaca tgttctttcc 4440

tgcgttatcc cctgattctg tggataaccg tattaccgcc tttgagtgag ctgataccgc 4500

tcgccgcagc cgaacgaccg agcgcagcga gtcagtgagc gaggaagcgg aagagcgccc 4560

aatacgcaaa ccgcctctcc ccgcgcgttg gccgattcat taatgcagct ggcacgacag 4620

gtttcccgac tggaaagcgg gcagtgagcg caacgcaatt aatgtgagtt agctcactca 4680

ttaggcaccc caggctttac actttatgct tccggctcgt atgttgtgtg gaattgtgag 4740

cggataacaa tttcacacag gaaacagcta tgaccatgat tacgaattcg agctcactct 4800

atcattgata gagtttgaaa ctctatcatt gatagagtat aatatctttg ttcatttaag 4860

ccatctacta aacaagtttt agagctagaa atagcaagtt aaaataaggc tagtccgtta 4920

tcaacttgaa aaagtggcac cgagtcggtg ctttttttga agcttg 4966

<210> 35
<211> 400
<212> DNA
<213> Artificial Sequence

<220>
<223> Fragment amont du gène catB

<400> 35
gtctttacac ttttgcccat taatttttga gttccttatt tttagggagc ttttattatt 60

tttatcatga aaatttcata aaatactcat aaactaagga tgtcttcata atcagattag 120

tactccattt tcaatccatt taatctggga atatgatatt ttaattacgt attatttaag 180

atatattaac gtgtaatata ataccccgca aatattaatt atcacataca tatcccccct 240

ttattggggc attttttgta cccattattt tagtattgtg cagtacttaa ataaaaaaat 300

gccgcaaatt catttttatt gaataatgcg gtatttcttc tattctttat ttttattact 360

ctataaataa tgtaatcaag acatgactat ctaaatatat 400

<210> 36
<211> 400
<212> DNA
<213> Artificial Sequence

<220>
<223> Fragment aval du gène catB

<400> 36
aattcataat tcgggcctcc taaaaatttt cgtaattcta ttttagaagg ctttttccg 60

tgacctagcc atttcaatct cctttttaca atgatattta cgctttagtt tattatagca 120

cattctgtaa taccgaacta ttcaattttc agagaccatt ttttattgat tcataactta 180

agaatactac gaattactct aatattttac tttttcttat ctcttgttat tttaacatcg 240

gaattactac taatattaat ttttattttt ccatccgcat ttgctccaac atttttttaa 300

ctatactttc cttttgttaa taaattatgt tattgttgaa caatataaga aaagtgcgta 360

acatttttta ttaaaaataa ttaggtattt ctatctgtgg 400

<210> 37
<211> 218
<212> PRT
<213> Clostridium beijerinckii

<400> 37

Met Asn Phe Asn Leu Ile Asp Ile Asn His Trp Ser Arg Lys Pro Tyr
1               5                   10                  15

Phe Glu His Tyr Leu Asn Asn Val Lys Cys Thr Tyr Ser Met Thr Ala

20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Lys
    50                  55                  60

His Lys Glu Phe Arg Ile Cys Asp His Glu Gly Ser Leu Gly Tyr Trp
65                  70                  75                  80

Asp Ser Met Asn Pro Ser Tyr Thr Ile Phe His Lys Glu Asn Glu Thr
                85                  90                  95

Phe Ser Ser Ile Trp Thr Glu Tyr Asn Lys Ser Phe Leu Arg Phe Tyr
            100                 105                 110

Ser Asp Tyr Leu Asp Asp Ile Lys Asn Tyr Gly Asn Ile Met Lys Phe
        115                 120                 125

Thr Pro Lys Ser Asn Glu Pro Asp Asn Thr Phe Ser Val Ser Ser Ile
    130                 135                 140

Pro Trp Val Ser Phe Thr Gly Phe Asn Leu Asn Val Tyr Asn Glu Gly
145                 150                 155                 160

Thr Tyr Leu Ile Pro Ile Phe Thr Ala Gly Lys Tyr Phe Lys Gln Glu
                165                 170                 175

Asn Lys Ile Phe Ile Pro Ile Ser Ile Gln Val His His Ala Ile Cys
            180                 185                 190

Asp Gly Tyr His Ala Ser Arg Phe Ile Asn Glu Met Gln Glu Leu Ala
        195                 200                 205

Phe Ser Phe Gln Glu Trp Leu Glu Asn Lys
    210                 215

<210>  38
<211>  9113
<212>  DNA
<213>  Artificial Sequence

<220>
<223>  pCas9ind-gRNA_catB

<400>  38
catggataaa aagtacagta ttggtctaga cataggaact aactctgttg ggtgggctgt        60

tataacagat gaatataaag ttccatcaaa aaaatttaaa gtattaggaa acactgatag       120

acattcaata aaaaaaaact tgataggtgc tttattattc gattcaggag agactgctga 180

agctacacgt ttaaaaagaa cagctagacg tagatataca agaagaaaaa ataggatatg 240

ttatcttcaa gaaattttta gtaatgaaat ggcaaaagtt gatgattcat tctttcacag 300

actagaagaa agtttcttag ttgaagaaga taagaagcat gaaagacacc ctatttttgg 360

taatatcgta gatgaagtag catatcatga gaagtatcca actatctatc atttaagaaa 420

gaaattagtt gattctacag ataaagctga tctgagatta atatatttag ctttagctca 480

tatgattaaa tttagaggac attttttaat agaaggtgat ttaaacccag acaacagcga 540

tgtagataaa ttatttatcc aattagttca aacttataat caattattcg aagagaatcc 600

aattaatgca agtggtgtag acgctaaggc tatattatca gctagattat caaaatctag 660

aagattagaa aatctaatag ctcaacttcc tggagaaaag aaaaatggac tttttgggaa 720

cctaatagct ctctcactcg gactaacacc aaattttaaa agcaattttg atcttgctga 780

agacgcaaag ttacaactat caaaggatac atacgatgat gatttagata atttgttagc 840

tcaaataggt gatcaatatg ctgatttgtt tcttgcagca aaaaacttaa gtgatgcaat 900

tttactatca gatatactta gagtaaatac agaaataaca aaggctcctt tatcagcaag 960

tatgattaaa cgatatgatg agcatcatca agatttaaca ttattaaagg cacttgtaag 1020

acaacaatta ccagaaaaat ataaagaaat tttctttgat caatctaaaa atggatatgc 1080

tggatatata gacggtggag caagtcaaga agagttttat aaatttataa agcctatttt 1140

agaaaaaatg gatggaactg aagaattact tgttaaactt aacagagaag atttacttag 1200

aaaacaaaga acttttgata atggttcaat tcctcaccaa attcatttag gagaattaca 1260

tgctatacta agaagacaag aagattttta tccatttctt aaagataata gagaaaaaat 1320

tgaaaaaatt ttaactttta gaataccata ttatgtagga ccacttgcaa ggggaaattc 1380

aagatttgca tggatgacta gaaaatcaga agaaactata accccgtgga attttgaaga 1440

agtagtagat aaaggagcta gtgctcaatc atttatagaa agaatgacaa attttgataa 1500

gaatcttcct aacgaaaagg ttttgccaaa gcatagcctt ctttatgagt attttacagt 1560

ttataatgag cttactaaag taaaatacgt tacagaagga atgagaaaac cagcattttt 1620

gtctggtgaa caaaagaaag caatagtaga cctattattt aaaacaaata ggaaggttac 1680

cgtaaagcaa cttaaagaag attacttcaa aaaaattgaa tgctttgata gtgttgaaat 1740

atcaggagtt gaagatagat ttaatgcttc acttggtaca tatcacgatc tcttaaaaat 1800

tataaaagat aaggattttt tagataatga agaaaatgaa gatattcttg aagatatagt 1860

attaacattg acactttttg aagatagaga aatgatagaa gaaagattaa aaacatatgc 1920

acatcttttt gatgataagg ttatgaagca acttaaaaga agaagatata caggttgggg 1980

acgtttgtca agaaagctaa ttaatggtat tagagataaa caatcaggaa agactattct 2040
cgattttctt aaatcagatg gatttgctaa tagaaacttt atgcaattaa ttcatgatga 2100
ttctcttact ttcaaagagg atattcaaaa ggctcaagtt tctggacaag gcgatagctt 2160
acacgaacac attgctaacc ttgcagggag ccccgctatc aaaaaaggaa ttttacaaac 2220
agttaaagtt gtagatgaac ttgttaaagt tatgggaaga cacaaacctg agaatatagt 2280
tatagaaatg gccagagaaa atcaaacaac acaaaaagga caaaaaaatt ctagagagag 2340
aatgaagaga attgaagaag gaataaaaga gctaggatca caaatattaa aagaacatcc 2400
agttgaaaat actcaattgc aaaatgaaaa gttatatttg tattacttac aaaatggaag 2460
agatatgtat gttgatcaag aactcgatat taatagatta agtgactatg atgttgatca 2520
tattgttcct caatcatttt taaaagatga ttcaatcgat aacaaagtat taactagatc 2580
agataaaaat agaggaaagt cagataatgt accatctgaa gaagttgtta aaaaaatgaa 2640
gaactattgg agacaacttt taaatgcaaa gctaattaca caaagaaaat ttgacaattt 2700
aacaaaagca gaaagaggag gattaagcga attagacaaa gctggattta taaaaagaca 2760
acttgttgag acaagacaaa taactaagca tgttgctcaa atacttgatt caagaatgaa 2820
tacaaaatat gatgaaaatg ataaattaat cagagaagta aaagtaataa cattaaagtc 2880
aaaattagta tcagatttca gaaaggattt tcaattttac aaagttcgtg aaataaataa 2940
ctatcatcat gctcatgatg catacttaaa tgctgttgta ggaactgctc ttattaagaa 3000
atatcctaaa ctagaaagcg aatttgttta tggagattat aaagtttatg atgtgcgcaa 3060
aatgatcgcg aaatccgaac aagaaatcgg taaggctaca gcaaaatatt tcttttatag 3120
taatataatg aattttttta agacagaaat aactttggct aatggtgaaa tcagaaaaag 3180
accacttatc gaaacaaatg gagagacagg agaaatagta tgggataaag gaagagattt 3240
tgctactgtt agaaaagtac taagtatgcc acaagtaaat atcgtaaaga aaactgaagt 3300
tcaaactgga ggtttctcta aggaatcaat tttacctaag agaaattcag ataagttaat 3360
tgcaaggaaa aaagattggg acccaaaaaa atacggtggt tttgatagtc caacagttgc 3420
ctatagtgtt cttgtagtag cgaaagttga gaaaggtaag tcaaaaaagt tgaaaagcgt 3480
aaaagaactt cttggtatca caattatgga aagatcttca tttgaaaaaa atccaattga 3540
ctttttagaa gctaagggtt ataaagaagt taaaaaggat ttaatcataa aactaccaaa 3600
gtatagtcta tttgaactcg aaaacggaag aaaacgaatg ctcgctagcg caggagaact 3660
tcaaaaagga aatgaacttg cgctgccatc aaagtatgta aatttcttat atttagcttc 3720
tcattatgag aaattaaaag gatcaccaga ggataatgaa caaaagcaac tatttgtaga 3780
acaacacaaa cattatttag atgaaataat agaacaaata tctgaatttt ctaaaagagt 3840
tatacttgcc gacgcaaatc tagataaggt gctttcagcg tataataaac acagagataa 3900

accaataaga gaacaagcag aaaacattat ccatcttttt acattaacta atcttggtgc 3960

accagctgca tttaagtact ttgatacaac aatagataga aaaagataca catctactaa 4020

agaagtatta gacgcaactt taatacatca atctattaca gggctttatg aaacaagaat 4080

tgatttaagt caactaggcg gagattaagt cgacaaagta ttgttaaaaa taactctgta 4140

gaattataaa ttagttctac agagttattt tttgacccgg gtatattgat aaaaataata 4200

atagtgggta taattaagtt gttaggaggt tagttagaat gatgtcaaga ttagataaaa 4260

gtaaagtgat taacagcgca ttagagctgc ttaatgaggt cggaatcgaa ggtttaacaa 4320

cccgtaaact cgcccagaag ctaggtgtag agcagcctac attgtattgg catgtaaaaa 4380

ataagcgggc tttgctcgac gccttagcca ttgagatgtt agataggcac catactcact 4440

tttgcccttt agaaggggaa agctggcaag attttttacg taataacgct aaaagtttta 4500

gatgtgcttt actaagtcat cgcgatggag caaaagtaca tttaggtaca cggcctacag 4560

aaaaacagta tgaaactctc gaaaatcaat tagccttttt atgccaacaa ggtttttcac 4620

tagagaatgc attatatgca ctcagcgctg tggggcattt tactttaggt tgcgtattgg 4680

aagatcaaga gcatcaagtc gctaaagaag aaagggaaac acctactact gatagtatgc 4740

cgccattatt acgacaagct atcgaattat ttgatcacca aggtgcagag ccagccttct 4800

tattcggcct tgaattgatc atatgcggat tagaaaaaca acttaaatgt gaaagtgggt 4860

cttaaaagca gcataacctt tttccgtgat ggtaacttca cggtaaccaa gatgtcgagt 4920

tgagctcgaa ttcgtaatca tggtcatagc tgtttcctgt gtgaaattgt tatccgctca 4980

caattccaca caacatacga gccggaagca taaagtgtaa agcctggggt gcctaatgag 5040

tgagctaact cacattaatt gcgttgcgct cactgcccgc tttccagtcg ggaaacctgt 5100

cgtgccagct gcattaatga atcggccaac gcgcggggag aggcggtttg cgtattgggc 5160

gctcttccgc ttcctcgctc actgactcgc tgcgctcggt cgttcggctg cggcgagcgg 5220

tatcagctca ctcaaaggcg gtaatacggt tatccacaga atcaggggat aacgcaggaa 5280

agaacatgtg agcaaaaggc cagcaaaagg ccaggaaccg taaaaaggcc gcgttgctgg 5340

cgtttttcca taggctccgc ccccctgacg agcatcacaa aaatcgacgc tcaagtcaga 5400

ggtggcgaaa cccgacagga ctataaagat accaggcgtt tccccctgga agctccctcg 5460

tgcgctctcc tgttccgacc ctgccgctta ccggatacct gtccgccttt ctcccttcgg 5520

gaagcgtggc gctttctcat agctcacgct gtaggtatct cagttcggtg taggtcgttc 5580

gctccaagct gggctgtgtg cacgaacccc ccgttcagcc cgaccgctgc gccttatccg 5640

gtaactatcg tcttgagtcc aacccggtaa gacacgactt atcgccactg gcagcagcca 5700

ctggtaacag gattagcaga gcgaggtatg taggcggtgc tacagagttc ttgaagtggt 5760

ggcctaacta cggctacact agaaggacag tatttggtat ctgcgctctg ctgaagccag 5820

ttaccttcgg aaaaagagtt ggtagctctt gatccggcaa acaaaccacc gctggtagcg 5880

gtggtttttt tgtttgcaag cagcagatta cgcgcagaaa aaaaggatct caagaagatc 5940

ctttgatctt ttctacgggg tctgacgctc agtggaacga aaactcacgt taagggattt 6000

tggtcatgag attatcaaaa aggatcttca cctagatcct tttaaattaa aaatgaagtt 6060

ttaaatcaat ctaaagtata tatgagtaaa cttggtctga cagttaccag gtccactgcc 6120

gggcctcttg cgggatcaaa agaaaaacga aatgatacac caatcagtgc aaaaaaagat 6180

ataatgggag ataagacggt tcgtgttcgt gctgacttgc accatatcat aaaaatcgaa 6240

acagcaaaga atggcggaaa cgtaaaagaa gttatggaaa taagacttag aagcaaactt 6300

aagagtgtgt tgatagtgca gtatcttaaa attttgtata ataggaattg aagttaaatt 6360

agatgctaaa aatttgtaat taagaaggag tgattacatg aacaaaaata taaaatattc 6420

tcaaaacttt ttaacgagtg aaaaagtact caaccaaata ataaaacaat tgaatttaaa 6480

agaaaccgat accgtttacg aaattggaac aggtaaaggg catttaacga cgaaactggc 6540

taaaataagt aaacaggtaa cgtctattga attagacagt catctattca acttatcgtc 6600

agaaaaatta aaactgaata ctcgtgtcac tttaattcac caagatattc tacagtttca 6660

attccctaac aaacagaggt ataaaattgt tgggagtatt ccttaccatt taagcacaca 6720

aattattaaa aaagtggttt ttgaaagcca tgcgtctgac atctatctga ttgttgaaga 6780

aggattctac aagcgtacct tggatattca ccgaacacta gggttgctct tgcacactca 6840

agtctcgatt cagcaattgc ttaagctgcc agcggaatgc tttcatccta aaccaaaagt 6900

aaacagtgtc ttaataaaac ttacccgcca taccacagat gttccagata aatattggaa 6960

gctatatacg tactttgttt caaaatgggt caatcgagaa tatcgtcaac tgtttactaa 7020

aaatcagttt catcaagcaa tgaaacacgc caaagtaaac aatttaagta ccgttactta 7080

tgagcaagta ttgtctattt ttaatagtta tctattattt aacggaggaa aataattcta 7140

tgagtcccta ggcaggcctc cgccattatt tttttgaaca attgacaatt catttcttat 7200

tttttattaa gtgatagtca aaaggcataa cagtgctgaa tagaaagaaa tttacagaaa 7260

agaaaattat agaatttagt atgattaatt atactcattt atgaatgttt aattgaatac 7320

aaaaaaaaat acttgttatg tattcaatta cgggttaaaa tatagacaag ttgaaaaatt 7380

taataaaaaa ataagtcctc agctcttata tattaagcta ccaacttagt atataagcca 7440

aaacttaaat gtgctaccaa cacatcaagc cgttagagaa ctctatctat agcaatattt 7500

caaatgtacc gacatacaag agaaacatta actatatata ttcaatttat gagattatct 7560

taacagatat aaatgtaaat tgcaataagt aagatttaga agtttatagc ctttgtgtat 7620

tggaagcagt acgcaaaggc ttttttattt gataaaaatt agaagtatat ttattttttc 7680

ataattaatt tatgaaaatg aaagggggtg agcaaagtga cagaggaaag cagtatctta 7740

tcaaataaca aggtattagc aatatcatta ttgactttag cagtaaacat tatgactttt 7800

atagtgcttg tagctaagta gtacgaaagg gggagcttta aaaagctcct tggaatacat 7860

agaattcata aattaattta tgaaaagaag ggcgtatatg aaaacttgta aaaattgcaa 7920

agagtttatt aaagatactg aaatatgcaa aatacattcg ttgatgattc atgataaaac 7980

agtagcaacc tattgcagta aatacaatga gtcaagatgt ttacataaag ggaaagtcca 8040

atgtattaat tgttcaaaga tgaaccgata tggatggtgt gccataaaaa tgagatgttt 8100

tacagaggaa gaacagaaaa aagaacgtac atgcattaaa tattatgcaa ggagctttaa 8160

aaaagctcat gtaaagaaga gtaaaaagaa aaaataattt atttattaat ttaatattga 8220

gagtgccgac acagtatgca ctaaaaaata tatctgtggt gtagtgagcc gatacaaaag 8280

gatagtcact cgcattttca taatacatct tatgttatga ttatgtgtcg gtgggacttc 8340

acgacgaaaa cccacaataa aaaaagagtt cggggtaggg ttaagcatag ttgaggcaac 8400

taaacaatca agctaggata tgcagtagca gaccgtaagg tcgttgttta ggtgtgttgt 8460

aatacatacg ctattaagat gtaaaaatac ggataccaat gaagggaaaa gtataatttt 8520

tggatgtagt ttgtttgttc atctatgggc aaactacgtc caaagccgtt tccaaatctg 8580

ctaaaaagta tatcctttct aaaatcaaag tcaagtatga aatcataaat aaagtttaat 8640

tttgaagtta ttatgatatt atgtttttct attaaaataa attaagtata tagaatagtt 8700

taataatagt atatacttaa tgtgataagt gtctgacagt gtcacagaaa ggatgattgt 8760

tatggattat aagcggctcg aggacgtcaa accatgttaa tcattgcttt tatcaaaaat 8820

aggatccact ctatcattga tagagtttga aactctatca ttgatagagt ataatatctt 8880

tgttcatgta catcatgcta tctgtgagtt ttagagctag aaatagcaag ttaaaataag 8940

gctagtccgt tatcaacttg aaaaagtggc accgagtcgg tgcttttttt gaagcttgtc 9000

tttacacttt tgcccctcga gtccctatca gtgatagatt gaaactctat cattgataga 9060

gtataatatc tttgttcatt agagcgataa acttgaattt gagagggaac ttc 9113

<210> 39
<211> 20
<212> DNA
<213> Artificial Sequence

<220>
<223> Amorce pNF2

<400> 39
gggcgcactt atacaccacc 20

<210> 40

<211>  20
<212>  DNA
<213>  Artificial Sequence

<220>
<223>  Amorce pNF2

<400>  40
tgctacgcac cccctaaagg                                                    20


<210>  41
<211>  50
<212>  DNA
<213>  Artificial Sequence

<220>
<223>  DeltacatB_gRNA_rev

<400>  41
aatctatcac tgatagggac tcgaggggca aaagtgtaaa gacaagcttc                    50


<210>  42
<211>  20
<212>  DNA
<213>  Artificial Sequence

<220>
<223>  Amorce pCas9ind_fwd

<400>  42
agctcttgat ccggcaaaca                                                    20


<210>  43
<211>  20
<212>  DNA
<213>  Artificial Sequence

<220>
<223>  Amorce pCas9ind _rev

<400>  43
gcaaccctag tgttcggtga                                                    20


<210>  44
<211>  219
<212>  PRT
<213>  Clostridium butyricum

<400>  44

Met Asn Phe Asn Leu Ile Asp Ile Asn His Trp Ser Arg Lys Pro Tyr
1               5                   10                  15


Phe Glu His Tyr Leu Asn Asn Val Lys Cys Thr Tyr Ser Met Thr Ala
            20                  25                  30


Asn Ile Glu Ile Thr Asp Leu Leu Tyr Glu Ile Lys Leu Lys Asn Ile

35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Lys Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Lys Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Ser Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Glu Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  45
<211>  219
<212>  PRT
<213>  Clostridium beijerinckii

<400>  45

Met Asn Phe Asn Leu Ile Asp Ile Asn His Trp Ile Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile

35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Lys Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Lys Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Ser Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Glu Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  46
<211>  219
<212>  PRT
<213>  Clostridium beijerinckii

<400>  46

Met Asn Phe Asn Leu Ile Asp Ile Asn His Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile

35                  40                  45

Lys Phe Tyr Pro Thr Leu Ile Tyr Met Ile Ala Thr Val Val Asn Asn
    50                  55                  60

His Glu Glu Phe Arg Ile Cys Phe Asp His Glu Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Lys Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Ser Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Gly Asn Lys Val Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  47
<211>  219
<212>  PRT
<213>  Clostridium beijerinckii

<400>  47

Met Asn Phe Asn Leu Ile Asp Ile Asn His Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile

35                  40                  45

Lys Phe Tyr Pro Thr Leu Ile Tyr Met Ile Ala Thr Val Val Asn Asn
    50                  55                  60

His Lys Glu Phe Ser Ile Cys Phe Asp His Glu Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Ser Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Gly Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  48
<211>  219
<212>  PRT
<213>  Artificial Sequence

<220>
<223>  Clostridium sp.2-1

<400>  48

Met Asn Phe Asn Leu Ile Asp Ile Asn His Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Asn Val Val Asn Asn
    50                  55                  60

His Lys Glu Phe Arg Ile Cys Phe Asp His Asn Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Gln Pro Asp Asn Thr Phe Ser Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Glu Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  49
<211>  219
<212>  PRT
<213>  Artificial Sequence

<220>
<223>  Clostridium diolis

<400>  49

Met Asn Phe Asn Leu Ile Asp Ile Asn His Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Asn Val Val Asn Asn
    50                  55                  60

His Lys Glu Phe Arg Ile Cys Phe Asp His Asn Gly Ser Leu Gly Tyr
65                  70                  75                  80

Trp Asp Ser Met Asn Pro Ser Tyr Thr Val Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Ser Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Gly Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  50
<211>  219
<212>  PRT
<213>  Clostridium beijerinckii

<400>  50

Met Asn Phe Asn Leu Ile Asp Ile Asn His Trp Ile Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Lys Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Lys Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Gln Pro Asp Asn Thr Phe Ser Val Ser Ser
    130                 135                 140

Ile Pro Trp Val Ser Phe Thr Gly Phe Asn Leu Asn Val Asn Asn Glu
145                 150                 155                 160

Gly Thr Tyr Leu Ile Pro Ile Phe Thr Ala Gly Lys Tyr Phe Lys Gln
                165                 170                 175

Glu Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Lys Glu Trp Leu Glu Asn Lys
    210                 215

<210>  51
<211>  221
<212>  PRT
<213>  Clostridium beijerinckii

<400>  51

Met Asn Phe Asn Leu Ile Asp Ile Asn Asn Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Asn Val Val Asn Asn
    50                  55                  60

His Lys Glu Phe Arg Ile Cys Phe Asp His Glu Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Pro Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Gly Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys Tyr Ile
    210                 215                 220

<210>  52
<211>  219
<212>  PRT
<213>  Clostridium beijerinckii

<400>  52

Met Asn Phe Asn Leu Ile Asp Ile Asn Asn Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Asn Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Pro Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Gly Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Arg Glu Trp Leu Glu Asn Lys
    210                 215

<210>  53
<211>  219
<212>  PRT
<213>  Clostridium saccharoperbutylacetonicum

<400>  53

Met Asn Phe Asn Leu Ile Asp Ile Asn His Trp Ser Arg Lys Pro Tyr
1               5                   10                  15

Phe Glu His Tyr Leu Asn Thr Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Lys Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Ile Asp Asp Ile Lys Asn Tyr Gly Asn Ile Met Lys
        115                 120                 125

Phe Thr Pro Lys Ser Asn Glu Pro Asp Asn Ile Phe Pro Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Glu Asn Lys Ile Phe Ile Pro Ile Ser Ile Gln Val His His Ala Val
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  54
<211>  219
<212>  PRT
<213>  Clostridium saccharoperbutylacetonicum

<400>  54

Met Asn Phe Asn Leu Ile Asp Ile Asn His Trp Ser Arg Lys Pro Tyr
1               5                   10                  15

Phe Glu His Tyr Leu Asn Thr Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Lys Gly Ser Leu Gly Tyr
65                  70                  75                  80

Trp Asp Ser Met Asn Pro Ser Tyr Thr Ile Phe Tyr Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Ile Asp Asp Ile Lys Asn Tyr Gly Asn Ile Met Lys
        115                 120                 125

Phe Thr Pro Lys Ser Asn Glu Pro Asp Asn Ile Phe Pro Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Glu Asn Lys Ile Phe Ile Pro Ile Ser Ile Gln Val His His Ala Val
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys
    210                 215

<210>  55
<211>  219
<212>  PRT
<213>  Clostridium beijerinckii

<400>  55

Met Asn Phe Asn Leu Ile Asp Ile Asn Asn Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Asn Gly Ser Leu Gly Tyr
65                  70                  75                  80

Trp Asp Ser Met Asn Pro Ser Tyr Thr Ile Phe His Lys Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Arg Ser Asp Asn Thr Phe Pro Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Ala Gly Lys Tyr Phe Lys Gln
                165                 170                 175

Gly Asn Lys Ile Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Arg Glu Trp Leu Glu Asn Lys
    210                 215

<210>  56
<211>  221
<212>  PRT
<213>  Clostridium beijerinckii

<400>  56

Met Asn Phe Asn Leu Ile Asp Ile Asn His Trp Asn Arg Lys Pro Phe
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Lys Leu Lys Asn Ile
        35                  40                  45

Lys Phe Tyr Pro Thr Leu Ile Tyr Met Ile Ala Thr Val Val Asn Asn
    50                  55                  60

His Lys Glu Phe Arg Ile Cys Phe Asp His Lys Gly Ser Leu Gly Tyr
65                  70                  75                  80

Trp Asp Ser Met Asn Pro Ser Tyr Thr Ile Phe His Glu Glu Asn Glu
                85                  90                  95

Thr Phe Ser Ser Ile Trp Thr Glu Tyr Asn Glu Ser Phe Leu Arg Phe
            100                 105                 110

Tyr Ser Asp Tyr Leu Asp Asp Ile Lys Asn Tyr Gly Asn Ile Met Lys
        115                 120                 125

Phe Thr Pro Lys Ser Asn Glu Pro Asp Asn Thr Phe Pro Val Ser Ser
    130                 135                 140

Ile Pro Trp Val Ser Phe Thr Gly Phe Asn Leu Asn Val Cys Asn Glu
145                 150                 155                 160

Gly Thr Tyr Leu Thr Pro Ile Phe Thr Ala Gly Lys Tyr Phe Lys Gln
                165                 170                 175

Glu Asn Lys Ile Phe Ile Pro Ile Ser Ile Gln Val His His Ser Ile
            180                 185                 190

Cys Asp Gly Tyr His Ala Ser Arg Phe Ile Asn Glu Met Gln Glu Leu
        195                 200                 205

Ala Phe Ser Phe Gln Glu Trp Leu Glu Asn Lys Tyr Ile
    210                 215                 220

<210>  57
<211>  219
<212>  PRT
<213>  Clostridium beijerinckii

<400>  57

Met Asn Phe Asn Leu Ile Asp Ile Lys His Trp Ser Arg Lys Pro Tyr
1               5                   10                  15

Phe Glu His Tyr Leu Asn Asn Val Lys Cys Thr Tyr Ser Met Thr Ala
            20                  25                  30

Asn Ile Glu Ile Thr Asp Leu Leu Tyr Glu Ile Arg Leu Lys Asn Ile
        35                  40                  45

Lys Leu Tyr Pro Thr Leu Ile Tyr Met Ile Ala Thr Val Val Asn Asn
    50                  55                  60

His Lys Glu Phe Arg Thr Cys Phe Asp His Ser Gly Ser Leu Gly Tyr
65                  70                  75                  80

Trp Asp Ser Met Ser Pro Ser Tyr Thr Ile Phe His Lys Glu Asn Glu
                85                  90                  95

Thr Phe Ser Ser Ile Trp Thr Glu Tyr Asn Glu Ser Phe Pro Arg Phe
            100                 105                 110

Tyr Ser Asp Tyr Phe Asp Asp Ile Lys Asn Tyr Gly Asn Ile Met Lys
        115                 120                 125

Phe Thr Pro Lys Leu Asn Glu Pro Asp Asn Thr Phe Pro Val Ser Ser
    130                 135                 140

Ile Pro Trp Val Ser Phe Thr Gly Phe Asn Leu Asn Val Tyr Asn Glu
145                 150                 155                 160

Gly Thr Tyr Leu Ile Pro Ile Phe Thr Thr Gly Lys Tyr Phe Lys Gln
                165                 170                 175

Glu Asn Lys Met Phe Ile Pro Ile Ser Ile Gln Val His His Ala Ile
            180                 185                 190

Cys Asp Gly Tyr His Ala Ser Arg Phe Ile Asn Glu Met Gln Glu Leu
        195                 200                 205

Ala Phe Ser Phe Gln Asp Trp Leu Glu Asn Lys
    210                 215

<210>  58
<211>  219
<212>  PRT
<213>  Clostridium botulinum

<400>  58

Met Lys Phe Asn Leu Ile Asp Ile Glu His Trp Asn Arg Lys Pro Tyr
1               5                   10                  15

Phe Glu Tyr Tyr Leu His Ser Val Arg Cys Thr Tyr Ser Met Thr Ala
            20                  25                  30

Asn Ile Glu Ile Thr Asn Leu Leu His Glu Ile Lys Leu Lys Lys Leu
        35                  40                  45

Lys Leu Tyr Pro Thr Leu Ile Tyr Ile Ile Ala Thr Val Val Asn Asn
    50                  55                  60

His Lys Glu Phe Arg Thr Cys Phe Asp Glu Asn Gly Asn Leu Gly Tyr
65                  70                  75                  80

Trp Asp Ser Met Ser Pro Ser Tyr Thr Ile Phe His Lys Asp Asn Glu
                85                  90                  95

Thr Phe Ser Ser Ile Trp Thr Asp Tyr Asp Glu Ser Phe Ser Cys Phe
            100                 105                 110

Tyr Asn Asp Tyr Leu Asp Asp Ile Lys Asn Tyr Gly Ala Ile Met Lys
        115                 120                 125

Phe Thr Pro Lys Leu Asn Glu Pro Ala Asn Thr Phe Pro Val Ser Ser
    130                 135                 140

Ile Pro Trp Val Asn Phe Thr Gly Phe Asn Leu Asn Val Tyr Asn Asn
145                 150                 155                 160

Gly Thr Tyr Leu Val Pro Ile Phe Thr Met Gly Lys Tyr Phe Glu Gln
                165                 170                 175

Asn Asn Lys Ile Phe Ile Pro Met Ser Ile Gln Val His His Ala Val
            180                 185                 190

Cys Asp Gly Tyr His Ile Ser Arg Phe Ile Asn Glu Val Gln Glu Leu
        195                 200                 205

Ala Leu Asn Ser Gln Thr Trp Leu Lys His Lys
    210                 215

<210>  59
<211>  219
<212>  PRT
<213>  Artificial Sequence

<220>
<223>  Anaerocolumna aminovalerica

<400>  59

Met Lys Phe Asn Leu Ile Asp Ile Glu Asn Trp Asn Arg Lys Pro Tyr
1               5                   10                  15

Phe Glu His Tyr Leu Asn Ser Val Arg Cys Thr Tyr Ser Met Thr Ala
            20                  25                  30

Asn Ile Glu Ile Thr Asn Leu Leu His Glu Ile Lys Leu Lys Asp Leu
        35                  40                  45

Lys Leu Tyr Pro Thr Leu Ile Tyr Ile Leu Ala Thr Val Val Asn Asn
    50                  55                  60

His Lys Glu Phe Arg Thr Cys Phe Asp Glu Asn Gly Asn Leu Gly Tyr
65                  70                  75                  80

Trp Asp Ser Met Ser Pro Ser Tyr Thr Ile Phe His Lys Glu Asn Glu
                85                  90                  95

Thr Phe Ser Ser Ile Trp Thr Glu Tyr Asp Glu Ser Phe Ser Arg Phe
            100                 105                 110

Tyr Thr Ala Tyr Leu Asp Asp Ile Lys Asn His Gly Asn Ile Met Lys
        115                 120                 125

Phe Thr Pro Lys Leu Asn Glu Pro Ala Asn Thr Phe Pro Ile Ser Ser
    130                 135                 140

Ile Pro Trp Val Ser Phe Thr Gly Phe Asn Leu Asn Val Tyr Asn Asp
145                 150                 155                 160

Gly Lys Tyr Leu Leu Pro Ile Phe Thr Thr Gly Lys Tyr Phe Glu Gln
                165                 170                 175

Asn Ser Lys Ile Phe Ile Pro Met Ser Val Gln Val His His Ala Val
            180                 185                 190

Cys Asp Gly Tyr His Ile Ser Arg Phe Ile Asn Glu Val Gln Glu Val
        195                 200                 205

Ile Leu Asn Tyr Gln Thr Trp Leu Gly Asp Lys
    210                 215

<210>  60
<211>  219
<212>  PRT
<213>  Artificial Sequence

<220>
<223>  Desnuesiella massiliensis

<400>  60

Met Lys Phe Asn Leu Ile Asp Ile Glu His Trp Asn Arg Lys Pro Tyr
1               5                   10                  15

Phe Glu His Tyr Leu Asn Ser Val Arg Cys Thr Tyr Ser Met Thr Ala
            20                  25                  30

Asn Ile Glu Ile Thr Asn Leu Leu His Asp Ile Lys Leu Lys Lys Leu
        35                  40                  45

Lys Leu Tyr Pro Thr Leu Ile Tyr Ile Ile Ala Thr Val Val Asn Asn
    50                  55                  60

His Glu Glu Phe Arg Thr Cys Phe Tyr Glu Asn Gly Asn Leu Gly Tyr
65                  70                  75                  80

Trp Asp Ser Met Ser Pro Ser Tyr Thr Ile Phe His Lys Asp Asn Glu
                85                  90                  95

Thr Phe Ser Glu Ile Trp Ser Glu Tyr Asp Glu Ser Phe Ser Cys Phe
            100                 105                 110

Tyr Ser Lys Tyr Leu Asp Asp Ile Lys Asn Tyr Gly Asp Ile Met Arg
        115                 120                 125

Phe Thr Pro Lys Leu Asn Glu Pro Ala Asn Thr Phe Pro Ile Ser Cys
    130                 135                 140

Ile Pro Trp Val Ser Phe Thr Gly Phe Asn Leu Asn Val Tyr Asn Asp
145                 150                 155                 160

Gly Arg Tyr Leu Val Pro Ile Phe Thr Ile Gly Lys Tyr Phe Glu Gln
                165                 170                 175

Asn Asn Lys Ile Phe Ile Pro Met Ser Ile Gln Val His His Ala Val
            180                 185                 190

Cys Asp Gly Tyr His Thr Ser Arg Phe Ile Asn Glu Val Gln Glu Leu
        195                 200                 205

Ala Leu Asn Ser Gln Thr Trp Leu Arg His Lys
    210                 215

<210>  61
<211>  219
<212>  PRT
<213>  Artificial Sequence

<220>
<223>  Clostridium sp. HMP27

<400>  61

Met Lys Phe Asn Leu Ile Asp Thr Glu His Trp Asn Arg Lys Pro Tyr
1               5                   10                  15

Phe Glu His Tyr Leu Asn Ser Val Arg Cys Thr Tyr Ser Ile Thr Ala
            20                  25                  30

Asn Ile Glu Ile Thr Asn Leu Leu His Asp Ile Lys Gln Lys Lys Leu
        35                  40                  45

Lys Leu Tyr Pro Thr Phe Ile Tyr Ile Ile Ala Thr Val Val Asn Thr
    50                  55                  60

His Lys Glu Phe Arg Thr Cys Phe Asp Glu Ser Gly Asn Leu Gly Tyr
65                  70                  75                  80

Trp Asp Ser Met Ser Pro Ser Tyr Thr Ile Phe His Lys Asp Asn Glu
                85                  90                  95

Thr Phe Ser Ser Ile Trp Thr Glu Tyr Asp Lys Ser Phe Ser Cys Phe
            100                 105                 110

Tyr Ser Lys Tyr Leu His Asp Ile Lys Asn Tyr Gly Asp Ile Met Ser
        115                 120                 125

Phe Thr Pro Lys Leu Asn Glu Pro Ala Asn Thr Phe Pro Ile Ser Cys
    130                 135                 140

Ile Pro Trp Val Ser Phe Thr Gly Phe Asn Leu Asn Ile Tyr Asn Asp
145                 150                 155                 160

Gly Thr Tyr Leu Val Pro Ile Phe Thr Ile Gly Lys Tyr Phe Lys Gln
                165                 170                 175

Ala Asp Lys Ile Leu Ile Pro Ile Ser Ile Gln Val His His Ala Val
            180                 185                 190

Cys Asp Gly Tyr His Thr Ser Arg Phe Ile Asn Glu Val Gln Glu Leu
        195                 200                 205

Ile Leu Asn Tyr Gln Thr Trp Leu Lys His Lys
    210                 215

<210>  62

<211>  219
<212>  PRT
<213>  Artificial Sequence

<220>
<223>  Clostridium drakei

<400>  62

Met Lys Phe Asn Leu Ile Asp Ile Glu Asn Trp Asn Arg Lys Pro Tyr
1               5                   10                  15

Phe Glu His Tyr Leu Asn Ala Val Arg Cys Thr Tyr Ser Met Thr Ala
            20                  25                  30

Asn Ile Glu Ile Thr Gly Leu Leu Arg Glu Ile Lys Leu Lys Gly Leu
        35                  40                  45

Lys Leu Tyr Pro Thr Leu Ile Tyr Ile Ile Thr Ala Val Ile Asn Arg
    50                  55                  60

His Lys Glu Phe Arg Thr Cys Phe Asp Glu Asn Arg Lys Leu Gly Tyr
65                  70                  75                  80

Trp Asp Ser Met Ser Pro Ser Tyr Thr Val Phe His Lys Glu Asp Glu
                85                  90                  95

Thr Phe Ser Ser Ile Trp Thr Glu Tyr Asp Glu Ser Phe Pro Arg Phe
            100                 105                 110

Tyr Asp Asn Tyr Leu Asp Asp Ile Lys Ser Tyr Gly Asp Val Leu Lys
        115                 120                 125

Phe Met Pro Lys Pro Asp Glu Pro Gly Asn Thr Phe Asn Val Ser Ser
    130                 135                 140

Ile Pro Trp Val Asn Phe Thr Gly Phe Asn Leu Asn Ile Tyr Asn Asp
145                 150                 155                 160

Ala Thr Tyr Leu Ile Pro Ile Phe Thr Met Gly Lys Phe Phe His Gln
                165                 170                 175

Asp Asn Lys Ile Phe Ile Pro Met Ser Ile Gln Val His His Ala Val
            180                 185                 190

Cys Asp Gly Tyr His Thr Ser Arg Phe Phe Asn Glu Val Gln Glu Leu
        195                 200                 205

Ser Ser Asn Phe Glu Thr Trp Leu Asp Glu Lys
    210                 215

<210>  63
<211>  219
<212>  PRT
<213>  Clostridium scatologenes

<400>  63

Met Lys Phe Asn Leu Ile Asp Ile Glu Asp Trp Asn Arg Lys Pro Tyr
1               5                   10                  15

Phe Glu His Tyr Leu Asn Ala Val Arg Cys Thr Tyr Ser Met Thr Ala
            20                  25                  30

Asn Ile Glu Ile Thr Gly Leu Leu Arg Glu Ile Lys Leu Lys Gly Leu
        35                  40                  45

Lys Leu Tyr Pro Thr Leu Ile Tyr Ile Ile Thr Ala Val Ile Asn Arg
    50                  55                  60

His Lys Glu Phe Arg Thr Cys Phe Asp Glu Asn Arg Lys Leu Gly Tyr
65                  70                  75                  80

Trp Asp Ser Met Ser Pro Ser Tyr Thr Val Phe His Lys Glu Asp Glu
                85                  90                  95

Thr Phe Ser Ser Ile Trp Thr Glu Tyr Asp Glu Ser Phe Pro Arg Phe
            100                 105                 110

Tyr Asp Asn Tyr Leu Asp Asp Ile Lys Ser Tyr Gly Asp Val Leu Lys
        115                 120                 125

Phe Met Pro Lys Pro Asp Glu Pro Gly Asn Thr Phe Asn Val Ser Ser
    130                 135                 140

Ile Pro Trp Val Asn Phe Thr Gly Phe Asn Leu Asn Ile Tyr Asn Asp
145                 150                 155                 160

Ala Thr Tyr Leu Ile Pro Ile Phe Thr Met Gly Lys Phe Phe His Gln
                165                 170                 175

Asp Asn Lys Ile Phe Ile Pro Met Ser Ile Gln Val His His Ala Val
            180                 185                 190

Cys Asp Gly Tyr His Thr Ser Arg Phe Phe Asn Glu Val Gln Glu Leu
        195                 200                 205

Ser Ser Asn Phe Glu Thr Trp Leu Gly Glu Lys
    210                 215

<210>  64
<211>  219
<212>  PRT
<213>  Artificial Sequence

<220>
<223>  Clostridium tunisiense

<400>  64

Met Lys Phe Asn Leu Ile Asp Thr Glu His Trp Asp Arg Lys Pro Tyr
1               5                   10                  15

Phe Glu His Tyr Phe Asn Ser Val Lys Cys Thr Tyr Ser Met Thr Ala
            20                  25                  30

Asn Ile Glu Ile Thr Asn Leu Leu Asn His Ile Arg Leu Lys Lys Leu
        35                  40                  45

Lys Leu Tyr Pro Thr Leu Ile Tyr Ile Ile Ala Thr Val Val Asn Asn
    50                  55                  60

His Glu Glu Phe Arg Ile Cys Phe Asp Glu Asn Asn Asn Leu Gly Tyr
65                  70                  75                  80

Trp Asp Ser Met Ser Pro Asn Tyr Thr Ile Phe His Glu Asp Asn Lys
                85                  90                  95

Thr Phe Ser Ser Ile Trp Thr Glu Tyr Glu Glu Ser Phe Ser Gly Phe
            100                 105                 110

Tyr Asn Lys Tyr Leu Glu Asp Ile Lys Thr Tyr Gly His Ile Met Ser
        115                 120                 125

Phe Glu Pro Lys Leu Asn Glu Ser Thr Asn Thr Phe Pro Ile Ser Cys
    130                 135                 140

Ile Pro Trp Val Ser Phe Thr Gly Phe Asn Leu Asn Ile Gln Asp Asp
145                 150                 155                 160

Gly Thr Tyr Leu Thr Pro Ile Phe Thr Leu Gly Lys Tyr Phe Glu Gln
                165                 170                 175

Asn Asn Lys Thr Phe Ile Pro Ile Ser Ile Gln Val His His Ala Val
            180                 185                 190

Cys Asp Gly Tyr His Thr Ser Arg Phe Ile Asn Glu Val Gln Glu Leu
        195                 200                 205

Ala Ser Asp Phe Gln Ile Trp Leu Thr Tyr Lys
    210                 215

<210>  65
<211>  219
<212>  PRT
<213>  Artificial Sequence

<220>
<223>  Lachnospiraceae

<400>  65

Met Lys Phe Asn Leu Ile Asp Ile Glu Asp Trp Asn Arg Lys Pro Tyr
1               5                   10                  15

Phe Glu His Tyr Leu Asn Ala Val Arg Cys Thr Tyr Ser Met Thr Ala
            20                  25                  30

Asn Ile Glu Ile Thr Gly Leu Leu Arg Glu Ile Lys Leu Lys Gly Leu
        35                  40                  45

Lys Leu Tyr Pro Thr Leu Ile Tyr Ile Ile Thr Thr Val Val Asn Arg
    50                  55                  60

His Lys Glu Phe Arg Thr Cys Phe Asp Gln Lys Gly Lys Leu Gly Tyr
65                  70                  75                  80

Trp Asp Ser Met Asn Pro Ser Tyr Thr Val Phe His Lys Asp Asn Glu
                85                  90                  95

Thr Phe Ser Ser Ile Trp Thr Glu Tyr Asp Glu Asn Phe Pro Arg Phe
            100                 105                 110

Tyr Tyr Asn Tyr Leu Glu Asp Ile Arg Asn Tyr Ser Asp Val Leu Asn
        115                 120                 125

Phe Met Pro Lys Thr Gly Glu Pro Ala Asn Thr Ile Asn Val Ser Ser
    130                 135                 140

Ile Pro Trp Val Asn Phe Thr Gly Phe Asn Leu Asn Ile Tyr Asn Asp
145                 150                 155                 160

Ala Thr Tyr Leu Ile Pro Ile Phe Thr Leu Gly Lys Tyr Phe Gln Gln
                165                 170                 175

Asp Asn Lys Ile Leu Leu Pro Met Ser Val Gln Val His His Ala Val
            180                 185                 190

Cys Asp Gly Tyr His Thr Ser Arg Phe Phe Asn Glu Ala Gln Glu Leu
        195                 200                 205

Ala Ser Asn Tyr Glu Thr Trp Leu Gly Glu Lys
    210                 215

<210>  66
<211>  219
<212>  PRT
<213>  Clostridium perfringens

<400>  66

Met Lys Phe Asn Leu Ile Asp Ile Glu Asp Trp Asn Arg Lys Pro Tyr
1               5                   10                  15

Phe Glu His Tyr Leu Asn Ala Val Arg Cys Thr Tyr Ser Met Thr Ala
            20                  25                  30

Asn Ile Glu Ile Thr Gly Leu Leu Arg Glu Ile Lys Leu Lys Gly Leu
        35                  40                  45

Lys Leu Tyr Pro Thr Leu Ile Tyr Ile Ile Thr Thr Val Val Asn Arg
    50                  55                  60

His Lys Glu Phe Arg Thr Cys Phe Asp Gln Lys Gly Lys Leu Gly Tyr
65                  70                  75                  80

Trp Asp Ser Met Asn Pro Ser Tyr Thr Val Phe His Lys Asp Asn Glu
                85                  90                  95

Thr Phe Ser Ser Ile Trp Thr Glu Tyr Asp Glu Asn Phe Pro Arg Phe
            100                 105                 110

Tyr Tyr Asn Tyr Leu Glu Asp Ile Arg Asn Tyr Ser Asp Val Leu Asn
        115                 120                 125

Phe Met Pro Lys Thr Gly Glu Pro Ala Asn Thr Ile Asn Val Ser Ser
    130                 135                 140

Ile Pro Trp Val Asn Phe Thr Gly Phe Asn Leu Asn Ile Tyr Asn Asp
145                 150                 155                 160

Ala Thr Tyr Leu Ile Pro Ile Phe Thr Leu Gly Lys Tyr Phe Gln Gln
                165                 170                 175

Asp Asn Lys Ile Leu Leu Pro Met Ser Val Gln Val His His Ala Val
            180                 185                 190

Cys Asp Gly Tyr His Ile Ser Arg Phe Phe Asn Glu Ala Gln Glu Leu
        195                 200                 205

Ala Ser Asn Tyr Glu Thr Trp Leu Gly Glu Lys
    210                 215

<210>  67
<211>  218
<212>  PRT
<213>  Artificial Sequence

<220>
<223>  Clostrdium sp. BL8

<400>  67

Met Lys Phe Asn Leu Ile Asp Ile Asp Gln Trp Asp Arg Lys Pro Tyr
1               5                   10                  15

Phe Glu His Tyr Phe Asn Ser Val Lys Cys Thr Tyr Ser Ile Thr Ala
            20                  25                  30

Asn Ile Glu Ile Thr Asn Leu Leu Lys Asp Ile Lys Ile Thr Lys Leu
        35                  40                  45

Lys Leu Tyr Pro Thr Leu Ile Tyr Ile Ile Ala Thr Val Ile Asn Asn
    50                  55                  60

His Glu Glu Phe Arg Thr Cys Phe Asp Glu Asn Asn Asn Leu Gly Tyr
65                  70                  75                  80

Trp Asp Ser Met Ser Pro Asn Tyr Thr Ile Phe His Glu Glu Thr Lys
                85                  90                  95

Thr Phe Ser Asn Ile Trp Thr Glu Tyr Asp Lys Ser Phe Ser Gly Phe
            100                 105                 110

Tyr Asn Lys Tyr Val Glu Asp Asn Lys Asn Tyr Gly Asn Ile Met Asn
        115                 120                 125

Phe Asp Pro Lys Leu Asn Glu Pro Ala Asn Thr Phe Pro Ile Ser Cys
    130                 135                 140

Ile Pro Trp Val Ser Phe Thr Gly Phe Asn Leu Asn Ile Gln Asp His
145                 150                 155                 160

Gly Thr Tyr Leu Thr Pro Ile Phe Thr Leu Gly Lys Tyr Phe Glu Glu
                165                 170                 175

Asn Asn Lys Val Phe Ile Pro Met Ser Ile Gln Val His His Ala Val

180                 185                 190

Cys Asp Gly Tyr His Thr Ser Arg Phe Ile Asn Glu Val Gln Glu Leu
        195                 200                 205

Ala Ser Asn Ser Gln Ser Trp Leu Lys His
    210                 215

<210>  68
<211>  660
<212>  DNA
<213>  Clostridium perfringens

<400>  68
atgaaattta atttgataga tattgaggat tggaatagaa agccatactt tgagcattat      60

ttaaatgcgg ttaggtgcac ttacagtatg actgcaaata tagagataac tggtttactg     120

cgtgaaatta aacttaaggg cctgaaactg taccctacgc ttatttatat catcacaact     180

gtggttaacc gtcacaagga gttccgcacc tgttttgatc aaaaaggtaa gttaggatac     240

tgggatagta tgaacccaag ttatactgtc tttcataagg ataacgaaac tttttcaagt     300

atttggacag agtatgacga gaacttccca cgtttttact ataattacct tgaggatatt     360

agaaactata gcgacgtttt gaatttcatg cctaagacag gtgaacctgc taatacaatt     420

aatgtgtcca gcattccttg ggtgaatttt accggattca acctgaatat atacaatgat     480

gcaacatatc taatccctat ttttactttg ggtaagtatt ttcagcagga taataaaatt     540

ttattaccta tgtctgtaca ggtgcatcat gcggtttgcg acggttatca tataagcaga     600

ttttttaatg aggcacagga attagcgtca aattatgaga catggttagg agaaaaataa     660

<210>  69
<211>  624
<212>  DNA
<213>  Clostridium difficile

<400>  69
atggtatttg aaaaaattga taaaaatagt tggaacagaa aagagtattt tgaccactac      60

tttgcaagtg taccttgtac atacagcatg accgttaaag tggatatcac acaaataaag     120

gaaaagggaa tgaaactata tcctgcaatg ctttattata ttgcaatgat tgtaaaccgc     180

cattcagagt ttaggacggc aatcaatcaa gatggtgaat tggggatata tgatgagatg     240

ataccaagct atacaatatt tcacaatgat actgaaacat tttccagcct ttggactgag     300

tgtaagtctg actttaaatc atttttagca gattatgaaa gtgatacgca acggtatgga     360

aacaatcata gaatggaagg aaagccaaat gctccggaaa acatttttaa tgtatctatg     420

ataccgtggt caaccttcga tggctttaat ctgaatttgc agaaaggata tgattatttg     480

attcctattt ttactatggg gaaatattat aaagaagata acaaaattat acttcctttg     540

gcaattcaag ttcatcacgc agtatgtgac ggatttcaca tttgccgttt tgtaaacgaa 600

ttgcaggaat tgataaatag ttaa 624

<210> 70
<211> 624
<212> DNA
<213> Clostridium perfringens

<400> 70
atggtatttg aaaaaattga taaaaatagt tggaacagaa aagagtattt tgaccactac 60

tttgcaagtg taccttgtac atacagcatg accgttaaag tggatatcac acaaataaag 120

gaaaagggaa tgaaactata tcctgcaatg ctttattata ttgcaatgat tgtaaaccgc 180

cattcagagt ttaggacggc aatcaatcaa gatggtgaat tggggatata tgatgagatg 240

ataccaagct atacaatatt tcacaatgat actgaaacat tttccagcct ttggactgag 300

tgtaagtctg actttaaatc atttttagca gattatgaaa gtgatacgca acggtatgga 360

aacaatcata gaatggaagg aaagccaaat gctccggaaa acatttttaa tgtatctatg 420

ataccgtggt caaccttcga tggctttaat ctgaatttgc agaaaggata tgattatttg 480

attcctattt ttactatggg gaaatattat aaagaagata acaaaattat acttcctttg 540

gcaattcaag ttcatcacgc agtatgtgac ggatttcaca tttgccgttt tgtaaacgaa 600

ttgcaggaat tgataaatag ttaa 624

<210> 71
<211> 3897
<212> DNA
<213> Artificial Sequence

<220>
<223> MAD7 optimisée

<400> 71
ctcgagtccc tatcagtgat agattgaaac tctatcattg atagagtata atatctttgt 60

tcattagagc gataaacttg aatttgagag ggaacttaga tgaacaacgg cacaaataat 120

tttcagaact tcatagggat atcaagtttg cagaaaacgt taagaaatgc tttaataccc 180

acggaaacca cgcaacagtt catagttaag aacggaataa ttaaagaaga tgagttaaga 240

ggcgagaaca gacagatttt aaaagatata atggatgact actacagagg attcatatct 300

gagactttaa gttctattga tgacatagat tggactagct tattcgaaaa aatggaaatt 360

cagttaaaaa atggtgataa taaagatacc ttaattaagg aacagacaga gtatagaaaa 420

gcaatacata aaaaatttgc gaacgacgat agatttaaga acatgtttag cgccaaatta 480

attagtgaca tattacctga atttgttata cacaacaata attattcggc atcagagaaa 540

gaggaaaaaa cccaggtgat aaaattgttt tcgagatttg cgactagctt taaagattac 600

ttcaagaaca gagcaaattg cttttcagcg gacgatattt catcaagcag ctgccataga 660

atagttaacg acaatgcaga gatattcttt tcaaatgcgt tagtttacag aagaatagta 720

aaatcgttaa gcaatgacga tataaacaaa atttcgggcg atatgaaaga ttcattaaaa 780

gaaatgagtt tagaagaaat atattcttac gagaagtatg gggaatttat tacccaggaa 840

ggcattagct tctataatga tatatgtggg aaagtgaatt cttttatgaa cttatattgt 900

cagaaaaata aagaaaacaa aaatttatac aaacttcaga aacttcacaa acagattcta 960

tgcattgcgg acactagcta tgaggttccg tataaatttg aaagtgacga ggaagtgtac 1020

caatcagtta acggcttcct tgataacatt agcagcaaac atatagttga aagattaaga 1080

aaaataggcg ataactataa cggctacaac ttagataaaa tttatatagt gtccaaattt 1140

tacgagagcg ttagccaaaa aacctacaga gactgggaaa caattaatac cgccttagaa 1200

attcattaca ataatatatt gccgggtaac ggtaaaagta aagccgacaa agtaaaaaaa 1260

gcggttaaga atgatttaca gaaatccata accgaaataa atgaactagt gtcaaactat 1320

aagttatgca gtgacgacaa cataaaagcg gagacttata tacatgagat tagccatata 1380

ttgaataact ttgaagcaca ggaattgaaa tacaatccgg aaattcacct agttgaatcc 1440

gagttaaaag cgagtgagct taaaaacgtg ttagacgtga taatgaatgc gtttcattgg 1500

tgttcggttt ttatgactga ggaacttgtt gataaagaca acaattttta tgcggaatta 1560

gaggagattt acgatgaaat ttatccagta attagtttat acaacttagt tagaaactac 1620

gttacccaga aaccgtacag cacgaaaaag attaaattga actttggaat accgacgtta 1680

gcagacggtt ggtcaaagtc caaagagtat tctaataacg ctataatatt aatgagagac 1740

aatttatatt atttaggcat atttaatgcg aagaataaac cggacaagaa gattatagag 1800

ggtaatacgt cagaaaataa gggtgactac aaaaagatga tttataattt gttaccgggt 1860

cccaacaaaa tgataccgaa agttttcttg agcagcaaga cgggggtgga aacgtataaa 1920

ccgagcgcct atatactaga ggggtataaa cagaataaac atataaagtc ttcaaaagac 1980

tttgatataa ctttctgtca tgatttaata gactacttca aaaactgtat tgcaattcat 2040

cccgagtgga aaaacttcgg ttttgatttt agcgacacca gtacttatga agacatttcc 2100

gggttttata gagaggtaga gttacaaggt tacaagattg attggacata cattagcgaa 2160

aaagacattg atttattaca ggaaaaaggt caattatatt tattccagat atataacaaa 2220

gatttttcga aaaaatcaac cgggaatgac aaccttcaca ccatgtactt aaaaaatctt 2280

ttctcagaag aaaatcttaa ggatatagtt ttaaaactta acggcgaagc ggaaatattc 2340

ttcaggaaga gcagcataaa gaacccaata attcataaaa aaggctcgat tttagttaac 2400

agaacctacg aagcagaaga aaaagaccag tttggcaaca ttcaaattgt gagaaaaaat 2460

attccggaaa acatttatca ggagttatac aaatacttca acgataaaag cgacaaagag 2520

ttatctgatg aagcagccaa attaaagaat gtagtgggac accacgaggc agcgacgaat 2580

atagttaagg actatagata cacgtatgat aaatacttcc ttcatatgcc tattacgata 2640

aatttcaaag ccaataaaac gggttttatt aatgatagga tattacagta tatagctaaa 2700

gaaaaagact tacatgtgat aggcattgat agaggcgaga gaaacttaat atacgtgtcc 2760

gtgattgata cttgtggtaa tatagttgaa cagaaaagct ttaacattgt aaacggctac 2820

gactatcaga taaaattaaa acaacaggag ggcgctagac agattgcgag aaaagaatgg 2880

aaagaaattg gtaaaattaa agagataaaa gagggctact taagcttagt aatacacgag 2940

atatctaaaa tggtaataaa atacaatgca attatagcga tggaggattt gtcttatggt 3000

tttaaaaaag ggagatttaa ggttgaaaga caagtttacc agaaatttga aaccatgtta 3060

ataaataaat taaactattt agtatttaaa gatatttcga ttaccgagaa tggcggttta 3120

ttaaaaggtt atcagttaac atacattcct gataaactta aaaacgtggg tcatcagtgc 3180

ggctgcattt tttatgtgcc tgctgcatac acgagcaaaa ttgatccgac caccggcttt 3240

gtgaatatat ttaaatttaa agacttaaca gtggacgcaa aaagagaatt cattaaaaaa 3300

tttgactcaa ttagatatga cagtgaaaaa aatttattct gctttacatt tgactacaat 3360

aactttatta cgcaaaacac ggttatgagc aaatcatcgt ggagtgtgta tacatacggc 3420

gtgagaataa aaagaagatt tgtgaacggc agattctcaa acgaaagtga taccattgac 3480

ataaccaaag atatggagaa aacgttggaa atgacggaca ttaactggag agatggccac 3540

gatcttagac aagacattat agattatgaa attgttcagc acatattcga aattttcaga 3600

ttaacagtgc aaatgagaaa ctccttgtct gaattagagg acagagatta cgatagatta 3660

atttcacctg tattaaacga aaataacatt ttttatgaca gcgcgaaagc gggggatgca 3720

cttcctaagg atgccgatgc aaatggtgcg tattgtattg cattaaaagg gttatatgaa 3780

attaaacaaa ttaccgaaaa ttggaaagaa gatggtaaat tttcgagaga taaattaaaa 3840

ataagcaata aagattggtt cgactttata cagaataaga gatatttata agtcgac 3897

<210> 72
<211> 1263
<212> PRT
<213> Artificial Sequence

<220>
<223> MAD7

<400> 72

Met Asn Asn Gly Thr Asn Asn Phe Gln Asn Phe Ile Gly Ile Ser Ser
1               5                   10                  15

Leu Gln Lys Thr Leu Arg Asn Ala Leu Ile Pro Thr Glu Thr Thr Gln
            20                  25                  30

Gln Phe Ile Val Lys Asn Gly Ile Ile Lys Glu Asp Glu Leu Arg Gly
        35                  40                  45

Glu Asn Arg Gln Ile Leu Lys Asp Ile Met Asp Asp Tyr Tyr Arg Gly
    50                  55                  60

Phe Ile Ser Glu Thr Leu Ser Ser Ile Asp Asp Ile Asp Trp Thr Ser
65                  70                  75                  80

Leu Phe Glu Lys Met Glu Ile Gln Leu Lys Asn Gly Asp Asn Lys Asp
                85                  90                  95

Thr Leu Ile Lys Glu Gln Thr Glu Tyr Arg Lys Ala Ile His Lys Lys
            100                 105                 110

Phe Ala Asn Asp Asp Arg Phe Lys Asn Met Phe Ser Ala Lys Leu Ile
        115                 120                 125

Ser Asp Ile Leu Pro Glu Phe Val Ile His Asn Asn Asn Tyr Ser Ala
    130                 135                 140

Ser Glu Lys Glu Glu Lys Thr Gln Val Ile Lys Leu Phe Ser Arg Phe
145                 150                 155                 160

Ala Thr Ser Phe Lys Asp Tyr Phe Lys Asn Arg Ala Asn Cys Phe Ser
                165                 170                 175

Ala Asp Asp Ile Ser Ser Ser Ser Cys His Arg Ile Val Asn Asp Asn
            180                 185                 190

Ala Glu Ile Phe Phe Ser Asn Ala Leu Val Tyr Arg Arg Ile Val Lys
        195                 200                 205

Ser Leu Ser Asn Asp Asp Ile Asn Lys Ile Ser Gly Asp Met Lys Asp
    210                 215                 220

Ser Leu Lys Glu Met Ser Leu Glu Glu Ile Tyr Ser Tyr Glu Lys Tyr
225                 230                 235                 240

Gly Glu Phe Ile Thr Gln Glu Gly Ile Ser Phe Tyr Asn Asp Ile Cys
                245                 250                 255

Gly Lys Val Asn Ser Phe Met Asn Leu Tyr Cys Gln Lys Asn Lys Glu
            260                 265                 270

Asn Lys Asn Leu Tyr Lys Leu Gln Lys Leu His Lys Gln Ile Leu Cys
        275                 280                 285

Ile Ala Asp Thr Ser Tyr Glu Val Pro Tyr Lys Phe Glu Ser Asp Glu
    290                 295                 300

Glu Val Tyr Gln Ser Val Asn Gly Phe Leu Asp Asn Ile Ser Ser Lys
305                 310                 315                 320

His Ile Val Glu Arg Leu Arg Lys Ile Gly Asp Asn Tyr Asn Gly Tyr
                325                 330                 335

Asn Leu Asp Lys Ile Tyr Ile Val Ser Lys Phe Tyr Glu Ser Val Ser
            340                 345                 350

Gln Lys Thr Tyr Arg Asp Trp Glu Thr Ile Asn Thr Ala Leu Glu Ile
        355                 360                 365

His Tyr Asn Asn Ile Leu Pro Gly Asn Gly Lys Ser Lys Ala Asp Lys
    370                 375                 380

Val Lys Lys Ala Val Lys Asn Asp Leu Gln Lys Ser Ile Thr Glu Ile
385                 390                 395                 400

Asn Glu Leu Val Ser Asn Tyr Lys Leu Cys Ser Asp Asp Asn Ile Lys
                405                 410                 415

Ala Glu Thr Tyr Ile His Glu Ile Ser His Ile Leu Asn Asn Phe Glu
            420                 425                 430

Ala Gln Glu Leu Lys Tyr Asn Pro Glu Ile His Leu Val Glu Ser Glu
        435                 440                 445

Leu Lys Ala Ser Glu Leu Lys Asn Val Leu Asp Val Ile Met Asn Ala
    450                 455                 460

Phe His Trp Cys Ser Val Phe Met Thr Glu Glu Leu Val Asp Lys Asp
465                 470                 475                 480

Asn Asn Phe Tyr Ala Glu Leu Glu Glu Ile Tyr Asp Glu Ile Tyr Pro
                485                 490                 495

Val Ile Ser Leu Tyr Asn Leu Val Arg Asn Tyr Val Thr Gln Lys Pro
            500                 505                 510

Tyr Ser Thr Lys Lys Ile Lys Leu Asn Phe Gly Ile Pro Thr Leu Ala
        515                 520                 525

Asp Gly Trp Ser Lys Ser Lys Glu Tyr Ser Asn Asn Ala Ile Ile Leu
    530                 535                 540

Met Arg Asp Asn Leu Tyr Tyr Leu Gly Ile Phe Asn Ala Lys Asn Lys
545                 550                 555                 560

Pro Asp Lys Lys Ile Ile Glu Gly Asn Thr Ser Glu Asn Lys Gly Asp
                565                 570                 575

Tyr Lys Lys Met Ile Tyr Asn Leu Leu Pro Gly Pro Asn Lys Met Ile
            580                 585                 590

Pro Lys Val Phe Leu Ser Ser Lys Thr Gly Val Glu Thr Tyr Lys Pro
        595                 600                 605

Ser Ala Tyr Ile Leu Glu Gly Tyr Lys Gln Asn Lys His Ile Lys Ser
    610                 615                 620

Ser Lys Asp Phe Asp Ile Thr Phe Cys His Asp Leu Ile Asp Tyr Phe
625                 630                 635                 640

Lys Asn Cys Ile Ala Ile His Pro Glu Trp Lys Asn Phe Gly Phe Asp
                645                 650                 655

Phe Ser Asp Thr Ser Thr Tyr Glu Asp Ile Ser Gly Phe Tyr Arg Glu
            660                 665                 670

Val Glu Leu Gln Gly Tyr Lys Ile Asp Trp Thr Tyr Ile Ser Glu Lys
        675                 680                 685

Asp Ile Asp Leu Leu Gln Glu Lys Gly Gln Leu Tyr Leu Phe Gln Ile
    690                 695                 700

Tyr Asn Lys Asp Phe Ser Lys Lys Ser Thr Gly Asn Asp Asn Leu His
705                 710                 715                 720

Thr Met Tyr Leu Lys Asn Leu Phe Ser Glu Glu Asn Leu Lys Asp Ile
                725                 730                 735

Val Leu Lys Leu Asn Gly Glu Ala Glu Ile Phe Phe Arg Lys Ser Ser
            740                 745                 750

Ile Lys Asn Pro Ile Ile His Lys Lys Gly Ser Ile Leu Val Asn Arg
        755                 760                 765

Thr Tyr Glu Ala Glu Glu Lys Asp Gln Phe Gly Asn Ile Gln Ile Val

770                 775                 780

Arg Lys Asn Ile Pro Glu Asn Ile Tyr Gln Glu Leu Tyr Lys Tyr Phe
785                 790                 795                 800

Asn Asp Lys Ser Asp Lys Glu Leu Ser Asp Glu Ala Ala Lys Leu Lys
                805                 810                 815

Asn Val Val Gly His His Glu Ala Ala Thr Asn Ile Val Lys Asp Tyr
            820                 825                 830

Arg Tyr Thr Tyr Asp Lys Tyr Phe Leu His Met Pro Ile Thr Ile Asn
        835                 840                 845

Phe Lys Ala Asn Lys Thr Gly Phe Ile Asn Asp Arg Ile Leu Gln Tyr
    850                 855                 860

Ile Ala Lys Glu Lys Asp Leu His Val Ile Gly Ile Asp Arg Gly Glu
865                 870                 875                 880

Arg Asn Leu Ile Tyr Val Ser Val Ile Asp Thr Cys Gly Asn Ile Val
                885                 890                 895

Glu Gln Lys Ser Phe Asn Ile Val Asn Gly Tyr Asp Tyr Gln Ile Lys
            900                 905                 910

Leu Lys Gln Gln Glu Gly Ala Arg Gln Ile Ala Arg Lys Glu Trp Lys
        915                 920                 925

Glu Ile Gly Lys Ile Lys Glu Ile Lys Glu Gly Tyr Leu Ser Leu Val
    930                 935                 940

Ile His Glu Ile Ser Lys Met Val Ile Lys Tyr Asn Ala Ile Ile Ala
945                 950                 955                 960

Met Glu Asp Leu Ser Tyr Gly Phe Lys Lys Gly Arg Phe Lys Val Glu
                965                 970                 975

Arg Gln Val Tyr Gln Lys Phe Glu Thr Met Leu Ile Asn Lys Leu Asn
            980                 985                 990

Tyr Leu Val Phe Lys Asp Ile Ser  Ile Thr Glu Asn Gly  Gly Leu Leu
        995                 1000                1005

Lys Gly  Tyr Gln Leu Thr Tyr  Ile Pro Asp Lys Leu  Lys Asn Val
    1010                1015                1020

Gly His Gln Cys Gly Cys Ile Phe Tyr Val Pro Ala Ala Tyr Thr
    1025                1030                1035

Ser Lys Ile Asp Pro Thr Thr Gly Phe Val Asn Ile Phe Lys Phe
    1040                1045                1050

Lys Asp Leu Thr Val Asp Ala Lys Arg Glu Phe Ile Lys Lys Phe
    1055                1060                1065

Asp Ser Ile Arg Tyr Asp Ser Glu Lys Asn Leu Phe Cys Phe Thr
    1070                1075                1080

Phe Asp Tyr Asn Asn Phe Ile Thr Gln Asn Thr Val Met Ser Lys
    1085                1090                1095

Ser Ser Trp Ser Val Tyr Thr Tyr Gly Val Arg Ile Lys Arg Arg
    1100                1105                1110

Phe Val Asn Gly Arg Phe Ser Asn Glu Ser Asp Thr Ile Asp Ile
    1115                1120                1125

Thr Lys Asp Met Glu Lys Thr Leu Glu Met Thr Asp Ile Asn Trp
    1130                1135                1140

Arg Asp Gly His Asp Leu Arg Gln Asp Ile Ile Asp Tyr Glu Ile
    1145                1150                1155

Val Gln His Ile Phe Glu Ile Phe Arg Leu Thr Val Gln Met Arg
    1160                1165                1170

Asn Ser Leu Ser Glu Leu Glu Asp Arg Asp Tyr Asp Arg Leu Ile
    1175                1180                1185

Ser Pro Val Leu Asn Glu Asn Asn Ile Phe Tyr Asp Ser Ala Lys
    1190                1195                1200

Ala Gly Asp Ala Leu Pro Lys Asp Ala Asp Ala Asn Gly Ala Tyr
    1205                1210                1215

Cys Ile Ala Leu Lys Gly Leu Tyr Glu Ile Lys Gln Ile Thr Glu
    1220                1225                1230

Asn Trp Lys Glu Asp Gly Lys Phe Ser Arg Asp Lys Leu Lys Ile
    1235                1240                1245

Ser Asn Lys Asp Trp Phe Asp Phe Ile Gln Asn Lys Arg Tyr Leu
    1250                1255                1260

<210>  73
<211>  363
<212>  DNA
<213>  Artificial Sequence

<220>
<223>  Promoteur CatB

<400>  73
taaaaaatgt tacgcacttt tcttatattg ttcaacaata acataattta ttaacaaaag      60

gaaagtatag ttaaaaaaat gttggagcaa atgcggatgg aaaaataaaa attaatatta     120

gtagtaattc cgatgttaaa ataacaagag ataagaaaaa gtaaaatatt agagtaattc     180

gtagtattct taagttatga atcaataaaa aatggtctct gaaaattgaa tagttcggta     240

ttacagaatg tgctataata aactaaagcg taaatatcat tgtaaaaagg agattgaaat     300

ggctaggtca cggaaaaaag ccttctaaaa tagaattacg aaaattttta ggaggcccga     360

att                                                                   363


<210>  74
<211>  322
<212>  DNA
<213>  Artificial Sequence

<220>
<223>  Promoteur CATQ

<400>  74
ctgcgtacac atccagacat cgctttagag tatggtgaat taaagatgga gcgggcttat      60

cgattctcag aggatattga aggctactgc actggtaagg atgcatttgt aaagcaacta     120

gaaaaggatg ctttgcgatg gtggcaaact gtctgttagg aggttattct caaaggattg     180

caagaagcag ttgaggataa tccgtataac taactattac acattcttaa cattgctggt     240

ttgtatcggt agaataacac gaattaacaa aggatatatt ttgtagtagc aagtgtattt     300

gttttatatt ctatgaacct at                                              322


<210>  75
<211>  1368
<212>  PRT
<213>  Streptococcus pyogenes

<400>  75

<210> 76
<211> 4107
<212> DNA
<213> Streptococcus pyogenes

<400> 76
atggataaga aatactcaat aggcttagat atcggcacaa atagcgtcgg atgggcggtg 60

atcactgatg aatataaggt tccgtctaaa aagttcaagg ttctgggaaa tacagaccgc 120

cacagtatca aaaaaaatct tataggggct cttttatttg acagtggaga gacagcggaa 180

gcgactcgtc tcaaacggac agctcgtaga aggtatacac gtcggaagaa tcgtatttgt 240

tatctacagg agattttttc aaatgagatg gcgaaagtag atgatagttt ctttcatcga 300

cttgaagagt cttttttggt ggaagaagac aagaagcatg aacgtcatcc tatttttgga 360

aatatagtag atgaagttgc ttatcatgag aaatatccaa ctatctatca tctgcgaaaa 420

aaattggtag attctactga taaagcggat ttgcgcttaa tctatttggc cttagcgcat 480

atgattaagt ttcgtggtca ttttttgatt gagggagatt taaatcctga taatagtgat 540

gtggacaaac tatttatcca gttggtacaa acctacaatc aattatttga agaaaaccct 600

attaacgcaa gtggagtaga tgctaaagcg attctttctg cacgattgag taaatcaaga 660

cgattagaaa atctcattgc tcagctcccc ggtgagaaga aaaatggctt atttgggaat 720

ctcattgctt tgtcattggg tttgacccct aattttaaat caaattttga tttggcagaa 780

gatgctaaat tacagctttc aaaagatact tacgatgatg atttagataa tttattggcg 840

caaattggag atcaatatgc tgatttgttt ttggcagcta agaatttatc agatgctatt 900

ttactttcag atatcctaag agtaaatact gaaataacta aggctcccct atcagcttca 960

atgattaaac gctacgatga acatcatcaa gacttgactc ttttaaaagc tttagttcga 1020

caacaacttc cagaaaagta taaagaaatc ttttttgatc aatcaaaaaa cggatatgca 1080

ggttatattg atgggggagc tagccaagaa gaattttata aatttatcaa accaatttta 1140

gaaaaaatgg atggtactga ggaattattg gtgaaactaa atcgtgaaga tttgctgcgc 1200

aagcaacgga cctttgacaa cggctctatt ccccatcaaa ttcacttggg tgagctgcat 1260

gctattttga gaagacaaga agacttttat ccatttttaa aagacaatcg tgagaagatt 1320

gaaaaaatct tgacttttcg aattccttat tatgttggtc cattggcgcg tggcaatagt 1380

cgttttgcat ggatgactcg gaagtctgaa gaaacaatta ccccatggaa ttttgaagaa 1440

gttgtcgata aaggtgcttc agctcaatca tttattgaac gcatgacaaa ctttgataaa 1500

aatcttccaa atgaaaaagt actaccaaaa catagtttgc tttatgagta ttttacggtt 1560

tataacgaat tgacaaaggt caaatatgtt actgaaggaa tgcgaaaacc agcatttctt 1620

tcaggtgaac agaagaaagc cattgttgat ttactcttca aaacaaatcg aaaagtaacc 1680

gttaagcaat taaaagaaga ttatttcaaa aaaatagaat gttttgatag tgttgaaatt 1740

tcaggagttg aagatagatt taatgcttca ttaggtacct accatgattt gctaaaaatt 1800

attaaagata aagatttttt ggataatgaa gaaaatgaag atatcttaga ggatattgtt 1860

ttaacattga ccttatttga agatagggag atgattgagg aaagacttaa aacatatgct 1920

cacctctttg atgataaggt gatgaaacag cttaaacgtc gccgttatac tggttgggga 1980

cgtttgtctc gaaaattgat taatggtatt agggataagc aatctggcaa aacaatatta 2040

gattttttga aatcagatgg ttttgccaat cgcaatttta tgcagctgat ccatgatgat 2100

agtttgacat ttaaagaaga cattcaaaaa gcacaagtgt ctggacaagg cgatagttta 2160

catgaacata ttgcaaattt agctggtagc cctgctatta aaaaaggtat tttacagact 2220

gtaaaagttg ttgatgaatt ggtcaaagta atggggcggc ataagccaga aaatatcgtt 2280

attgaaatgg cacgtgaaaa tcagacaact caaaagggcc agaaaaattc gcgagagcgt 2340

atgaaacgaa tcgaagaagg tatcaaagaa ttaggaagtc agattcttaa agagcatcct 2400

gttgaaaata ctcaattgca aaatgaaaag ctctatctct attatctcca aaatggaaga 2460

gacatgtatg tggaccaaga attagatatt aatcgtttaa gtgattatga tgtcgatcac 2520

attgttccac aaagtttcct taaagacgat tcaatagaca ataaggtctt aacgcgttct 2580

gataaaaatc gtggtaaatc ggataacgtt ccaagtgaag aagtagtcaa aaagatgaaa 2640

aactattgga gacaacttct aaacgccaag ttaatcactc aacgtaagtt tgataattta 2700

acgaaagctg aacgtggagg tttgagtgaa cttgataaag ctggttttat caaacgccaa 2760

ttggttgaaa ctcgccaaat cactaagcat gtggcacaaa ttttggatag tcgcatgaat 2820

actaaatacg atgaaaatga taaacttatt cgagaggtta aagtgattac cttaaaatct 2880

aaattagttt ctgacttccg aaaagatttc caattctata aagtacgtga gattaacaat 2940

taccatcatg cccatgatgc gtatctaaat gccgtcgttg gaactgcttt gattaagaaa 3000

tatccaaaac ttgaatcgga gtttgtctat ggtgattata aagtttatga tgttcgtaaa 3060

atgattgcta agtctgagca agaaataggc aaagcaaccg caaaatattt cttttactct 3120

aatatcatga acttcttcaa aacagaaatt acacttgcaa atggagagat tcgcaaacgc 3180

cctctaatcg aaactaatgg ggaaactgga gaaattgtct gggataaagg gcgagatttt 3240

gccacagtgc gcaaagtatt gtccatgccc caagtcaata ttgtcaagaa aacagaagta 3300

cagacaggcg gattctccaa ggagtcaatt ttaccaaaaa gaaattcgga caagcttatt 3360

gctcgtaaaa aagactggga tccaaaaaaa tatggtggtt ttgatagtcc aacggtagct 3420

tattcagtcc tagtggttgc taaggtggaa aaagggaaat cgaagaagtt aaaatccgtt 3480

aaagagttac tagggatcac aattatggaa agaagttcct ttgaaaaaaa tccgattgac 3540

tttttagaag ctaaaggata taaggaagtt aaaaaagact taatcattaa actacctaaa 3600

tatagtcttt ttgagttaga aaacggtcgt aaacggatgc tggctagtgc cggagaatta 3660

caaaaaggaa atgagctggc tctgccaagc aaatatgtga attttttata tttagctagt 3720

cattatgaaa agttgaaggg tagtccagaa gataacgaac aaaaacaatt gtttgtggag 3780

cagcataagc attatttaga tgagattatt gagcaaatca gtgaattttc taagcgtgtt 3840

attttagcag atgccaattt agataaagtt cttagtgcat ataacaaaca tagagacaaa 3900

ccaatacgtg aacaagcaga aaatattatt catttattta cgttgacgaa tcttggagct 3960

cccgctgctt ttaaatattt tgatacaaca attgatcgta aacgatatac gtctacaaaa 4020

gaagttttag atgccactct tatccatcaa tccatcactg gtctttatga aacacgcatt 4080

gatttgagtc agctaggagg tgactga 4107

<210> 77
<211> 1170
<212> DNA
<213> Artificial Sequence

<220>
<223> bdhA

<400> 77
atgctaagtt ttgattattc aataccaact aaagtttttt ttggaaaagg aaaaatagac 60

gtaattggag aagaaattaa gaaatatggc tcaagagtgc ttatagttta tggcggagga 120

agtataaaaa ggaacggtat atatgataga gcaacagcta tattaaaaga aaacaatata 180

gctttctatg aactttcagg agtagagcca aatcctagga taacaacagt aaaaaaaggc 240

atagaaatat gtagagaaaa taatgtggat ttagtattag caataggggg aggaagtgca 300

atagactgtt ctaaggtaat tgcagctgga gtttattatg atggcgatac atgggacatg 360

gttaaagatc catctaaaat aactaaagtt cttccaattg caagtatact tactctttca 420

gcaacagggt ctgaaatgga tcaaattgca gtaatttcaa atatggagac taatgaaaag 480

cttggagtag gacatgatga tatgagacct aaattttcag tgttagatcc tacatatact 540

tttacagtac ctaaaaatca aacagcagcg ggaacagctg acattatgag tcacaccttt 600

gaatcttact ttagtggtgt tgaaggtgct tatgtgcagg acggtatagc agaagcaatc 660

ttaagaacat gtataaagta tggaaaaata gcaatggaga agactgatga ttacgaggct 720

agagctaatt tgatgtgggc ttcaagttta gctataaatg gtctattatc acttggtaag 780

gatagaaaat ggagttgtca tcctatggaa cacgagttaa gtgcatatta tgatataaca 840

catggtgtag gacttgcaat tttaacacct aattggatgg aatatattct aaatgacgat 900

acacttcata aatttgtttc ttatggaata aatgtttggg gaatagacaa gaacaaagat 960

aactatgaaa tagcacgaga ggctattaaa aatacgagag aatactttaa ttcattgggt 1020

attccttcaa agcttagaga agttggaata ggaaaagata aactagaact aatggcaaag 1080

caagctgtta gaaattctgg aggaacaata ggaagtttaa gaccaataaa tgcagaggat 1140

gttcttgaga tatttaaaaa atcttattaa 1170

<210> 78
<211> 1173
<212> DNA
<213> Artificial Sequence

<220>
<223> bdhB

<400> 78
gtggttgatt tcgaatattc aataccaact agaatttttt tcggtaaaga taagataaat 60

gtacttggaa gagagcttaa aaaatatggt tctaaagtgc ttatagttta tggtggagga 120

agtataaaga gaaatggaat atatgataaa gctgtaagta tacttgaaaa aaacagtatt 180

aaattttatg aacttgcagg agtagagcca aatccaagag taactacagt tgaaaaagga 240

gttaaaatat gtagagaaaa tggagttgaa gtagtactag ctataggtgg aggaagtgca 300

atagattgcg caaaggttat agcagcagca tgtgaatatg atggaaatcc atgggatatt 360

gtgttagatg gctcaaaaat aaaaagggtg cttcctatag ctagtatatt aaccattgct 420

gcaacaggat cagaaatgga tacgtgggca gtaataaata atatggatac aaacgaaaaa 480

ctaattgcgg cacatccaga tatggctcct aagttttcta tattagatcc aacgtatacg 540

tataccgtac ctaccaatca aacagcagca ggaacagctg atattatgag tcatatattt 600

gaggtgtatt ttagtaatac aaaaacagca tatttgcagg atagaatggc agaagcgtta 660

ttaagaactt gtattaaata tggaggaata gctcttgaga agccggatga ttatgaggca 720

agagccaatc taatgtgggc ttcaagtctt gcgataaatg gacttttaac atatggtaaa 780

gacactaatt ggagtgtaca cttaatggaa catgaattaa gtgcttatta cgacataaca 840

cacggcgtag ggcttgcaat tttaacacct aattggatgg agtatatttt aaataatgat 900

acagtgtaca agtttgttga atatggtgta aatgtttggg gaatagacaa agaaaaaaat 960

cactatgaca tagcacatca agcaatacaa aaaacaagag attactttgt aaatgtacta 1020

ggtttaccat ctagactgag agatgttgga attgaagaag aaaaattgga cataatggca 1080

aaggaatcag taaagcttac aggaggaacc ataggaaacc taagaccagt aaacgcctcc 1140

gaagtcctac aaatattcaa aaaatctgtg taa 1173

<210> 79
<211> 6560
<212> DNA
<213> Artificial Sequence

<220>
<223> pGRNA-deltabdhB

<400> 79
gatccccggg taccgagctc gaattcgtaa tcatggtcat agctgtttcc tgtgtgaaat 60

tgttatccgc tcacaattcc acacaacata cgagccggaa gcataaagtg taaagcctgg 120

ggtgcctaat gagtgagcta actcacatta attgcgttgc gctcactgcc cgctttccag 180

tcgggaaacc tgtcgtgcca gctgcattaa tgaatcggcc aacgcgcggg gagaggcggt 240

ttgcgtattg ggcgctcttc cgcttcctcg ctcactgact cgctgcgctc ggtcgttcgg 300

ctgcggcgag cggtatcagc tcactcaaag gcggtaatac ggttatccac agaatcaggg 360

gataacgcag gaaagaacat gtgagcaaaa ggccagcaaa aggccaggaa ccgtaaaaag 420

gccgcgttgc tggcgttttt ccataggctc cgcccccctg acgagcatca caaaaatcga 480

cgctcaagtc agaggtggcg aaacccgaca ggactataaa gataccaggc gtttccccct 540

ggaagctccc tcgtgcgctc tcctgttccg accctgccgc ttaccggata cctgtccgcc 600

tttctccctt cgggaagcgt ggcgctttct catagctcac gctgtaggta tctcagttcg 660

gtgtaggtcg ttcgctccaa gctgggctgt gtgcacgaac cccccgttca gcccgaccgc 720

tgcgccttat ccggtaacta tcgtcttgag tccaacccgg taagacacga cttatcgcca 780

ctggcagcag ccactggtaa caggattagc agagcgaggt atgtaggcgg tgctacagag 840

ttcttgaagt ggtggcctaa ctacggctac actagaagaa cagtatttgg tatctgcgct 900

ctgctgaagc cagttacctt cggaaaaaga gttggtagct cttgatccgg caaacaaacc 960

accgctggta gcggtggttt ttttgtttgc aagcagcaga ttacgcgcag aaaaaaagga 1020

tctcaagaag atcctttgat cttttctacg gggtctgacg ctcagtggaa cgaaaactca 1080

cgttaaggga ttttggtcat gagattatca aaaaggatct tcacctagat ccttttaaat 1140

taaaaatgaa gttttaaatc aatctaaagt atatatgagt aaacttggtc tgacagttac 1200

caaagctagc ttaatactag tatatactta atgtgataag tgtctgacag ctgaccggtc 1260

taaagaggtc cctagcgcct acggggaatt tgtatcgata aggggtacaa attcccacta 1320

agcgctcggc cggggatcga tccccgggta cgtacccggc agtttttctt tttcggcaag 1380

tgttcaagaa gttattaagt cgggagtgca gtcgaagtgg gcaagttgaa aaattcacaa 1440

aaatgtggta taatatcttt gttcattaga gcgataaact tgaatttgag agggaactta 1500

gatggtattt gaaaaaattg ataaaaatag ttggaacaga aaagagtatt ttgaccacta 1560

ctttgcaagt gtaccttgta cctacagcat gaccgttaaa gtggatatca cacaaataaa 1620

ggaaaaggga atgaaactat atcctgcaat gctttattat attgcaatga ttgtaaaccg 1680

ccattcagag tttaggacgg caatcaatca agatggtgaa ttggggatat atgatgagat 1740

gataccaagc tatacaatat ttcacaatga tactgaaaca ttttccagcc tttggactga 1800

gtgtaagtct gactttaaat catttttagc agattatgaa agtgatacgc aacggtatgg 1860

aaacaatcat agaatggaag gaaagccaaa tgctccggaa aacattttta atgtatctat 1920

gataccgtgg tcaaccttcg atggctttaa tctgaatttg cagaaaggat atgattattt 1980

gattcctatt tttactatgg ggaaatatta taaagaagat aacaaaatta tacttccttt 2040

ggcaattcaa gttcatcacg cagtatgtga cggatttcac atttgccgtt ttgtaaacga 2100

attgcaggaa ttgataaata gttaacttca ggtttgtctg taactaaaaa ctagtattta 2160

acctaggatc aaaaaaattt ccaataatcc cactctaagc cacaaacacg ccctataaaa 2220

tcccgcttta atcccacttt gagacacatg taatattact ttacgcccta gtatagtgat 2280

aattttttac attcaatgcc acgcaaaaaa ataaaggggc actataataa aagttccttc 2340

ggaactaact aaagtaaaaa attatcttta caacctcccc aaaaaaaaga acaggtacaa 2400

agtaccctat aatacaagcg taaaaaaaat gagggtaaaa ataaaaaaat aaaaaaataa 2460

aaaaataaaa aaataaaaaa ataaaaaaat aaaaaaatat aaaaataaaa aaatataaaa 2520

ataaaaaaat ataaaaataa aaaaataaaa aaatataaaa ataaaaaaat aaaaaaatat 2580

aaaaatattt tttatttaaa gtttgaaaaa aattttttta tattatataa tctttgaaga 2640

aaagaatata aaaaatgagc ctttataaaa gcccattttt tttcatatac gtaatatgac 2700

gttctaatgt ttttattggt acttctaaca ttagagtaat ttctttattt ttaaagcctt 2760

tttctttaag ggcttttatt ttttttctta atacatttaa ttcctctttt tttgttgctt 2820

ttcctttagc ttttaattgc tcttgataat tttttttacc tctaatattt tctcttctct 2880

tatattcctt tttagaaatt attattgtca tatatttttg ttcttcttct gtaatttcta 2940

ataactctat aagagtttca ttcttatact tatattgctt atttttatct aaataacatc 3000

tttcagcact tctagttgct cttataactt ctctttcact taaatgttgt ctaaacatac 3060

tattaagttc taaaacatca tttaatgcct tctcaatgtc ttctgtaaag ctacaaagat 3120

aatatctata taaaaataat ataagctctc tgtgtccttt taaatcatat tctcttagtt 3180

cacaaagttt tattatgtct tgtattcttc cataatataa acttctttct ctataaatat 3240

aatttatttt gcttggtcta ccctttttcc tttcatatgg ttttaattca ggtaaaaatc 3300

cattttgtat ttctcttaag tcataaatat attcgtactc atctaatata ttgactactg 3360

tttttgattt agagtttata cttcctggaa ctcttaatat tctcgttgca tctaaggctt 3420

gtctatctgc tccaaagtat tttaattgat tatataaata ttcttgaacc gctttccata 3480

atggtaatgc tttactaggt actgcattta ttatccatat taaatacatt cctcttccac 3540

tatctattac atagtttggt ataggaatac tttgattaaa ataattcttt tctaagtcca 3600

ttaatacctg gtctttagtt ttgccagttt tataataatc caagtctata aacagtgtat 3660

ttaactcttt tatattttct aatcgcctac acggcttata aaaggtattt agagttatat 3720

agatattttc atcactcata tctaaatctt ttaattcagc gtatttatag tgccattggc 3780

tatatccttt tttatctata acgctcctgg ttatccaccc tttacttcta ctatgaatat 3840

tatctatata gttcttttta ttcagcttta atgcgtttct cacttattca cctccccttc 3900

tgtaaaacta agaaaattat atcatatttt caataattat taactattct taaactctta 3960

ataaaaaata gagtaagtcc ccaattgaaa cttaatctat tttttatgtt ttaatttatt 4020

atttttatta aaatatttta aactaaatta aatgattctt tttaattttt tactatttca 4080

ttccataata tattactata attatttaca aataatattt cttcatttgt aatatttaga 4140

tgatttacta attttagttt ttatatatta aataattaat gtataattta tataaaaaat 4200

caaaggagct tataaattat gattatttcc aaagatacta aagatttaat ttttttcaat 4260

tttaacaata ctttttgtaa tattatgttt aaatttaatt gtattttttt catataataa 4320

agccgttgaa gtaaaccaat ccattttcct tatgatgtta ttattaaatt taagttttat 4380

aataatatct ttattatatt tattgttttt aaaaaaacta gtgaaatttc tagtgaaatt 4440

tccggcttta ttaaacttat ttttaggaat tttattttca ttttcatctt tacaggattt 4500

gattatatct ttaaatatgt tttatcaaat attatctttt tctaaattta tatatatttt 4560

tattatattt attattatat atattttatt tttaagtttc tttctaacag ctattaaaaa 4620

gaaacttaaa aataaaaaca cgtactctaa accaataaat aaaactattt ttattattgc 4680

tgccttgatt ggaatagttt ttagtaaaat taatttcaat attccacaat attatattat 4740

aagctagcac gcctcgagac tctatcattg atagagtttg aaactctatc attgatagag 4800

tataatatct ttgttcatgc ttattacgac ataacacagt tttagagcta gaaatagcaa 4860

gttaaaataa ggctagtccg ttatcaactt gaaaaagtgg caccgagtcg gtgctttttt 4920

tgaagcttct cgagatctcc atggacgcgt gacgtcgact cttaagaaca tgtataaagt 4980

atggaaaaat agcaatggag aagactgatg attacgaggc tagagctaat ttgatgtggg 5040

cttcaagttt agctataaat ggtctattat cacttggtaa ggatagaaaa tggagttgtc 5100

atcctatgga acacgagtta agtgcatatt atgatataac acatggtgta ggacttgcaa 5160

ttttaacacc taattggatg gaatatattc taaatgacga tacacttcat aaatttgttt 5220

cttatggaat aaatgtttgg ggaatagaca agaacaaaga taactatgaa atagcacgag 5280

aggctattaa aaatacgaga gaatacttta attcattggg tattccttca aagcttagag 5340

aagttggaat aggaaaagat aaactagaac taatggcaaa gcaagctgtt agaaattctg 5400

gaggaacaat aggaagttta agaccaataa atgcagagga tgttcttgag atatttaaaa 5460

aatcttatta atagaaactg tagaggtatt tttataattt aaaagatgtt aaagagtgag 5520

gagtaatttt gttctaacgc ctcactcttt tcattttatg attaaatgta tgctgattta 5580

cgctaactta aatcctaaat aataacctaa tgttaatatt ttgtaacaaa tggataaaag 5640

cgtaaaaata ttattgtaat aattttaagt aggtttaaaa tatatataat gtagaagcat 5700

tcctacatta tattatttaa ataataatct aaacaggagg ggttaaagtg gttgatttca 5760

aatctgtgta aacctaccgg ggtttgggcg tagccattat attcatgaac tccaagaaag 5820

cagtatgcta gcaaagaaat aaaactcaaa gcagagagaa aatttagaca ttcaactata 5880

aataaaaaat acccccaaa gcattaatat cttggggagt atttttattt ttgaagtatt 5940

ctgttcagct aaatattctt ctaaggtaat acctctgttc ataatttctt gtgaggcagg 6000

aagaccgata tatcttacat gccatggctc aaaattatac tttgttatgt tttctttatc 6060

cttaggatat cttattatga aaccatattt accacaattt tgttgaagcc atttataaga 6120

atttgtattc ataaatccat catctaaaga agagtattcg gttgatagta agtccattgc 6180

caatccagtt tgatgctcac ttgtaccagg ttcagctaca tatttatcag cttcggcttt 6240

tccgtctcgt gctacttttt cattatataa tttttgctga tacgaataag gtctataacc 6300

tgaaacagct agaagtgtaa gaccatcctt tgatgctgca ttaaacatat tttcaagtcc 6360

tgttgcagct tcgctctcca tttgatttac attaggatca gaactactaa taaatttaac 6420

gttaggagtt ctcaaatttt gaggtatata gtttcctgat aatttacttt gcttgtttac 6480

aagtaggatg ttctgtttct ttacctcggg tttcttggct tgttttttag gtgtagaaac 6540

tttctttttg ggttcgtttg 6560

<210> 80
<211> 6560
<212> DNA
<213> Artificial Sequence

<220>
<223>  pGRNA_deltabdhA_deltabdhB

<400>  80
gatccccggg taccgagctc gaattcgtaa tcatggtcat agctgtttcc tgtgtgaaat      60

tgttatccgc tcacaattcc acacaacata cgagccggaa gcataaagtg taaagcctgg     120

ggtgcctaat gagtgagcta actcacatta attgcgttgc gctcactgcc cgctttccag     180

tcgggaaacc tgtcgtgcca gctgcattaa tgaatcggcc aacgcgcggg gagaggcggt     240

ttgcgtattg ggcgctcttc cgcttcctcg ctcactgact cgctgcgctc ggtcgttcgg     300

ctgcggcgag cggtatcagc tcactcaaag gcggtaatac ggttatccac agaatcaggg     360

gataacgcag gaaagaacat gtgagcaaaa ggccagcaaa aggccaggaa ccgtaaaaag     420

gccgcgttgc tggcgttttt ccataggctc cgcccccctg acgagcatca caaaaatcga     480

cgctcaagtc agaggtggcg aaacccgaca ggactataaa gataccaggc gtttccccct     540

ggaagctccc tcgtgcgctc tcctgttccg accctgccgc ttaccggata cctgtccgcc     600

tttctccctt cgggaagcgt ggcgctttct catagctcac gctgtaggta tctcagttcg     660

gtgtaggtcg ttcgctccaa gctgggctgt gtgcacgaac cccccgttca gcccgaccgc     720

tgcgccttat ccggtaacta tcgtcttgag tccaacccgg taagacacga cttatcgcca     780

ctggcagcag ccactggtaa caggattagc agagcgaggt atgtaggcgg tgctacagag     840

ttcttgaagt ggtggcctaa ctacggctac actagaagaa cagtatttgg tatctgcgct     900

ctgctgaagc cagttacctt cggaaaaaga gttggtagct cttgatccgg caaacaaacc     960

accgctggta gcggtggttt ttttgtttgc aagcagcaga ttacgcgcag aaaaaaagga    1020

tctcaagaag atcctttgat cttttctacg gggtctgacg ctcagtggaa cgaaaactca    1080

cgttaaggga ttttggtcat gagattatca aaaaggatct tcacctagat ccttttaaat    1140

taaaaatgaa gttttaaatc aatctaaagt atatatgagt aaacttggtc tgacagttac    1200

caaagctagc ttaatactag tatatactta atgtgataag tgtctgacag ctgaccggtc    1260

taaagaggtc cctagcgcct acggggaatt tgtatcgata aggggtacaa attcccacta    1320

agcgctcggc cggggatcga tccccgggta cgtacccggc agtttttctt tttcggcaag    1380

tgttcaagaa gttattaagt cgggagtgca gtcgaagtgg gcaagttgaa aaattcacaa    1440

aaatgtggta taatatcttt gttcattaga gcgataaact tgaatttgag agggaactta    1500

gatggtattt gaaaaaattg ataaaaatag ttggaacaga aaagagtatt ttgaccacta    1560

ctttgcaagt gtaccttgta cctacagcat gaccgttaaa gtggatatca cacaaataaa    1620

ggaaaaggga atgaaactat atcctgcaat gctttattat attgcaatga ttgtaaaccg    1680

ccattcagag tttaggacgg caatcaatca agatggtgaa ttggggatat atgatgagat    1740

gataccaagc tatacaatat ttcacaatga tactgaaaca ttttccagcc tttggactga    1800

gtgtaagtct gactttaaat catttttagc agattatgaa agtgatacgc aacggtatgg 1860

aaacaatcat agaatggaag gaaagccaaa tgctccggaa aacattttta atgtatctat 1920

gataccgtgg tcaaccttcg atggctttaa tctgaatttg cagaaaggat atgattattt 1980

gattcctatt tttactatgg ggaaatatta taaagaagat aacaaaatta tacttccttt 2040

ggcaattcaa gttcatcacg cagtatgtga cggatttcac atttgccgtt ttgtaaacga 2100

attgcaggaa ttgataaata gttaacttca ggtttgtctg taactaaaaa ctagtattta 2160

acctaggatc aaaaaaattt ccaataatcc cactctaagc cacaaacacg ccctataaaa 2220

tcccgcttta atcccacttt gagacacatg taatattact ttacgcccta gtatagtgat 2280

aattttttac attcaatgcc acgcaaaaaa ataaaggggc actataataa aagttccttc 2340

ggaactaact aaagtaaaaa attatcttta caacctcccc aaaaaaaaga acaggtacaa 2400

agtaccctat aatacaagcg taaaaaaaat gagggtaaaa ataaaaaaat aaaaaaataa 2460

aaaaataaaa aaataaaaaa ataaaaaaat aaaaaaatat aaaaataaaa aaatataaaa 2520

ataaaaaaat ataaaaataa aaaaataaaa aaatataaaa ataaaaaaat aaaaaaatat 2580

aaaaatattt tttatttaaa gtttgaaaaa aattttttta tattatataa tctttgaaga 2640

aaagaatata aaaaatgagc ctttataaaa gcccattttt tttcatatac gtaatatgac 2700

gttctaatgt ttttattggt acttctaaca ttagagtaat ttctttattt ttaaagcctt 2760

tttctttaag ggcttttatt ttttttctta atacatttaa ttcctctttt tttgttgctt 2820

ttcctttagc ttttaattgc tcttgataat tttttttacc tctaatattt tctcttctct 2880

tatattcctt tttagaaatt attattgtca tatatttttg ttcttcttct gtaatttcta 2940

ataactctat aagagtttca ttcttatact tatattgctt atttttatct aaataacatc 3000

tttcagcact tctagttgct cttataactt ctctttcact taaatgttgt ctaaacatac 3060

tattaagttc taaaacatca tttaatgcct tctcaatgtc ttctgtaaag ctacaaagat 3120

aatatctata taaaaataat ataagctctc tgtgtccttt taaatcatat tctcttagtt 3180

cacaaagttt tattatgtct tgtattcttc cataatataa acttctttct ctataaatat 3240

aatttatttt gcttggtcta ccctttttcc tttcatatgg ttttaattca ggtaaaaatc 3300

cattttgtat ttctcttaag tcataaatat attcgtactc atctaatata ttgactactg 3360

tttttgattt agagtttata cttcctggaa ctcttaatat tctcgttgca tctaaggctt 3420

gtctatctgc tccaaagtat tttaattgat tatataaata ttcttgaacc gctttccata 3480

atggtaatgc tttactaggt actgcattta ttatccatat taaatacatt cctcttccac 3540

tatctattac atagtttggt ataggaatac tttgattaaa ataattcttt tctaagtcca 3600

ttaatacctg gtctttagtt ttgccagttt tataataatc caagtctata aacagtgtat 3660

ttaactcttt tatattttct aatcgcctac acggcttata aaaggtattt agagttatat 3720

agatattttc atcactcata tctaaatctt ttaattcagc gtatttatag tgccattggc 3780

tatatccttt tttatctata acgctcctgg ttatccaccc tttacttcta ctatgaatat 3840

tatctatata gttcttttta ttcagcttta atgcgtttct cacttattca cctccccttc 3900

tgtaaaacta agaaaattat atcatatttt caataattat taactattct taaactctta 3960

ataaaaaata gagtaagtcc ccaattgaaa cttaatctat tttttatgtt ttaatttatt 4020

atttttatta aaatatttta aactaaatta aatgattctt tttaattttt tactatttca 4080

ttccataata tattactata attatttaca aataatattt cttcatttgt aatatttaga 4140

tgatttacta attttagttt ttatatatta aataattaat gtataattta tataaaaaat 4200

caaaggagct tataaattat gattatttcc aaagatacta aagatttaat ttttttcaat 4260

tttaacaata ctttttgtaa tattatgttt aaatttaatt gtattttttt catataataa 4320

agccgttgaa gtaaaccaat ccattttcct tatgatgtta ttattaaatt taagttttat 4380

aataatatct ttattatatt tattgttttt aaaaaaacta gtgaaatttc tagtgaaatt 4440

tccggcttta ttaaacttat ttttaggaat tttattttca ttttcatctt tacaggattt 4500

gattatatct ttaaatatgt tttatcaaat attatctttt tctaaattta tatatatttt 4560

tattatattt attattatat atattttatt tttaagtttc tttctaacag ctattaaaaa 4620

gaaacttaaa aataaaaaca cgtactctaa accaataaat aaaactattt ttattattgc 4680

tgccttgatt ggaatagttt ttagtaaaat taatttcaat attccacaat attatattat 4740

aagctagcac gcctcgagac tctatcattg atagagtttg aaactctatc attgatagag 4800

tataatatct ttgttcatgc ttattacgac ataacacagt tttagagcta gaaatagcaa 4860

gttaaaataa ggctagtccg ttatcaactt gaaaaagtgg caccgagtcg gtgctttttt 4920

tgaagcttct cgagatctcc atggacgcgt gacgtcgacc ttctaatctc ctctactatt 4980

ttagggttag ctacattagc taaataggta atagctacag ttgtctttga attctcacct 5040

aaagtaagtt cttccacttt aaaatcagtg cttctaattt tttttcttaa aagggctaca 5100

tttgtggtta aagattcagt gaagccctct ctaggacctc ttattacagt ttcaacagtt 5160

ggttctgtta tagctctttc agggggtttt ccaatactta taataattgc tttactttca 5220

ccatctagga ataatgctat acttcctttt aaaatggaca atataacatc atccatgctt 5280

ttatatacat ttttatcatt aacagcaaaa attgattttg tatattcaaa tatgtttaaa 5340

tggggatggt tattgtaatc ttcttctata agttttttta taacagagga ttctattaca 5400

tcagattgga taagattatt tatgtagaca atcattgcag aaaaatttct attattagct 5460

attttaaatt ctctaatcgt taaatctgag caatttgtaa ataaggtttc tatagtatgt 5520

ttatttgttt taaggctagt tgaaaccgtc ttcgcgttat ttttagatgc ttcttcttta 5580

ttaaaaattt tattaaacaa cgaaaaattc accccctcaa tttatttata taatagtagt 5640

ttgcatgaaa tttcgttgtt tattcatatt agatgcttgt attaaaataa taaaatagta 5700

aaatataagt agacaaacta taaatctatt actaggaggt aagaagtatg ctaagtttta 5760

aatctgtgta aacctaccgg ggtttgggcg tagccattat attcatgaac tccaagaaag 5820

cagtatgcta gcaaagaaat aaaactcaaa gcagagagaa aatttagaca ttcaactata 5880

aataaaaaat accccccaaa gcattaatat cttggggagt attttttatt ttgaagtatt 5940

ctgttcagct aaatattctt ctaaggtaat acctctgttc ataatttctt gtgaggcagg 6000

aagaccgata tatcttacat gccatggctc aaaattatac tttgttatgt tttctttatc 6060

cttaggatat cttattatga aaccatattt accacaattt tgttgaagcc atttataaga 6120

atttgtattc ataaatccat catctaaaga agagtattcg gttgatagta agtccattgc 6180

caatccagtt tgatgctcac ttgtaccagg ttcagctaca tatttatcag cttcggcttt 6240

tccgtctcgt gctacttttt cattatataa tttttgctga tacgaataag gtctataacc 6300

tgaaacagct agaagtgtaa gaccatcctt tgatgctgca ttaaacatat tttcaagtcc 6360

tgttgcagct tcgctctcca tttgatttac attaggatca gaactactaa taaatttaac 6420

gttaggagtt ctcaaatttt gaggtatata gtttcctgat aatttacttt gcttgtttac 6480

aagtaggatg ttctgtttct ttacctcggg tttcttggct tgttttttag gtgtagaaac 6540

tttctttttg ggttcgtttg 6560

<210> 81
<211> 1654
<212> DNA
<213> Artificial Sequence

<220>
<223> Cassette bgaR acrIIA4

<400> 81
aaaaagtata acagaggttt taatttacgc ctctgttata ctttttattt ttgaaatttt 60

tttgttttaa agctgtattt taaatttata tacttggttt atttacttga ttatttctgt 120

aatttagtgg agacattgaa aaatgttttg aaaaagtttt tgaaaataac agggagtcac 180

tataacctac actacttgcg acttctccta taggaagttt agtgcttttt aataaaaggg 240

tggctttgta cattctaagg tttattaaat atctttgagg agaaattcca aggtttttta 300

tgaacatttt atataaataa cttctactta agttcacata atcagcaatt tcttgaacag 360

ttatgctatg catgtaatta gaattaatga aattaagagc atcttgaata tatgtgtgta 420

attccttatc tttgtattca aaaggttttg ggaattcttc tataagtgcg tacaataatg 480

agtaaagttc ttttagtaat agtatgtcat cagatcttga aggattataa gtttttgata 540

tttcgcacat atttaatatt atctgtggaa tttttgagtt ttcttcacaa ttagcaacac 600

aggagttagt aatagaagtt ctatttaaat actcattagc atttgaacca ctaaatccta 660

tccagtagta ttcccaagga tcatcaatag aagccacata ctcaacttgc atacctttta 720

gtagtataaa aatatcacct tgttttaagt tatatacctt accattaaat ttaaaagttc 780

catatccctt agttacgtaa tgaataacag catttttcaa tacttcatag ttatatccta 840

atcctggtat accttgttct ataccacatt catctacatt catttcaaag ttttctttaa 900

catacttttt ccacaatatt tgcatttcta cctcctaacc tataaaatta gccaatttta 960

tagtagtctt atattaaaca tttacatgag agctttgcaa agcagtttat caacataaaa 1020

gctttttatt ttaaaataaa ttcttctaaa tataagaata ttttaaagaa atatctttat 1080

atattagtta ttaaaattta taagattata agaaacatta taacatattt tagaactttt 1140

taactattct aaaagattaa tttacatatt aacatttaat tatgggtaaa aactattttg 1200

aaaaatgatt tatatggaat tatgtttctt aaatatacaa tcatgtttca tgaatacata 1260

attattttaa atgtattggg agggtaaaat gatattaaaa aatgaatacc atgaagatac 1320

tgcagaatct agaatccgcg gtagtcgacg tggaattgtg agcggataac aatttcacag 1380

gagggctgaa atgaatatta atgacttaat tagagaaata aaaaacaaag attacacagt 1440

gaaattgagt ggtacggata gcaatagtat aacacagcta attattagag ttaataatga 1500

tggaaacgag tatgtaattt ctgaaagtga aaatgaatca atagttgaaa aattcatatc 1560

tgcatttaaa aacggttgga atcaagaata cgaggatgaa gaagaatttt ataatgacat 1620

gcaaacaatc accttaaaaa gtgagttgaa ctaa 1654

<210> 82
<211> 4984
<212> DNA
<213> Artificial Sequence

<220>
<223> pGRNAind

<400> 82
   caagcttcaa aaaaagcacc gactcggtgc cactttttca agttgataac ggactagcct 60
   tattttaact tgctatttct agctctaaaa cagagaccgc tagcgatatc cccgggagat 120
   ctggtctcaa tgaacaaaga tattatactc tatcaatgat agagtttcaa actctatcaa 180
   tgatagagtg agctcgaatt cgtaatcatg gtcatagctg tttcctgtgt gaaattgtta 240
   tccgctcaca attccacaca acatacgagc cggaagcata aagtgtaaag cctggggtgc 300
   ctaatgagtg agctaactca cattaattgc gttgcgctca ctgcccgctt tccagtcggg 360
   aaacctgtcg tgccagctgc attaatgaat cggccaacgc gcggggagag gcggtttgcg 420
   tattgggcgc tcttccgctt cctcgctcac tgactcgctg cgctcggtcg ttcggctgcg 480
   gcgagcggta tcagctcact caaaggcggt aatacggtta tccacagaat caggggataa 540
   cgcaggaaag aacatgtgag caaaaggcca gcaaaaggcc aggaaccgta aaaaggccgc 600
   gttgctggcg tttttccata ggctccgccc ccctgacgag catcacaaaa atcgacgctc 660
   aagtcagagg tggcgaaacc cgacaggact ataaagatac caggcgtttc cccctggaag 720
   ctccctcgtg cgctctcctg ttccgaccct gccgcttacc ggatacctgt ccgcctttct 780
   cccttcggga agcgtggcgc tttctcatag ctcacgctgt aggtatctca gttcggtgta 840
   ggtcgttcgc tccaagctgg gctgtgtgca cgaacccccc gttcagcccg accgctgcgc 900
   cttatccggt aactatcgtc ttgagtccaa cccggtaaga cacgacttat cgccactggc 960
   agcagccact ggtaacagga ttagcagagc gaggtatgta ggcggtgcta cagagttctt 1020
   gaagtggtgg cctaactacg gctacactag aagaacagta tttggtatct gcgctctgct 1080
   gaagccagtt accttcggaa aaagagttgg tagctcttga tccggcaaac aaaccaccgc 1140
   tggtagcggt ggtttttttg tttgcaagca gcagattacg cgcagaaaaa aaggatctca 1200
   agaagatcct ttgatctttt ctacggggtc tgacgctcag tggaacgaaa actcacgtta 1260
   agggattttg gtcatgagat tatcaaaaag gatcttcacc tagatccttt taaattaaaa 1320
   atgaagtttt aaatcaatct aaagtatata tgagtaaact tggtctgaca gttaccaaag 1380
   ctagcttaat actagtatat acttaatgtg ataagtgtct gacagctgac cggtctaaag 1440
   aggtccctag cgcctacggg gaatttgtat cgataagggg tacaaattcc cactaagcgc 1500
   tcggccgggg atcgatcccc gggtacgtac ccggcagttt ttctttttcg gcaagtgttc 1560
   aagaagttat taagtcggga gtgcagtcga agtgggcaag ttgaaaaatt cacaaaaatg 1620
   tggtataata tctttgttca ttagagcgat aaacttgaat ttgagaggga acttagatgg 1680
   tatttgaaaa aattgataaa aatagttgga acagaaaaga gtattttgac cactactttg 1740
   caagtgtacc ttgtacctac agcatgaccg ttaaagtgga tatcacacaa ataaaggaaa 1800
   agggaatgaa actatatcct gcaatgcttt attatattgc aatgattgta aaccgccatt 1860
   cagagtttag gacggcaatc aatcaagatg gtgaattggg gatatatgat gagatgatac 1920
   caagctatac aatatttcac aatgatactg aaacattttc cagcctttgg actgagtgta 1980
   agtctgactt taaatcattt ttagcagatt atgaaagtga tacgcaacgg tatggaaaca 2040
   atcatagaat ggaaggaaag ccaaatgctc cggaaaacat ttttaatgta tctatgatac 2100
   cgtggtcaac cttcgatggc tttaatctga atttgcagaa aggatatgat tatttgattc 2160
   ctatttttac tatggggaaa tattataaag aagataacaa aattatactt cctttggcaa 2220
   ttcaagttca tcacgcagta tgtgacggat ttcacatttg ccgttttgta aacgaattgc 2280
   aggaattgat aaatagttaa cttcaggttt gtctgtaact aaaaactagt atttaaccta 2340
   ggatcaaaaa aatttccaat aatcccactc taagccacaa acacgcccta taaaatcccg 2400
   ctttaatccc actttgagac acatgtaata ttactttacg ccctagtata gtgataattt 2460
   tttacattca atgccacgca aaaaaataaa ggggcactat aataaaagtt ccttcggaac 2520
   taactaaagt aaaaaattat ctttacaacc tccccaaaaa aaagaacagg tacaaagtac 2580
   cctataatac aagcgtaaaa aaaatgaggg taaaaataaa aaaataaaaa aataaaaaaa 2640
   taaaaaaata aaaaaataaa aaaataaaaa aatataaaaa taaaaaaata taaaaataaa 2700
   aaaatataaa aataaaaaaa taaaaaaata taaaaataaa aaaataaaaa aatataaaaa 2760
   tattttttat ttaaagtttg aaaaaaattt ttttatatta tataatcttt gaagaaaaga 2820
   atataaaaaa tgagccttta taaaagccca ttttttttca tatacgtaat atgacgttct 2880
   aatgttttta ttggtacttc taacattaga gtaatttctt tatttttaaa gccttttctt 2940
   ttaagggctt ttattttttt tcttaataca tttaattcct ctttttttgt tgcttttcct 3000
   ttagctttta attgctcttg ataatttttt ttacctctaa tattttctct tctcttatat 3060
   tcctttttag aaattattat tgtcatatat ttttgttctt cttctgtaat ttctaataac 3120
   tctataagag tttcattctt atacttatat tgcttatttt tatctaaata acatctttca 3180
   gcacttctag ttgctcttat aacttctctt tcacttaaat gttgtctaaa catactatta 3240
   agttctaaaa catcatttaa tgccttctca atgtcttctg taaagctaca aagataatat 3300
   ctatataaaa ataatataag ctctctgtgt ccttttaaat catattctct tagttcacaa 3360
   agttttatta tgtcttgtat tcttccataa tataaacttc tttctctata aatataattt 3420
   attttgcttg gtctaccctt tttcctttca tatggtttta attcaggtaa aaatccattt 3480
   tgtatttctc ttaagtcata aatatattcg tactcatcta atatattgac tactgttttt 3540
   gatttagagt ttatacttcc tggaactctt aatattctcg ttgcatctaa ggcttgtcta 3600
   tctgctccaa agtattttaa ttgattatat aaatattctt gaaccgcttt ccataatggt 3660
   aatgctttac taggtactgc atttattatc catattaaat acattcctct tccactatct 3720
   attacatagt ttggtatagg aatactttga ttaaaataat tcttttctaa gtccattaat 3780
   acctggtctt tagttttgcc agttttataa taatccaagt ctataaacag tgtatttaac 3840
   tcttttatat tttctaatcg cctacacggc ttataaaagg tatttagagt tatatagata 3900
   ttttcatcac tcatatctaa atcttttaat tcagcgtatt tatagtgcca ttggctatat 3960
   ccttttttat ctataacgct cctggttatc caccctttac ttctactatg aatattatct 4020
   atatagttct ttttattcag ctttaatgcg tttctcactt attcacctcc ccttctgtaa 4080
   aactaagaaa attatatcat attttcaata attattaact attcttaaac tcttaataaa 4140
   aaatagagta agtccccaat tgaaacttaa tctatttttt atgttttaat ttattatttt 4200
   tattaaaata ttttaaacta aattaaatga ttctttttaa ttttttacta tttcattcca 4260
   taatatatta ctataattat ttacaaataa tatttcttca tttgtaatat ttagatgatt 4320
   tactaatttt agtttttata tattaaataa ttaatgtata atttatataa aaaatcaaag 4380
   gagcttataa attatgatta tttccaaaga tactaaagat ttaatttttt tcaattttaa 4440
   caatactttt tgtaatatta tgtttaaatt taattgtatt tttttcatat aataaagccg 4500
   ttgaagtaaa ccaatccatt ttccttatga tgttattatt aaatttaagt tttataataa 4560
   tatctttatt atatttattg tttttaaaaa aactagtgaa atttctagtg aaatttccgg 4620
   ctttattaaa cttattttta ggaattttat tttcattttc atctttacag gatttgatta 4680
   tatctttaaa tatgttttat caaatattat ctttttctaa atttatatat atttttatta 4740
   tatttattat tatatatatt ttatttttaa gtttctttct aacagctatt aaaaagaaac 4800
   ttaaaaataa aaacacgtac tctaaaccaa taaataaaac tatttttatt attgctgcct 4860
   tgattggaat agtttttagt aaaattaatt tcaatattcc acaatattat attataagct 4920
   agcacgcctc gagatctcca tggacgcgtg acgtcgactc tagaggatcc ccgggtaccg 4980
   agct 4984

<210> 83
<211> 200
<212> DNA
<213> Artificial Sequence

<220>
<223> Cassette ARNg

<400> 83
   gagctcactc tatcattgat agagtttgaa actctatcat tgatagagta taatatcttt 60
   gttcattgag accagatctc ccggggatat cgctagcggt ctctgtttta gagctagaaa 120
   tagcaagtta aaataaggct agtccgttat caacttgaaa aagtggcacc gagtcggtgc 180
   tttttttgaa gcttgagctc 200

<210> 84
<211> 24
<212> DNA
<213> Artificial Sequence

<220>
<223> primer

<400> 84
tcatgatttc tccatattag ctag 24

<210> 85
<211> 24
<212> DNA
<213> Artificial Sequence

<220>
<223>  Primer

<400>  85
aaacctagct aatatggaga aatc                                              24


<210>  86
<211>  24
<212>  DNA
<213>  Artificial Sequence

<220>
<223>  Primer

<400>  86
tcatgttaca cttggaacag gcgt                                              24


<210>  87
<211>  24
<212>  DNA
<213>  Artificial Sequence

<220>
<223>  Primer

<400>  87
aaacacgcct gttccaagtg taac                                              24


<210>  88
<211>  24
<212>  DNA
<213>  Artificial Sequence

<220>
<223>  Primer

<400>  88
tcatttccgg cagtaggatc ccca                                              24


<210>  89
<211>  24
<212>  DNA
<213>  Artificial Sequence

<220>
<223>  Primer

<400>  89
aaactgggga tcctactgcc ggaa                                              24


<210>  90
<211>  24
<212>  DNA
<213>  Artificial Sequence

<220>
<223>  Primer

<400>  90
tcatgcttat tacgacataa caca                                              24


<210>  91
<211>  24
<212>  DNA
<213>  Artificial Sequence

<220>
<223>  Primer

<400>  91
aaactgtgtt atgtcgtaat aagc                                              24


<210>  92
<211>  36
<212>  DNA
<213>  Artificial Sequence

<220>
<223>  Primer

<400>  92
atgcatggat ccaaacgaac ccaaaaagaa agtttc                                 36


<210>  93
<211>  30
<212>  DNA
<213>  Artificial Sequence

<220>
<223>  Primer

<400>  93
ggttgatttc aaatctgtgt aaacctaccg                                        30


<210>  94
<211>  30
<212>  DNA
<213>  Artificial Sequence

<220>
<223>  Primer

<400>  94
acacagattt gaaatcaacc actttaaccc                                        30


<210>  95
<211>  37
<212>  DNA
<213>  Artificial Sequence

<220>
<223>  Primer

<400>  95
atgcatgtcg actcttaaga acatgtataa agtatgg                                37

<210>  96
<211>  36
<212>  DNA
<213>  Artificial Sequence

<220>
<223>  Primer

<400>  96
atgcatggat ccaaacgaac ccaaaaagaa agtttc                                36


<210>  97
<211>  30
<212>  DNA
<213>  Artificial Sequence

<220>
<223>  Primer

<400>  97
gctaagtttt aaatctgtgt aaacctaccg                                       30


<210>  98
<211>  32
<212>  DNA
<213>  Artificial Sequence

<220>
<223>  Primer

<400>  98
acacagattt aaaacttagc atacttctta cc                                    32


<210>  99
<211>  37
<212>  DNA
<213>  Artificial Sequence

<220>
<223>  Primer

<400>  99
atgcatgtcg accttctaat ctcctctact attttag                               37


<210>  100
<211>  20
<212>  DNA
<213>  Artificial Sequence

<220>
<223>  Primer

<400>  100
acacattgaa gggagctttt                                                  20


<210>  101
<211>  20

<212>  DNA
<213>  Artificial Sequence

<220>
<223>  Primer

<400>  101
ggcaacaaca tcaggccttt                                                   20


<210>  102
<211>  4966
<212>  DNA
<213>  Artificial Sequence

<220>
<223>  pGRNA-xylB

<400>  102
atcaaaaaaa tttccaataa tcccactcta agccacaaac acgccctata aaatcccgct      60

ttaatcccac tttgagacac atgtaatatt actttacgcc ctagtatagt gataattttt     120

tacattcaat gccacgcaaa aaaataaagg ggcactataa taaaagttcc ttcggaacta     180

actaaagtaa aaaattatct ttacaacctc cccaaaaaaa agaacaggta caaagtaccc     240

tataatacaa gcgtaaaaaa aatgagggta aaaataaaaa aataaaaaaa taaaaaaata     300

aaaaaataaa aaaataaaaa aataaaaaaa tataaaaata aaaaaatata aaaataaaaa     360

aatataaaaa taaaaaaata aaaaaatata aaaataaaaa aataaaaaaa tataaaaata     420

ttttttattt aaagtttgaa aaaaattttt ttatattata taatctttga agaaaagaat     480

ataaaaaatg agcctttata aaagcccatt ttttttcata tacgtaatat gacgttctaa     540

tgtttttatt ggtacttcta acattagagt aatttcttta tttttaaagc ctttttcttt     600

aagggctttt attttttttc ttaatacatt taattcctct ttttttgttg cttttccttt     660

agcttttaat tgctcttgat aatttttttt acctctaata ttttctcttc tcttatattc     720

ctttttagaa attattattg tcatatattt ttgttcttct tctgtaattt ctaataactc     780

tataagagtt tcattcttat acttatattg cttattttta tctaaataac atctttcagc     840

acttctagtt gctcttataa cttctctttc acttaaatgt tgtctaaaca tactattaag     900

ttctaaaaca tcatttaatg ccttctcaat gtcttctgta aagctacaaa gataatatct     960

atataaaaat aatataagct ctctgtgtcc ttttaaatca tattctctta gttcacaaag    1020

ttttattatg tcttgtattc ttccataata taaacttctt tctctataaa tataatttat    1080

tttgcttggt ctaccctttt tcctttcata tggttttaat tcaggtaaaa atccattttg    1140

tatttctctt aagtcataaa tatattcgta ctcatctaat atattgacta ctgtttttga    1200

tttagagttt atacttcctg gaactcttaa tattctcgtt gcatctaagg cttgtctatc    1260

tgctccaaag tattttaatt gattatataa atattcttga accgctttcc ataatggtaa    1320

tgctttacta ggtactgcat ttattatcca tattaaatac attcctcttc cactatctat 1380

tacatagttt ggtataggaa tactttgatt aaaataattc ttttctaagt ccattaatac 1440

ctggtcttta gttttgccag ttttataata atccaagtct ataaacagtg tatttaactc 1500

ttttatattt tctaatcgcc tacacggctt ataaaaggta tttagagtta tatagatatt 1560

ttcatcactc atatctaaat cttttaattc agcgtattta tagtgccatt ggctatatcc 1620

ttttttatct ataacgctcc tggttatcca ccctttactt ctactatgaa tattatctat 1680

atagttcttt ttattcagct ttaatgcgtt tctcacttat tcacctcccc ttctgtaaaa 1740

ctaagaaaat tatatcatat tttcaataat tattaactat tcttaaactc ttaataaaaa 1800

atagagtaag tccccaattg aaacttaatc tattttttat gttttaattt attattttta 1860

ttaaaatatt ttaaactaaa ttaaatgatt ctttttaatt ttttactatt tcattccata 1920

atatattact ataattattt acaaataata tttcttcatt tgtaatattt agatgattta 1980

ctaattttag tttttatata ttaaataatt aatgtataat ttatataaaa aatcaaagga 2040

gcttataaat tatgattatt tccaaagata ctaaagattt aatttttttc aattttaaca 2100

atactttttg taatattatg tttaaattta attgtatttt tttcatataa taaagccgtt 2160

gaagtaaacc aatccatttt ccttatgatg ttattattaa atttaagttt tataataata 2220

tctttattat atttattgtt tttaaaaaaa ctagtgaaat ttctagtgaa atttccggct 2280

ttattaaact tatttttagg aattttattt tcattttcat ctttacagga tttgattata 2340

tctttaaata tgttttatca aatattatct ttttctaaat ttatatatat ttttattata 2400

tttattatta tatatatttt atttttaagt ttctttctaa cagctattaa aaagaaactt 2460

aaaaataaaa acacgtactc taaaccaata aataaaacta tttttattat tgctgccttg 2520

attggaatag tttttagtaa aattaatttc aatattccac aatattatat tataagctag 2580

cacgcctcga gaagcttcaa aaaaagcacc gactcggtgc cactttttca agttgataac 2640

ggactagcct tattttaact tgctatttct agctctaaaa cctagctaat atggagaaat 2700

catgaacaaa gatattatac tctatcaatg atagagtttc aaactctatc aatgatagag 2760

tctcgagatc tccatggacg cgtgacgtcg actctagagg atccccgggt accgagctcg 2820

aattcgtaat catggtcata gctgtttcct gtgtgaaatt gttatccgct cacaattcca 2880

cacaacatac gagccggaag cataaagtgt aaagcctggg gtgcctaatg agtgagctaa 2940

ctcacattaa ttgcgttgcg ctcactgccc gctttccagt cgggaaacct gtcgtgccag 3000

ctgcattaat gaatcggcca acgcgcgggg agaggcggtt tgcgtattgg gcgctcttcc 3060

gcttcctcgc tcactgactc gctgcgctcg gtcgttcggc tgcggcgagc ggtatcagct 3120

cactcaaagg cggtaatacg gttatccaca gaatcagggg ataacgcagg aaagaacatg 3180

tgagcaaaag gccagcaaaa ggccaggaac cgtaaaaagg ccgcgttgct ggcgtttttc 3240

cataggctcc gcccccctga cgagcatcac aaaaatcgac gctcaagtca gaggtggcga 3300

aacccgacag gactataaag ataccaggcg tttccccctg gaagctccct cgtgcgctct 3360

cctgttccga ccctgccgct taccggatac ctgtccgcct ttctcccttc gggaagcgtg 3420

gcgctttctc atagctcacg ctgtaggtat ctcagttcgg tgtaggtcgt tcgctccaag 3480

ctgggctgtg tgcacgaacc ccccgttcag cccgaccgct gcgccttatc cggtaactat 3540

cgtcttgagt ccaacccggt aagacacgac ttatcgccac tggcagcagc cactggtaac 3600

aggattagca gagcgaggta tgtaggcggt gctacagagt tcttgaagtg gtggcctaac 3660

tacggctaca ctagaagaac agtatttggt atctgcgctc tgctgaagcc agttaccttc 3720

ggaaaaagag ttggtagctc ttgatccggc aaacaaacca ccgctggtag cggtggtttt 3780

tttgtttgca agcagcagat tacgcgcaga aaaaaaggat ctcaagaaga tcctttgatc 3840

ttttctacgg ggtctgacgc tcagtggaac gaaaactcac gttaagggat tttggtcatg 3900

agattatcaa aaaggatctt cacctagatc cttttaaatt aaaaatgaag ttttaaatca 3960

atctaaagta tatatgagta aacttggtct gacagttacc aaagctagct taatactagt 4020

atatacttaa tgtgataagt gtctgacagc tgaccggtct aaagaggtcc ctagcgccta 4080

cggggaattt gtatcgataa ggggtacaaa ttcccactaa gcgctcggcc ggggatcgat 4140

ccccgggtac gtacccggca gtttttcttt ttcggcaagt gttcaagaag ttattaagtc 4200

gggagtgcag tcgaagtggg caagttgaaa aattcacaaa aatgtggtat aatatctttg 4260

ttcattagag cgataaactt gaatttgaga gggaacttag atggtatttg aaaaaattga 4320

taaaaatagt tggaacagaa aagagtattt tgaccactac tttgcaagtg taccttgtac 4380

ctacagcatg accgttaaag tggatatcac acaaataaag gaaaagggaa tgaaactata 4440

tcctgcaatg ctttattata ttgcaatgat tgtaaaccgc cattcagagt ttaggacggc 4500

aatcaatcaa gatggtgaat tggggatata tgatgagatg ataccaagct atacaatatt 4560

tcacaatgat actgaaacat tttccagcct ttggactgag tgtaagtctg actttaaatc 4620

atttttagca gattatgaaa gtgatacgca acggtatgga aacaatcata gaatggaagg 4680

aaagccaaat gctccggaaa acatttttaa tgtatctatg ataccgtggt caaccttcga 4740

tggctttaat ctgaatttgc agaaaggata tgattatttg attcctattt ttactatggg 4800

gaaatattat aaagaagata acaaaattat acttcctttg gcaattcaag ttcatcacgc 4860

agtatgtgac ggatttcaca tttgccgttt tgtaaacgaa ttgcaggaat tgataaatag 4920

ttaacttcag gtttgtctgt aactaaaaac tagtatttaa cctagg 4966

<210> 103
<211> 4966
<212> DNA

<213>  Artificial Sequence

<220>
<223>  pGRNA-xylR

<400>  103
atcaaaaaaa tttccaataa tcccactcta agccacaaac acgccctata aaatcccgct      60

ttaatcccac tttgagacac atgtaatatt actttacgcc ctagtatagt gataattttt     120

tacattcaat gccacgcaaa aaaataaagg ggcactataa taaaagttcc ttcggaacta     180

actaaagtaa aaaattatct ttacaacctc cccaaaaaaa agaacaggta caaagtaccc     240

tataatacaa gcgtaaaaaa aatgagggta aaaataaaaa aataaaaaaa taaaaaaata     300

aaaaaataaa aaaataaaaa aataaaaaaa tataaaaata aaaaaatata aaaataaaaa     360

aatataaaaa taaaaaaata aaaaaatata aaaataaaaa aataaaaaaa tataaaaata     420

ttttttattt aaagtttgaa aaaaattttt ttatattata taatctttga agaaaagaat     480

ataaaaaatg agcctttata aaagcccatt ttttttcata tacgtaatat gacgttctaa     540

tgtttttatt ggtacttcta acattagagt aatttcttta tttttaaagc ctttttcttt     600

aagggctttt attttttttc ttaatacatt taattcctct ttttttgttg cttttccttt     660

agcttttaat tgctcttgat aatttttttt acctctaata ttttctcttc tcttatattc     720

ctttttagaa attattattg tcatatattt ttgttcttct tctgtaattt ctaataactc     780

tataagagtt tcattcttat acttatattg cttattttta tctaaataac atctttcagc     840

acttctagtt gctcttataa cttctctttc acttaaatgt tgtctaaaca tactattaag     900

ttctaaaaca tcatttaatg ccttctcaat gtcttctgta aagctacaaa gataatatct     960

atataaaaat aatataagct ctctgtgtcc ttttaaatca tattctctta gttcacaaag    1020

ttttattatg tcttgtattc ttccataata taaacttctt tctctataaa tataatttat    1080

tttgcttggt ctaccctttt tcctttcata tggttttaat tcaggtaaaa atccattttg    1140

tatttctctt aagtcataaa tatattcgta ctcatctaat atattgacta ctgtttttga    1200

tttagagttt atacttcctg gaactcttaa tattctcgtt gcatctaagg cttgtctatc    1260

tgctccaaag tattttaatt gattatataa atattcttga accgctttcc ataatggtaa    1320

tgctttacta ggtactgcat ttattatcca tattaaatac attcctcttc cactatctat    1380

tacatagttt ggtataggaa tactttgatt aaaataattc ttttctaagt ccattaatac    1440

ctggtcttta gttttgccag ttttataata atccaagtct ataaacagtg tatttaactc    1500

ttttatattt tctaatcgcc tacacggctt ataaaaggta tttagagtta tatagatatt    1560

ttcatcactc atatctaaat cttttaattc agcgtattta tagtgccatt ggctatatcc    1620

ttttttatct ataacgctcc tggttatcca ccctttactt ctactatgaa tattatctat    1680

atagttcttt ttattcagct ttaatgcgtt tctcacttat tcacctcccc ttctgtaaaa    1740

ctaagaaaat tatatcatat tttcaataat tattaactat tcttaaactc ttaataaaaa 1800

atagagtaag tccccaattg aaacttaatc tattttttat gttttaattt attattttta 1860

ttaaaatatt ttaaactaaa ttaaatgatt ctttttaatt ttttactatt tcattccata 1920

atatattact ataattattt acaaataata tttcttcatt tgtaatattt agatgattta 1980

ctaattttag tttttatata ttaaataatt aatgtataat ttatataaaa aatcaaagga 2040

gcttataaat tatgattatt tccaaagata ctaaagattt aatttttttc aattttaaca 2100

atactttttg taatattatg tttaaattta attgtatttt tttcatataa taaagccgtt 2160

gaagtaaacc aatccatttt ccttatgatg ttattattaa atttaagttt tataataata 2220

tctttattat atttattgtt tttaaaaaaa ctagtgaaat ttctagtgaa atttccggct 2280

ttattaaact tatttttagg aattttattt tcattttcat ctttacagga tttgattata 2340

tctttaaata tgttttatca aatattatct ttttctaaat ttatatatat ttttattata 2400

tttattatta tatatatttt atttttaagt ttctttctaa cagctattaa aaagaaactt 2460

aaaaataaaa acacgtactc taaaccaata aataaaacta tttttattat tgctgccttg 2520

attggaatag tttttagtaa aattaatttc aatattccac aatattatat tataagctag 2580

cacgcctcga gactctatca ttgatagagt ttgaaactct atcattgata gagtataata 2640

tctttgttca tgttacactt ggaacaggcg tgttttagag ctagaaatag caagttaaaa 2700

taaggctagt ccgttatcaa cttgaaaaag tggcaccgag tcggtgcttt ttttgaagct 2760

tctcgagatc tccatggacg cgtgacgtcg actctagagg atccccgggt accgagctcg 2820

aattcgtaat catggtcata gctgtttcct gtgtgaaatt gttatccgct cacaattcca 2880

cacaacatac gagccggaag cataaagtgt aaagcctggg gtgcctaatg agtgagctaa 2940

ctcacattaa ttgcgttgcg ctcactgccc gctttccagt cgggaaacct gtcgtgccag 3000

ctgcattaat gaatcggcca acgcgcgggg agaggcggtt tgcgtattgg gcgctcttcc 3060

gcttcctcgc tcactgactc gctgcgctcg gtcgttcggc tgcggcgagc ggtatcagct 3120

cactcaaagg cggtaatacg gttatccaca gaatcagggg ataacgcagg aaagaacatg 3180

tgagcaaaag gccagcaaaa ggccaggaac cgtaaaaagg ccgcgttgct ggcgtttttc 3240

cataggctcc gcccccctga cgagcatcac aaaaatcgac gctcaagtca gaggtggcga 3300

aacccgacag gactataaag ataccaggcg tttccccctg gaagctccct cgtgcgctct 3360

cctgttccga ccctgccgct taccggatac ctgtccgcct ttctcccttc gggaagcgtg 3420

gcgctttctc atagctcacg ctgtaggtat ctcagttcgg tgtaggtcgt tcgctccaag 3480

ctgggctgtg tgcacgaacc ccccgttcag cccgaccgct gcgccttatc cggtaactat 3540

cgtcttgagt ccaacccggt aagacacgac ttatcgccac tggcagcagc cactggtaac 3600

aggattagca gagcgaggta tgtaggcggt gctacagagt tcttgaagtg gtggcctaac 3660

tacggctaca ctagaagaac agtatttggt atctgcgctc tgctgaagcc agttaccttc 3720

ggaaaaagag ttggtagctc ttgatccggc aaacaaacca ccgctggtag cggtggtttt 3780

tttgtttgca agcagcagat tacgcgcaga aaaaaaggat ctcaagaaga tcctttgatc 3840

ttttctacgg ggtctgacgc tcagtggaac gaaaactcac gttaagggat tttggtcatg 3900

agattatcaa aaaggatctt cacctagatc cttttaaatt aaaaatgaag ttttaaatca 3960

atctaaagta tatatgagta aacttggtct gacagttacc aaagctagct taatactagt 4020

atatacttaa tgtgataagt gtctgacagc tgaccggtct aaagaggtcc ctagcgccta 4080

cggggaattt gtatcgataa ggggtacaaa ttcccactaa gcgctcggcc ggggatcgat 4140

ccccgggtac gtacccggca gtttttcttt ttcggcaagt gttcaagaag ttattaagtc 4200

gggagtgcag tcgaagtggg caagttgaaa aattcacaaa aatgtggtat aatatctttg 4260

ttcattagag cgataaactt gaatttgaga gggaacttag atggtatttg aaaaaattga 4320

taaaaatagt tggaacagaa aagagtattt tgaccactac tttgcaagtg taccttgtac 4380

ctacagcatg accgttaaag tggatatcac acaaataaag gaaaagggaa tgaaactata 4440

tcctgcaatg ctttattata ttgcaatgat tgtaaaccgc cattcagagt ttaggacggc 4500

aatcaatcaa gatggtgaat tggggatata tgatgagatg ataccaagct atacaatatt 4560

tcacaatgat actgaaacat tttccagcct ttggactgag tgtaagtctg actttaaatc 4620

atttttagca gattatgaaa gtgatacgca acggtatgga aacaatcata gaatggaagg 4680

aaagccaaat gctccggaaa acatttttaa tgtatctatg ataccgtggt caaccttcga 4740

tggctttaat ctgaatttgc agaaaggata tgattatttg attcctattt ttactatggg 4800

gaaatattat aaagaagata acaaaattat acttcctttg gcaattcaag ttcatcacgc 4860

agtatgtgac ggatttcaca tttgccgttt tgtaaacgaa ttgcaggaat tgataaatag 4920

ttaacttcag gtttgtctgt aactaaaaac tagtatttaa cctagg 4966

<210> 104
<211> 4966
<212> DNA
<213> Artificial Sequence

<220>
<223> pGRNA-glcG

<400> 104
agctcggtac ccggggatcc tctagagtcg acgtcacgcg tccatggaga tctcgaggcg 60

tgctagctta taatataata ttgtggaata ttgaaattaa ttttactaaa aactattcca 120

atcaaggcag caataataaa aatagtttta tttattggtt tagagtacgt gtttttattt 180

ttaagtttct ttttaatagc tgttagaaag aaacttaaaa ataaaatata tataataata 240

aatataataa aaatatatat aaatttagaa aaagataata tttgataaaa catatttaaa 300

gatataatca aatcctgtaa agatgaaaat gaaaataaaa ttcctaaaaa taagtttaat 360

aaagccggaa atttcactag aaatttcact agttttttta aaaacaataa atataataaa 420

gatattatta taaaacttaa atttaataat aacatcataa ggaaaatgga ttggtttact 480

tcaacggctt tattatatga aaaaaataca attaaattta aacataatat tacaaaaagt 540

attgttaaaa ttgaaaaaaa ttaaatcttt agtatctttg gaaataatca taatttataa 600

gctcctttga ttttttatat aaattataca ttaattattt aatatataaa aactaaaatt 660

agtaaatcat ctaaatatta caaatgaaga aatattattt gtaaataatt atagtaatat 720

attatggaat gaaatagtaa aaaattaaaa agaatcattt aatttagttt aaaatatttt 780

aataaaaata ataaattaaa acataaaaaa tagattaagt ttcaattggg gacttactct 840

attttttatt aagagtttaa gaatagttaa taattattga aaatatgata taattttctt 900

agttttacag aaggggaggt gaataagtga gaaacgcatt aaagctgaat aaaaagaact 960

atatagataa tattcatagt agaagtaaag ggtggataac caggagcgtt atagataaaa 1020

aaggatatag ccaatggcac tataaatacg ctgaattaaa agatttagat atgagtgatg 1080

aaaatatcta tataactcta aatacctttt ataagccgtg taggcgatta gaaaatataa 1140

aagagttaaa tacactgttt atagacttgg attattataa aactggcaaa actaaagacc 1200

aggtattaat ggacttagaa aagaattatt ttaatcaaag tattcctata ccaaactatg 1260

taatagatag tggaagagga atgtatttaa tatggataat aaatgcagta cctagtaaag 1320

cattaccatt atggaaagcg gttcaagaat atttatataa tcaattaaaa tactttggag 1380

cagatagaca agccttagat gcaacgagaa tattaagagt tccaggaagt ataaactcta 1440

aatcaaaaac agtagtcaat atattagatg agtacgaata tatttatgac ttaagagaaa 1500

tacaaaatgg atttttacct gaattaaaac catatgaaag gaaaaagggt agaccaagca 1560

aaataaatta tatttataga gaaagaagtt tatattatgg aagaatacaa gacataataa 1620

aactttgtga actaagagaa tatgatttaa aaggacacag agagcttata ttatttttat 1680

atagatatta tctttgtagc tttacagaag acattgagaa ggcattaaat gatgttttag 1740

aacttaatag tatgtttaga caacatttaa gtgaaagaga agttataaga gcaactagaa 1800

gtgctgaaag atgttattta gataaaaata agcaatataa gtataagaat gaaactctta 1860

tagagttatt agaaattaca gaagaagaac aaaaatatat gacaataata atttctaaaa 1920

aggaatataa gagaagagaa aatattagag gtaaaaaaaa ttatcaagag caattaaaag 1980

ctaaggaaaa agcaacaaaa aaagaggaat taaatgtatt aagaaaaaaa ataaaagccc 2040

ttaaagaaaa aggctttaaa aataaagaaa ttactctaat gttagaagta ccaataaaaa 2100

cattagaacg tcatattacg tatatgaaaa aaaatgggct tttataaagg ctcatttttt 2160

atattctttt cttcaaagat tatataatat aaaaaaattt ttttcaaact ttaaataaaa 2220

aatattttta tattttttta tttttttatt tttatatttt tttatttttt tatttttata 2280

tttttttatt tttatatttt tttattttta tattttttta tttttttatt tttttatttt 2340

tttatttttt tattttttta tttttttatt tttaccctca ttttttttac gcttgtatta 2400

tagggtactt tgtacctgtt cttttttttg gggaggttgt aaagataatt ttttacttta 2460

gttagttccg aaggaacttt tattatagtg cccctttatt tttttgcgtg gcattgaatg 2520

taaaaaatta tcactatact agggcgtaaa gtaatattac atgtgtctca aagtgggatt 2580

aaagcgggat tttatagggc gtgtttgtgg cttagagtgg gattattgga aatttttttg 2640

atcctaggtt aaatactagt ttttagttac agacaaacct gaagttaact atttatcaat 2700

tcctgcaatt cgtttacaaa acggcaaatg tgaaatccgt cacatactgc gtgatgaact 2760

tgaattgcca aaggaagtat aattttgtta tcttctttat aatatttccc catagtaaaa 2820

ataggaatca aataatcata tcctttctgc aaattcagat taaagccatc gaaggttgac 2880

cacggtatca tagatacatt aaaaatgttt tccggagcat ttggctttcc ttccattcta 2940

tgattgtttc cataccgttg cgtatcactt tcataatctg ctaaaaatga tttaaagtca 3000

gacttacact cagtccaaag gctggaaaat gtttcagtat cattgtgaaa tattgtatag 3060

cttggtatca tctcatcata tatccccaat tcaccatctt gattgattgc cgtcctaaac 3120

tctgaatggc ggtttacaat cattgcaata taataaagca ttgcaggata tagtttcatt 3180

cccttttcct ttatttgtgt gatatccact ttaacggtca tgctgtaggt acaaggtaca 3240

cttgcaaagt agtggtcaaa atactctttt ctgttccaac tatttttatc aattttttca 3300

aataccatct aagttccctc tcaaattcaa gtttatcgct ctaatgaaca aagatattat 3360

accacatttt tgtgaatttt tcaacttgcc cacttcgact gcactcccga cttaataact 3420

tcttgaacac ttgccgaaaa agaaaaactg ccgggtacgt acccggggat cgatccccgg 3480

ccgagcgctt agtgggaatt tgtacccctt atcgatacaa attccccgta ggcgctaggg 3540

acctctttag accggtcagc tgtcagacac ttatcacatt aagtatatac tagtattaag 3600

ctagctttgg taactgtcag accaagttta ctcatatata ctttagattg atttaaaact 3660

tcatttttaa tttaaaagga tctaggtgaa gatccttttt gataatctca tgaccaaaat 3720

cccttaacgt gagttttcgt tccactgagc gtcagacccc gtagaaaaga tcaaaggatc 3780

ttcttgagat cctttttttc tgcgcgtaat ctgctgcttg caaacaaaaa aaccaccgct 3840

accagcggtg gtttgtttgc cggatcaaga gctaccaact ctttttccga aggtaactgg 3900

cttcagcaga gcgcagatac caaatactgt tcttctagtg tagccgtagt taggccacca 3960

cttcaagaac tctgtagcac cgcctacata cctcgctctg ctaatcctgt taccagtggc 4020

tgctgccagt ggcgataagt cgtgtcttac cgggttggac tcaagacgat agttaccgga 4080

taaggcgcag cggtcgggct gaacgggggg ttcgtgcaca cagcccagct tggagcgaac 4140

gacctacacc gaactgagat acctacagcg tgagctatga gaaagcgcca cgcttcccga 4200

agggagaaag gcggacaggt atccggtaag cggcagggtc ggaacaggag agcgcacgag 4260

ggagcttcca gggggaaacg cctggtatct ttatagtcct gtcgggtttc gccacctctg 4320

acttgagcgt cgatttttgt gatgctcgtc aggggggcgg agcctatgga aaaacgccag 4380

caacgcggcc tttttacggt tcctggcctt ttgctggcct tttgctcaca tgttctttcc 4440

tgcgttatcc cctgattctg tggataaccg tattaccgcc tttgagtgag ctgataccgc 4500

tcgccgcagc cgaacgaccg agcgcagcga gtcagtgagc gaggaagcgg aagagcgccc 4560

aatacgcaaa ccgcctctcc ccgcgcgttg gccgattcat taatgcagct ggcacgacag 4620

gtttcccgac tggaaagcgg gcagtgagcg caacgcaatt aatgtgagtt agctcactca 4680

ttaggcaccc caggctttac actttatgct tccggctcgt atgttgtgtg gaattgtgag 4740

cggataacaa tttcacacag gaaacagcta tgaccatgat tacgaattcg agctcactct 4800

atcattgata gagtttgaaa ctctatcatt gatagagtat aatatctttg ttcatttccg 4860

gcagtaggat ccccagtttt agagctagaa atagcaagtt aaaataaggc tagtccgtta 4920

tcaacttgaa aaagtggcac cgagtcggtg ctttttttga agcttg 4966

<210> 105
<211> 4938
<212> DNA
<213> Artificial Sequence

<220>
<223> pGRNA-bdhB

<400> 105
atcaaaaaaa tttccaataa tcccactcta agccacaaac acgccctata aaatcccgct 60

ttaatcccac tttgagacac atgtaatatt actttacgcc ctagtatagt gataattttt 120

tacattcaat gccacgcaaa aaaataaagg ggcactataa taaaagttcc ttcggaacta 180

actaaagtaa aaaattatct ttacaacctc cccaaaaaaa agaacaggta caaagtaccc 240

tataatacaa gcgtaaaaaa aatgagggta aaaataaaaa aataaaaaaa taaaaaaata 300

aaaaaataaa aaaataaaaa aataaaaaaa tataaaaata aaaaaatata aaaataaaaa 360

aatataaaaa taaaaaaata aaaaaatata aaaataaaaa aataaaaaaa tataaaaata 420

ttttttattt aaagtttgaa aaaaattttt ttatattata taatctttga agaaaagaat 480

ataaaaaatg agcctttata aaagcccatt ttttttcata tacgtaatat gacgttctaa 540

tgtttttatt ggtacttcta acattagagt aatttcttta tttttaaagc ctttttcttt 600

aagggctttt attttttttc ttaatacatt taattcctct ttttttgttg cttttccttt 660

agcttttaat tgctcttgat aatttttttt acctctaata ttttctcttc tcttatattc 720

ctttttagaa attattattg tcatatattt ttgttcttct tctgtaattt ctaataactc 780

tataagagtt tcattcttat acttatattg cttattttta tctaaataac atctttcagc 840

acttctagtt gctcttataa cttctctttc acttaaatgt tgtctaaaca tactattaag 900

ttctaaaaca tcatttaatg ccttctcaat gtcttctgta aagctacaaa gataatatct 960

atataaaaat aatataagct ctctgtgtcc ttttaaatca tattctctta gttcacaaag 1020

ttttattatg tcttgtattc ttccataata taaacttctt tctctataaa tataatttat 1080

tttgcttggt ctaccctttt tcctttcata tggttttaat tcaggtaaaa atccattttg 1140

tatttctctt aagtcataaa tatattcgta ctcatctaat atattgacta ctgtttttga 1200

tttagagttt atacttcctg gaactcttaa tattctcgtt gcatctaagg cttgtctatc 1260

tgctccaaag tattttaatt gattatataa atattcttga accgctttcc ataatggtaa 1320

tgctttacta ggtactgcat ttattatcca tattaaatac attcctcttc cactatctat 1380

tacatagttt ggtataggaa tactttgatt aaaataattc ttttctaagt ccattaatac 1440

ctggtcttta gttttgccag ttttataata atccaagtct ataaacagtg tatttaactc 1500

ttttatattt tctaatcgcc tacacggctt ataaaaggta tttagagtta tatagatatt 1560

ttcatcactc atatctaaat cttttaattc agcgtattta tagtgccatt ggctatatcc 1620

ttttttatct ataacgctcc tggttatcca ccctttactt ctactatgaa tattatctat 1680

atagttcttt ttattcagct ttaatgcgtt tctcacttat tcacctcccc ttctgtaaaa 1740

ctaagaaaat tatatcatat tttcaataat tattaactat tcttaaactc ttaataaaaa 1800

atagagtaag tccccaattg aaacttaatc tattttttat gttttaattt attattttta 1860

ttaaaatatt ttaaactaaa ttaaatgatt ctttttaatt ttttactatt tcattccata 1920

atatattact ataattattt acaaataata tttcttcatt tgtaatattt agatgattta 1980

ctaattttag tttttatata ttaaataatt aatgtataat ttatataaaa aatcaaagga 2040

gcttataaat tatgattatt tccaaagata ctaaagattt aatttttttc aattttaaca 2100

atactttttg taatattatg tttaaattta attgtatttt tttcatataa taaagccgtt 2160

gaagtaaacc aatccatttt ccttatgatg ttattattaa atttaagttt tataataata 2220

tctttattat atttattgtt tttaaaaaaa ctagtgaaat ttctagtgaa atttccggct 2280

ttattaaact tatttttagg aattttattt tcattttcat ctttacagga tttgattata 2340

tctttaaata tgttttatca aatattatct ttttctaaat ttatatatat ttttattata 2400

tttattatta tatatatttt atttttaagt ttctttctaa cagctattaa aaagaaactt 2460

aaaaataaaa acacgtactc taaaccaata aataaaacta tttttattat tgctgccttg 2520

attggaatag tttttagtaa aattaatttc aatattccac aatattatat tataagctag 2580

cacgcctcga gtatattgat aaaaataata atagtgggta taattaagtt gttaggaggt 2640

tagttagagc ttattacgac ataacacagt tttagagcta gaaatagcaa gttaaaataa 2700

ggctagtccg ttatcaactt gaaaaagtgg caccgagtcg gtgctttttt tgaagcttgt 2760

cgactctaga ggatccccgg gtaccgagct cgaattcgta atcatggtca tagctgtttc 2820

ctgtgtgaaa ttgttatccg ctcacaattc cacacaacat acgagccgga agcataaagt 2880

gtaaagcctg gggtgcctaa tgagtgagct aactcacatt aattgcgttg cgctcactgc 2940

ccgctttcca gtcgggaaac ctgtcgtgcc agctgcatta atgaatcggc caacgcgcgg 3000

ggagaggcgg tttgcgtatt gggcgctctt ccgcttcctc gctcactgac tcgctgcgct 3060

cggtcgttcg gctgcggcga gcggtatcag ctcactcaaa ggcggtaata cggttatcca 3120

cagaatcagg ggataacgca ggaaagaaca tgtgagcaaa aggccagcaa aaggccagga 3180

accgtaaaaa ggccgcgttg ctggcgtttt tccataggct ccgcccccct gacgagcatc 3240

acaaaaatcg acgctcaagt cagaggtggc gaaacccgac aggactataa agataccagg 3300

cgtttccccc tggaagctcc ctcgtgcgct ctcctgttcc gaccctgccg cttaccggat 3360

acctgtccgc ctttctccct tcgggaagcg tggcgctttc tcatagctca cgctgtaggt 3420

atctcagttc ggtgtaggtc gttcgctcca agctgggctg tgtgcacgaa ccccccgttc 3480

agcccgaccg ctgcgcctta tccggtaact atcgtcttga gtccaacccg gtaagacacg 3540

acttatcgcc actggcagca gccactggta acaggattag cagagcgagg tatgtaggcg 3600

gtgctacaga gttcttgaag tggtggccta actacggcta cactagaaga acagtatttg 3660

gtatctgcgc tctgctgaag ccagttacct tcggaaaaag agttggtagc tcttgatccg 3720

gcaaacaaac caccgctggt agcggtggtt tttttgtttg caagcagcag attacgcgca 3780

gaaaaaaagg atctcaagaa gatcctttga tcttttctac ggggtctgac gctcagtgga 3840

acgaaaactc acgttaaggg attttggtca tgagattatc aaaaaggatc ttcacctaga 3900

tccttttaaa ttaaaaatga agttttaaat caatctaaag tatatatgag taaacttggt 3960

ctgacagtta ccaaagctag cttaatacta gtatatactt aatgtgataa gtgtctgaca 4020

gctgaccggt ctaaagaggt ccctagcgcc tacggggaat ttgtatcgat aaggggtaca 4080

aattcccact aagcgctcgg ccggggatcg atccccgggt acgtacccgg cagtttttct 4140

ttttcggcaa gtgttcaaga agttattaag tcgggagtgc agtcgaagtg ggcaagttga 4200

aaaattcaca aaaatgtggt ataatatctt tgttcattag agcgataaac ttgaatttga 4260

gagggaactt agatggtatt tgaaaaaatt gataaaaata gttggaacag aaaagagtat 4320

tttgaccact actttgcaag tgtaccttgt acctacagca tgaccgttaa agtggatatc 4380

acacaaataa aggaaaaggg aatgaaacta tatcctgcaa tgctttatta tattgcaatg 4440

attgtaaacc gccattcaga gtttaggacg gcaatcaatc aagatggtga attggggata 4500

tatgatgaga tgataccaag ctatacaata tttcacaatg atactgaaac attttccagc 4560

ctttggactg agtgtaagtc tgactttaaa tcatttttag cagattatga aagtgatacg 4620

caacggtatg gaaacaatca tagaatggaa ggaaagccaa atgctccgga aaacattttt 4680

aatgtatcta tgataccgtg gtcaaccttc gatggcttta atctgaattt gcagaaagga 4740

tatgattatt tgattcctat ttttactatg gggaaatatt ataaagaaga taacaaaatt 4800

atacttcctt tggcaattca agttcatcac gcagtatgtg acggatttca catttgccgt 4860

tttgtaaacg aattgcagga attgataaat agttaacttc aggtttgtct gtaactaaaa 4920

actagtattt aacctagg 4938

<210> 106
<211> 4790
<212> DNA
<213> Artificial Sequence

<220>
<223> pEC750C

<400> 106
atcaaaaaaa tttccaataa tcccactcta agccacaaac acgccctata aaatcccgct 60

ttaatcccac tttgagacac atgtaatatt actttacgcc ctagtatagt gataattttt 120

tacattcaat gccacgcaaa aaaataaagg ggcactataa taaaagttcc ttcggaacta 180

actaaagtaa aaaattatct ttacaacctc cccaaaaaaa agaacaggta caaagtaccc 240

tataatacaa gcgtaaaaaa aatgagggta aaaataaaaa aataaaaaaa taaaaaaata 300

aaaaaataaa aaaataaaaa aataaaaaaa tataaaaata aaaaaatata aaaataaaaa 360

aatataaaaa taaaaaaata aaaaaatata aaaataaaaa aataaaaaaa tataaaaata 420

ttttttattt aaagtttgaa aaaaattttt ttatattata taatctttga agaaaagaat 480

ataaaaaatg agcctttata aaagcccatt ttttttcata tacgtaatat gacgttctaa 540

tgtttttatt ggtacttcta acattagagt aatttcttta tttttaaagc ctttttcttt 600

aagggctttt attttttttc ttaatacatt taattcctct ttttttgttg cttttccttt 660

agcttttaat tgctcttgat aatttttttt acctctaata ttttctcttc tcttatattc 720

ctttttagaa attattattg tcatatattt ttgttcttct tctgtaattt ctaataactc 780

tataagagtt tcattcttat acttatattg cttattttta tctaaataac atctttcagc 840

acttctagtt gctcttataa cttctctttc acttaaatgt tgtctaaaca tactattaag 900

ttctaaaaca tcatttaatg ccttctcaat gtcttctgta aagctacaaa gataatatct 960

atataaaaat aatataagct ctctgtgtcc ttttaaatca tattctctta gttcacaaag 1020

ttttattatg tcttgtattc ttccataata taaacttctt tctctataaa tataatttat 1080

tttgcttggt ctaccctttt tcctttcata tggttttaat ttcaggtaaa aatccatttg 1140

tatttctctt aagtcataaa tatattcgta ctcatctaat atattgacta ctgtttttga 1200

tttagagttt atacttcctg gaactcttaa tattctcgtt gcatctaagg cttgtctatc 1260

tgctccaaag tattttaatt gattatataa atattcttga accgctttcc ataatggtaa 1320

tgctttacta ggtactgcat ttattatcca tattaaatac attcctcttc cactatctat 1380

tacatagttt ggtataggaa tactttgatt aaaataattc ttttctaagt ccattaatac 1440

ctggtcttta gttttgccag ttttataata atccaagtct ataaacagtg tatttaactc 1500

ttttatattt tctaatcgcc tacacggctt ataaaaggta tttagagtta tatagatatt 1560

ttcatcactc atatctaaat cttttaattc agcgtattta tagtgccatt ggctatatcc 1620

ttttttatct ataacgctcc tggttatcca ccctttactt ctactatgaa tattatctat 1680

atagttcttt ttattcagct ttaatgcgtt tctcacttat tcacctcccc ttctgtaaaa 1740

ctaagaaaat tatatcatat tttcaataat tattaactat tcttaaactc ttaataaaaa 1800

atagagtaag tccccaattg aaacttaatc tattttttat gttttaattt attattttta 1860

ttaaaatatt ttaaactaaa ttaaatgatt ctttttaatt ttttactatt tcattccata 1920

atatattact ataattattt acaaataata tttcttcatt tgtaatattt agatgattta 1980

ctaattttag tttttatata ttaaataatt aatgtataat ttatataaaa aatcaaagga 2040

gcttataaat tatgattatt tccaaagata ctaaagattt aatttttttc aattttaaca 2100

atactttttg taatattatg tttaaattta attgtatttt tttcatataa taaagccgtt 2160

gaagtaaacc aatccatttt ccttatgatg ttattattaa atttaagttt tataataata 2220

tctttattat atttattgtt tttaaaaaaa ctagtgaaat ttctagtgaa atttccggct 2280

ttattaaact tatttttagg aattttattt tcattttcat ctttacagga tttgattata 2340

tctttaaata tgttttatca aatattatct ttttctaaat ttatatatat ttttattata 2400

tttattatta tatatatttt atttttaagt ttctttctaa cagctattaa aaagaaactt 2460

aaaaataaaa acacgtactc taaaccaata aataaaacta tttttattat tgctgccttg 2520

attggaatag tttttagtaa aattaatttc aatattccac aatattatat tataagctag 2580

cacgcctcga gatctccatg gacgcgtgac gtcgactcta gaggatcccc gggtaccgag 2640

ctcgaattcg taatcatggt catagctgtt tcctgtgtga aattgttatc cgctcacaat 2700

tccacacaac atacgagccg gaagcataaa gtgtaaagcc tggggtgcct aatgagtgag 2760

ctaactcaca ttaattgcgt tgcgctcact gcccgctttc cagtcgggaa acctgtcgtg 2820

ccagctgcat taatgaatcg gccaacgcgc ggggagaggc ggtttgcgta ttgggcgctc 2880

ttccgcttcc tcgctcactg actcgctgcg ctcggtcgtt cggctgcggc gagcggtatc 2940

agctcactca aaggcggtaa tacggttatc cacagaatca ggggataacg caggaaagaa 3000

catgtgagca aaaggccagc aaaaggccag gaaccgtaaa aaggccgcgt tgctggcgtt 3060

tttccatagg ctccgccccc ctgacgagca tcacaaaaat cgacgctcaa gtcagaggtg 3120

gcgaaacccg acaggactat aaagatacca ggcgtttccc cctggaagct ccctcgtgcg 3180

ctctcctgtt ccgaccctgc cgcttaccgg atacctgtcc gcctttctcc cttcgggaag 3240

cgtggcgctt tctcatagct cacgctgtag gtatctcagt tcggtgtagg tcgttcgctc 3300

caagctgggc tgtgtgcacg aaccccccgt tcagcccgac cgctgcgcct tatccggtaa 3360

ctatcgtctt gagtccaacc cggtaagaca cgacttatcg ccactggcag cagccactgg 3420

taacaggatt agcagagcga ggtatgtagg cggtgctaca gagttcttga agtggtggcc 3480

taactacggc tacactagaa gaacagtatt tggtatctgc gctctgctga agccagttac 3540

cttcggaaaa agagttggta gctcttgatc cggcaaacaa accaccgctg gtagcggtgg 3600

tttttttgtt tgcaagcagc agattacgcg cagaaaaaaa ggatctcaag aagatccttt 3660

gatcttttct acggggtctg acgctcagtg gaacgaaaac tcacgttaag ggattttggt 3720

catgagatta tcaaaaagga tcttcaccta gatcctttta aattaaaaat gaagttttaa 3780

atcaatctaa agtatatatg agtaaacttg gtctgacagt taccaaagct agcttaatac 3840

tagtatatac ttaatgtgat aagtgtctga cagctgaccg gtctaaagag gtccctagcg 3900

cctacgggga atttgtatcg ataaggggta caaattccca ctaagcgctc ggccggggat 3960

cgatccccgg gtacgtaccc ggcagttttt ctttttcggc aagtgttcaa gaagttatta 4020

agtcgggagt gcagtcgaag tgggcaagtt gaaaaattca caaaaatgtg gtataatatc 4080

tttgttcatt agagcgataa acttgaattt gagagggaac ttagatggta tttgaaaaaa 4140

ttgataaaaa tagttggaac agaaaagagt attttgacca ctactttgca agtgtacctt 4200

gtacctacag catgaccgtt aaagtggata tcacacaaat aaaggaaaag ggaatgaaac 4260

tatatcctgc aatgctttat tatattgcaa tgattgtaaa ccgccattca gagtttagga 4320

cggcaatcaa tcaagatggt gaattgggga tatatgatga gatgatacca agctatacaa 4380

tatttcacaa tgatactgaa acattttcca gcctttggac tgagtgtaag tctgacttta 4440

aatcattttt agcagattat gaaagtgata cgcaacggta tggaaacaat catagaatgg 4500

aaggaaagcc aaatgctccg gaaaacattt ttaatgtatc tatgataccg tggtcaacct 4560

tcgatggctt taatctgaat ttgcagaaag gatatgatta tttgattcct atttttacta 4620

tggggaaata ttataaagaa gataacaaaa ttatacttcc tttggcaatt caagttcatc 4680

acgcagtatg tgacggattt cacatttgcc gttttgtaaa cgaattgcag gaattgataa 4740

atagttaact tcaggtttgt ctgtaactaa aaactagtat ttaacctagg 4790

<210>  107
<211>  35
<212>  DNA
<213>  Artificial Sequence

<220>
<223>  Primer

<400>  107
acttgggtcg accacgataa aacaaggttt taagg                                  35


<210>  108
<211>  42
<212>  DNA
<213>  Artificial Sequence

<220>
<223>  Primer

<400>  108
taccagggat ccgtattaat gtaactatga tatcaattct tg                          42


<210>  109
<211>  46
<212>  DNA
<213>  Artificial Sequence

<220>
<223>  Primer

<400>  109
atgcatggtc ccaatgaata ggtttacact tactttagtt ttatgg                      46


<210>  110
<211>  39
<212>  DNA
<213>  Artificial Sequence

<220>
<223>  Primer

<400>  110
atgcgagtta acaacttcta aaatctgatt accaattag                              39


<210>  111
<211>  47
<212>  DNA
<213>  Artificial Sequence

<220>
<223>  Primer

<400>  111
atgcatggat cccaatgaat aggtttacac ttactttagt tttatgg                     47


<210>  112

<211>  39
<212>  DNA
<213>  Artificial Sequence

<220>
<223>  Primer

<400>  112
atgcgagagc tcaacttcta aaatctgatt accaattag                              39


<210>  113
<211>  32
<212>  DNA
<213>  Artificial Sequence

<220>
<223>  Primer

<400>  113
atgcatggat ccgtctgaca gttaccaggt cc                                     32


<210>  114
<211>  39
<212>  DNA
<213>  Artificial Sequence

<220>
<223>  Primer

<400>  114
atgcgagagc tccaattgtt caaaaaaata atggcggag                              39


<210>  115
<211>  32
<212>  DNA
<213>  Artificial Sequence

<220>
<223>  Primer

<400>  115
atgcatggat cccggcagtt tttctttttc gg                                     32


<210>  116
<211>  40
<212>  DNA
<213>  Artificial Sequence

<220>
<223>  Primer

<400>  116
atgcgagagc tcggttaaat actagttttt agttacagac                             40


<210>  117
<211>  2686
<212>  DNA
<213>  Artificial Sequence

<220>
<223>  pUC19

<400>  117
gcgcccaata cgcaaaccgc ctctccccgc gcgttggccg attcattaat gcagctggca      60

cgacaggttt cccgactgga aagcgggcag tgagcgcaac gcaattaatg tgagttagct     120

cactcattag gcaccccagg ctttacactt tatgcttccg gctcgtatgt tgtgtggaat     180

tgtgagcgga taacaatttc acacaggaaa cagctatgac catgattacg ccaagcttgc     240

atgcctgcag gtcgactcta gaggatcccc gggtaccgag ctcgaattca ctggccgtcg     300

ttttacaacg tcgtgactgg gaaaaccctg gcgttaccca acttaatcgc cttgcagcac     360

atcccccttt cgccagctgg cgtaatagcg aagaggcccg caccgatcgc ccttcccaac     420

agttgcgcag cctgaatggc gaatggcgcc tgatgcggta ttttctcctt acgcatctgt     480

gcggtatttc acaccgcata tggtgcactc tcagtacaat ctgctctgat gccgcatagt     540

taagccagcc ccgacacccg ccaacacccg ctgacgcgcc ctgacgggct tgtctgctcc     600

cggcatccgc ttacagacaa gctgtgaccg tctccgggag ctgcatgtgt cagaggtttt     660

caccgtcatc accgaaacgc gcgagacgaa agggcctcgt gatacgccta tttttatagg     720

ttaatgtcat gataataatg gtttcttaga cgtcaggtgg cacttttcgg ggaaatgtgc     780

gcggaacccc tatttgttta tttttctaaa tacattcaaa tatgtatccg ctcatgagac     840

aataaccctg ataaatgctt caataatatt gaaaaaggaa gagtatgagt attcaacatt     900

tccgtgtcgc ccttattccc ttttttgcgg cattttgcct tcctgttttt gctcacccag     960

aaacgctggt gaaagtaaaa gatgctgaag atcagttggg tgcacgagtg ggttacatcg    1020

aactggatct caacagcggt aagatccttg agagttttcg ccccgaagaa cgttttccaa    1080

tgatgagcac ttttaaagtt ctgctatgtg gcgcggtatt atcccgtatt gacgccgggc    1140

aagagcaact cggtcgccgc atacactatt ctcagaatga cttggttgag tactcaccag    1200

tcacagaaaa gcatcttacg gatggcatga cagtaagaga attatgcagt gctgccataa    1260

ccatgagtga taacactgcg gccaacttac ttctgacaac gatcggagga ccgaaggagc    1320

taaccgcttt tttgcacaac atgggggatc atgtaactcg ccttgatcgt tgggaaccgg    1380

agctgaatga agccatacca aacgacgagc gtgacaccac gatgcctgta gcaatggcaa    1440

caacgttgcg caaactatta actggcgaac tacttactct agcttcccgg caacaattaa    1500

tagactggat ggaggcggat aaagttgcag gaccacttct gcgctcggcc cttccggctg    1560

gctggtttat tgctgataaa tctggagccg gtgagcgtgg gtctcgcggt atcattgcag    1620

cactggggcc agatggtaag ccctcccgta tcgtagttat ctacacgacg gggagtcagg    1680

caactatgga tgaacgaaat agacagatcg ctgagatagg tgcctcactg attaagcatt    1740

ggtaactgtc agaccaagtt tactcatata tactttagat tgatttaaaa cttcattttt 1800

aatttaaaag gatctaggtg aagatccttt ttgataatct catgaccaaa atcccttaac 1860

gtgagttttc gttccactga gcgtcagacc ccgtagaaaa gatcaaagga tcttcttgag 1920

atcctttttt tctgcgcgta atctgctgct tgcaaacaaa aaaaccaccg ctaccagcgg 1980

tggtttgttt gccggatcaa gagctaccaa ctctttttcc gaaggtaact ggcttcagca 2040

gagcgcagat accaaatact gttcttctag tgtagccgta gttaggccac cacttcaaga 2100

actctgtagc accgcctaca tacctcgctc tgctaatcct gttaccagtg gctgctgcca 2160

gtggcgataa gtcgtgtctt accgggttgg actcaagacg atagttaccg gataaggcgc 2220

agcggtcggg ctgaacgggg ggttcgtgca cacagcccag cttggagcga acgacctaca 2280

ccgaactgag atacctacag cgtgagctat gagaaagcgc cacgcttccc gaagggagaa 2340

aggcggacag gtatccggta agcggcaggg tcggaacagg agagcgcacg agggagcttc 2400

cagggggaaa cgcctggtat ctttatagtc ctgtcgggtt tcgccacctc tgacttgagc 2460

gtcgattttt gtgatgctcg tcaggggggc ggagcctatg gaaaaacgcc agcaacgcgg 2520

cctttttacg gttcctggcc ttttgctggc cttttgctca catgttcttt cctgcgttat 2580

cccctgattc tgtggataac cgtattaccg cctttgagtg agctgatacc gctcgccgca 2640

gccgaacgac cgagcgcagc gagtcagtga gcgaggaagc ggaaga 2686

<210> 118
<211> 4282
<212> DNA
<213> Artificial Sequence

<220>
<223> pNF2

<400> 118
ctggagagga ttgtccttat acttatcata agcatgaagg acttgttatt cctagataga 60

gaattaatta tgttaaagag atataataaa ctcattataa ttataatttt tagtataatt 120

attattgcaa ttttttcgta taaatatcta ataatgccaa aagagcatag aatagaaatt 180

tcaacattat caaacataga agtttttaaa tttaatagtt tttcaaagtt tagtaacgaa 240

aaaatgtata ctattaatga tagtgataag ttaataaaat tcaaaacact atttaataat 300

ttagataaat caaaagatat aaaaaagatt agtattccgg aaagtgaaaa tttaaatgca 360

tttaaatttt ctgcacatat aaaacttaac tttaactatg ttaataaaga tagccaaata 420

actgaaggtg cttttcttat gtatattttg gtagacaatt tagaagggaa gtcatatatg 480

acttttttag gacaagattc aagctatata ttagatagta atgaaactaa cattttaaga 540

gaaatattta tgaattcaga gattaattaa tttatgaatt cataaatatt atctaagcac 600

gataaaacaa ggttttaagg ataagaaaag tcatgagatt tatagtaaat cttgtgactt 660

tttttattga atagtagaga gagttcggaa gtataacacg ctatattctt gatattttta 720

gaatagcaag cattggattt gtcctgacac tttcccaaaa attaaggagt tattccttaa 780

accaaaaaga ttaatgtggg aacaaattta gtgtatccat ttttgaaggg cgcacttata 840

caccaccaaa atggtgtgtg cgaaatcttt aaaaaagatt tatcaaaaag cttttttaaa 900

gctgggacat ttagaaaatc aataatgttt tttgcccaat acgctagtct taaaatctgc 960

aaggttgata actatttagt cccaggtatt agaatggggc atatatatac aaagtatata 1020

tatgcgtaaa tatatgtggg actgtgggaa caaaattgcg tgctaaaatt gtattgaaaa 1080

ggtaatgaaa aggtcatgct ttggtattgc taacgtatag aaaaggtaat gaaaagctca 1140

tggttctata aaaaagatgt acccacgaaa ataataggct ttgcctattt ccccatgtaa 1200

tatgggggca gttttctctt atgctctttc ttaacatatt gaataaatac aaaatgcagc 1260

tttgtgggaa taaaaatatt tttgttttta ttcttatagt tagacaaaat tttaatcttt 1320

tttgtgctat aacaagatta aaatttgtgg gaacattaag aaatattgtt gtcacaaata 1380

aaaaggagag tgggaacaat tgctataaaa aacgcagaaa ttaagattag agttacaaaa 1440

gagcaaaaag aattatttaa gaaaattgca aaagctgaaa atatgagtat gagtgaattt 1500

attattgtga ccacagaata tttagccaga aaaaaagatg aaaatatgaa atcaaaagac 1560

atgatcgaga gaagagctgc gaagactgaa gaaaaaatta tgaagctaaa aaagaaacta 1620

aataaaaaca ggtaatatag attacagttt taagcttgtt ttccctatag actagagtaa 1680

atatataaat atacctgtca agggcttata agccccttta gggggtgcgt agcacccttg 1740

acaggtatat ttatatattt tagggtgcca ttaagggaaa caagctttaa aatgccttta 1800

aaggcatttt aaaataaata aaaaaaagat ggtttttacc atctttttta actcccgaaa 1860

gggagttctt tcttttcttg atactatacg taactatttc gatttgccct gaacctaatc 1920

aaagctagat aaattcagta ttagggcata aaaaaacttg ctttttcggg tggaaatctg 1980

tataatttaa attgcttaga taaaaattac caattccata cgaaaggagc aagttttaca 2040

taaggttaaa gccttatgtg aattctcatt taattacatg aataataata acacagaaag 2100

tgaagaatta aaagagcaaa gtcaactatt gcttgacaaa tgcacaaaaa agaaaaagaa 2160

aaatcctaaa tttagtagtt atatagaacc attagtaagc aagaaattat ctgaaagaat 2220

aaaggaatgt ggtgactttt tgcagatgtt atctgattta aaccttgaaa attcgaaact 2280

gcatagagca agtttttgtg gtaacagatt ttgtcctatg tgtagctggc gtattgcttg 2340

taaggatagt ttggaaatat ctattctcat ggagcattta cgcaaagagg aaagcaaaga 2400

atttatcttt ttgaccttaa caactccaaa tgtgaaaggt gcggaccttg ataattccat 2460

aaaagcatac aataaagcat ttaaaaagtt aatggaacgc aaagaggtca agagcatagt 2520

aaaaggctac ataagaaagc tagaagtaac ctataatttg gacaagagtt ccaaatcata 2580

taatacttat cacccacatt tccatgtggt actagcagtc aatagaagtt actttaaaaa 2640

gcaaaatcta tatataaacc atcatagatg gcttagtttg tggcaagagt caactggtga 2700

ttattcgata actcaagttg atgtaagaaa ggctaaaatt aacgattata aagaggttta 2760

tgagcttgct aagtattcgg ctaaggattc cgactattta atcaatagag aagtgtttac 2820

ggtattctac aaatctttaa agggtaaaca ggtacttgta tttagtggat tatttaaaga 2880

cgctcataaa atgtataaga atggagagct agatctgtat aagaagttgg atactatcga 2940

atatgcttat atggtaagtt ataactggct taaaaagaag tatgatactt caaatattag 3000

agaattaact gaggaagaaa agcagaaatt caataaaaat ttaatcgaag atgtggatat 3060

tgagtaggtg ggattatatc tcaccttttt tattgtcttt tcatgttgaa attttgacgc 3120

ttaatgcatg aagtattgac aagtttaaaa attacggttt ttaatcctta gttgattagc 3180

aggattatgg ccggaatgct ccgtccagtc ctgttaagga attaaaattc cctaaaaccc 3240

ttggctatga tttatagcga gaatcgtcaa ttaaaaattt aataggtgct atgaaagtcg 3300

attaataatt aattttaaaa tgcaatatga aacataatta caagaatttg acttttaata 3360

caagaattga tatcatagtt acattaatac atttattttg aagggggaaa atgttttatg 3420

aaaagactac ttaaactacc tattttatca ttattaggat tatttttaat tggatcaact 3480

ccaacattag ctttaactaa agataataat caaaatttag atactatgaa agtaaactta 3540

tatactgaaa cagtagatgt gtttgataaa gatgcattta aacaaacatt tactaataaa 3600

gatataaaat ttctagagga ttctttgaat gcaaaaataa attattcagg taaatctgtt 3660

acagtaacaa tgaaaaacaa aattaagcca tctactaaac aagggcttgt tttatatgta 3720

aatggaaaat cagttaatgt tgattcagat ggcagtataa aagtacctaa agatactaag 3780

aaaatttcta aattaaataa agataaatca atgatggatg gatcaatgat ggataaatca 3840

ttacatgatg agaattgtgt agtatcagat agtttttata atgctgatgt taataatata 3900

aattcaaaag aagcagaagc tgtatttaaa gtaagttctg gtgaattatt agctaaaatg 3960

gatgaaaaag aagatgatta catacaaaag aactcatcta aaattctagc agctgcttat 4020

cataagggat atggggacaa gtactatgaa ggagattggg ttcattgcaa taggtttaat 4080

ggtcaactta cagatgatgt tcactataat tggagaactg gaagtgtttc agaaaaagca 4140

gctgcaatga gaaattttta tggcagtgat tgtcatatag cattagttca agcaggtagt 4200

ggatgtacaa gtataggttc atgcgaatgc aatacagatc aaatagctgc gtattgttca 4260

ggtttcgtaa aagataaaaa ta 4282

<210> 119
<211> 5473

<212>  DNA
<213>  Artificial Sequence

<220>
<223>  pNF3

<400>  119
gcgcccaata cgcaaaccgc ctctccccgc gcgttggccg attcattaat gcagctggca      60

cgacaggttt cccgactgga aagcgggcag tgagcgcaac gcaattaatg tgagttagct     120

cactcattag gcaccccagg ctttacactt tatgcttccg gctcgtatgt tgtgtggaat     180

tgtgagcgga taacaatttc acacaggaaa cagctatgac catgattacg ccaagcttgc     240

atgcctgcag gtcgaccacg ataaaacaag gttttaagga taagaaaagt catgagattt     300

atagtaaatc ttgtgacttt ttttattgaa tagtagagag agttcggaag tataacacgc     360

tatattcttg atatttttag aatagcaagc attggatttg tcctgacact ttcccaaaaa     420

ttaaggagtt attccttaaa ccaaaaagat taatgtggga acaaatttag tgtatccatt     480

tttgaagggc gcacttatac accaccaaaa tggtgtgtgc gaaatcttta aaaaagattt     540

atcaaaaagc ttttttaaag ctgggacatt tagaaaatca ataatgtttt ttgcccaata     600

cgctagtctt aaaatctgca aggttgataa ctatttagtc ccaggtatta gaatggggca     660

tatatataca aagtatatat atgcgtaaat atatgtggga ctgtgggaac aaaattgcgt     720

gctaaaattg tattgaaaag gtaatgaaaa ggtcatgctt tggtattgct aacgtataga     780

aaaggtaatg aaaagctcat ggttctataa aaaagatgta cccacgaaaa taataggctt     840

tgcctatttc cccatgtaat atgggggcag ttttctctta tgctctttct taacatattg     900

aataaataca aaatgcagct ttgtgggaat aaaaatattt ttgtttttat tcttatagtt     960

agacaaaatt ttaatctttt ttgtgctata acaagattaa aatttgtggg aacattaaga    1020

aatattgttg tcacaaataa aaaggagagt gggaacaatt gctataaaaa acgcagaaat    1080

taagattaga gttacaaaag agcaaaaaga attatttaag aaaattgcaa aagctgaaaa    1140

tatgagtatg agtgaattta ttattgtgac cacagaatat ttagccagaa aaaaagatga    1200

aaatatgaaa tcaaaagaca tgatcgagag aagagctgcg aagactgaag aaaaaattat    1260

gaagctaaaa aagaaactaa ataaaaacag gtaatataga ttacagtttt aagcttgttt    1320

tccctataga ctagagtaaa tatataaata tacctgtcaa gggcttataa gcccctttag    1380

ggggtgcgta gcacccttga caggtatatt tatatatttt agggtgccat taagggaaac    1440

aagctttaaa atgcctttaa aggcatttta aaataaataa aaaaaagatg gtttttacca    1500

tcttttttaa ctcccgaaag ggagttcttt cttttcttga tactatacgt aactatttcg    1560

atttgccctg aacctaatca aagctagata aattcagtat tagggcataa aaaaacttgc    1620

tttttcgggt ggaaatctgt ataatttaaa ttgcttagat aaaaattacc aattccatac    1680

gaaaggagca agttttacat aaggttaaag ccttatgtga attctcattt aattacatga 1740

ataataataa cacagaaagt gaagaattaa aagagcaaag tcaactattg cttgacaaat 1800

gcacaaaaaa gaaaaagaaa aatcctaaat ttagtagtta tatagaacca ttagtaagca 1860

agaaattatc tgaaagaata aaggaatgtg gtgacttttt gcagatgtta tctgatttaa 1920

accttgaaaa ttcgaaactg catagagcaa gtttttgtgg taacagattt tgtcctatgt 1980

gtagctggcg tattgcttgt aaggatagtt tggaaatatc tattctcatg gagcatttac 2040

gcaaagagga aagcaaagaa tttatctttt tgaccttaac aactccaaat gtgaaaggtg 2100

cggaccttga taattccata aaagcataca ataaagcatt taaaaagtta atggaacgca 2160

aagaggtcaa gagcatagta aaaggctaca taagaaagct agaagtaacc tataatttgg 2220

acaagagttc caaatcatat aatacttatc acccacattt ccatgtggta ctagcagtca 2280

atagaagtta ctttaaaaag caaaatctat atataaacca tcatagatgg cttagtttgt 2340

ggcaagagtc aactggtgat tattcgataa ctcaagttga tgtaagaaag gctaaaatta 2400

acgattataa agaggtttat gagcttgcta agtattcggc taaggattcc gactatttaa 2460

tcaatagaga agtgtttacg gtattctaca aatctttaaa gggtaaacag gtacttgtat 2520

ttagtggatt atttaaagac gctcataaaa tgtataagaa tggagagcta gatctgtata 2580

agaagttgga tactatcgaa tatgcttata tggtaagtta taactggctt aaaaagaagt 2640

atgatacttc aaatattaga gaattaactg aggaagaaaa gcagaaattc aataaaaatt 2700

taatcgaaga tgtggatatt gagtaggtgg gattatatct cacctttttt attgtctttt 2760

catgttgaaa ttttgacgct taatgcatga agtattgaca agtttaaaaa ttacggtttt 2820

taatccttag ttgattagca ggattatggc cggaatgctc cgtccagtcc tgttaaggaa 2880

ttaaaattcc ctaaaaccct tggctatgat ttatagcgag aatcgtcaat taaaaattta 2940

ataggtgcta tgaaagtcga ttaataatta attttaaaat gcaatatgaa acataattac 3000

aagaatttga cttttaatac aagaattgat atcatagtta cattaatacg gatccccggg 3060

taccgagctc gaattcactg gccgtcgttt tacaacgtcg tgactgggaa aaccctggcg 3120

ttacccaact taatcgcctt gcagcacatc cccctttcgc cagctggcgt aatagcgaag 3180

aggcccgcac cgatcgccct tcccaacagt tgcgcagcct gaatggcgaa tggcgcctga 3240

tgcggtattt tctccttacg catctgtgcg gtatttcaca ccgcatatgg tgcactctca 3300

gtacaatctg ctctgatgcc gcatagttaa gccagccccg acacccgcca acacccgctg 3360

acgcgccctg acgggcttgt ctgctcccgg catccgctta cagacaagct gtgaccgtct 3420

ccgggagctg catgtgtcag aggttttcac cgtcatcacc gaaacgcgcg agacgaaagg 3480

gcctcgtgat acgcctattt ttataggtta atgtcatgat aataatggtt tcttagacgt 3540

caggtggcac ttttcgggga aatgtgcgcg gaacccctat ttgtttattt ttctaaatac 3600

attcaaatat gtatccgctc atgagacaat aaccctgata aatgcttcaa taatattgaa 3660

aaaggaagag tatgagtatt caacatttcc gtgtcgccct tattcccttt tttgcggcat 3720

tttgccttcc tgtttttgct cacccagaaa cgctggtgaa agtaaaagat gctgaagatc 3780

agttgggtgc acgagtgggt tacatcgaac tggatctcaa cagcggtaag atccttgaga 3840

gttttcgccc cgaagaacgt tttccaatga tgagcacttt taaagttctg ctatgtggcg 3900

cggtattatc ccgtattgac gccgggcaag agcaactcgg tcgccgcata cactattctc 3960

agaatgactt ggttgagtac tcaccagtca cagaaaagca tcttacggat ggcatgacag 4020

taagagaatt atgcagtgct gccataacca tgagtgataa cactgcggcc aacttacttc 4080

tgacaacgat cggaggaccg aaggagctaa ccgctttttt gcacaacatg ggggatcatg 4140

taactcgcct tgatcgttgg gaaccggagc tgaatgaagc cataccaaac gacgagcgtg 4200

acaccacgat gcctgtagca atggcaacaa cgttgcgcaa actattaact ggcgaactac 4260

ttactctagc ttcccggcaa caattaatag actggatgga ggcggataaa gttgcaggac 4320

cacttctgcg ctcggccctt ccggctggct ggtttattgc tgataaatct ggagccggtg 4380

agcgtgggtc tcgcggtatc attgcagcac tggggccaga tggtaagccc tcccgtatcg 4440

tagttatcta cacgacgggg agtcaggcaa ctatggatga acgaaataga cagatcgctg 4500

agataggtgc ctcactgatt aagcattggt aactgtcaga ccaagtttac tcatatatac 4560

tttagattga tttaaaactt catttttaat ttaaaaggat ctaggtgaag atcctttttg 4620

ataatctcat gaccaaaatc ccttaacgtg agttttcgtt ccactgagcg tcagaccccg 4680

tagaaaagat caaaggatct tcttgagatc ctttttttct gcgcgtaatc tgctgcttgc 4740

aaacaaaaaa accaccgcta ccagcggtgg tttgtttgcc ggatcaagag ctaccaactc 4800

tttttccgaa ggtaactggc ttcagcagag cgcagatacc aaatactgtt cttctagtgt 4860

agccgtagtt aggccaccac ttcaagaact ctgtagcacc gcctacatac ctcgctctgc 4920

taatcctgtt accagtggct gctgccagtg gcgataagtc gtgtcttacc gggttggact 4980

caagacgata gttaccggat aaggcgcagc ggtcgggctg aacggggggt tcgtgcacac 5040

agcccagctt ggagcgaacg acctacaccg aactgagata cctacagcgt gagctatgag 5100

aaagcgccac gcttcccgaa gggagaaagg cggacaggta tccggtaagc ggcagggtcg 5160

gaacaggaga gcgcacgagg gagcttccag ggggaaacgc ctggtatctt tatagtcctg 5220

tcgggtttcg ccacctctga cttgagcgtc gatttttgtg atgctcgtca ggggggcgga 5280

gcctatggaa aaacgccagc aacgcggcct ttttacggtt cctggccttt tgctggcctt 5340

ttgctcacat gttctttcct gcgttatccc ctgattctgt ggataaccgt attaccgcct 5400

ttgagtgagc tgataccgct cgccgcagcc gaacgaccga gcgcagcgag tcagtgagcg 5460

aggaagcgga aga                                                          5473

<210>  120
<211>  9128
<212>  DNA
<213>  Artificial Sequence

<220>
<223>  pMTL007S-E1

<400>  120
gatcgggccc cctgcagggt gtagtagcct gtgaaataag taaggaaaaa aaagaagtaa      60

gtgttatata tgatgattat tttgtagatg tagataggat aatagaatcc atagaaaata     120

taggttatac agttatataa aaattacttt aaaaattaat aaaaacatgg taaaatataa     180

atcgtataaa gttgtgtaat ttttaagctt gagctcataa caatttcaca caggaaacag     240

ctatgaccat gattacggat tcactggccg tcgttttaca acgtcgtgac tgggaaaacc     300

ctggcgttac ccaacttaat cgccttgcag cacatccccc tttcgccagc tggcgtaata     360

gcgaagaggc ccgcaccgat cgcccttccc aacagttgcg cagcctgaat ggcgaatggc     420

gctaataaag atcttgtaca atctgtagga gaacctatgg gaacgaaacg aaagcgatgc     480

cgagaatctg aatttaccaa gacttaacac taactgggga taccctaaac aagaatgcct     540

aatagaaagg aggaaaaagg ctatagcact agagcttgaa aatcttgcaa gggtacggag     600

tactcgtagt agtctgagaa gggtaacgcc ctttacatgg caaaggggta cagttattgt     660

gtactaaaat taaaaattga ttagggagga aaacctcaaa atgaaaccaa caatggcaat     720

tttagaaaga atcagtaaaa attcacaaga aaatatagac gaagttttta caagacttta     780

tcgttatctt ttacgtccag atatttatta cgtggcgacg cgtgcgactc atagaattat     840

ttcctcccgt taaataatag ataactatta aaaatagaca atacttgctc ataagtaacg     900

gtacttaaat tgtttacttt ggcgtgtttc attgcttgat gaaactgatt tttagtaaac     960

agttgacgat attctcgatt gacccatttt gaaacaaagt acgtatatag cttccaatat    1020

ttatctggaa catctgtggt atggcgggta agttttatta agacactgtt tacttttggt    1080

ttaggatgaa agcattccgc tggcagctta agcaattgct gaatcgagac ttgagtgtgc    1140

aagagcaacc ctagtgttcg gtgaatatcc aaggtacgct tgtagaatcc ttcttcaaca    1200

atcagataga tgtcagacgc atggctttca aaaaccactt ttttaataat ttgtgtgctt    1260

aaatggtaag gaatactccc aacaatttta tacctctgtt tgttagggaa ttgaaactgt    1320

agaatatctt ggtgaattaa agtgacacga gtattcagtt ttaatttttc tgacgataag    1380

ttgaatagat gactgtctaa ttcaatagac gttacctgtt tacttatttt agccagtttc    1440

gtcgttaaat gccctttacc tgttccaatt tcgtaaacgg tatcggtttc ttttaaattc    1500

aattgtttta ttatttggtt gagtactttt tcactcgtta aaaagttttg agaatatttt    1560

atatttttgt tcataccagc accagaagca ccagcatctc ttgggttaat tgaggcctga 1620

gtataaggtg acttatactt gtaatctatc taaacgggga acctctctag tagacaatcc 1680

cgtgctaaat tgtaggactg ccctttaata aatacttcta tatttaaaga ggtatttatg 1740

aaaagcggaa tttatcagat taaaaatact ttctctagag aaaatttcgt ctggattagt 1800

tacttatcgt gtaaaatctg ataaatggaa ttggttctac ataaatgcct aacgactatc 1860

cctttgggga gtagggtcaa gtgactcgaa acgatagaca acttgcttta acaagttgga 1920

gatatagtct gctctgcatg gtgacatgca gctggatata attccggggt aagattaacg 1980

accttatctg aacataatgc catatgaatc cctcctaatt tatacgtttt ctctaacaac 2040

ttaattatac ccactattat tatttttatc aatataacgc gttgggaaat ggcaatgata 2100

gcgaaacaac gtaaaactct tgttgtatgc tttcattgtc atcgtcacgt gattcataaa 2160

cacaagtgaa tgtcgacagt gaatttttac gaacgaacaa taacagagcc gtatactccg 2220

agaggggtac gtacggttcc cgaagagggt ggtgcaaacc agtcacagta atgtgaacaa 2280

ggcggtacct ccctacttca ccatatcatt ttctgcagcc ccctagaaat aattttgttt 2340

aactttaaga aggagatata catatatggc tagatcgtcc attccgacag catcgccagt 2400

cactatggcg tgctgctagc gctatatgcg ttgatgcaat ttctatgcac tcgtagtagt 2460

ctgagaaggg taacgccctt tacatggcaa aggggtacag ttattgtgta ctaaaattaa 2520

aaattgatta gggaggaaaa cctcaaaatg aaaccaacaa tggcaatttt agaaagaatc 2580

agtaaaaatt cacaagaaaa tatagacgaa gtttttacaa gactttatcg ttatctttta 2640

cgtccagata tttattacgt ggcgtatcaa aatttatatt ccaataaagg agcttccaca 2700

aaaggaatat tagatgatac agcggatggc tttagtgaag aaaaaataaa aaagattatt 2760

caatctttaa aagacggaac ttactatcct caacctgtac gaagaatgta tattgcaaaa 2820

aagaattcta aaaagatgag acctttagga attccaactt tcacagataa attgatccaa 2880

gaagctgtga gaataattct tgaatctatc tatgaaccgg tattcgaaga tgtgtctcac 2940

ggttttagac ctcaacgaag ctgtcacaca gctttgaaaa caatcaaaag agagtttggc 3000

ggcgcaagat ggtttgtgga gggagatata aaaggctgct tcgataatat agaccacgtt 3060

acactcattg gactcatcaa tcttaaaatc aaagatatga aaatgagcca attgatttat 3120

aaatttctaa aagcaggtta tctggaaaac tggcagtatc acaaaactta cagcggaaca 3180

cctcaaggtg gaattctatc tcctcttttg gccaacatct atcttcatga attggataag 3240

tttgttttac aactcaaaat gaagtttgac cgagaaagtc cagaaagaat aacacctgaa 3300

tatcgggagc tccacaatga gataaaaaga atttctcacc gtctcaagaa gttggagggt 3360

gaagaaaaag ctaaagttct tttagaatat caagaaaaac gtaaaagatt acccacactc 3420

ccctgtacct cacagacaaa taaagtattg aaatacgtcc ggtatgcgga cgacttcatt 3480

atctctgtta aaggaagcaa agaggactgt caatggataa aagaacaatt aaaacttttt 3540

attcataaca agctaaaaat ggaattgagt gaagaaaaaa cactcatcac acatagcagt 3600

caacccgctc gttttctggg atatgatata cgagtaagga gatctggaac gataaaacga 3660

tctggtaaag tcaaaaagag aacactcaat gggagtgtag aactccttat tcctcttcaa 3720

gacaaaattc gtcaatttat ttttgacaag aaaatagcta tccaaaagaa agatagctca 3780

tggtttccag ttcacaggaa atatcttatt cgttcaacag acttagaaat catcacaatt 3840

tataattctg aactccgcgg gatttgtaat tactacggtc tagcaagtaa ttttaaccag 3900

ctcaattatt ttgcttatct tatggaatac agctgtctaa aaacgatagc ctccaaacat 3960

aagggaacac tttcaaaaac catttccatg tttaaagatg gaagtggttc gtgggggatc 4020

ccgtatgaga taaagcaagg taagcagcgc cgttattttg caaattttag tgaatgtaaa 4080

tccccttatc aatttacgga tgagataagt caagctcctg tattgtatgg ctatgcccgg 4140

aatactcttg aaaacaggtt aaaagctaaa tgttgtgaat tatgtgggac gtctgatgaa 4200

aatacttcct atgaaattca ccatgtcaat aaggtcaaaa atcttaaagg caaagaaaaa 4260

tgggaaatgg caatgatagc gaaacaacgt aaaactcttg ttgtatgctt tcattgtcat 4320

cgtcacgtga ttcataaaca caagtgaatg tcgagcaccc gttctcggag cactgtccga 4380

ccgctttggc cgccgcccag tcctgctcgc ttcgctactt ggagccacta tcgactacgc 4440

gatcatggcg accacacccg tcctgtggat cgccaagccg ccgatggtag tgtggggtct 4500

ccccatgcga gagtagggaa ctgccaggca tcaaataaaa cgaaaggctc agtcgaaaga 4560

ctgggccttt cgttttatct gttgtttgtc ggtgaacgct ctcctgagta ggacaaatcc 4620

gccgggagcg gatttgaacg ttgcgaagca acggcccgga gggtggcggg caggacgccc 4680

gccataaact gccaggcatc aaattaagca gaaggccatc ctgacggatg gcctttttgc 4740

gtttctacaa actcttcctg tcgtcatatc tacaagccat ccccccacag atacgggcgc 4800

gccgccatta tttttttgaa caattgacaa ttcatttctt attttttatt aagtgatagt 4860

caaaaggcat aacagtgctg aatagaaaga aatttacaga aaagaaaatt atagaattta 4920

gtatgattaa ttatactcat ttatgaatgt ttaattgaat acaaaaaaaa atacttgtta 4980

tgtattcaat tacgggttaa aatatagaca agttgaaaaa tttaataaaa aaataagtcc 5040

tcagctctta tatattaagc taccaactta gtatataagc caaaacttaa atgtgctacc 5100

aacacatcaa gccgttagag aactctatct atagcaatat ttcaaatgta ccgacataca 5160

agagaaacat taactatata tattcaattt atgagattat cttaacagat ataaatgtaa 5220

attgcaataa gtaagattta gaagtttata gcctttgtgt attggaagca gtacgcaaag 5280

gcttttttat ttgataaaaa ttagaagtat atttattttt tcataattaa tttatgaaaa 5340

tgaaaggggg tgagcaaagt gacagaggaa agcagtatct tatcaaataa caaggtatta 5400

gcaatatcat tattgacttt agcagtaaac attatgactt ttatagtgct tgtagctaag 5460

tagtacgaaa gggggagctt taaaaagctc cttggaatac atagaattca taaattaatt 5520

tatgaaaaga agggcgtata tgaaaacttg taaaaattgc aaagagttta ttaaagatac 5580

tgaaatatgc aaaatacatt cgttgatgat tcatgataaa acagtagcaa cctattgcag 5640

taaatacaat gagtcaagat gtttacataa agggaaagtc caatgtatta attgttcaaa 5700

gatgaaccga tatggatggt gtgccataaa aatgagatgt tttacagagg aagaacagaa 5760

aaaagaacgt acatgcatta aatattatgc aaggagcttt aaaaaagctc atgtaaagaa 5820

gagtaaaaag aaaaaataat ttatttatta atttaatatt gagagtgccg acacagtatg 5880

cactaaaaaa tatatctgtg gtgtagtgag ccgatacaaa aggatagtca ctcgcatttt 5940

cataatacat cttatgttat gattatgtgt cggtgggact tcacgacgaa aacccacaat 6000

aaaaaaagag ttcggggtag ggttaagcat agttgaggca actaaacaat caagctagga 6060

tatgcagtag cagaccgtaa ggtcgttgtt taggtgtgtt gtaatacata cgctattaag 6120

atgtaaaaat acggatacca atgaagggaa aagtataatt tttggatgta gtttgtttgt 6180

tcatctatgg gcaaactacg tccaaagccg tttccaaatc tgctaaaaag tatatccttt 6240

ctaaaatcaa agtcaagtat gaaatcataa ataaagttta attttgaagt tattatgata 6300

ttatgttttt ctattaaaat aaattaagta tatagaatag tttaataata gtatatactt 6360

aatgtgataa gtgtctgaca gtgtcacaga aaggatgatt gttatggatt ataagcggcc 6420

ggcccaatga ataggtttac acttacttta gttttatgga aatgaaagat catatcatat 6480

ataatctaga ataaaattaa ctaaaataat tattatctag ataaaaaatt tagaagccaa 6540

tgaaatctat aaataaacta aattaagttt atttaattaa caactatgga tataaaatag 6600

gtactaatca aaatagtgag gaggatatat ttgaatacat acgaacaaat taataaagtg 6660

aaaaaaatac ttcggaaaca tttaaaaaat aaccttattg gtacttacat gtttggatca 6720

ggagttgaga gtggactaaa accaaatagt gatcttgact ttttagtcgt cgtatctgaa 6780

ccattgacag atcaaagtaa agaaatactt atacaaaaaa ttagacctat ttcaaagaaa 6840

ataggagata aaagcaactt acgatatatt gaattaacaa ttattattca gcaagaaatg 6900

gtaccgtgga atcatcctcc caaacaagaa tttatttatg gagaatggtt acaagagctt 6960

tatgaacaag gatacattcc tcagaaggaa ttaaattcag atttaaccat aatgctttac 7020

caagcaaaac gaaaaaataa aagaatatac ggaaattatg acttagagga attactacct 7080

gatattccat tttctgatgt gagaagagcc attatggatt cgtcagagga attaatagat 7140

aattatcagg atgatgaaac caactctata ttaactttat gccgtatgat tttaactatg 7200

gacacgggta aaatcatacc aaaagatatt gcgggaaatg cagtggctga atcttctcca 7260

ttagaacata gggagagaat tttgttagca gttcgtagtt atcttggaga gaatattgaa 7320

tggactaatg aaaatgtaaa tttaactata aactatttaa ataacagatt aaaaaaatta 7380

taaaaaaatt gaaaaaatgg tggaaacact tttttcaatt tttttgtttt attatttaat 7440

atttgggaaa tattcattct aattggtaat cagattttag aagtttaaac tcctttttga 7500

taatctcatg accaaaatcc cttaacgtga gttttcgttc cactgagcgt cagaccccgt 7560

agaaaagatc aaaggatctt cttgagatcc tttttttctg cgcgtaatct gctgcttgca 7620

aacaaaaaaa ccaccgctac cagcggtggt ttgtttgccg gatcaagagc taccaactct 7680

ttttccgaag gtaactggct tcagcagagc gcagatacca aatactgttc ttctagtgta 7740

gccgtagtta ggccaccact tcaagaactc tgtagcaccg cctacatacc tcgctctgct 7800

aatcctgtta ccagtggctg ctgccagtgg cgataagtcg tgtcttaccg ggttggactc 7860

aagacgatag ttaccggata aggcgcagcg gtcgggctga acggggggtt cgtgcacaca 7920

gcccagcttg gagcgaacga cctacaccga actgagatac ctacagcgtg agctatgaga 7980

aagcgccacg cttcccgaag ggagaaaggc ggacaggtat ccggtaagcg gcagggtcgg 8040

aacaggagag cgcacgaggg agcttccagg gggaaacgcc tggtatcttt atagtcctgt 8100

cgggtttcgc cacctctgac ttgagcgtcg atttttgtga tgctcgtcag gggggcggag 8160

cctatggaaa aacgccagca acgcggcctt tttacggttc ctggcctttt gctggccttt 8220

tgctcacatg ttctttcctg cgttatcccc tgattctgtg gataaccgta ttaccgcctt 8280

tgagtgagct gataccgctc gccgcagccg aacgaccgag cgcagcgagt cagtgagcga 8340

ggaagcggaa gagcgcccaa tacgcagggc cccctgcttc ggggtcatta tagcgatttt 8400

ttcggtatat ccatcctttt tcgcacgata tacaggattt tgccaaaggg ttcgtgtaga 8460

ctttccttgg tgtatccaac ggcgtcagcc gggcaggata ggtgaagtag gcccacccgc 8520

gagcgggtgt tccttcttca ctgtccctta ttcgcacctg gcggtgctca acgggaatcc 8580

tgctctgcga ggctggccgg ctaccgccgg cgtaacagat gagggcaagc ggatggctga 8640

tgaaaccaag ccaaccagga agggcagccc acctatcaag gtgtactgcc ttccagacga 8700

acgaagagcg attgaggaaa aggcggcggc ggccggcatg agcctgtcgg cctacctgct 8760

ggccgtcggc cagggctaca aaatcacggg cgtcgtggac tatgagcacg tccgcgagct 8820

ggcccgcatc aatggcgacc tgggccgcct gggcggcctg ctgaaactct ggctcaccga 8880

cgacccgcgc acggcgcggt tcggtgatgc cacgatcctc gccctgctgg cgaagatcga 8940

agagaagcag gacgagcttg gcaaggtcat gatgggcgtg gtccgcccga gggcagagcc 9000

atgacttttt tagccgctaa aacggccggg gggtgcgcgt gattgccaag cacgtcccca 9060

tgcgctccat caagaagagc gacttcgcgg agctggtgaa gtacatcacc gacgagcaag 9120

gcaagacc                                                          9128

<210>  121
<211>  5002
<212>  DNA
<213>  Artificial Sequence

<220>
<223>  pEC751S

<400>  121
atcaaaaaaa tttccaataa tcccactcta agccacaaac acgccctata aaatcccgct      60

ttaatcccac tttgagacac atgtaatatt actttacgcc ctagtatagt gataattttt     120

tacattcaat gccacgcaaa aaaataaagg ggcactataa taaaagttcc ttcggaacta     180

actaaagtaa aaaattatct ttacaacctc cccaaaaaaa agaacaggta caaagtaccc     240

tataatacaa gcgtaaaaaa aatgagggta aaaataaaaa aataaaaaaa taaaaaaata     300

aaaaaataaa aaaataaaaa aataaaaaaa tataaaaata aaaaaatata aaaataaaaa     360

aatataaaaa taaaaaaata aaaaaatata aaaataaaaa aataaaaaaa tataaaaata     420

ttttttattt aaagtttgaa aaaaattttt ttatattata taatctttga agaaaagaat     480

ataaaaaatg agcctttata aaagcccatt ttttttcata tacgtaatat gacgttctaa     540

tgtttttatt ggtacttcta acattagagt aatttcttta tttttaaagc ctttttcttt     600

aagggctttt attttttttc ttaatacatt taattcctct ttttttgttg cttttccttt     660

agcttttaat tgctcttgat aatttttttt acctctaata ttttctcttc tcttatattc     720

ctttttagaa attattattg tcatatattt ttgttcttct tctgtaattt ctaataactc     780

tataagagtt tcattcttat acttatattg cttattttta tctaaataac atctttcagc     840

acttctagtt gctcttataa cttctctttc acttaaatgt tgtctaaaca tactattaag     900

ttctaaaaca tcatttaatg ccttctcaat gtcttctgta aagctacaaa gataatatct     960

atataaaaat aatataagct ctctgtgtcc ttttaaatca tattctctta gttcacaaag    1020

ttttattatg tcttgtattc ttccataata taaacttctt tctctataaa tataatttat    1080

tttgcttggt ctaccctttt tcctttcata tggttttaat tcaggtaaaa atccattttg    1140

tatttctctt aagtcataaa tatattcgta ctcatctaat atattgacta ctgtttttga    1200

tttagagttt atacttcctg gaactcttaa tattctcgtt gcatctaagg cttgtctatc    1260

tgctccaaag tattttaatt gattatataa atattcttga accgctttcc ataatggtaa    1320

tgctttacta ggtactgcat ttattatcca tattaaatac attcctcttc cactatctat    1380

tacatagttt ggtataggaa tactttgatt aaaataattc ttttctaagt ccattaatac    1440

ctggtcttta gttttgccag ttttataata atccaagtct ataaacagtg tatttaactc    1500

ttttatattt tctaatcgcc tacacggctt ataaaaggta tttagagtta tatagatatt 1560

ttcatcactc atatctaaat cttttaattc agcgtattta tagtgccatt ggctatatcc 1620

ttttttatct ataacgctcc tggttatcca ccctttactt ctactatgaa tattatctat 1680

atagttcttt ttattcagct ttaatgcgtt tctcacttat tcacctcccc ttctgtaaaa 1740

ctaagaaaat tatatcatat tttcaataat tattaactat tcttaaactc ttaataaaaa 1800

atagagtaag tccccaattg aaacttaatc tattttttat gttttaattt attattttta 1860

ttaaaatatt ttaaactaaa ttaaatgatt ctttttaatt ttttactatt tcattccata 1920

atatattact ataattattt acaaataata tttcttcatt tgtaatattt agatgattta 1980

ctaattttag tttttatata ttaaataatt aatgtataat ttatataaaa aatcaaagga 2040

gcttataaat tatgattatt tccaaagata ctaaagattt aatttttttc aattttaaca 2100

atactttttg taatattatg tttaaattta attgtatttt tttcatataa taaagccgtt 2160

gaagtaaacc aatccatttt ccttatgatg ttattattaa atttaagttt tataataata 2220

tctttattat atttattgtt tttaaaaaaa ctagtgaaat ttctagtgaa atttccggct 2280

ttattaaact tatttttagg aattttattt tcattttcat ctttacagga tttgattata 2340

tctttaaata tgttttatca aatattatct ttttctaaat ttatatatat ttttattata 2400

tttattatta tatatatttt atttttaagt ttctttctaa cagctattaa aaagaaactt 2460

aaaaataaaa acacgtactc taaaccaata aataaaacta tttttattat tgctgccttg 2520

attggaatag tttttagtaa aattaatttc aatattccac aatattatat tataagctag 2580

cacgcctcga gatctccatg gacgcgtgac gtcgactcta gaggatcccc gggtaccgag 2640

ctcgaattcg taatcatggt catagctgtt tcctgtgtga aattgttatc cgctcacaat 2700

tccacacaac atacgagccg gaagcataaa gtgtaaagcc tggggtgcct aatgagtgag 2760

ctaactcaca ttaattgcgt tgcgctcact gcccgctttc cagtcgggaa acctgtcgtg 2820

ccagctgcat taatgaatcg gccaacgcgc ggggagaggc ggtttgcgta ttgggcgctc 2880

ttccgcttcc tcgctcactg actcgctgcg ctcggtcgtt cggctgcggc gagcggtatc 2940

agctcactca aaggcggtaa tacggttatc cacagaatca ggggataacg caggaaagaa 3000

catgtgagca aaaggccagc aaaaggccag gaaccgtaaa aaggccgcgt tgctggcgtt 3060

tttccatagg ctccgccccc ctgacgagca tcacaaaaat cgacgctcaa gtcagaggtg 3120

gcgaaacccg acaggactat aaagatacca ggcgtttccc cctggaagct ccctcgtgcg 3180

ctctcctgtt ccgaccctgc cgcttaccgg atacctgtcc gcctttctcc cttcgggaag 3240

cgtggcgctt tctcatagct cacgctgtag gtatctcagt tcggtgtagg tcgttcgctc 3300

caagctgggc tgtgtgcacg aaccccccgt tcagcccgac cgctgcgcct tatccggtaa 3360

ctatcgtctt gagtccaacc cggtaagaca cgacttatcg ccactggcag cagccactgg 3420

taacaggatt agcagagcga ggtatgtagg cggtgctaca gagttcttga agtggtggcc 3480

taactacggc tacactagaa gaacagtatt tggtatctgc gctctgctga agccagttac 3540

cttcggaaaa agagttggta gctcttgatc cggcaaacaa accaccgctg gtagcggtgg 3600

tttttttgtt tgcaagcagc agattacgcg cagaaaaaaa ggatctcaag aagatccttt 3660

gatcttttct acggggtctg acgctcagtg gaacgaaaac tcacgttaag ggattttggt 3720

catgagatta tcaaaaagga tcttcaccta gatcctttta aattaaaaat gaagttttaa 3780

atcaatctaa agtatatatg agtaaacttg gtctgacagt taccaaagct agcttaatac 3840

tagtatatac ttaatgtgat aagtgtctga cagctgaccg gtctaaagag gtcccaatga 3900

ataggtttac acttacttta gttttatgga aatgaaagat catatcatat ataatctaga 3960

ataaaattaa ctaaaataat tattatctag ataaaaaatt tagaagccaa tgaaatctat 4020

aaataaacta aattaagttt atttaattaa caactatgga tataaaatag gtactaatca 4080

aaatagtgag gaggatatat ttgaatacat acgaacaaat taataaagtg aaaaaaatac 4140

ttcggaaaca tttaaaaaat aaccttattg gtacttacat gtttggatca ggagttgaga 4200

gtggactaaa accaaatagt gatcttgact ttttagtcgt cgtatctgaa ccattgacag 4260

atcaaagtaa agaaatactt atacaaaaaa ttagacctat ttcaaagaaa ataggagata 4320

aaagcaactt acgatatatt gaattaacaa ttattattca gcaagaaatg gtaccgtgga 4380

atcatcctcc caaacaagaa tttatttatg gagaatggtt acaagagctt tatgaacaag 4440

gatacattcc tcagaaggaa ttaaattcag atttaaccat aatgctttac caagcaaaac 4500

gaaaaaataa aagaatatac ggaaattatg acttagagga attactacct gatattccat 4560

tttctgatgt gagaagagcc attatggatt cgtcagagga attaatagat aattatcagg 4620

atgatgaaac caactctata ttaactttat gccgtatgat tttaactatg gacacgggta 4680

aaatcatacc aaaagatatt gcgggaaatg cagtggctga atcttctcca ttagaacata 4740

gggagagaat tttgttagca gttcgtagtt atcttggaga gaatattgaa tggactaatg 4800

aaaatgtaaa tttaactata aactatttaa ataacagatt aaaaaaatta taaaaaaatt 4860

gaaaaaatgg tggaaacact tttttcaatt tttttgtttt attatttaat atttgggaaa 4920

tattcattct aattggtaat cagattttag aagttgttaa cttcaggttt gtctgtaact 4980

aaaaactagt atttaaccta gg 5002

<210> 122
<211> 3907
<212> DNA
<213> Artificial Sequence

<220>
<223> pFW01

<400> 122
tcgagatctc catggacgcg tgacgtcgac tctagaggat ccccgggtac cgagctcgaa 60

ttcgtaatca tggtcatagc tgtttcctgt gtgaaattgt tatccgctca caattccaca 120

caacatacga gccggaagca taaagtgtaa agcctggggt gcctaatgag tgagctaact 180

cacattaatt gcgttgcgct cactgcccgc tttccagtcg ggaaacctgt cgtgccagct 240

gcattaatga atcggccaac gcgcggggag aggcggtttg cgtattgggc gctcttccgc 300

ttcctcgctc actgactcgc tgcgctcggt cgttcggctg cggcgagcgg tatcagctca 360

ctcaaaggcg gtaatacggt tatccacaga atcaggggat aacgcaggaa agaacatgtg 420

agcaaaaggc cagcaaaagg ccaggaaccg taaaaaggcc gcgttgctgg cgtttttcca 480

taggctccgc ccccctgacg agcatcacaa aaatcgacgc tcaagtcaga ggtggcgaaa 540

cccgacagga ctataaagat accaggcgtt tccccctgga agctccctcg tgcgctctcc 600

tgttccgacc ctgccgctta ccggatacct gtccgccttt ctcccttcgg gaagcgtggc 660

gctttctcat agctcacgct gtaggtatct cagttcggtg taggtcgttc gctccaagct 720

gggctgtgtg cacgaacccc ccgttcagcc cgaccgctgc gccttatccg gtaactatcg 780

tcttgagtcc aacccggtaa gacacgactt atcgccactg gcagcagcca ctggtaacag 840

gattagcaga gcgaggtatg taggcggtgc tacagagttc ttgaagtggt ggcctaacta 900

cggctacact agaaggacag tatttggtat ctgcgctctg ctgaagccag ttaccttcgg 960

aaaaagagtt ggtagctctt gatccggcaa acaaaccacc gctggtagcg gtggtttttt 1020

tgtttgcaag cagcagatta cgcgcagaaa aaaaggatct caagaagatc ctttgatctt 1080

ttctacgggg tctgacgctc agtggaacga aaactcacgt taagggattt tggtcatgag 1140

attatcaaaa aggatcttca cctagatcct tttaaattaa aaatgaagtt ttaaatcaat 1200

ctaaagtata tatgagtaaa cttggtctga cagttaccag gtccactgcc gggcctcttg 1260

cgggatcaaa agaaaaacga aatgatacac caatcagtgc aaaaaaagat ataatgggag 1320

ataagacggt tcgtgttcgt gctgacttgc accatatcat aaaaatcgaa acagcaaaga 1380

atggcggaaa cgtaaaagaa gttatggaaa taagacttag aagcaaactt aagagtgtgt 1440

tgatagtgca gtatcttaaa attttgtata ataggaattg aagttaaatt agatgctaaa 1500

aatttgtaat taagaaggag tgattacatg aacaaaaata taaaatattc tcaaaacttt 1560

ttaacgagtg aaaaagtact caaccaaata ataaaacaat tgaatttaaa agaaaccgat 1620

accgtttacg aaattggaac aggtaaaggg catttaacga cgaaactggc taaaataagt 1680

aaacaggtaa cgtctattga attagacagt catctattca acttatcgtc agaaaaatta 1740

aaactgaata ctcgtgtcac tttaattcac caagatattc tacagtttca attccctaac 1800

aaacagaggt ataaaattgt tgggagtatt ccttaccatt taagcacaca aattattaaa 1860

aaagtggttt ttgaaagcca tgcgtctgac atctatctga ttgttgaaga aggattctac 1920

aagcgtacct tggatattca ccgaacacta gggttgctct tgcacactca agtctcgatt 1980

cagcaattgc ttaagctgcc agcggaatgc tttcatccta aaccaaaagt aaacagtgtc 2040

ttaataaaac ttacccgcca taccacagat gttccagata aatattggaa gctatatacg 2100

tactttgttt caaaatgggt caatcgagaa tatcgtcaac tgtttactaa aaatcagttt 2160

catcaagcaa tgaaacacgc caaagtaaac aatttaagta ccgttactta tgagcaagta 2220

ttgtctattt ttaatagtta tctattattt aacgggagga aataattcta tgagtcccta 2280

ggcaggcctc cgccattatt tttttgaaca attgacaatt catttcttat tttttattaa 2340

gtgatagtca aaaggcataa cagtgctgaa tagaaagaaa tttacagaaa agaaaattat 2400

agaatttagt atgattaatt atactcattt atgaatgttt aattgaatac aaaaaaaaat 2460

acttgttatg tattcaatta cgggttaaaa tatagacaag ttgaaaaatt taataaaaaa 2520

ataagtcctc agctcttata tattaagcta ccaacttagt atataagcca aaacttaaat 2580

gtgctaccaa cacatcaagc cgttagagaa ctctatctat agcaatattt caaatgtacc 2640

gacatacaag agaaacatta actatatata ttcaatttat gagattatct taacagatat 2700

aaatgtaaat tgcaataagt aagatttaga agtttatagc ctttgtgtat tggaagcagt 2760

acgcaaaggc ttttttattt gataaaaatt agaagtatat ttattttttc ataattaatt 2820

tatgaaaatg aaagggggtg agcaaagtga cagaggaaag cagtatctta tcaaataaca 2880

aggtattagc aatatcatta ttgactttag cagtaaacat tatgactttt atagtgcttg 2940

tagctaagta gtacgaaagg gggagcttta aaaagctcct tggaatacat agaattcata 3000

aattaattta tgaaaagaag ggcgtatatg aaaacttgta aaaattgcaa agagtttatt 3060

aaagatactg aaatatgcaa aatacattcg ttgatgattc atgataaaac agtagcaacc 3120

tattgcagta aatacaatga gtcaagatgt ttacataaag ggaaagtcca atgtattaat 3180

tgttcaaaga tgaaccgata tggatggtgt gccataaaaa tgagatgttt tacagaggaa 3240

gaacagaaaa aagaacgtac atgcattaaa tattatgcaa ggagctttaa aaaagctcat 3300

gtaaagaaga gtaaaaagaa aaaataattt atttattaat ttaatattga gagtgccgac 3360

acagtatgca ctaaaaaata tatctgtggt gtagtgagcc gatacaaaag gatagtcact 3420

cgcattttca taatacatct tatgttatga ttatgtgtcg gtgggacttc acgacgaaaa 3480

cccacaataa aaaaagagtt cggggtaggg ttaagcatag ttgaggcaac taaacaatca 3540

agctaggata tgcagtagca gaccgtaagg tcgttgttta ggtgtgttgt aatacatacg 3600

ctattaagat gtaaaaatac ggataccaat gaagggaaaa gtataatttt tggatgtagt 3660

ttgtttgttc atctatgggc aaactacgtc caaagccgtt tccaaatctg ctaaaaagta 3720

tatcctttct aaaatcaaag tcaagtatga aatcataaat aaagtttaat tttgaagtta 3780

ttatgatatt atgtttttct attaaaataa attaagtata tagaatagtt taataatagt 3840

atatacttaa tgtgataagt gtctgacagt gtcacagaaa ggatgattgt tatggattat 3900

aagcggc 3907

<210> 123
<211> 6525
<212> DNA
<213> Artificial Sequence

<220>
<223> pNF3S

<400> 123
gcgcccaata cgcaaaccgc ctctccccgc gcgttggccg attcattaat gcagctggca 60

cgacaggttt cccgactgga aagcgggcag tgagcgcaac gcaattaatg tgagttagct 120

cactcattag gcaccccagg ctttacactt tatgcttccg gctcgtatgt tgtgtggaat 180

tgtgagcgga taacaatttc acacaggaaa cagctatgac catgattacg ccaagcttgc 240

atgcctgcag gtcgaccacg ataaaacaag gttttaagga taagaaaagt catgagattt 300

atagtaaatc ttgtgacttt ttttattgaa tagtagagag agttcggaag tataacacgc 360

tatattcttg atatttttag aatagcaagc attggatttg tcctgacact ttcccaaaaa 420

ttaaggagtt attccttaaa ccaaaaagat taatgtggga acaaatttag tgtatccatt 480

tttgaagggc gcacttatac accaccaaaa tggtgtgtgc gaaatcttta aaaaagattt 540

atcaaaaagc ttttttaaag ctgggacatt tagaaaatca ataatgtttt ttgcccaata 600

cgctagtctt aaaatctgca aggttgataa ctatttagtc ccaggtatta gaatggggca 660

tatatataca aagtatatat atgcgtaaat atatgtggga ctgtgggaac aaaattgcgt 720

gctaaaattg tattgaaaag gtaatgaaaa ggtcatgctt tggtattgct aacgtataga 780

aaaggtaatg aaaagctcat ggttctataa aaaagatgta cccacgaaaa taataggctt 840

tgcctatttc cccatgtaat atgggggcag ttttctctta tgctctttct taacatattg 900

aataaataca aaatgcagct ttgtgggaat aaaaatattt ttgtttttat tcttatagtt 960

agacaaaatt ttaatctttt ttgtgctata acaagattaa aatttgtggg aacattaaga 1020

aatattgttg tcacaaataa aaaggagagt gggaacaatt gctataaaaa acgcagaaat 1080

taagattaga gttacaaaag agcaaaaaga attatttaag aaaattgcaa aagctgaaaa 1140

tatgagtatg agtgaattta ttattgtgac cacagaatat ttagccagaa aaaaagatga 1200

aaatatgaaa tcaaaagaca tgatcgagag aagagctgcg aagactgaag aaaaaattat 1260

gaagctaaaa aagaaactaa ataaaaacag gtaatataga ttacagtttt aagcttgttt 1320

tccctataga ctagagtaaa tatataaata tacctgtcaa gggcttataa gcccctttag 1380

ggggtgcgta gcacccttga caggtatatt tatatatttt agggtgccat taagggaaac 1440

aagctttaaa atgcctttaa aggcatttta aaataaataa aaaaaagatg gtttttacca 1500

tcttttttaa ctcccgaaag ggagttcttt cttttcttga tactatacgt aactatttcg 1560

atttgccctg aacctaatca aagctagata aattcagtat tagggcataa aaaaacttgc 1620

tttttcgggt ggaaatctgt ataatttaaa ttgcttagat aaaaattacc aattccatac 1680

gaaaggagca agttttacat aaggttaaag ccttatgtga attctcattt aattacatga 1740

ataataataa cacagaaagt gaagaattaa aagagcaaag tcaactattg cttgacaaat 1800

gcacaaaaaa gaaaaagaaa aatcctaaat ttagtagtta tatagaacca ttagtaagca 1860

agaaattatc tgaaagaata aaggaatgtg gtgacttttt gcagatgtta tctgatttaa 1920

accttgaaaa ttcgaaactg catagagcaa gtttttgtgg taacagattt tgtcctatgt 1980

gtagctggcg tattgcttgt aaggatagtt tggaaatatc tattctcatg gagcatttac 2040

gcaaagagga aagcaaagaa tttatctttt tgaccttaac aactccaaat gtgaaaggtg 2100

cggaccttga taattccata aaagcataca ataaagcatt taaaaagtta atggaacgca 2160

aagaggtcaa gagcatagta aaaggctaca taagaaagct agaagtaacc tataatttgg 2220

acaagagttc caaatcatat aatacttatc acccacattt ccatgtggta ctagcagtca 2280

atagaagtta ctttaaaaag caaaatctat atataaacca tcatagatgg cttagtttgt 2340

ggcaagagtc aactggtgat tattcgataa ctcaagttga tgtaagaaag gctaaaatta 2400

acgattataa agaggtttat gagcttgcta agtattcggc taaggattcc gactatttaa 2460

tcaatagaga agtgtttacg gtattctaca aatctttaaa gggtaaacag gtacttgtat 2520

ttagtggatt atttaaagac gctcataaaa tgtataagaa tggagagcta gatctgtata 2580

agaagttgga tactatcgaa tatgcttata tggtaagtta taactggctt aaaaagaagt 2640

atgatacttc aaatattaga gaattaactg aggaagaaaa gcagaaattc aataaaaatt 2700

taatcgaaga tgtggatatt gagtaggtgg gattatatct cacctttttt attgtctttt 2760

catgttgaaa ttttgacgct taatgcatga agtattgaca agtttaaaaa ttacggtttt 2820

taatccttag ttgattagca ggattatggc cggaatgctc cgtccagtcc tgttaaggaa 2880

ttaaaattcc ctaaaaccct tggctatgat ttatagcgag aatcgtcaat taaaaattta 2940

ataggtgcta tgaaagtcga ttaataatta attttaaaat gcaatatgaa acataattac 3000

aagaatttga cttttaatac aagaattgat atcatagtta cattaatacg gatcccaatg 3060

aataggttta cacttacttt agttttatgg aaatgaaaga tcatatcata tataatctag 3120

aataaaatta actaaaataa ttattatcta gataaaaaat ttagaagcca atgaaatcta 3180

taaataaact aaattaagtt tatttaatta acaactatgg atataaaata ggtactaatc 3240

aaaatagtga ggaggatata tttgaataca tacgaacaaa ttaataaagt gaaaaaaata 3300

cttcggaaac atttaaaaaa taaccttatt ggtacttaca tgtttggatc aggagttgag 3360

agtggactaa aaccaaatag tgatcttgac tttttagtcg tcgtatctga accattgaca 3420

gatcaaagta aagaaatact tatacaaaaa attagaccta tttcaaagaa aataggagat 3480

aaaagcaact tacgatatat tgaattaaca attattattc agcaagaaat ggtaccgtgg 3540

aatcatcctc ccaaacaaga atttatttat ggagaatggt tacaagagct ttatgaacaa 3600

ggatacattc ctcagaagga attaaattca gatttaacca taatgcttta ccaagcaaaa 3660

cgaaaaaata aaagaatata cggaaattat gacttagagg aattactacc tgatattcca 3720

ttttctgatg tgagaagagc cattatggat tcgtcagagg aattaataga taattatcag 3780

gatgatgaaa ccaactctat attaacttta tgccgtatga ttttaactat ggacacgggt 3840

aaaatcatac caaaagatat tgcgggaaat gcagtggctg aatcttctcc attagaacat 3900

agggagagaa ttttgttagc agttcgtagt tatcttggag agaatattga atggactaat 3960

gaaaatgtaa atttaactat aaactattta aataacagat taaaaaaatt ataaaaaaat 4020

tgaaaaaatg gtggaaacac ttttttcaat ttttttgttt tattatttaa tatttgggaa 4080

atattcattc taattggtaa tcagatttta gaagttgagc tcgaattcac tggccgtcgt 4140

tttacaacgt cgtgactggg aaaaccctgg cgttacccaa cttaatcgcc ttgcagcaca 4200

tccccctttc gccagctggc gtaatagcga agaggcccgc accgatcgcc cttcccaaca 4260

gttgcgcagc ctgaatggcg aatggcgcct gatgcggtat tttctcctta cgcatctgtg 4320

cggtatttca caccgcatat ggtgcactct cagtacaatc tgctctgatg ccgcatagtt 4380

aagccagccc cgacacccgc caacacccgc tgacgcgccc tgacgggctt gtctgctccc 4440

ggcatccgct tacagacaag ctgtgaccgt ctccgggagc tgcatgtgtc agaggttttc 4500

accgtcatca ccgaaacgcg cgagacgaaa gggcctcgtg atacgcctat ttttataggt 4560

taatgtcatg ataataatgg tttcttagac gtcaggtggc acttttcggg gaaatgtgcg 4620

cggaacccct atttgtttat ttttctaaat acattcaaat atgtatccgc tcatgagaca 4680

ataaccctga taaatgcttc aataatattg aaaaaggaag agtatgagta ttcaacattt 4740

ccgtgtcgcc cttattccct tttttgcggc attttgcctt cctgtttttg ctcacccaga 4800

aacgctggtg aaagtaaaag atgctgaaga tcagttgggt gcacgagtgg gttacatcga 4860

actggatctc aacagcggta agatccttga gagttttcgc cccgaagaac gttttccaat 4920

gatgagcact tttaaagttc tgctatgtgg cgcggtatta tcccgtattg acgccgggca 4980

agagcaactc ggtcgccgca tacactattc tcagaatgac ttggttgagt actcaccagt 5040

cacagaaaag catcttacgg atggcatgac agtaagagaa ttatgcagtg ctgccataac 5100

catgagtgat aacactgcgg ccaacttact tctgacaacg atcggaggac cgaaggagct 5160

aaccgctttt ttgcacaaca tgggggatca tgtaactcgc cttgatcgtt gggaaccgga 5220

gctgaatgaa gccataccaa acgacgagcg tgacaccacg atgcctgtag caatggcaac 5280

aacgttgcgc aaactattaa ctggcgaact acttactcta gcttcccggc aacaattaat 5340

agactggatg gaggcggata aagttgcagg accacttctg cgctcggccc ttccggctgg 5400

ctggtttatt gctgataaat ctggagccgg tgagcgtggg tctcgcggta tcattgcagc 5460

actggggcca gatggtaagc cctcccgtat cgtagttatc tacacgacgg ggagtcaggc 5520

aactatggat gaacgaaata gacagatcgc tgagataggt gcctcactga ttaagcattg 5580

gtaactgtca gaccaagttt actcatatat actttagatt gatttaaaac ttcattttta 5640

atttaaaagg atctaggtga agatcctttt tgataatctc atgaccaaaa tcccttaacg 5700

tgagttttcg ttccactgag cgtcagaccc cgtagaaaag atcaaaggat cttcttgaga 5760

tccttttttt ctgcgcgtaa tctgctgctt gcaaacaaaa aaaccaccgc taccagcggt 5820

ggtttgtttg ccggatcaag agctaccaac tctttttccg aaggtaactg gcttcagcag 5880

agcgcagata ccaaatactg ttcttctagt gtagccgtag ttaggccacc acttcaagaa 5940

ctctgtagca ccgcctacat acctcgctct gctaatcctg ttaccagtgg ctgctgccag 6000

tggcgataag tcgtgtctta ccgggttgga ctcaagacga tagttaccgg ataaggcgca 6060

gcggtcgggc tgaacggggg gttcgtgcac acagcccagc ttggagcgaa cgacctacac 6120

cgaactgaga tacctacagc gtgagctatg agaaagcgcc acgcttcccg aagggagaaa 6180

ggcggacagg tatccggtaa gcggcagggt cggaacagga gagcgcacga gggagcttcc 6240

agggggaaac gcctggtatc tttatagtcc tgtcgggttt cgccacctct gacttgagcg 6300

tcgatttttg tgatgctcgt caggggggcg gagcctatgg aaaaacgcca gcaacgcggc 6360

ctttttacgg ttcctggcct tttgctggcc ttttgctcac atgttctttc ctgcgttatc 6420

ccctgattct gtggataacc gtattaccgc ctttgagtga gctgataccg ctcgccgcag 6480

ccgaacgacc gagcgcagcg agtcagtgag cgaggaagcg gaaga 6525

<210> 124
<211> 6554
<212> DNA
<213> Artificial Sequence

<220>
<223> pNF3E

<400> 124
gcgcccaata cgcaaaccgc ctctccccgc gcgttggccg attcattaat gcagctggca 60

cgacaggttt cccgactgga aagcgggcag tgagcgcaac gcaattaatg tgagttagct 120

cactcattag gcaccccagg ctttacactt tatgcttccg gctcgtatgt tgtgtggaat 180

tgtgagcgga taacaatttc acacaggaaa cagctatgac catgattacg ccaagcttgc 240

atgcctgcag gtcgaccacg ataaaacaag gttttaagga taagaaaagt catgagattt 300

atagtaaatc ttgtgacttt ttttattgaa tagtagagag agttcggaag tataacacgc 360

tatattcttg atatttttag aatagcaagc attggatttg tcctgacact ttcccaaaaa 420

ttaaggagtt attccttaaa ccaaaaagat taatgtggga acaaatttag tgtatccatt 480

tttgaagggc gcacttatac accaccaaaa tggtgtgtgc gaaatcttta aaaaagattt 540

atcaaaaagc ttttttaaag ctgggacatt tagaaaatca ataatgtttt ttgcccaata 600

cgctagtctt aaaatctgca aggttgataa ctatttagtc ccaggtatta gaatggggca 660

tatatataca aagtatatat atgcgtaaat atatgtggga ctgtgggaac aaaattgcgt 720

gctaaaattg tattgaaaag gtaatgaaaa ggtcatgctt tggtattgct aacgtataga 780

aaaggtaatg aaaagctcat ggttctataa aaaagatgta cccacgaaaa taataggctt 840

tgcctatttc cccatgtaat atgggggcag ttttctctta tgctctttct taacatattg 900

aataaataca aaatgcagct ttgtgggaat aaaaatattt ttgtttttat tcttatagtt 960

agacaaaatt ttaatctttt ttgtgctata acaagattaa aatttgtggg aacattaaga 1020

aatattgttg tcacaaataa aaaggagagt gggaacaatt gctataaaaa acgcagaaat 1080

taagattaga gttacaaaag agcaaaaaga attatttaag aaaattgcaa aagctgaaaa 1140

tatgagtatg agtgaattta ttattgtgac cacagaatat ttagccagaa aaaaagatga 1200

aaatatgaaa tcaaaagaca tgatcgagag aagagctgcg aagactgaag aaaaaattat 1260

gaagctaaaa aagaaactaa ataaaaacag gtaatataga ttacagtttt aagcttgttt 1320

tccctataga ctagagtaaa tatataaata tacctgtcaa gggcttataa gcccctttag 1380

ggggtgcgta gcacccttga caggtatatt tatatatttt agggtgccat taagggaaac 1440

aagctttaaa atgcctttaa aggcatttta aaataaataa aaaaaagatg gtttttacca 1500

tcttttttaa ctcccgaaag ggagttcttt cttttcttga tactatacgt aactatttcg 1560

atttgccctg aacctaatca aagctagata aattcagtat tagggcataa aaaaacttgc 1620

tttttcgggt ggaaatctgt ataatttaaa ttgcttagat aaaaattacc aattccatac 1680

gaaaggagca agttttacat aaggttaaag ccttatgtga attctcattt aattacatga 1740

ataataataa cacagaaagt gaagaattaa aagagcaaag tcaactattg cttgacaaat 1800

gcacaaaaaa gaaaaagaaa aatcctaaat ttagtagtta tatagaacca ttagtaagca 1860

agaaattatc tgaaagaata aaggaatgtg gtgacttttt gcagatgtta tctgatttaa 1920

accttgaaaa ttcgaaactg catagagcaa gtttttgtgg taacagattt tgtcctatgt 1980

gtagctggcg tattgcttgt aaggatagtt tggaaatatc tattctcatg gagcatttac 2040

gcaaagagga aagcaaagaa tttatctttt tgaccttaac aactccaaat gtgaaaggtg 2100

cggaccttga taattccata aaagcataca ataaagcatt taaaaagtta atggaacgca 2160

aagaggtcaa gagcatagta aaaggctaca taagaaagct agaagtaacc tataatttgg 2220

acaagagttc caaatcatat aatacttatc acccacattt ccatgtggta ctagcagtca 2280

atagaagtta ctttaaaaag caaaatctat atataaacca tcatagatgg cttagtttgt 2340

ggcaagagtc aactggtgat tattcgataa ctcaagttga tgtaagaaag gctaaaatta 2400

acgattataa agaggtttat gagcttgcta agtattcggc taaggattcc gactatttaa 2460

tcaatagaga agtgtttacg gtattctaca aatctttaaa gggtaaacag gtacttgtat 2520

ttagtggatt atttaaagac gctcataaaa tgtataagaa tggagagcta gatctgtata 2580

agaagttgga tactatcgaa tatgcttata tggtaagtta taactggctt aaaaagaagt 2640

atgatacttc aaatattaga gaattaactg aggaagaaaa gcagaaattc aataaaaatt 2700

taatcgaaga tgtggatatt gagtaggtgg gattatatct cacctttttt attgtctttt 2760

catgttgaaa ttttgacgct taatgcatga agtattgaca agtttaaaaa ttacggtttt 2820

taatccttag ttgattagca ggattatggc cggaatgctc cgtccagtcc tgttaaggaa 2880

ttaaaattcc ctaaaaccct tggctatgat ttatagcgag aatcgtcaat taaaaattta 2940

ataggtgcta tgaaagtcga ttaataatta attttaaaat gcaatatgaa acataattac 3000

aagaatttga cttttaatac aagaattgat atcatagtta cattaatacg gatccgtctg 3060

acagttacca ggtccactgc cgggcctctt gcgggatcaa aagaaaaacg aaatgataca 3120

ccaatcagtg caaaaaaaga tataatggga gataagacgg ttcgtgttcg tgctgacttg 3180

caccatatca taaaaatcga aacagcaaag aatggcggaa acgtaaaaga agttatggaa 3240

ataagactta gaagcaaact taagagtgtg ttgatagtgc agtatcttaa aattttgtat 3300

aataggaatt gaagttaaat tagatgctaa aaatttgtaa ttaagaagga gtgattacat 3360

gaacaaaaat ataaaatatt ctcaaaactt tttaacgagt gaaaaagtac tcaaccaaat 3420

aataaaacaa ttgaatttaa aagaaaccga taccgtttac gaaattggaa caggtaaagg 3480

gcatttaacg acgaaactgg ctaaaataag taaacaggta acgtctattg aattagacag 3540

tcatctattc aacttatcgt cagaaaaatt aaaactgaat actcgtgtca ctttaattca 3600

ccaagatatt ctacagtttc aattccctaa caaacagagg tataaaattg ttgggagtat 3660

tccttaccat ttaagcacac aaattattaa aaaagtggtt tttgaaagcc atgcgtctga 3720

catctatctg attgttgaag aaggattcta caagcgtacc ttggatattc accgaacact 3780

agggttgctc ttgcacactc aagtctcgat tcagcaattg cttaagctgc cagcggaatg 3840

ctttcatcct aaaccaaaag taaacagtgt cttaataaaa cttacccgcc ataccacaga 3900

tgttccagat aaatattgga agctatatac gtactttgtt tcaaaatggg tcaatcgaga 3960

atatcgtcaa ctgtttacta aaaatcagtt tcatcaagca atgaaacacg ccaaagtaaa 4020

caatttaagt accgttactt atgagcaagt attgtctatt tttaatagtt atctattatt 4080

taacgggagg aaataattct atgagtccct aggcaggcct ccgccattat ttttttgaac 4140

aattggagct cgaattcact ggccgtcgtt ttacaacgtc gtgactggga aaaccctggc 4200

gttacccaac ttaatcgcct tgcagcacat ccccctttcg ccagctggcg taatagcgaa 4260

gaggcccgca ccgatcgccc ttcccaacag ttgcgcagcc tgaatggcga atggcgcctg 4320

atgcggtatt ttctccttac gcatctgtgc ggtatttcac accgcatatg gtgcactctc 4380

agtacaatct gctctgatgc cgcatagtta agccagcccc gacacccgcc aacacccgct 4440

gacgcgccct gacgggcttg tctgctcccg gcatccgctt acagacaagc tgtgaccgtc 4500

tccgggagct gcatgtgtca gaggttttca ccgtcatcac cgaaacgcgc gagacgaaag 4560

ggcctcgtga tacgcctatt tttataggtt aatgtcatga taataatggt ttcttagacg 4620

tcaggtggca cttttcgggg aaatgtgcgc ggaaccccta tttgtttatt tttctaaata 4680

cattcaaata tgtatccgct catgagacaa taaccctgat aaatgcttca ataatattga 4740

aaaaggaaga gtatgagtat tcaacatttc cgtgtcgccc ttattccctt ttttgcggca 4800

ttttgccttc ctgtttttgc tcacccagaa acgctggtga aagtaaaaga tgctgaagat 4860

cagttgggtg cacgagtggg ttacatcgaa ctggatctca acagcggtaa gatccttgag 4920

agttttcgcc ccgaagaacg ttttccaatg atgagcactt ttaaagttct gctatgtggc 4980

gcggtattat cccgtattga cgccgggcaa gagcaactcg gtcgccgcat acactattct 5040

cagaatgact tggttgagta ctcaccagtc acagaaaagc atcttacgga tggcatgaca 5100

gtaagagaat tatgcagtgc tgccataacc atgagtgata acactgcggc caacttactt 5160

ctgacaacga tcggaggacc gaaggagcta accgcttttt tgcacaacat gggggatcat 5220

gtaactcgcc ttgatcgttg ggaaccggag ctgaatgaag ccataccaaa cgacgagcgt 5280

gacaccacga tgcctgtagc aatggcaaca acgttgcgca aactattaac tggcgaacta 5340

cttactctag cttcccggca acaattaata gactggatgg aggcggataa agttgcagga 5400

ccacttctgc gctcggccct tccggctggc tggtttattg ctgataaatc tggagccggt 5460

gagcgtgggt ctcgcggtat cattgcagca ctggggccag atggtaagcc ctcccgtatc 5520

gtagttatct acacgacggg gagtcaggca actatggatg aacgaaatag acagatcgct 5580

gagataggtg cctcactgat taagcattgg taactgtcag accaagttta ctcatatata 5640

ctttagattg atttaaaact tcatttttaa tttaaaagga tctaggtgaa gatccttttt 5700

gataatctca tgaccaaaat cccttaacgt gagttttcgt tccactgagc gtcagacccc 5760

gtagaaaaga tcaaaggatc ttcttgagat cctttttttc tgcgcgtaat ctgctgcttg 5820

caaacaaaaa aaccaccgct accagcggtg gtttgtttgc cggatcaaga gctaccaact 5880

ctttttccga aggtaactgg cttcagcaga gcgcagatac caaatactgt tcttctagtg 5940

tagccgtagt taggccacca cttcaagaac tctgtagcac cgcctacata cctcgctctg 6000

ctaatcctgt taccagtggc tgctgccagt ggcgataagt cgtgtcttac cgggttggac 6060

tcaagacgat agttaccgga taaggcgcag cggtcgggct gaacgggggg ttcgtgcaca 6120

cagcccagct tggagcgaac gacctacacc gaactgagat acctacagcg tgagctatga 6180

gaaagcgcca cgcttcccga agggagaaag gcggacaggt atccggtaag cggcagggtc 6240

ggaacaggag agcgcacgag ggagcttcca gggggaaacg cctggtatct ttatagtcct 6300

gtcgggtttc gccacctctg acttgagcgt cgatttttgt gatgctcgtc aggggggcgg 6360

agcctatgga aaaacgccag caacgcggcc tttttacggt tcctggcctt ttgctggcct 6420

tttgctcaca tgttctttcc tgcgttatcc cctgattctg tggataaccg tattaccgcc 6480

tttgagtgag ctgataccgc tcgccgcagc cgaacgaccg agcgcagcga gtcagtgagc 6540

gaggaagcgg aaga 6554

<210> 125
<211> 6271
<212> DNA
<213> Artificial Sequence

<220>
<223> pNF3C

<400> 125
gcgcccaata cgcaaaccgc ctctccccgc gcgttggccg attcattaat gcagctggca 60

cgacaggttt cccgactgga aagcgggcag tgagcgcaac gcaattaatg tgagttagct 120

cactcattag gcaccccagg ctttacactt tatgcttccg gctcgtatgt tgtgtggaat 180

tgtgagcgga taacaatttc acacaggaaa cagctatgac catgattacg ccaagcttgc 240

atgcctgcag gtcgaccacg ataaaacaag gttttaagga taagaaaagt catgagattt 300

atagtaaatc ttgtgacttt ttttattgaa tagtagagag agttcggaag tataacacgc 360

tatattcttg atatttttag aatagcaagc attggatttg tcctgacact ttcccaaaaa 420

ttaaggagtt attccttaaa ccaaaaagat taatgtggga acaaatttag tgtatccatt 480

tttgaagggc gcacttatac accaccaaaa tggtgtgtgc gaaatcttta aaaaagattt 540

atcaaaaagc ttttttaaag ctgggacatt tagaaaatca ataatgtttt ttgcccaata 600

cgctagtctt aaaatctgca aggttgataa ctatttagtc ccaggtatta gaatggggca 660

tatatataca aagtatatat atgcgtaaat atatgtggga ctgtgggaac aaaattgcgt 720

gctaaaattg tattgaaaag gtaatgaaaa ggtcatgctt tggtattgct aacgtataga 780

aaaggtaatg aaaagctcat ggttctataa aaaagatgta cccacgaaaa taataggctt 840

tgcctatttc cccatgtaat atgggggcag ttttctctta tgctctttct taacatattg 900

aataaataca aaatgcagct ttgtgggaat aaaaatattt ttgtttttat tcttatagtt 960

agacaaaatt ttaatctttt ttgtgctata acaagattaa aatttgtggg aacattaaga 1020

aatattgttg tcacaaataa aaaggagagt gggaacaatt gctataaaaa acgcagaaat 1080

taagattaga gttacaaaag agcaaaaaga attatttaag aaaattgcaa aagctgaaaa 1140

tatgagtatg agtgaattta ttattgtgac cacagaatat ttagccagaa aaaaagatga 1200

aaatatgaaa tcaaaagaca tgatcgagag aagagctgcg aagactgaag aaaaaattat 1260

gaagctaaaa aagaaactaa ataaaaacag gtaatataga ttacagtttt aagcttgttt 1320

tccctataga ctagagtaaa tatataaata tacctgtcaa gggcttataa gcccctttag 1380

ggggtgcgta gcacccttga caggtatatt tatatatttt agggtgccat taagggaaac 1440

aagctttaaa atgcctttaa aggcatttta aaataaataa aaaaaagatg gtttttacca 1500

tcttttttaa ctcccgaaag ggagttcttt cttttcttga tactatacgt aactatttcg 1560

atttgccctg aacctaatca aagctagata aattcagtat tagggcataa aaaaacttgc 1620

tttttcgggt ggaaatctgt ataatttaaa ttgcttagat aaaaattacc aattccatac 1680

gaaaggagca agttttacat aaggttaaag ccttatgtga attctcattt aattacatga 1740

ataataataa cacagaaagt gaagaattaa aagagcaaag tcaactattg cttgacaaat 1800

gcacaaaaaa gaaaaagaaa aatcctaaat ttagtagtta tatagaacca ttagtaagca 1860

agaaattatc tgaaagaata aaggaatgtg gtgacttttt gcagatgtta tctgatttaa 1920

accttgaaaa ttcgaaactg catagagcaa gtttttgtgg taacagattt tgtcctatgt 1980

gtagctggcg tattgcttgt aaggatagtt tggaaatatc tattctcatg gagcatttac 2040

gcaaagagga aagcaaagaa tttatctttt tgaccttaac aactccaaat gtgaaaggtg 2100

cggaccttga taattccata aaagcataca ataaagcatt taaaaagtta atggaacgca 2160

aagaggtcaa gagcatagta aaaggctaca taagaaagct agaagtaacc tataatttgg 2220

acaagagttc caaatcatat aatacttatc acccacattt ccatgtggta ctagcagtca 2280

atagaagtta ctttaaaaag caaaatctat atataaacca tcatagatgg cttagtttgt 2340

ggcaagagtc aactggtgat tattcgataa ctcaagttga tgtaagaaag gctaaaatta 2400

acgattataa agaggtttat gagcttgcta agtattcggc taaggattcc gactatttaa 2460

tcaatagaga agtgtttacg gtattctaca aatctttaaa gggtaaacag gtacttgtat 2520

ttagtggatt atttaaagac gctcataaaa tgtataagaa tggagagcta gatctgtata 2580

agaagttgga tactatcgaa tatgcttata tggtaagtta taactggctt aaaaagaagt 2640

atgatacttc aaatattaga gaattaactg aggaagaaaa gcagaaattc aataaaaatt 2700

taatcgaaga tgtggatatt gagtaggtgg gattatatct cacctttttt attgtctttt 2760

catgttgaaa ttttgacgct taatgcatga agtattgaca agtttaaaaa ttacggtttt 2820

taatccttag ttgattagca ggattatggc cggaatgctc cgtccagtcc tgttaaggaa 2880

ttaaaattcc ctaaaaccct tggctatgat ttatagcgag aatcgtcaat taaaaattta 2940

ataggtgcta tgaaagtcga ttaataatta attttaaaat gcaatatgaa acataattac 3000

aagaatttga cttttaatac aagaattgat atcatagtta cattaatacg gatcccggca 3060

gtttttcttt ttcggcaagt gttcaagaag ttattaagtc gggagtgcag tcgaagtggg 3120

caagttgaaa aattcacaaa aatgtggtat aatatctttg ttcattagag cgataaactt 3180

gaatttgaga gggaacttag atggtatttg aaaaaattga taaaaatagt tggaacagaa 3240

aagagtattt tgaccactac tttgcaagtg taccttgtac ctacagcatg accgttaaag 3300

tggatatcac acaaataaag gaaaagggaa tgaaactata tcctgcaatg ctttattata 3360

ttgcaatgat tgtaaaccgc cattcagagt ttaggacggc aatcaatcaa gatggtgaat 3420

tggggatata tgatgagatg ataccaagct atacaatatt tcacaatgat actgaaacat 3480

tttccagcct ttggactgag tgtaagtctg actttaaatc atttttagca gattatgaaa 3540

gtgatacgca acggtatgga aacaatcata gaatggaagg aaagccaaat gctccggaaa 3600

acatttttaa tgtatctatg ataccgtggt caaccttcga tggctttaat ctgaatttgc 3660

agaaaggata tgattatttg attcctattt ttactatggg gaaatattat aaagaagata 3720

acaaaattat acttcctttg gcaattcaag ttcatcacgc agtatgtgac ggatttcaca 3780

tttgccgttt tgtaaacgaa ttgcaggaat tgataaatag ttaacttcag gtttgtctgt 3840

aactaaaaac tagtatttaa ccgagctcga attcactggc cgtcgtttta caacgtcgtg 3900

actgggaaaa ccctggcgtt acccaactta atcgccttgc agcacatccc cctttcgcca 3960

gctggcgtaa tagcgaagag gcccgcaccg atcgcccttc ccaacagttg cgcagcctga 4020

atggcgaatg gcgcctgatg cggtattttc tccttacgca tctgtgcggt atttcacacc 4080

gcatatggtg cactctcagt acaatctgct ctgatgccgc atagttaagc cagccccgac 4140

acccgccaac acccgctgac gcgccctgac gggcttgtct gctcccggca tccgcttaca 4200

gacaagctgt gaccgtctcc gggagctgca tgtgtcagag gttttcaccg tcatcaccga 4260

aacgcgcgag acgaaagggc ctcgtgatac gcctattttt ataggttaat gtcatgataa 4320

taatggtttc ttagacgtca ggtggcactt ttcggggaaa tgtgcgcgga acccctattt 4380

gtttattttt ctaaatacat tcaaatatgt atccgctcat gagacaataa ccctgataaa 4440

tgcttcaata atattgaaaa aggaagagta tgagtattca acatttccgt gtcgccctta 4500

ttcccttttt tgcggcattt tgccttcctg tttttgctca cccagaaacg ctggtgaaag 4560

taaaagatgc tgaagatcag ttgggtgcac gagtgggtta catcgaactg gatctcaaca 4620

gcggtaagat ccttgagagt tttcgccccg aagaacgttt tccaatgatg agcactttta 4680

aagttctgct atgtggcgcg gtattatccc gtattgacgc cgggcaagag caactcggtc 4740

gccgcataca ctattctcag aatgacttgg ttgagtactc accagtcaca gaaaagcatc 4800

ttacggatgg catgacagta agagaattat gcagtgctgc cataaccatg agtgataaca 4860

ctgcggccaa cttacttctg acaacgatcg gaggaccgaa ggagctaacc gcttttttgc 4920

acaacatggg ggatcatgta actcgccttg atcgttggga accggagctg aatgaagcca 4980

taccaaacga cgagcgtgac accacgatgc ctgtagcaat ggcaacaacg ttgcgcaaac 5040

tattaactgg cgaactactt actctagctt cccggcaaca attaatagac tggatggagg 5100

cggataaagt tgcaggacca cttctgcgct cggcccttcc ggctggctgg tttattgctg 5160

ataaatctgg agccggtgag cgtgggtctc gcggtatcat tgcagcactg gggccagatg 5220

gtaagccctc ccgtatcgta gttatctaca cgacggggag tcaggcaact atggatgaac 5280

gaaatagaca gatcgctgag ataggtgcct cactgattaa gcattggtaa ctgtcagacc 5340

aagtttactc atatatactt tagattgatt taaaacttca tttttaattt aaaaggatct 5400

aggtgaagat cctttttgat aatctcatga ccaaaatccc ttaacgtgag ttttcgttcc 5460

actgagcgtc agaccccgta gaaaagatca aaggatcttc ttgagatcct ttttttctgc 5520

gcgtaatctg ctgcttgcaa acaaaaaaac caccgctacc agcggtggtt tgtttgccgg 5580

atcaagagct accaactctt tttccgaagg taactggctt cagcagagcg cagataccaa 5640

atactgttct tctagtgtag ccgtagttag gccaccactt caagaactct gtagcaccgc 5700

ctacatacct cgctctgcta atcctgttac cagtggctgc tgccagtggc gataagtcgt 5760

gtcttaccgg gttggactca agacgatagt taccggataa ggcgcagcgg tcgggctgaa 5820

cggggggttc gtgcacacag cccagcttgg agcgaacgac ctacaccgaa ctgagatacc 5880

tacagcgtga gctatgagaa agcgccacgc ttcccgaagg gagaaaggcg gacaggtatc 5940

cggtaagcgg cagggtcgga acaggagagc gcacgaggga gcttccaggg ggaaacgcct 6000

ggtatcttta tagtcctgtc gggtttcgcc acctctgact tgagcgtcga tttttgtgat 6060

gctcgtcagg ggggcggagc ctatggaaaa acgccagcaa cgcggccttt ttacggttcc 6120

tggccttttg ctggcctttt gctcacatgt tctttcctgc gttatcccct gattctgtgg 6180

ataaccgtat taccgccttt gagtgagctg ataccgctcg ccgcagccga acgaccgagc 6240

gcagcgagtc agtgagcgag gaagcggaag a 6271

<210> 126
<211> 2793
<212> DNA
<213> Artificial Sequence

<220>

<223>  OREP

<400>  126
cacgataaaa caaggtttta aggataagaa aagtcatgag atttatagta aatcttgtga      60

ctttttttat tgaatagtag agagagttcg gaagtataac acgctatatt cttgatattt      120

ttagaatagc aagcattgga tttgtcctga cactttccca aaaattaagg agttattcct      180

taaaccaaaa agattaatgt gggaacaaat ttagtgtatc catttttgaa gggcgcactt      240

atacaccacc aaaatggtgt gtgcgaaatc tttaaaaaag atttatcaaa aagctttttt      300

aaagctggga catttagaaa atcaataatg ttttttgccc aatacgctag tcttaaaatc      360

tgcaaggttg ataactattt agtcccaggt attagaatgg ggcatatata tacaaagtat      420

atatatgcgt aaatatatgt gggactgtgg gaacaaaatt gcgtgctaaa attgtattga      480

aaaggtaatg aaaaggtcat gctttggtat tgctaacgta tagaaaaggt aatgaaaagc      540

tcatggttct ataaaaaaga tgtacccacg aaaataatag gctttgccta tttccccatg      600

taatatgggg gcagttttct cttatgctct ttcttaacat attgaataaa tacaaaatgc      660

agctttgtgg gaataaaaat atttttgttt ttattcttat agttagacaa aattttaatc      720

ttttttgtgc tataacaaga ttaaaatttg tgggaacatt aagaaatatt gttgtcacaa      780

ataaaaagga gagtgggaac aattgctata aaaaacgcag aaattaagat tagagttaca      840

aaagagcaaa aagaattatt taagaaaatt gcaaaagctg aaaatatgag tatgagtgaa      900

tttattattg tgaccacaga atatttagcc agaaaaaaag atgaaaatat gaaatcaaaa      960

gacatgatcg agagaagagc tgcgaagact gaagaaaaaa ttatgaagct aaaaaagaaa      1020

ctaaataaaa acaggtaata tagattacag ttttaagctt gttttcccta tagactagag      1080

taaatatata aatatacctg tcaagggctt ataagcccct ttagggggtg cgtagcaccc      1140

ttgacaggta tatttatata ttttagggtg ccattaaggg aaacaagctt taaaatgcct      1200

ttaaaggcat tttaaaataa ataaaaaaaa gatggttttt accatctttt ttaactcccg      1260

aaagggagtt ctttcttttc ttgatactat acgtaactat ttcgatttgc cctgaaccta      1320

atcaaagcta gataaattca gtattagggc ataaaaaaac ttgctttttc gggtggaaat      1380

ctgtataatt taaattgctt agataaaaat taccaattcc atacgaaagg agcaagtttt      1440

acataaggtt aaagccttat gtgaattctc atttaattac atgaataata ataacacaga      1500

aagtgaagaa ttaaaagagc aaagtcaact attgcttgac aaatgcacaa aaaagaaaaa      1560

gaaaaatcct aaatttagta gttatataga accattagta agcaagaaat tatctgaaag      1620

aataaaggaa tgtggtgact ttttgcagat gttatctgat ttaaaccttg aaaattcgaa      1680

actgcataga gcaagttttt gtggtaacag attttgtcct atgtgtagct ggcgtattgc      1740

ttgtaaggat agtttggaaa tatctattct catggagcat ttacgcaaag aggaaagcaa      1800

agaatttatc tttttgacct taacaactcc aaatgtgaaa ggtgcggacc ttgataattc 1860

cataaaagca tacaataaag catttaaaaa gttaatggaa cgcaaagagg tcaagagcat 1920

agtaaaaggc tacataagaa agctagaagt aacctataat ttggacaaga gttccaaatc 1980

atataatact tatcacccac atttccatgt ggtactagca gtcaatagaa gttactttaa 2040

aaagcaaaat ctatatataa accatcatag atggcttagt ttgtggcaag agtcaactgg 2100

tgattattcg ataactcaag ttgatgtaag aaaggctaaa attaacgatt ataaagaggt 2160

ttatgagctt gctaagtatt cggctaagga ttccgactat ttaatcaata gagaagtgtt 2220

tacggtattc tacaaatctt taaagggtaa acaggtactt gtatttagtg gattatttaa 2280

agacgctcat aaaatgtata agaatggaga gctagatctg tataagaagt tggatactat 2340

cgaatatgct tatatggtaa gttataactg gcttaaaaag aagtatgata cttcaaatat 2400

tagagaatta actgaggaag aaaagcagaa attcaataaa aatttaatcg aagatgtgga 2460

tattgagtag gtgggattat atctcacctt ttttattgtc ttttcatgtt gaaattttga 2520

cgcttaatgc atgaagtatt gacaagttta aaaattacgg tttttaatcc ttagttgatt 2580

agcaggatta tggccggaat gctccgtcca gtcctgttaa ggaattaaaa ttccctaaaa 2640

cccttggcta tgatttatag cgagaatcgt caattaaaaa tttaataggt gctatgaaag 2700

tcgattaata attaatttta aaatgcaata tgaaacataa ttacaagaat ttgactttta 2760

atacaagaat tgatatcata gttacattaa tac 2793

<210> 127
<211> 2793
<212> DNA
<213> Clostridium beijerinckii

<400> 127
cacgataaaa caaggtttta aggataagaa aagtcatgag atttatagta aatcttgtga 60

ctttttttat tgaatagtag agagagttcg gaagtataac acgctatatt cttgatattt 120

ttagaatagc aagcattgga tttgtcctga cactttccca aaaattaagg agttattcct 180

taaaccaaaa agattaatgt gggaacaaat ttagtgtatc catttttgaa gggcgcactt 240

atacaccacc aaaatggtgt gtgcgaaatc tttaaaaaag atttatcaaa aagctttttt 300

aaagctggga catttagaaa atcaataatg ttttttgccc aatacgctag tcttaaaatc 360

tgcaaggttg ataactattt agtcccaggt attagaatgg ggcatatata tacaaagtat 420

atatatgcgt aaatatatgt gggactgtgg gaacaaaatt gcgtgctaaa attgtattga 480

aaaggtaatg aaaaggtcat gctttggtat tgctaacgta tagaaaaggt aatgaaaagc 540

tcatggttct ataaaaaaga tgtacccacg aaaataatag gctttgccta tttccccatg 600

taatatgggg gcagttttct cttatgctct ttcttaacat attgaataaa tacaaaatgc 660

agctttgtgg gaataaaaat atttttgttt ttattcttat agttagacaa aattttaatc 720
ttttttgtgc tataacaaga ttaaaatttg tgggaacatt aagaaatatt gttgtcacaa 780
ataaaaagga gagtgggaac aattgctata aaaaacgcag aaattaagat tagagttaca 840
aaagagcaaa aagaattatt taagaaaatt gcaaaagctg aaaatatgag tatgagtgaa 900
tttattattg tgaccacaga atatttagcc agaaaaaaag atgaaaatat gaaatcaaaa 960
gacatgatcg agagaagagc tgcgaagact gaagaaaaaa ttatgaagct aaaaaagaaa 1020
ctaaataaaa acaggtaata tagattacag ttttaagctt gttttcccta tagactagag 1080
taaatatata aatatacctg tcaagggctt ataagcccct ttagggggtg cgtagcaccc 1140
ttgacaggta tatttatata ttttagggtg ccattaaggg aaacaagctt taaaatgcct 1200
ttaaaggcat tttaaaataa ataaaaaaaa gatggttttt accatctttt ttaactcccg 1260
aaagggagtt ctttcttttc ttgatactat acgtaactat ttcgatttgc cctgaaccta 1320
atcaaagcta gataaattca gtattagggc ataaaaaaac ttgctttttc gggtggaaat 1380
ctgtataatt taaattgctt agataaaaat taccaattcc atacgaaagg agcaagtttt 1440
acataaggtt aaagccttat gtgaattctc atttaattac atgaataata ataacacaga 1500
aagtgaagaa ttaaaagagc aaagtcaact attgcttgac aaatgcacaa aaaagaaaaa 1560
gaaaaatcct aaatttagta gttatataga accattagta agcaagaaat tatctgaaag 1620
aataaaggaa tgtggtgact ttttgcagat gttatctgat ttaaaccttg aaaattcgaa 1680
actgcataga gcaagttttt gtggtaacag attttgtcct atgtgtagct ggcgtattgc 1740
ttgtaaggat agtttggaaa tatctattct catggagcat ttacgcaaag aggaaagcaa 1800
agaatttatc tttttgacct taacaactcc aaatgtgaaa ggtgcggacc ttgataattc 1860
cataaaagca tacaataaag catttaaaaa gttaatggaa cgcaaagagg tcaagagcat 1920
agtaaaaggc tacataagaa agctagaagt aacctataat ttggacaaga gttccaaatc 1980
atataatact tatcacccac atttccatgt ggtactagca gtcaatagaa gttactttaa 2040
aaagcaaaat ctatatataa accatcatag atggcttagt ttgtggcaag agtcaactgg 2100
tgattattcg ataactcaag ttgatgtaag aaaggctaaa attaacgatt ataaagaggt 2160
ttatgagctt gctaagtatt cggctaagga ttccgactat ttaatcaata gagaagtgtt 2220
tacggtattc tacaaatctt taaagggtaa acaggtactt gtatttagtg gattatttaa 2280
agacgctcat aaaatgtata agaatggaga gctagatctg tataagaagt tggatactat 2340
cgaatatgct tatatggtaa gttataactg gcttaaaaag aagtatgata cttcaaatat 2400
tagagaatta actgaggaag aaaagcagaa attcaataaa aatttaatcg aagatgtgga 2460
tattgagtag gtgggattat atctcacctt ttttattgtc ttttcatgtt gaaattttga 2520
cgcttaatgc atgaagtatt gacaagttta aaaattacgg tttttaatcc ttagttgatt 2580

agcaggatta tggccggaat gctccgtcca gtcctgttaa ggaattaaaa ttccctaaaa 2640

cccttggcta tgatttatag cgagaatcgt caattaaaaa tttaataggt gctatgaaag 2700

tcgattaata attaatttta aaatgcaata tgaaacataa ttacaagaat ttgactttta 2760

atacaagaat tgatatcata gttacattaa tac 2793

<210> 128
<211> 329
<212> PRT
<213> Clostridium beijerinckii

<400> 128

Met Asn Asn Asn Asn Thr Glu Ser Glu Glu Leu Lys Glu Gln Ser Gln
1               5                   10                  15

Leu Leu Leu Asp Lys Cys Thr Lys Lys Lys Lys Lys Asn Pro Lys Phe
            20                  25                  30

Ser Ser Tyr Ile Glu Pro Leu Val Ser Lys Lys Leu Ser Glu Arg Ile
        35                  40                  45

Lys Glu Cys Gly Asp Phe Leu Gln Met Leu Ser Asp Leu Asn Leu Glu
    50                  55                  60

Asn Ser Lys Leu His Arg Ala Ser Phe Cys Gly Asn Arg Phe Cys Pro
65                  70                  75                  80

Met Cys Ser Trp Arg Ile Ala Cys Lys Asp Ser Leu Glu Ile Ser Ile
                85                  90                  95

Leu Met Glu His Leu Arg Lys Glu Glu Ser Lys Glu Phe Ile Phe Leu
            100                 105                 110

Thr Leu Thr Thr Pro Asn Val Lys Gly Ala Asp Leu Asp Asn Ser Ile
        115                 120                 125

Lys Ala Tyr Asn Lys Ala Phe Lys Lys Leu Met Glu Arg Lys Glu Val
    130                 135                 140

Lys Ser Ile Val Lys Gly Tyr Ile Arg Lys Leu Glu Val Thr Tyr Asn
145                 150                 155                 160

Leu Asp Lys Ser Ser Lys Ser Tyr Asn Thr Tyr His Pro His Phe His
                165                 170                 175

Val Val Leu Ala Val Asn Arg Ser Tyr Phe Lys Lys Gln Asn Leu Tyr
            180                 185                 190

Ile Asn His His Arg Trp Leu Ser Leu Trp Gln Glu Ser Thr Gly Asp
        195                 200                 205

Tyr Ser Ile Thr Gln Val Asp Val Arg Lys Ala Lys Ile Asn Asp Tyr
    210                 215                 220

Lys Glu Val Tyr Glu Leu Ala Lys Tyr Ser Ala Lys Asp Ser Asp Tyr
225                 230                 235                 240

Leu Ile Asn Arg Glu Val Phe Thr Val Phe Tyr Lys Ser Leu Lys Gly
                245                 250                 255

Lys Gln Val Leu Val Phe Ser Gly Leu Phe Lys Asp Ala His Lys Met
            260                 265                 270

Tyr Lys Asn Gly Glu Leu Asp Leu Tyr Lys Lys Leu Asp Thr Ile Glu
        275                 280                 285

Tyr Ala Tyr Met Val Ser Tyr Asn Trp Leu Lys Lys Lys Tyr Asp Thr
    290                 295                 300

Ser Asn Ile Arg Glu Leu Thr Glu Glu Glu Lys Gln Lys Phe Asn Lys
305                 310                 315                 320

Asn Leu Ile Glu Asp Val Asp Ile Glu
                325

<210>  129
<211>  256
<212>  PRT
<213>  Artificial Sequence

<220>
<223>  Consensus COG5655

<400>  129

Met Cys Gln Lys Arg Ser Asp Tyr Ser Asp Glu Lys Ala Trp Leu Lys
1               5                   10                  15

Asp Lys Ser Lys Asp Gly Lys Val Glu Pro Trp Arg Glu Lys Lys Glu
            20                  25                  30

Ala Asn Val Lys Tyr Phe Glu Leu Leu Lys Ile Leu Met Phe Lys Lys
        35                  40                  45

Ala Glu Arg Val Tyr Arg Cys Asn Glu Leu Leu Glu Leu Gln Lys Val
    50                  55                  60

Asn Glu Thr Gly Glu Asn Lys Leu Cys Pro Asn Trp Phe Cys Lys Ser
65                  70                  75                  80

Leu Leu Cys Pro Met Cys Asn Trp Arg Lys Pro Met Lys Ser Asp Leu
                85                  90                  95

Gln Asp Gly Leu Tyr Val Lys Arg Val Ile Ser Tyr Gly Pro Leu Leu
            100                 105                 110

Lys Trp Lys His Leu Lys Leu Asn Leu Lys Asn Val Glu Asp Gly Asp
        115                 120                 125

Leu Leu Asn Lys Ser Leu Asp Glu Met Ala Leu Gly Phe Lys Arg Thr
    130                 135                 140

Met Gly Phe Lys Lys Ile Ala Lys Asn Phe Val Gly Phe Met Lys Ser
145                 150                 155                 160

Thr Glu Ile Thr Tyr Asn Glu Lys Asp Asn Ser Tyr Asn Gln His Met
                165                 170                 175

His Val Leu Phe Cys Ser Glu Gln Thr Tyr Phe Lys Asn Phe Ile Asn
            180                 185                 190

Asn Thr Pro Gln Glu Phe Trp Asn Lys Arg Trp Ser Lys Ala Met Lys
        195                 200                 205

Leu Asp Tyr Asp Pro Gln Val Met Lys Leu Trp Thr Met Tyr Lys Lys
    210                 215                 220

Glu Ile Lys Asn Tyr Ile Gln Thr Ala Leu Gln Glu Thr Ala Lys Tyr
225                 230                 235                 240

Asp Val Lys Asp Met Asp Ser Ala Thr Ile Asp Asp Glu Lys Ser Leu
                245                 250                 255

<210>  130
<211>  768
<212>  DNA
<213>  Enterococcus faecalis

<400>  130
gtgaggagga tatatttgaa tacatacgaa caaattaata aagtgaaaaa aatacttcgg      60

aaacatttaa aaaataacct tattggtact tacatgtttg gatcaggagt tgagagtgga     120

ctaaaaccaa atagtgatct tgacttttta gtcgtcgtat ctgaaccatt gacagatcaa     180

agtaaagaaa tacttataca aaaaattaga cctatttcaa agaaaatagg agataaaagc     240

aacttacgat atattgaatt aacaattatt attcagcaag aaatggtacc gtggaatcat 300

cctcccaaac aagaatttat ttatggagaa tggttacaag agctttatga acaaggatac 360

attcctcaga aggaattaaa ttcagattta accataatgc tttaccaagc aaaacgaaaa 420

aataaaagaa tatacggaaa ttatgactta gaggaattac tacctgatat tccattttct 480

gatgtgagaa gagccattat ggattcgtca gaggaattaa tagataatta tcaggatgat 540

gaaaccaact ctatattaac tttatgccgt atgattttaa ctatggacac gggtaaaatc 600

ataccaaaag atattgcggg aaatgcagtg gctgaatctt ctccattaga acatagggag 660

agaattttgt tagcagttcg tagttatctt ggagagaata ttgaatggac taatgaaaat 720

gtaaatttaa ctataaacta tttaaataac agattaaaaa aattataa 768

<210> 131
<211> 738
<212> DNA
<213> Clostridium difficile

<400> 131
atgaacaaaa atataaaata ttctcaaaac tttttaacga gtgaaaaagt actcaaccaa 60

ataataaaac aattgaattt aaaagaaacc gataccgttt acgaaattgg aacaggtaaa 120

gggcatttaa cgacgaaact ggctaaaata agtaaacagg taacgtctat tgaattagac 180

agtcatctat tcaacttatc gtcagaaaaa ttaaaactga atactcgtgt cactttaatt 240

caccaagata ttctacagtt tcaattccct aacaaacaga ggtataaaat tgttgggagt 300

attccttacc atttaagcac acaaattatt aaaaaagtgg tttttgaaag ccatgcgtct 360

gacatctatc tgattgttga agaaggattc tacaagcgta ccttggatat tcaccgaaca 420

ctagggttgc tcttgcacac tcaagtctcg attcagcaat tgcttaagct gccagcggaa 480

tgctttcatc ctaaaccaaa agtaaacagt gtcttaataa aacttacccg ccataccaca 540

gatgttccag ataaatattg gaagctatat acgtactttg tttcaaaatg ggtcaatcga 600

gaatatcgtc aactgtttac taaaaatcag tttcatcaag caatgaaaca cgccaaagta 660

aacaatttaa gtaccgttac ttatgagcaa gtattgtcta tttttaatag ttatctatta 720

tttaacggga ggaaataa 738

<210> 132
<211> 3792
<212> DNA
<213> Artificial Sequence

<220>
<223> Optimized Mad7 CDS for B. subtilis

<400> 132
atgaacaacg gcacaaataa ttttcagaac tttattggca tttcatcatt gcagaaaacg 60

ttaagaaatg ctttaattcc gacggaaaca acgcaacagt ttattgttaa aaacggaatt 120
attaaagaag atgaattaag aggcgaaaac agacagattt taaaagatat tatggatgac 180
tactacagag gatttatttc tgaaacatta tcatctattg atgacattga ttggacaagc 240
ttatttgaaa aaatggaaat tcagttaaaa aatggtgata ataaagatac attaattaaa 300
gaacagacag aatatagaaa agcaattcat aaaaaatttg cgaacgacga tagatttaaa 360
aacatgttta gcgccaaatt aatttcagac attttacctg aatttgttat tcataacaat 420
aattattcag catcagaaaa agaagaaaaa acacaggtga ttaaattgtt ttcaagattt 480
gcgacaagct ttaaagatta ctttaaaaac agagcaaatt gcttttcagc ggacgatatt 540
tcatcaagca gctgccatag aattgttaac gacaatgcag aaattttttt ttcaaatgcg 600
ttagtttaca gaagaattgt aaaatcatta agcaatgacg atattaacaa aatttcaggc 660
gatatgaaag attcattaaa agaaatgtca ttagaagaaa tttattctta cgaaaaatat 720
ggcgaattta ttacacagga aggcattagc ttttataatg atatttgtgg caaagtgaat 780
tcttttatga acttatattg tcagaaaaat aaagaaaaca aaaatttata caaacttcag 840
aaacttcata aacagattct gtgcattgcg gacacaagct atgaagttcc gtataaattt 900
gaatcagacg aagaagtgta ccaatcagtt aacggctttc ttgataacat tagcagcaaa 960
catattgttg aaagattaag aaaaattggc gataactata acggctacaa cttagataaa 1020
atttatattg tgtccaaatt ttacgaaagc gttagccaaa aaacatacag agactgggaa 1080
acaattaata cagccttaga aattcattac aataatattt tgccgggtaa cggtaaatca 1140
aaagccgaca aagtaaaaaa agcggttaaa aatgatttac agaaatccat tacagaaatt 1200
aatgaactgg tgtcaaacta taaattatgc tcagacgaca acattaaagc ggaaacatat 1260
attcatgaaa ttagccatat tttgaataac tttgaagcac aggaattgaa atacaatccg 1320
gaaattcatc tggttgaatc cgaattaaaa gcgtcagaac ttaaaaacgt gttagacgtg 1380
attatgaatg cgtttcattg gtgttcagtt tttatgacag aagaacttgt tgataaagac 1440
aacaattttt atgcggaatt agaagaaatt tacgatgaaa tttatccggt aatttcatta 1500
tacaacttag ttagaaacta cgttacacag aaaccgtaca gcacgaaaaa aattaaattg 1560
aactttggaa ttccgacgtt agcagacggt tggtcaaaat ccaaagaata ttctaataac 1620
gctattattt taatgagaga caatttatat tatttaggca tttttaatgc gaaaaataaa 1680
ccggacaaaa aaattattga aggtaatacg tcagaaaata aaggtgacta caaaaaaatg 1740
atttataatt tgttaccggg tccgaacaaa atgattccga aagttttttt gagcagcaaa 1800
acgggcgtgg aaacgtataa accgagcgcc tatattctgg aaggctataa acagaataaa 1860
catattaaat cttcaaaaga ctttgatatt acattttgtc atgatttaat tgactacttt 1920
aaaaactgta ttgcaattca tccggaatgg aaaaactttg gttttgattt tagcgacaca 1980

tcaacatatg aagacatttc cggcttttat agagaagtag aattacaagg ttacaaaatt 2040

gattggacat acattagcga aaaagacatt gatttattac aggaaaaagg tcaattatat 2100

ttatttcaga tttataacaa agatttttca aaaaaatcaa caggcaatga caaccttcat 2160

acaatgtact taaaaaatct tttttcagaa gaaaatctta aagatattgt tttaaaactt 2220

aacggcgaag cggaaatttt ttttagaaaa agcagcatta aaaacccgat tattcataaa 2280

aaaggctcaa ttttagttaa cagaacatac gaagcagaag aaaaagacca gtttggcaac 2340

attcaaattg tgagaaaaaa tattccggaa aacatttatc aggaattata caaatacttt 2400

aacgataaaa gcgacaaaga attatctgat gaagcagcca aattaaaaaa tgtagtggga 2460

catcatgaag cagcgacgaa tattgttaaa gactatagat acacgtatga taaatacttt 2520

cttcatatgc ctattacgat taattttaaa gccaataaaa cgggttttat taatgataga 2580

attttacagt atattgctaa agaaaaagac ttacatgtga ttggcattga tagaggcgaa 2640

agaaacttaa tttacgtgtc cgtgattgat acatgtggta atattgttga acagaaaagc 2700

tttaacattg taaacggcta cgactatcag attaaattaa aacaacagga aggcgctaga 2760

cagattgcga gaaaagaatg gaaagaaatt ggtaaaatta aagaaattaa agaaggctac 2820

ttaagcttag taattcatga aatttctaaa atggtaatta aatacaatgc aattattgcg 2880

atggaagatt tgtcttatgg ttttaaaaaa ggcagattta aagttgaaag acaagtttac 2940

cagaaatttg aaacaatgtt aattaataaa ttaaactatt tagtatttaa agatatttca 3000

attacagaaa atggcggttt attaaaaggt tatcagttaa catacattcc tgataaactt 3060

aaaaacgtgg gtcatcagtg cggctgcatt ttttatgtgc ctgctgcata cacgagcaaa 3120

attgatccga caacaggctt tgtgaatatt tttaaattta aagacttaac agtggacgca 3180

aaaagagaat ttattaaaaa atttgactca attagatatg actcagaaaa aaatttattt 3240

tgctttacat ttgactacaa taactttatt acgcaaaaca cggttatgag caaatcatca 3300

tggtcagtgt atacatacgg cgtgagaatt aaaagaagat ttgtgaacgg cagattttca 3360

aacgaatcag atacaattga cattacaaaa gatatggaaa aaacgttgga aatgacggac 3420

attaactgga gagatggcca tgatcttaga caagacatta ttgattatga aattgttcag 3480

catatttttg aaatttttag attaacagtg caaatgagaa actccttgtc tgaattagaa 3540

gacagagatt acgatagatt aatttcacct gtattaaacg aaaataacat tttttatgac 3600

agcgcgaaag cgggcgatgc acttcctaaa gatgccgatg caaatggtgc gtattgtatt 3660

gcattaaaag gcttatatga aattaaacaa attacagaaa attggaaaga agatggtaaa 3720

ttttcaagag ataaattaaa aattagcaat aaagattggt ttgactttat tcagaataaa 3780

agatatttat aa 3792

<210>  133
<211>  10469
<212>  DNA
<213>  Artificial Sequence

<220>
<223>  pCas9cond

<400>  133
catggataaa aagtacagta ttggtctaga cataggaact aactctgttg ggtgggctgt      60

tataacagat gaatataaag ttccatcaaa aaaatttaaa gtattaggaa acactgatag     120

acattcaata aaaaaaaact tgataggtgc tttattattc gattcaggag agactgctga     180

agctacacgt ttaaaaagaa cagctagacg tagatataca agaagaaaaa ataggatatg     240

ttatcttcaa gaaattttta gtaatgaaat ggcaaaagtt gatgattcat tctttcacag     300

actagaagaa agtttcttag ttgaagaaga taagaagcat gaaagacacc ctatttttgg     360

taatatcgta gatgaagtag catatcatga gaagtatcca actatctatc atttaagaaa     420

gaaattagtt gattctacag ataaagctga tctgagatta atatatttag ctttagctca     480

tatgattaaa tttagaggac attttttaat agaaggtgat ttaaacccag acaacagcga     540

tgtagataaa ttatttatcc aattagttca aacttataat caattattcg aagagaatcc     600

aattaatgca agtggtgtag acgctaaggc tatattatca gctagattat caaaatctag     660

aagattagaa aatctaatag ctcaacttcc tggagaaaag aaaaatggac tttttgggaa     720

cctaatagct ctctcactcg gactaacacc aaattttaaa agcaattttg atcttgctga     780

agacgcaaag ttacaactat caaaggatac atacgatgat gatttagata atttgttagc     840

tcaaataggt gatcaatatg ctgatttgtt tcttgcagca aaaaacttaa gtgatgcaat     900

tttactatca gatatactta gagtaaatac agaaataaca aaggctcctt tatcagcaag     960

tatgattaaa cgatatgatg agcatcatca agatttaaca ttattaaagg cacttgtaag    1020

acaacaatta ccagaaaaat ataaagaaat tttctttgat caatctaaaa atggatatgc    1080

tggatatata gacggtggag caagtcaaga agagttttat aaatttataa agcctatttt    1140

agaaaaaatg gatggaactg aagaattact tgttaaactt aacagagaag atttacttag    1200

aaaacaaaga acttttgata atggttcaat tcctcaccaa attcatttag gagaattaca    1260

tgctatacta agaagacaag aagattttta tccatttctt aaagataata gagaaaaaat    1320

tgaaaaaatt ttaactttta gaataccata ttatgtagga ccacttgcaa ggggaaattc    1380

aagatttgca tggatgacta gaaaatcaga agaaactata accccgtgga attttgaaga    1440

agtagtagat aaaggagcta gtgctcaatc atttatagaa agaatgacaa attttgataa    1500

gaatcttcct aacgaaaagg ttttgccaaa gcatagcctt ctttatgagt attttacagt    1560

ttataatgag cttactaaag taaaatacgt tacagaagga atgagaaaac cagcattttt    1620

gtctggtgaa caaaagaaag caatagtaga cctattattt aaaacaaata ggaaggttac 1680

cgtaaagcaa cttaaagaag attacttcaa aaaaattgaa tgctttgata gtgttgaaat 1740

atcaggagtt gaagatagat ttaatgcttc acttggtaca tatcacgatc tcttaaaaat 1800

tataaaagat aaggattttt tagataatga agaaaatgaa gatattcttg aagatatagt 1860

attaacattg acactttttg aagatagaga aatgatagaa gaaagattaa aaacatatgc 1920

acatcttttt gatgataagg ttatgaagca acttaaaaga agaagatata caggttgggg 1980

acgtttgtca agaaagctaa ttaatggtat tagagataaa caatcaggaa agactattct 2040

cgattttctt aaatcagatg gatttgctaa tagaaacttt atgcaattaa ttcatgatga 2100

ttctcttact ttcaaagagg atattcaaaa ggctcaagtt tctggacaag gcgatagctt 2160

acacgaacac attgctaacc ttgcagggag ccccgctatc aaaaaaggaa ttttacaaac 2220

agttaaagtt gtagatgaac ttgttaaagt tatgggaaga cacaaacctg agaatatagt 2280

tatagaaatg gccagagaaa atcaaacaac acaaaaagga caaaaaaatt ctagagagag 2340

aatgaagaga attgaagaag gaataaaaga gctaggatca caaatattaa aagaacatcc 2400

agttgaaaat actcaattgc aaaatgaaaa gttatatttg tattacttac aaaatggaag 2460

agatatgtat gttgatcaag aactcgatat taatagatta agtgactatg atgttgatca 2520

tattgttcct caatcatttt taaaagatga ttcaatcgat aacaaagtat taactagatc 2580

agataaaaat agaggaaagt cagataatgt accatctgaa gaagttgtta aaaaaatgaa 2640

gaactattgg agacaacttt taaatgcaaa gctaattaca caaagaaaat ttgacaattt 2700

aacaaaagca gaaagaggag gattaagcga attagacaaa gctggattta taaaaagaca 2760

acttgttgag acaagacaaa taactaagca tgttgctcaa atacttgatt caagaatgaa 2820

tacaaaatat gatgaaaatg ataaattaat cagagaagta aaagtaataa cattaaagtc 2880

aaaattagta tcagatttca gaaaggattt tcaattttac aaagttcgtg aaataaataa 2940

ctatcatcat gctcatgatg catacttaaa tgctgttgta ggaactgctc ttattaagaa 3000

atatcctaaa ctagaaagcg aatttgttta tggagattat aaagtttatg atgtgcgcaa 3060

aatgatcgcg aaatccgaac aagaaatcgg taaggctaca gcaaaatatt tcttttatag 3120

taatataatg aattttttta agacagaaat aactttggct aatggtgaaa tcagaaaaag 3180

accacttatc gaaacaaatg gagagacagg agaaatagta tgggataaag gaagagattt 3240

tgctactgtt agaaaagtac taagtatgcc acaagtaaat atcgtaaaga aaactgaagt 3300

tcaaactgga ggtttctcta aggaatcaat tttacctaag agaaattcag ataagttaat 3360

tgcaaggaaa aaagattggg acccaaaaaa atacggtggt tttgatagtc caacagttgc 3420

ctatagtgtt cttgtagtag cgaaagttga gaaaggtaag tcaaaaaagt tgaaaagcgt 3480

aaaagaactt cttggtatca caattatgga aagatcttca tttgaaaaaa atccaattga 3540
ctttttagaa gctaagggtt ataaagaagt taaaaaggat ttaatcataa aactaccaaa 3600
gtatagtcta tttgaactcg aaaacggaag aaaacgaatg ctcgctagcg caggagaact 3660
tcaaaaagga aatgaacttg cgctgccatc aaagtatgta aatttcttat atttagcttc 3720
tcattatgag aaattaaaag gatcaccaga ggataatgaa caaaagcaac tatttgtaga 3780
acaacacaaa cattatttag atgaaataat agaacaaata tctgaatttt ctaaaagagt 3840
tatacttgcc gacgcaaatc tagataaggt gctttcagcg tataataaac acagagataa 3900
accaataaga gaacaagcag aaaacattat ccatcttttt acattaacta atcttggtgc 3960
accagctgca tttaagtact ttgatacaac aatagataga aaaagataca catctactaa 4020
agaagtatta gacgcaactt taatacatca atctattaca gggctttatg aaacaagaat 4080
tgatttaagt caactaggcg gagattaagt cgacaaagta ttgttaaaaa taactctgta 4140
gaattataaa ttagttctac agagttattt tttgacccgg gtaccgagct cgaattcgta 4200
atcatggtca tagctgtttc ctgtgtgaaa ttgttatccg ctcacaattc cacacaacat 4260
acgagccgga agcataaagt gtaaagcctg gggtgcctaa tgagtgagct aactcacatt 4320
aattgcgttg cgctcactgc ccgctttcca gtcgggaaac ctgtcgtgcc agctgcatta 4380
atgaatcggc caacgcgcgg ggagaggcgg tttgcgtatt gggcgctctt ccgcttcctc 4440
gctcactgac tcgctgcgct cggtcgttcg gctgcggcga gcggtatcag ctcactcaaa 4500
ggcggtaata cggttatcca cagaatcagg ggataacgca ggaaagaaca tgtgagcaaa 4560
aggccagcaa aaggccagga accgtaaaaa ggccgcgttg ctggcgtttt tccataggct 4620
ccgcccccct gacgagcatc acaaaaatcg acgctcaagt cagaggtggc gaaacccgac 4680
aggactataa agataccagg cgtttccccc tggaagctcc ctcgtgcgct ctcctgttcc 4740
gaccctgccg cttaccggat acctgtccgc ctttctccct tcgggaagcg tggcgctttc 4800
tcatagctca cgctgtaggt atctcagttc ggtgtaggtc gttcgctcca agctgggctg 4860
tgtgcacgaa ccccccgttc agcccgaccg ctgcgcctta tccggtaact atcgtcttga 4920
gtccaacccg gtaagacacg acttatcgcc actggcagca gccactggta acaggattag 4980
cagagcgagg tatgtaggcg gtgctacaga gttcttgaag tggtggccta actacggcta 5040
cactagaagg acagtatttg gtatctgcgc tctgctgaag ccagttacct tcggaaaaag 5100
agttggtagc tcttgatccg gcaaacaaac caccgctggt agcggtggtt tttttgtttg 5160
caagcagcag attacgcgca gaaaaaaagg atctcaagaa gatcctttga tcttttctac 5220
ggggtctgac gctcagtgga acgaaaactc acgttaaggg attttggtca tgagattatc 5280
aaaaaggatc ttcacctaga tccttttaaa ttaaaaatga agttttaaat caatctaaag 5340
tatatatgag taaacttggt ctgacagtta ccaatgctta atcagtgagg cacctatctc 5400

agcgatctgt ctatttcgtt catccatagt tgcctgactc cccgtcgtgt agataactac 5460

gatacgggag ggcttaccat ctggccccag tgctgcaatg ataccgcgag acccacgctc 5520

accggctcca gatttatcag caataaacca gccagccgga agggccgagc gcagaagtgg 5580

tcctgcaact ttatccgcct ccatccagtc tattaattgt tgccgggaag ctagagtaag 5640

tagttcgcca gttaatagtt tgcgcaacgt tgttgccatt gctacaggca tcgtggtgtc 5700

acgctcgtcg tttggtatgg cttcattcag ctccggttcc caacgatcaa ggcgagttac 5760

atgatccccc atgttgtgca aaaaagcggt tagctccttc ggtcctccga tcgttgtcag 5820

aagtaagttg gccgcagtgt tatcactcat ggttatggca gcactgcata attctcttac 5880

tgtcatgcca tccgtaagat gcttttctgt gactggtgag tactcaacca agtcattctg 5940

agaatagtgt atgcggcgac cgagttgctc ttgcccggcg tcaatacggg ataataccgc 6000

gccacatagc agaactttaa aagtgctcat cattggaaaa cgttcttcgg ggcgaaaact 6060

ctcaaggatc ttaccgctgt tgagatccag ttcgatgtaa cccactcgtg cacccaactg 6120

atcttcagca tcttttactt tcaccagcgt ttctgggtga gcaaaaacag gaaggcaaaa 6180

tgccgcaaaa aagggaataa gggcgacacg gaaatgttga atactcatac tcttcctttt 6240

tcaatattat tgaagcattt atcagggtta ttgtctcatg agcggataca tatttgaatg 6300

tatttagaaa aataaacaaa taggggttcc gcgcacattt ccccgaaaag tgccacctga 6360

ctgccgggcc tcttgcggga tcaaaagaaa aacgaaatga tacaccaatc agtgcaaaaa 6420

aagatataat gggagataag acggttcgtg ttcgtgctga cttgcaccat atcataaaaa 6480

tcgaaacagc aaagaatggc ggaaacgtaa aagaagttat ggaaataaga cttagaagca 6540

aacttaagag tgtgttgata gtgcagtatc ttaaaatttt gtataatagg aattgaagtt 6600

aaattagatg ctaaaaattt gtaattaaga aggagtgatt acatgaacaa aaatataaaa 6660

tattctcaaa actttttaac gagtgaaaaa gtactcaacc aaataataaa acaattgaat 6720

ttaaaagaaa ccgataccgt ttacgaaatt ggaacaggta aagggcattt aacgacgaaa 6780

ctggctaaaa taagtaaaca ggtaacgtct attgaattag acagtcatct attcaactta 6840

tcgtcagaaa aattaaaact gaatactcgt gtcactttaa ttcaccaaga tattctacag 6900

tttcaattcc ctaacaaaca gaggtataaa attgttggga gtattcctta ccatttaagc 6960

acacaaatta ttaaaaaagt ggtttttgaa agccatgcgt ctgacatcta tctgattgtt 7020

gaagaaggat tctacaagcg taccttggat attcaccgaa cactagggtt gctcttgcac 7080

actcaagtct cgattcagca attgcttaag ctgccagcgg aatgctttca tcctaaacca 7140

aaagtaaaca gtgtcttaat aaaacttacc cgccatacca cagatgttcc agataaatat 7200

tggaagctat atacgtactt tgtttcaaaa tgggtcaatc gagaatatcg tcaactgttt 7260

actaaaaatc agtttcatca agcaatgaaa cacgccaaag taaacaattt aagtaccgtt 7320

acttatgagc aagtattgtc tatttttaat agttatctat tatttaacgg gaggaaataa 7380

ttctatgagt ccctaggccc aactaactca acgctagtag tggatttaat cccaaatgag 7440

ccaacagaac cagaaccaga aacagaatca gaacaagtaa cattggattt agaaatggaa 7500

gaagaaaaaa gcaatgactt cgtgtgaata atgcacgaaa tcgttgctta ttttttttta 7560

aaagcggtat actagatata acgaaacaac gaactgaata gaaacgaaaa aagagccatg 7620

acacatttat aaaatgtttg acgacatttt ataaatgcat agcccgataa gattgccaaa 7680

ccaacgctta tcagttagtc agatgaactc ttccctcgta agaagttatt taattaactt 7740

tgtttgaaga cggtatataa ccgtactatc attatatagg gaaatcagag agttttcaag 7800

tatctaagct actgaattta agaattgtta agcaatcaat cggaaatcgt ttgattgctt 7860

tttttgtatt catttataga aggtggagtt tgtatgaatc atgatgaatg taaaacttat 7920

ataaaaaata gtttattgga gataagaaaa ttagcaaata tctatacact agaaacgttt 7980

aagaaagagt tagaaaagag aaatatctac ttagaaacaa aatcagataa gtatttttct 8040

tcggaggggg aagattatat atataagtta atagaaaata acaaaataat ttattcgatt 8100

agtggaaaaa aattgactta taaaggaaaa aaatcttttt caaaacatgc aatattgaaa 8160

cagttgaatg aaaaagcaaa ccaagttaat taaacaacct attttatagg atttatagga 8220

aaggagaaca gctgaatgaa tatccctttt gttgtagaaa ctgtgcttca tgacggcttg 8280

ttaaagtaca aatttaaaaa tagtaaaatt cgctcaatca ctaccaagcc aggtaaaagc 8340

aaaggggcta tttttgcgta tcgctcaaaa tcaagcatga ttggcggtcg tggtgttgtt 8400

ctgacttccg aggaagcgat tcaagaaaat caagatacat ttacacattg gacacccaac 8460

gtttatcgtt atggaacgta tgcagacgaa aaccgttcat acacgaaagg acattctgaa 8520

aacaatttaa gacaaatcaa taccttcttt attgattttg atattcacac ggcaaaagaa 8580

actatttcag caagcgatat tttaacaacc gctattgatt taggttttat gcctactatg 8640

attatcaaat ctgataaagg ttatcaagca tattttgttt tagaaacgcc agtctatgtg 8700

acttcaaaat cagaatttaa atctgtcaaa gcagccaaaa taatttcgca aaatatccga 8760

gaatattttg gaaagtcttt gccagttgat ctaacgtgta atcattttgg tattgctcgc 8820

ataccaagaa cggacaatgt agaatttttt gatcctaatt accgttattc tttcaaagaa 8880

tggcaagatt ggtctttcaa acaaacagat aataagggct ttactcgttc aagtctaacg 8940

gttttaagcg gtacagaagg caaaaaacaa gtagatgaac cctggtttaa tctcttattg 9000

cacgaaacga aattttcagg agaaaagggt ttaatagggc gtaataacgt catgtttacc 9060

ctctctttag cctactttag ttcaggctat tcaatcgaaa cgtgcgaata taatatgttt 9120

gagtttaata atcgattaga tcaaccctta gaagaaaaag aagtaatcaa aattgttaga 9180

agtgcctatt cagaaaacta tcaaggggct aatagggaat acattaccat tctttgcaaa 9240

gcttgggtat caagtgattt aaccagtaaa gatttatttg tccgtcaagg gtggtttaaa 9300

ttcaagaaaa aaagaagcga acgtcaacgt gttcatttgt cagaatggaa agaagattta 9360

atggcttata ttagcgaaaa aagcgatgta tacaagcctt atttagtgac gaccaaaaaa 9420

gagattagag aagtgctagg cattcctgaa cggacattag ataaattgct gaaggtactg 9480

aaggcgaatc aggaaatttt ctttaagatt aaaccaggaa gaaatggtgg cattcaactt 9540

gctagtgtta aatcattgtt gctatcgatc attaaagtaa aaaaagaaga aaaagaaagc 9600

tatataaagg cgctgacaaa ttcttttgac ttagagcata cattcattca agagacttta 9660

aacaagctag cagaacgccc taaaacggac acacaactcg atttgtttag ctatgataca 9720

ggctgaaaat aaaacccgca ctatgccatt acatttatat ctatgatacg tgtttgtttt 9780

ttctttgctg tttagcgaat gattagcaga aatatacaga gtaagatttt aattaattat 9840

tagggggaga aggagagagt agcccgaaaa cttttagttg gcttggactg aacgaagtga 9900

gggaaaggct actaaaacgt cgaggggcag tgagagcgaa gcgaacactt gattttttaa 9960

ttttctatct tttataggtc attagagtat acttatttgt cctataaact atttagcagc 10020

ataatagatt tattgaatag gtcatttaag ttgagcatat tagaggagga aaatcttgga 10080

gaaatatttg aagaacccga ttacatggat tggattagtt cttgtggtta cgtggttttt 10140

aactaaaagt agtgaatttt tgatttttgg tgtgtgtgtc ttgttgttag tatttgctag 10200

tcaaagtgat taaatagaat tctagcgcca ttcgccattc aggctgcgca actgttggga 10260

agggcgatcg gtgcgggcct cttcgctatt acgccagctg gcgaaagggg gatgtgctgc 10320

aaggcgatta agttgggtaa cgccagggtt ttcccagtca cgacgttgta aaacgacggc 10380

cagtgccaag cttgcatgcc tgcaggcctc gagtatattg ataaaaataa taatagtggg 10440

tataattaag ttgttaggag gttagttac 10469

<210> 134
<211> 8559
<212> DNA
<213> Artificial Sequence

<220>
<223> pMAD7

<400> 134
tcgagtccct atcagtgata gattgaaact ctatcattga tagagtataa tatctttgtt 60

cattagagcg ataaacttga atttgagagg gaacttagat gaacaacggc acaaataatt 120

ttcagaactt catagggata tcaagtttgc agaaaacgtt aagaaatgct ttaataccca 180

cggaaaccac gcaacagttc atagttaaga acggaataat taaagaagat gagttaagag 240

gcgagaacag acagatttta aaagatataa tggatgacta ctacagagga ttcatatctg 300

agactttaag ttctattgat gacatagatt ggactagctt attcgaaaaa atggaaattc 360

agttaaaaaa tggtgataat aaagatacct taattaagga acagacagag tatagaaaag 420

caatacataa aaaatttgcg aacgacgata gatttaagaa catgtttagc gccaaattaa 480

ttagtgacat attacctgaa tttgttatac acaacaataa ttattcggca tcagagaaag 540

aggaaaaaac ccaggtgata aaattgtttt cgagatttgc gactagcttt aaagattact 600

tcaagaacag agcaaattgc ttttcagcgg acgatatttc atcaagcagc tgccatagaa 660

tagttaacga caatgcagag atattctttt caaatgcgtt agtttacaga agaatagtaa 720

aatcgttaag caatgacgat ataaacaaaa tttcgggcga tatgaaagat tcattaaaag 780

aaatgagttt agaagaaata tattcttacg agaagtatgg ggaatttatt acccaggaag 840

gcattagctt ctataatgat atatgtggga aagtgaattc ttttatgaac ttatattgtc 900

agaaaaataa agaaaacaaa aatttataca aacttcagaa acttcacaaa cagattctat 960

gcattgcgga cactagctat gaggttccgt ataaatttga aagtgacgag gaagtgtacc 1020

aatcagttaa cggcttcctt gataacatta gcagcaaaca tatagttgaa agattaagaa 1080

aaataggcga taactataac ggctacaact tagataaaat ttatatagtg tccaaatttt 1140

acgagagcgt tagccaaaaa acctacagag actgggaaac aattaatacc gccttagaaa 1200

ttcattacaa taatatattg ccgggtaacg gtaaaagtaa agccgacaaa gtaaaaaaag 1260

cggttaagaa tgatttacag aaatccataa ccgaaataaa tgaactagtg tcaaactata 1320

agttatgcag tgacgacaac ataaaagcgg agacttatat acatgagatt agccatatat 1380

tgaataactt tgaagcacag gaattgaaat acaatccgga aattcaccta gttgaatccg 1440

agttaaaagc gagtgagctt aaaaacgtgt tagacgtgat aatgaatgcg tttcattggt 1500

gttcggtttt tatgactgag gaacttgttg ataaagacaa caatttttat gcggaattag 1560

aggagattta cgatgaaatt tatccagtaa ttagtttata caacttagtt agaaactacg 1620

ttacccagaa accgtacagc acgaaaaaga ttaaattgaa ctttggaata ccgacgttag 1680

cagacggttg gtcaaagtcc aaagagtatt ctaataacgc tataatatta atgagagaca 1740

atttatatta tttaggcata tttaatgcga agaataaacc ggacaagaag attatagagg 1800

gtaatacgtc agaaaataag ggtgactaca aaaagatgat ttataatttg ttaccgggtc 1860

ccaacaaaat gataccgaaa gttttcttga gcagcaagac gggggtggaa acgtataaac 1920

cgagcgccta tatactagag gggtataaac agaataaaca tataaagtct tcaaaagact 1980

ttgatataac tttctgtcat gatttaatag actacttcaa aaactgtatt gcaattcatc 2040

ccgagtggaa aaacttcggt tttgatttta gcgacaccag tacttatgaa gacatttccg 2100

ggttttatag agaggtagag ttacaaggtt acaagattga ttggacatac attagcgaaa 2160

aagacattga tttattacag gaaaaaggtc aattatattt attccagata tataacaaag 2220

atttttcgaa aaaatcaacc gggaatgaca accttcacac catgtactta aaaaatcttt 2280

tctcagaaga aaatcttaag gatatagttt taaaacttaa cggcgaagcg gaaatattct 2340

tcaggaagag cagcataaag aacccaataa ttcataaaaa aggctcgatt ttagttaaca 2400

gaacctacga agcagaagaa aaagaccagt ttggcaacat tcaaattgtg agaaaaaata 2460

ttccggaaaa catttatcag gagttataca aatacttcaa cgataaaagc gacaaagagt 2520

tatctgatga agcagccaaa ttaaagaatg tagtgggaca ccacgaggca gcgacgaata 2580

tagttaagga ctatagatac acgtatgata aatacttcct tcatatgcct attacgataa 2640

atttcaaagc caataaaacg ggttttatta atgataggat attacagtat atagctaaag 2700

aaaaagactt acatgtgata ggcattgata gaggcgagag aaacttaata tacgtgtccg 2760

tgattgatac ttgtggtaat atagttgaac agaaaagctt taacattgta aacggctacg 2820

actatcagat aaaattaaaa caacaggagg gcgctagaca gattgcgaga aaagaatgga 2880

aagaaattgg taaaattaaa gagataaaag agggctactt aagcttagta atacacgaga 2940

tatctaaaat ggtaataaaa tacaatgcaa ttatagcgat ggaggatttg tcttatggtt 3000

ttaaaaaagg gagatttaag gttgaaagac aagtttacca gaaatttgaa accatgttaa 3060

taaataaatt aaactattta gtatttaaag atatttcgat taccgagaat ggcggtttat 3120

taaaaggtta tcagttaaca tacattcctg ataaacttaa aaacgtgggt catcagtgcg 3180

gctgcatttt ttatgtgcct gctgcataca cgagcaaaat tgatccgacc accggctttg 3240

tgaatatatt taaatttaaa gacttaacag tggacgcaaa aagagaattc attaaaaaat 3300

ttgactcaat tagatatgac agtgaaaaaa atttattctg ctttacattt gactacaata 3360

actttattac gcaaaacacg gttatgagca aatcatcgtg gagtgtgtat acatacggcg 3420

tgagaataaa aagaagattt gtgaacggca gattctcaaa cgaaagtgat accattgaca 3480

taaccaaaga tatggagaaa acgttggaaa tgacggacat taactggaga gatggccacg 3540

atcttagaca agacattata gattatgaaa ttgttcagca catattcgaa attttcagat 3600

taacagtgca aatgagaaac tccttgtctg aattagagga cagagattac gatagattaa 3660

tttcacctgt attaaacgaa aataacattt tttatgacag cgcgaaagcg ggggatgcac 3720

ttcctaagga tgccgatgca aatggtgcgt attgtattgc attaaaaggg ttatatgaaa 3780

ttaaacaaat taccgaaaat tggaaagaag atggtaaatt ttcgagagat aaattaaaaa 3840

taagcaataa agattggttc gactttatac agaataagag atatttataa gtcgacaaag 3900

tattgttaaa aataactctg tagaattata aattagttct acagagttat tttttgaccc 3960

gggtatattg ataaaaataa taatagtggg tataattaag ttgttaggag gttagttaga 4020

atgatgtcaa gattagataa aagtaaagtg attaacagcg cattagagct gcttaatgag 4080

gtcggaatcg aaggtttaac aacccgtaaa ctcgcccaga agctaggtgt agagcagcct 4140

acattgtatt ggcatgtaaa aaataagcgg gctttgctcg acgccttagc cattgagatg 4200

ttagataggc accatactca cttttgccct ttagaagggg aaagctggca agatttttta 4260

cgtaataacg ctaaaagttt tagatgtgct ttactaagtc atcgcgatgg agcaaaagta 4320

catttaggta cacggcctac agaaaaacag tatgaaactc tcgaaaatca attagccttt 4380

ttatgccaac aaggtttttc actagagaat gcattatatg cactcagcgc tgtggggcat 4440

tttactttag gttgcgtatt ggaagatcaa gagcatcaag tcgctaaaga agaaagggaa 4500

acacctacta ctgatagtat gccgccatta ttacgacaag ctatcgaatt atttgatcac 4560

caaggtgcag agccagcctt cttattcggc cttgaattga tcatatgcgg attagaaaaa 4620

caacttaaat gtgaaagtgg gtcttaaaag cagcataacc tttttccgtg atggtaactt 4680

cacggtaacc aagatgtcga gttgagctcg aattcgtaat catggtcata gctgtttcct 4740

gtgtgaaatt gttatccgct cacaattcca cacaacatac gagccggaag cataaagtgt 4800

aaagcctggg gtgcctaatg agtgagctaa ctcacattaa ttgcgttgcg ctcactgccc 4860

gctttccagt cgggaaacct gtcgtgccag ctgcattaat gaatcggcca acgcgcgggg 4920

agaggcggtt tgcgtattgg gcgctcttcc gcttcctcgc tcactgactc gctgcgctcg 4980

gtcgttcggc tgcggcgagc ggtatcagct cactcaaagg cggtaatacg gttatccaca 5040

gaatcagggg ataacgcagg aaagaacatg tgagcaaaag gccagcaaaa ggccaggaac 5100

cgtaaaaagg ccgcgttgct ggcgtttttc cataggctcc gcccccctga cgagcatcac 5160

aaaaatcgac gctcaagtca gaggtggcga aacccgacag gactataaag ataccaggcg 5220

tttccccctg gaagctccct cgtgcgctct cctgttccga ccctgccgct taccggatac 5280

ctgtccgcct ttctcccttc gggaagcgtg gcgctttctc atagctcacg ctgtaggtat 5340

ctcagttcgg tgtaggtcgt tcgctccaag ctgggctgtg tgcacgaacc ccccgttcag 5400

cccgaccgct gcgccttatc cggtaactat cgtcttgagt ccaacccggt aagacacgac 5460

ttatcgccac tggcagcagc cactggtaac aggattagca gagcgaggta tgtaggcggt 5520

gctacagagt tcttgaagtg gtggcctaac tacggctaca ctagaaggac agtatttggt 5580

atctgcgctc tgctgaagcc agttaccttc ggaaaaagag ttggtagctc ttgatccggc 5640

aaacaaacca ccgctggtag cggtggtttt tttgtttgca agcagcagat tacgcgcaga 5700

aaaaaaggat ctcaagaaga tcctttgatc ttttctacgg ggtctgacgc tcagtggaac 5760

gaaaactcac gttaagggat tttggtcatg agattatcaa aaaggatctt cacctagatc 5820

cttttaaatt aaaaatgaag ttttaaatca atctaaagta tatatgagta aacttggtct 5880

gacagttacc aggtccactg ccgggcctct tgcgggatca aaagaaaaac gaaatgatac 5940

accaatcagt gcaaaaaaag atataatggg agataagacg gttcgtgttc gtgctgactt 6000

gcaccatatc ataaaaatcg aaacagcaaa gaatggcgga aacgtaaaag aagttatgga 6060

aataagactt agaagcaaac ttaagagtgt gttgatagtg cagtatctta aaattttgta 6120

taataggaat tgaagttaaa ttagatgcta aaaatttgta attaagaagg agtgattaca 6180

tgaacaaaaa tataaaatat tctcaaaact ttttaacgag tgaaaaagta ctcaaccaaa 6240

taataaaaca attgaattta aaagaaaccg ataccgttta cgaaattgga acaggtaaag 6300

ggcatttaac gacgaaactg gctaaaataa gtaaacaggt aacgtctatt gaattagaca 6360

gtcatctatt caacttatcg tcagaaaaat taaaactgaa tactcgtgtc actttaattc 6420

accaagatat tctacagttt caattcccta acaaacagag gtataaaatt gttgggagta 6480

ttccttacca tttaagcaca caaattatta aaaaagtggt ttttgaaagc catgcgtctg 6540

acatctatct gattgttgaa gaaggattct acaagcgtac cttggatatt caccgaacac 6600

tagggttgct cttgcacact caagtctcga ttcagcaatt gcttaagctg ccagcggaat 6660

gctttcatcc taaaccaaaa gtaaacagtg tcttaataaa acttacccgc cataccacag 6720

atgttccaga taaatattgg aagctatata cgtactttgt ttcaaaatgg gtcaatcgag 6780

aatatcgtca actgtttact aaaaatcagt ttcatcaagc aatgaaacac gccaaagtaa 6840

acaatttaag taccgttact tatgagcaag tattgtctat ttttaatagt tatctattat 6900

ttaacgggag gaaataattc tatgagtccc taggcaggcc tccgccatta tttttttgaa 6960

caattgacaa ttcatttctt attttttatt aagtgatagt caaaaggcat aacagtgctg 7020

aatagaaaga aatttacaga aaagaaaatt atagaattta gtatgattaa ttatactcat 7080

ttatgaatgt ttaattgaat acaaaaaaaa atacttgtta tgtattcaat tacgggttaa 7140

aatatagaca agttgaaaaa tttaataaaa aaataagtcc tcagctctta tatattaagc 7200

taccaactta gtatataagc caaaacttaa atgtgctacc aacacatcaa gccgttagag 7260

aactctatct atagcaatat ttcaaatgta ccgacataca agagaaacat taactatata 7320

tattcaattt atgagattat cttaacagat ataaatgtaa attgcaataa gtaagattta 7380

gaagtttata gcctttgtgt attggaagca gtacgcaaag gcttttttat ttgataaaaa 7440

ttagaagtat atttattttt tcataattaa tttatgaaaa tgaaaggggg tgagcaaagt 7500

gacagaggaa agcagtatct tatcaaataa caaggtatta gcaatatcat tattgacttt 7560

agcagtaaac attatgactt ttatagtgct tgtagctaag tagtacgaaa gggggagctt 7620

taaaaagctc cttggaatac atagaattca taaattaatt tatgaaaaga agggcgtata 7680

tgaaaacttg taaaaattgc aaagagttta ttaaagatac tgaaatatgc aaaatacatt 7740

cgttgatgat tcatgataaa acagtagcaa cctattgcag taaatacaat gagtcaagat 7800

gtttacataa agggaaagtc caatgtatta attgttcaaa gatgaaccga tatggatggt 7860

gtgccataaa aatgagatgt tttacagagg aagaacagaa aaaagaacgt acatgcatta 7920

aatattatgc aaggagcttt aaaaaagctc atgtaaagaa gagtaaaaag aaaaaataat 7980

ttatttatta atttaatatt gagagtgccg acacagtatg cactaaaaaa tatatctgtg 8040

gtgtagtgag ccgatacaaa aggatagtca ctcgcatttt cataatacat cttatgttat 8100

gattatgtgt cggtgggact tcacgacgaa aacccacaat aaaaaaagag ttcggggtag 8160

ggttaagcat agttgaggca actaaacaat caagctagga tatgcagtag cagaccgtaa 8220

ggtcgttgtt taggtgtgtt gtaatacata cgctattaag atgtaaaaat acggatacca 8280

atgaagggaa aagtataatt tttggatgta gtttgtttgt tcatctatgg gcaaactacg 8340

tccaaagccg tttccaaatc tgctaaaaag tatatccttt ctaaaatcaa agtcaagtat 8400

gaaatcataa ataaagttta attttgaagt tattatgata ttatgttttt ctattaaaat 8460

aaattaagta tatagaatag tttaataata gtatatactt aatgtgataa gtgtctgaca 8520

gtgtcacaga aaggatgatt gttatggatt ataagcggc 8559